# EUROPEAN PATENT APPLICATION

(11) **EP 4 814 017 A1**
(43) Date of publication of application: **30.09.2026**
(21) Application number: 26166665.5
(22) Date of filing: 23.03.2026
(51) Int. Cl.: C07K 16/28

(54) **THERAPEUTIC ANTI-BAFF-R ANTIBODIES**

(30) Priority: 24.03.2025 US 202563776536 P
(71) Applicant: Novartis AG, 4056 Basel (CH)
(72) Inventor: Bonal, Claire, 4056 Basel (CH); Cirera Salinas, Daniel, 4056 Basel (CH); Flores Ortiz, Luis Francisco, 6336 Langkampfen (AT); Folkers, Mareike Ute, 6336 Langkampfen (AT); Gellermann, Gerald, 4056 Basel (CH); Isnardi, Isabelle, 4056 Basel (CH); Kern, Wolfram, 4056 Basel (CH); Kirsch, Stephan, 6336 Langkampfen (AT); Liu, Hejun, San Diego, 92121 (US); Milicic, Goran, 1000 Ljubljana (SI); Müller, Wolfgang, 6336 Langkampfen (AT); Omersa, Neza, 1000 Ljubljana (SI); Petricoul, Olivier, 4056 Basel (CH); Pohl, Thomas, 4056 Basel (CH); Porenta, Tine, 1000 Ljubljana (SI); Süssenbacher, Iris, 6336 Langkampfen (AT)
(74) Representative: Bieri, Simona Roxana

(57) **Abstract**

The present invention relates to compositions of anti-BAFF-R antibodies, such as ianalumab, that can exert potent therapeutic efficacy through BAFF-R binding and antibody-dependent cell-mediated cytotoxicity (ADCC), and method of producing the same and method of treatment using the compositions.

## Description

### SEQUENCE LISTING

The instant application contains a Sequence Listing which has been submitted electronically and is hereby incorporated by reference in its entirety.

### BACKGROUND

B cell-activating factor receptor (BAFF-R) (also known as BR3, TNFRSF13C, or CD268) is a member of the tumor necrosis factor receptor superfamily. It is expressed predominantly on B-lymphocytes and on a subset of T-cells. BAFF-R specifically binds the tumor necrosis factor family member BLyS (also known as BAFF, CD257, TALL-1, THANK, TNFSF13B, ZTNF4) which can be expressed by a variety of different cell types, most notably myeloid cells. Functionally, the BLyS/BAFF-R ligand-receptor pair is critically involved in the maturation of immature transitional B-cells and for survival, migration and activation of mature B-cells including isotype class switching. BLyS can act alone or in concert with B-cell receptor (BCR), interleukin-4, interleukin-21 or CD40 ligand. Due to the presence of BAFF-R on some T-cells, BLyS can act as costimulatory factor for T-cell activation. BLyS can also bind to two additional receptors found on B-cells, TACI and BCMA.

lanalumab is a fully human monoclonal antibody of the IgG1/kappa subclass, specifically targeting the ligand-binding domain of the human BAFF-R. BAFF-R is predominantly expressed on immature and mature B cells, which is maintained in autoimmune diseases. lanalumab can inhibit the interaction between BAFF-R and its cognate ligand, the B cell-activating factor (BAFF), which is key for B cell differentiation and proliferation. lanalumab has been shown to inhibit BAFF-mediated activation and survival of B cells and is currently under development for various human B-cell-related disorders. The ability of ianalumab to mediate ADCC was confirmed in vitro using primary human B cells from healthy individuals and chronic lymphocytic leukemia (CLL) patients.

It is well recognized that the biological activity of a protein containing oligosaccharide chains, known as a glycoprotein, is dependent upon not only the integral structure of the protein, but also the properties of the oligosaccharide covalently attached to the protein. Glycosylation can affect solubility, resistance to proteolytic attack and thermal inactivation, quaternary structure, activity, targeting, antigenicity, functional activity, and half-life of the protein. Mammalian glycosylation patterns in general are described in Fukuda et al. (1994), Molecular Glycobiology, IRL Press, New York, incorporated herein by reference. Therapeutic monoclonal antibodies (mAbs) produced in mammalian cells are heterogeneous as a result of post-translational modifications (PTMs). PTMs can occur during mAb production, purification, storage, and post-administration. PTMs may be directly linked to variations in drug efficacy and safety. For example, core fucose has been demonstrated to have a very significant impact on FcγRIIIa binding affinity, leading to substantial changes in ADCC activity. It is vital to the biopharmaceutical industry to ensure consistent product quality which reduces potential impacts on drug safety and efficacy. There still is a need in the biopharmaceutical industry for simple and efficient methods to manufacture a particular antibody having a specific level of effector function based on the given glycoform profile for that antibody composition.

lanalumab's mechanism of action relies, at least in part, on its Fc receptor and glycan compositions thereon.

Accordingly, there is a need in the field for anti-BAFF-R antibody compositions that are suitable to confer biological functions necessary for beneficial therapeutic effects.

### SUMMARY OF THE INVENTION

Provided herein are compositions of anti-BAFF-R antibodies, such as ianalumab, that can exert potent therapeutic efficacy through BAFF-R binding and antibody-dependent cell-mediated cytotoxicity (ADCC).

In one aspect, provided are compositions of anti-BAFF-R antibodies (e.g. monoclonal anti-BAFF-R antibodies) having N-glycosylated Fc regions, wherein about 10% or less (e.g., 0% to 10%) of the N-glycosylated Fc regions comprise a high mannose glycan. In some embodiments, at least 95% of the anti-BAFF-R antibodies have N-glycosylated Fc regions (e.g., at least 96%, at least 97%, at least 98%, at least 99% or 100% of the anti-BAFF-R antibodies have N-glycosylated Fc regions). In some embodiments, at least 90% of the N-glycosylated Fc regions are afucosylated (e.g., at least 90%, at least 91%, at least 92%, at least 93%, or at least 94% of the N-glycosylated Fc regions are afucosylated). In some embodiments, at least 95% of the N-glycosylated Fc regions are afucosylated (e.g., at least 96%, at least 97%, at least 98%, or at least 99% of the N-glycosylated Fc regions are afucosylated). In some embodiments, about 49% or less of the N-glycosylated Fc regions are galactosylated (e.g., about 45% or less, about 41% or less, about 40% or less, about 35% or less, or about 30% or less, e.g., about 25% or less of the N-glycosylated Fc regions are galactosylated; e.g., from about 10% to about 40%, from about 15% to about 35%, or from about 20% to about 25% of the N-glycosylated Fc regions are galactosylated). In some embodiments, about 10-40% of the N-glycosylated Fc regions are galactosylated and less than 20% of the N-glycosylated Fc regions have a high mannose glycan (e.g., less than 15%, less than 10%, less than 8%, less than 6%, less than 5%, less than 4%, less than 3%, less than 2%, or less than 1%; e.g., from 0.1% to 6%, from 0.5% to 3.5%, or from 1% to 3%; e.g., from 0.1% to 1%, from 1% to 2%, from 2% to 3%, from 3% to 4%, from 4% to 5%, or from 5% to 6% of the N-glycosylated Fc regions have a high mannose glycan). In some embodiments, less than 20% of the N-glycosylated Fc regions have a high mannose glycan (e.g., less than 15%, less than 10%, less than 8%, less than 6%, less than 5%, less than 4%, less than 3%, less than 2%, or less than 1%; e.g., from 0.1% to 6%, from 0.5% to 3.5%, or from 1% to 3%; e.g., from 0.1% to 1%, from 1% to 2%, from 2% to 3%, from 3% to 4%, from 4% to 5%, or from 5% to 6% of the N-glycosylated Fc regions have a high mannose glycan) and about 10-40% of the N-glycosylated Fc regions are galactosylated (e.g., about 15% to 38% of the N-glycosylated Fc regions are galactosylated, e.g., about 21% to 25% of the N-glycosylated Fc regions are galactosylated). In some embodiments, about 0.1% to about 7% (e.g., from about 0.5% to about 3.5%, e.g., about 3% to about 4.8%) of the N-glycosylated Fc regions have a high mannose glycan and about 15% to about 38% (e.g., about 21% to about 25%) of the N-glycosylated Fc regions are galactosylated.

In some embodiments, the composition (e.g., a composition of anti-BAFF-R antibodies, e.g. monoclonal anti-BAFF-R antibodies, of any of the preceding embodiments, e.g., wherein the monoclonal anti-BAFF-R antibody is an afucosylated IgG₁ antibody and/or wherein the monoclonal anti-BAFF-R antibody is ianalumab or has any one or more of the amino acid sequences of ianalumab described herein) has a relative antibody-dependent cellular cytotoxicity (ADCC) potency of about 70% to about 130% (e.g., from about 75% to about 125%, from about 80% to about 120%, from about 80% to about 116%, from about 85% to about 115%, from about 90% to about 110%, from about 95% to about 105%, or about 100%) in a cell-based ADCC assay (e.g., an NK cell-based ADCC potency assay, e.g., as described herein) compared to a reference standard (e.g., a commercial reference standard, e.g., ianalumab reference standard), which is set as a 100% ADCC potency as described herein.

In some embodiments, the composition of anti-BAFF-R antibodies (e.g., a composition of monoclonal anti-BAFF-R antibodies of any of the preceding embodiments, e.g., wherein the monoclonal anti-BAFF-R antibody is an afucosylated IgG₁ antibody and/or wherein the monoclonal anti-BAFF-R antibody is ianalumab or has any one or more of the amino acid sequences of ianalumab described herein) has an ADCC potency within a reference range of ADCC potencies from two or more lots of a reference standard (e.g., a reference range of ADCC potencies from two, three, four, five, six, or more lots of a reference standard of ianalumab, e.g., commercially available ianalumab).

In some embodiments, the composition anti-BAFF-R antibodies (e.g., a composition of monoclonal anti-BAFF-R antibodies of any of the preceding embodiments, e.g., wherein the monoclonal anti-BAFF-R antibody is an afucosylated IgG₁ antibody and/or wherein the monoclonal anti-BAFF-R antibody is ianalumab or has any one or more of the amino acid sequences of ianalumab described herein) depletes human B cells through ADCC with a half-maximal efficacy (EC₅₀): (a) from 10 pM to 1 nM (e.g., from about 50 pM to about 500 pM, from about 100 pM to about 400 pM, or from about 150 pM to about 250 pM; e.g., about 196 pM) in an *in vitro* culture of human whole blood and/or (b) from 0.2 pM to 100 pM (e.g., from about 1.0 pM to about 50 pM, from about 1.5 pM to about 10 pM, or from 2.0 pM to about 5 pM; e.g., about 2.7 pM) in an *in vitro* culture of human peripheral blood mononuclear cells.

In some embodiments, the monoclonal anti-BAFF-R antibodies of the composition (e.g., the monoclonal anti-BAFF-R antibodies of any of the preceding embodiments, e.g., wherein the monoclonal anti-BAFF-R antibody is an afucosylated IgG₁ antibody and/or wherein the monoclonal anti-BAFF-R antibody is ianalumab or has any one or more of the amino acid sequences of ianalumab described herein) have: (a) a binding affinity (K_{D}) to FcγRIIIa^{F158} from 1.3 nM to 3.9 nM (e.g., from about 1.5 nM to about 3.5 nM, e.g., from about 2.0 nM to about 3.0 nM, e.g., about 2.6 nM); (b) a binding affinity to FcγRIIIa^{V158} from 0.52 nM to 1.59 nM; or (c) a relative binding affinity to FcγRIIIa^{F158} and/or FcγRIIIa^{V158} from about 70% to about 130% compared to a commercial reference standard.

In some embodiments, the anti-BAFF-R antibodies, e.g. monoclonal anti-BAFF-R antibodies, of the composition (e.g., the monoclonal anti-BAFF-R antibodies of any of the preceding embodiments, e.g., wherein the monoclonal anti-BAFF-R antibody is an afucosylated IgG₁ antibody and/or wherein the monoclonal anti-BAFF-R antibody is ianalumab or has any one or more of the amino acid sequences of ianalumab described herein) have a serum half-life (e.g., in humans) of at least 5 days (e.g., at least 8 days, or at least 9 days).

In some embodiments of any of the preceding embodiments, the anti-BAFF-R antibodies, e.g. monoclonal anti-BAFF-R antibodies, of the composition (e.g., the monoclonal anti-BAFF-R antibodies of any of the preceding embodiments, e.g., wherein the monoclonal anti-BAFF-R antibody is an afucosylated IgG1) have a CDR-H1, a CDR-H2, and a CDR-H3 having the amino acid sequences of SEQ ID NO: 5, SEQ ID NO: 6, and SEQ ID NO: 7, and/or a CDR-L1, a CDR-L2, and a CDR-L3 having the amino acid sequences of SEQ ID NO: 8, SEQ ID NO: 9, and SEQ ID NO: 10, respectively. In some embodiments, the monoclonal anti-BAFF-R antibodies of the composition (e.g., the monoclonal anti-BAFF-R antibodies of any of the preceding embodiments, e.g., wherein the monoclonal anti-BAFF-R antibody is an afucosylated IgG) have a CDR-H1, a CDR-H2, and a CDR-H3 having the amino acid sequences of SEQ ID NO: 5, SEQ ID NO: 6, and SEQ ID NO: 7, and a CDR-L1, a CDR-L2, and a CDR-L3 having the amino acid sequences of SEQ ID NO: 8, SEQ ID NO: 9, and SEQ ID NO: 10, respectively. In some embodiments, the monoclonal anti-BAFF-R antibodies of the composition (e.g., the monoclonal anti-BAFF-R antibodies of any of the preceding embodiments, e.g., wherein the monoclonal anti-BAFF-R antibody is an afucosylated IgG1) bind to a BAFF-R epitope comprising one or more of residues D26, L27, L28, V29, R30, H31, C32, V33, A34, and C35; and/or have one or more anti-BAFF-R paratopic residues comprising one or more of heavy chain residues N32, S33, A34, A35, R52, Y54, R56, S57, Y60, Y102, D103, W104, V110 and/or one or more of light chain residues L92, Y93, and S94.

In some embodiments, the monoclonal anti-BAFF-R antibodies of the composition (e.g., the monoclonal anti-BAFF-R antibodies of any of the preceding embodiments, e.g., wherein the monoclonal anti-BAFF-R antibody is an afucosylated IgG₁ antibody and/or wherein the monoclonal anti-BAFF-R antibody is ianalumab or has any one or more of the amino acid sequences of ianalumab described herein) includes at least one variant selected from a high mannose N-glycan variant, a galactosyalated N-glycan variant, a high molecular weight variant, a deamidated variant, a tryptophan-oxidized variant, a methionine-oxidized variant, a fragmented variant, and a clipped variant.

In some embodiments, the anti-BAFF-R antibodies of the composition includes at least one variant (e.g., one, two, three, four, five, or all six variants) selected from a high molecular weight variant, a deamidated variant, a tryptophan-oxidized variant, a methionine-oxidized variant, a fragmented variant, and a clipped variant. In some embodiments, the monoclonal anti-BAFF-R antibodies of the composition (e.g., the monoclonal anti-BAFF-R antibodies of any of the preceding embodiments, e.g., wherein the monoclonal anti-BAFF-R antibody is an afucosylated IgG₁ antibody and/or wherein the monoclonal anti-BAFF-R antibody is ianalumab or has any one or more of the amino acid sequences of ianalumab described herein) includes at least one variant (e.g., one, two, three, four, five, or all six variants) selected from a high molecular weight variant, a deamidated variant, a tryptophan-oxidized variant, a methionine-oxidized variant, a fragmented variant, and a clipped variant. In some embodiments, the monoclonal anti-BAFF-R antibodies include a high molecular weight variant, wherein the high molecular weight variant accounts for about 11% or less, about 6% or less, or about 2.4% or less, preferably about 1.5% or less or about 1% or less, of the anti-BAFF-R antibodies in the composition. In some embodiments, the monoclonal anti-BAFF-R antibodies include a deamidated variant, wherein the deamidated variant comprises deamidation at position N332 (heavy chain residue 332), and optionally wherein the deamidated variant accounts for up to about 42.6%, up to about 23%, about 9.7% or less, or about 4.3% or less, preferably about 3.5% or less, of the anti-BAFF-R antibodies in the composition. In some embodiments, the monoclonal anti-BAFF-R antibodies include a tryptophan-oxidized variant, e.g., including tryptophan oxidation at heavy chain residue 104 and/or heavy chain residue 59 (H-W104 and/or H-W59), e.g., wherein the tryptophan-oxidized variant accounts for up to about 23.8%, up to about 16%, about 10.5%, about 5.6% or less, preferably about 3.51% or less or about 3% or less, of the anti-BAFF-R antibodies in the composition. In some embodiments, the anti-BAFF-R antibodies comprise a methionine-oxidized variant, wherein the methionine-oxidized variant comprises one or more of light chain residue M97, heavy chain residue M259, heavy chain residue M365, and heavy chain residue M435 (e.g., one, two, three, or all four of light chain residue M97, heavy chain residue M259, heavy chain residue M365, and heavy chain residue M435). In some embodiments, the anti-BAFF-R antibodies comprise a methionine-oxidized variant, wherein the methionine-oxidized variant comprises oxidation of M259 on both heavy chains, optionally the methionine-oxidized variant accounting for up to about 99.7%, about 56.3% or less, about 27% of less, or about 12.4% or less, preferably about 6.7% or less, of the anti-BAFF-R antibodies in the composition. In some embodiments, the anti-BAFF-R antibodies comprise a methionine-oxidized variant, wherein the methionine-oxidized variant has oxidation of M435 on both heavy chains, optionally wherein the amount of the methionine-oxidized variant is up to about 98.5% or less, about 37.3% or less, or about 12.4% or less, preferably about 6.7% or less, of the anti-BAFF-R antibodies in the composition. In some embodiments, the anti-BAFF-R antibodies comprise a clipped variant, e.g., wherein the clipped variant includes clipping between R56 and S57 in the CDR2 loop of the heavy chain, and optionally wherein the amount of the clipped variant is about 10% or less or about 8.8% or less, preferably about 6.0% or less, of the anti-BAFF-R antibodies in the composition. In some embodiments, the afucosylated anti-BAFF-R antibodies include a high molecular weight variant, e.g., wherein the high molecular weight variant accounts for about 11% or less (e.g., about 6% or less, e.g., about 1% or less) of the anti-BAFF-R antibodies in the composition. In some embodiments, the afucosylated anti-BAFF-R antibodies comprise a deamidated variant, e.g., wherein the deamidated variant comprises deamidation at position N332 (heavy chain residue 332), e.g., wherein the deamidated variant accounts for about 4.3% or less, preferably about 3.5% or less, of the anti-BAFF-R antibodies in the composition. In some embodiments, the afucosylated anti-BAFF-R antibodies include a tryptophan-oxidized variant, e.g., wherein the tryptophan-oxidized variant comprises H-W104 and/or H-W59, e.g., wherein the tryptophan-oxidized variant accounts for up to about 23.8% of the anti-BAFF-R antibodies in the composition, e.g., up to about 16% of the anti-BAFF-R antibodies in the composition, e.g., up to about 3% of the anti-BAFF-R antibodies in the composition. In some embodiments, the afucosylated anti-BAFF-R antibodies include a methionine-oxidized variant, e.g., wherein the methionine-oxidized variant comprises one or more of light chain M97, heavy chain M259, heavy chain M365, and heavy chain M435, e.g., wherein the methionine-oxidized variant accounts for about 10% or less, about 5.6% or less, about 4% or less, preferably about 3.5% or less of the anti-BAFF-R antibodies in the composition. In some embodiments, the afucosylated anti-BAFF-R antibodies include a methionine-oxidized variant, wherein the methionine-oxidized variant comprises one or more of light chain M97, heavy chain M259, heavy chain M365, and heavy chain M435, and wherein the methionine-oxidized variant has simultaneous oxidation of M259 on both heavy chains, optionally wherein the amount of the methionine-oxidized variant is up to about 99.7%, about 56.3% or less, about 12.4% or less, preferably about 6.7% or less, of the anti-BAFF-R antibodies in the composition. In some embodiments, the afucosylated anti-BAFF-R antibodies include a methionine-oxidized variant, wherein the methionine-oxidized variant has simultaneous oxidation of M435 on both heavy chains, optionally wherein the amount of the methionine-oxidized variant is up to about 98.5%, about 37.3% or less, about 12.4% or less, preferably about 6.7% or less, of the anti-BAFF-R antibodies in the composition. In some embodiments, the afucosylated anti-BAFF-R antibodies include a clipped variant, e.g., wherein the clipped variant comprises clipping between R56 and S57 in the CDR2 loop of the heavy chain, and optionally wherein the amount of the clipped variant is up to about 13.5%, about 10% or less, about 8.8% or less, preferably about 6.0% or less, of the anti-BAFF-R antibodies in the composition. In some embodiments, the afucosylated anti-BAFF-R antibodies include a fragmented variant, with primary fragmentation site is in the hinge region, leading to the formation of antibody species missing one Fab arm and the corresponding Fab fragment, and to less extent, upon disruption of peptide bonds or disulfide bridges, resulting in an anti-BAFF-R antibody missing one light chain (LC), single LC or heavy chain (HC) fragments, and optionally wherein the amount of the fragmented variant is up to about 19.2%, about 9.7% or less, about 6.3% or less, preferably about 4.5% or less, of the anti-BAFF-R antibodies in the composition.

In another aspect, provided herein is a composition of afucosylated anti-BAFF-R antibodies composition (e.g., monoclonal anti-BAFF-R antibodies of any of the preceding embodiments, e.g., wherein the monoclonal anti-BAFF-R antibody is an afucosylated IgG₁ antibody and/or wherein the monoclonal anti-BAFF-R antibody is ianalumab or has any one or more of the amino acid sequences of ianalumab described herein), wherein the afucosylated anti-BAFF-R antibodies comprise a deamidated variant, e.g., wherein the deamidated variant comprises deamidation at position N332, e.g., wherein the deamidated variant accounts for up to about 42.6%, up to about 23%, or about 9.7% or less, optionally of about 4.3% or less, preferably of about 3.5% or less or about 1% or less, of the anti-BAFF-R antibodies in the composition. In some embodiments, the afucosylated anti-BAFF-R antibodies include a high molecular weight variant, e.g., wherein the high molecular weight variant accounts for about 11% or less, about 6% or less, or about 2.4% or less, preferably of about 1.5% or less or about 1% or less, of the anti-BAFF-R antibodies in the composition. In some embodiments, the afucosylated anti-BAFF-R antibodies comprise a tryptophan-oxidized variant, e.g., wherein the tryptophan-oxidized variant comprises H-W104 and/or H-W59, e.g., wherein the tryptophan-oxidized variant accounts for up to about 23.8%, up to about 16%, or up to about 10.5%, optionally about 5.6% or less, preferably about 3.5% or less or about 3% or less, of the anti-BAFF-R antibodies in the composition. In some embodiment, the afucosylated anti-BAFF-R antibodies comprise a methionine-oxidized variant, e.g., wherein the methionine-oxidized variant comprises one or more of M97, M259, M365, and M435, e.g., wherein the methionine-oxidized variant comprises M259 and M435, e.g., wherein the methionine-oxidized variant accounts for about of about 12.4% or less, preferably of about 6.7% or less, or of about 4% or less of the anti-BAFF-R antibodies in the composition. In some embodiments, the anti-BAFF-R antibodies comprise a methionine-oxidized variant, wherein the methionine-oxidized variant has simultaneous oxidation of M259 on both heavy chains, optionally wherein the amount of the methionine-oxidized variant is of up to 99.7%, of about 56.3%, optionally of about 12.4% or less, preferably of about 6.7% or less, of the anti-BAFF-R antibodies in the composition. In some embodiments, the anti-BAFF-R antibodies comprise a methionine-oxidized variant, wherein the methionine-oxidized variant has simultaneous oxidation of M435 on both heavy chains, optionally wherein the amount of the methionine-oxidized variant is of up to 98.5%, of about 37.3%, optionally of about 12.4% or less, preferably of about 6.7% or less, of the anti-BAFF-R antibodies in the composition. In some embodiments, the afucosylated anti-BAFF-R antibodies include a clipped variant, e.g., wherein the clipped variant comprises clipping between R56 and S57 in the CDR2 loop of the heavy chain, and optionally wherein the amount of the clipped variant is of about 6.0% or less, of the anti-BAFF-R antibodies in the composition. In some embodiments, the afucosylated anti-BAFF-R antibodies include a fragmented variant, with primary fragmentation site is in the hinge region, leading to the formation of antibody species missing one Fab arm and the corresponding Fab fragment, and to less extent, upon disruption of peptide bonds or disulfide bridges, resulting in an anti-BAFF-R antibody missing one light chain (LC), single LC or heavy chain (HC) fragments, and optionally wherein the amount of the fragmented variant is of up to 19.2%, of about 9.7%, optionally of about 6.3% or less, preferably of about 4.5% or less, of the anti-BAFF-R antibodies in the composition.

In another aspect of the invention, provided are compositions having afucosylated anti-BAFF-R antibodies (e.g., monoclonal anti-BAFF-R antibodies of any of the preceding embodiments, e.g., wherein the monoclonal anti-BAFF-R antibody is an afucosylated IgG₁ antibody and/or wherein the monoclonal anti-BAFF-R antibody is ianalumab or has any one or more of the amino acid sequences of ianalumab described herein), wherein the afucosylated anti-BAFF-R antibodies comprise a tryptophan-oxidized variant, e.g., wherein the tryptophan-oxidized variant comprises H-W104 and/or H-W59, and optionally wherein the tryptophan-oxidized variant accounts for up to about 23.8%, up to about 16%, or up to about 10.5%, optionally about 5.6% or less, preferably about 3.5% or less, or about 3% or less of the anti-BAFF-R antibodies in the composition. In some embodiments, the afucosylated anti-BAFF-R antibodies further include a deamidated variant, e.g., wherein the deamidated variant comprises deamidation at position N332, and optionally wherein the deamidated variant accounts for up to about 42.6%, up to about 23%, or about 9.7% or less, optionally or of about 4.3% or less, preferably of about 3.5% or less or about 1% or less, of the anti-BAFF-R antibodies in the composition. In some embodiments, the afucosylated anti-BAFF-R antibodies further include a high molecular weight variant, e.g., wherein the high molecular weight variant accounts for about 11% or less, about 6% or less, or about 2.4% or less, preferably of about 1.5% or less, of the anti-BAFF-R antibodies in the composition. In some embodiments, the afucosylated anti-BAFF-R antibodies further include a methionine-oxidized variant, e.g., wherein the methionine-oxidized variant comprises one or more of M97, M259, M365, and M435, and optionally wherein the methionine-oxidized variant accounts for about 4% or less of the anti-BAFF-R antibodies in the composition. In some embodiments, the anti-BAFF-R antibodies comprise a methionine-oxidized variant, wherein the methionine-oxidized variant has simultaneous oxidation of M259 on both heavy chains, optionally wherein the amount of the methionine-oxidized variant is of up to 99.7%, of about 56.3%, optionally of about 12.4% or less, preferably of about 6.7% or less, of the anti-BAFF-R antibodies in the composition. In some embodiments, the anti-BAFF-R antibodies comprise a methionine-oxidized variant, wherein the methionine-oxidized variant has simultaneous oxidation of M435 on both heavy chains, optionally wherein the amount of the methionine-oxidized variant is of up to 98.5%, of about 37.3%, optionally of about 12.4% or less, preferably of about 6.7% or less, of the anti-BAFF-R antibodies in the composition. In some embodiments, the afucosylated anti-BAFF-R antibodies further comprise a clipped variant, e.g., wherein the clipped variant comprises clipping between R56 and S57 in the CDR2 loop of the heavy chain, and optionally wherein the amount of the clipped variant is of up to about 13.5%, about 10% or less, about 8.8% or less, preferably of about 6.0% or less, of the anti-BAFF-R antibodies in the composition. In some embodiments, the afucosylated anti-BAFF-R antibodies include a fragmented variant, with primary fragmentation site is in the hinge region, leading to the formation of antibody species missing one Fab arm and the corresponding Fab fragment, and to less extent, upon disruption of peptide bonds or disulfide bridges, resulting in an anti-BAFF-R antibody missing one light chain (LC), single LC or heavy chain (HC) fragments, and optionally wherein the amount of the fragmented variant is of up to about 19.2%, of about 9.7%, optionally of about 6.3% or less, preferably of about 4.5% or less, of the anti-BAFF-R antibodies in the composition.

In another aspect, provided are compositions including afucosylated anti-BAFF-R antibodies (e.g., monoclonal anti-BAFF-R antibodies of any of the preceding embodiments, e.g., wherein the monoclonal anti-BAFF-R antibody is an afucosylated IgG₁ antibody and/or wherein the monoclonal anti-BAFF-R antibody is ianalumab or has any one or more of the amino acid sequences of ianalumab described herein), wherein the afucosylated anti-BAFF-R antibodies comprise a methionine-oxidized variant comprising methionine oxidation at one or more of light chain M97, heavy chain M259, heavy chain M365, and heavy chain M435. In some embodiments, the anti-BAFF-R antibodies comprise a methionine-oxidized variant, wherein the methionine-oxidized variant has simultaneous oxidation of M259 on both heavy chains, optionally wherein the amount of the methionine-oxidized variant is of up to 99.7%, of about 56.3%, optionally of about 12.4% or less, preferably of about 6.7% or less, of the anti-BAFF-R antibodies in the composition. In some embodiments, the anti-BAFF-R antibodies comprise a methionine-oxidized variant, wherein the methionine-oxidized variant has simultaneous oxidation of M435 on both heavy chains, optionally wherein the amount of the methionine-oxidized variant is of up to 98.5%, of about 37.3%, optionally of about 12.4% or less, preferably of about 6.7% or less, of the anti-BAFF-R antibodies in the composition. In some embodiments, the afucosylated anti-BAFF-R antibodies further include a deamidated variant, e.g., wherein the deamidated variant comprises deamidation at position N332, and optionally wherein the deamidated variant accounts for about 42.6%, up to about 23%, or up to about 9.7%, optionally about 4.3% or less, preferably about 3.5% or less or about 1% or less, of the anti-BAFF-R antibodies in the composition. In some embodiments, the afucosylated anti-BAFF-R antibodies further include a tryptophan-oxidized variant, e.g., wherein the tryptophan-oxidized variant comprises tryptophan oxidation at heavy chain residue 104 (H-W104) and/or heavy chain residue 59 (H-W59), and optionally wherein the tryptophan-oxidized variant accounts for up to about 23.8%, up to about 16%, up to about 10.5%, optionally about 5.6% or less, preferably of about 3.5% or less or about 3% or less, of the anti-BAFF-R antibodies in the composition. In some embodiments, the afucosylated anti-BAFF-R antibodies include a high molecular weight variant, e.g., wherein the high molecular weight variant accounts for about 11% or less, about 6% or less, or about 2.4% or less, preferably of about 1.5% or less, of the anti-BAFF-R antibodies in the composition. In some embodiments, the afucosylated anti-BAFF-R antibodies further include a clipped variant, wherein the clipped variant comprises clipping between R56 and S57 in the CDR2 loop of the heavy chain, and optionally wherein the amount of the clipped variant is up to about 13.5%, about 10% or less, about 8.8% or less, preferably of about 6.0% or less, of the anti-BAFF-R antibodies in the composition. In some embodiments, the afucosylated anti-BAFF-R antibodies include a fragmented variant, with primary fragmentation site is in the hinge region, leading to the formation of antibody species missing one Fab arm and the corresponding Fab fragment, and to less extent, upon disruption of peptide bonds or disulfide bridges, resulting in an anti-BAFF-R antibody missing one light chain (LC), single LC or heavy chain (HC) fragments, and optionally wherein the amount of the fragmented variant is of up to about 19.2%, of about 9.7%, optionally of about 6.3% or less, preferably of about 4.5% or less, of the anti-BAFF-R antibodies in the composition.

In another aspect, the invention provides a composition comprising afucosylated anti-BAFF-R antibodies (e.g., monoclonal anti-BAFF-R antibodies of any of the preceding embodiments, e.g., wherein the monoclonal anti-BAFF-R antibody is an afucosylated IgG₁ antibody and/or wherein the monoclonal anti-BAFF-R antibody is ianalumab or has any one or more of the amino acid sequences of ianalumab described herein), wherein the afucosylated anti-BAFF-R antibodies comprise a clipped variant comprising clipping between R56 and S57 in the CDR2 loop of the heavy chain, and optionally wherein the amount of the clipped variant is up to about 13.5% about 10% or less, about 8.8% or less, preferably of about 6.0% or less, of the anti-BAFF-R antibodies in the composition. In some embodiments, the afucosylated anti-BAFF-R antibodies further include a deamidated variant, e.g., wherein the deamidated variant comprises deamidation at position N332, and optionally wherein the deamidated variant accounts for up to about 42.6%, up to about 23%, or about 9.7% or less, optionally about 4.3% or less, preferably about 3.5% or less or about 1% or less, of the anti-BAFF-R antibodies in the composition. In some embodiments, the afucosylated anti-BAFF-R antibodies further comprise a tryptophan-oxidized variant comprising tryptophan oxidation at heavy chain residue 104 (H-W104) and/or at heavy chain residue 59 (H-W59), and optionally wherein the tryptophan-oxidized variant accounts for up to about 23.8%, up to about 16%, up to about 10.5%, optionally about 5.6% or less, preferably about 3.5% or less or about 3% or less, of the anti-BAFF-R antibodies in the composition. In some embodiments, the afucosylated anti-BAFF-R antibodies further comprise a methionine-oxidized variant, e.g., wherein the methionine-oxidized variant comprises one or more of light chain M97, heavy chain M259, heavy chain M365, and heavy chain M435, and optionally wherein the methionine-oxidized variant accounts for about 4% or less of the anti-BAFF-R antibodies in the composition. In some embodiments, the anti-BAFF-R antibodies comprise a methionine-oxidized variant, wherein the methionine-oxidized variant has simultaneous oxidation of M259 on both heavy chains, optionally wherein the amount of the methionine-oxidized variant is of up to 99.7%, of about 56.3%, optionally of about 12.4% or less, preferably of about 6.7% or less, of the anti-BAFF-R antibodies in the composition. In some embodiments, the anti-BAFF-R antibodies comprise a methionine-oxidized variant, wherein the methionine-oxidized variant has simultaneous oxidation of M435 on both heavy chains, optionally wherein the amount of the methionine-oxidized variant is of up to 98.5%, of about 37.3%, optionally of about 12.4% or less, preferably of about 6.7% or less, of the anti-BAFF-R antibodies in the composition. In some embodiments, the afucosylated anti-BAFF-R antibodies further comprise a high molecular weight variant, wherein the high molecular weight variant accounts for about 11% or less, about 6% or less, or about 2.4% or less, preferably of about 1.5% or less or about 1% or less, of the anti-BAFF-R antibodies in the composition. In some embodiments, the afucosylated anti-BAFF-R antibodies include a fragmented variant, with primary fragmentation site is in the hinge region, leading to the formation of antibody species missing one Fab arm and the corresponding Fab fragment, and to less extent, upon disruption of peptide bonds or disulfide bridges, resulting in an anti-BAFF-R antibody missing one light chain (LC), single LC or heavy chain (HC) fragments, and optionally wherein the amount of the fragmented variant is of up to about 19.2%, of about 9.7%, optionally of about 6.3% or less, preferably of about 4.5% or less, of the anti-BAFF-R antibodies in the composition.

In some embodiments of any of the preceding aspects, at least 90% of N-glycosylated Fc regions in the anti-BAFF-R antibodies are afucosylated. In some embodiments of any of the preceding aspects, at least 95% of N-glycosylated Fc regions in the anti-BAFF-R antibodies are afucosylated. In some embodiments of any of the preceding aspects, at least 98% of N-glycosylated Fc regions in the anti-BAFF-R antibodies are afucosylated. In some embodiments of any of the preceding aspects, at least 99% of N-glycosylated Fc regions in the anti-BAFF-R antibodies are afucosylated (e.g., 100% of the N-glycosylated Fc regions in the anti-BAFF-R antibodies are afucosylated).

In some embodiments of any of the preceding aspects or embodiments, about 10% or less of N-glycosylated Fc regions in the afucosylated anti-BAFF-R antibodies comprise a high mannose glycan.

In some embodiments of any of the preceding aspects or embodiments, 50% or less of the N-glycosylated Fc regions are galactosylated (e.g., 0% to 49% of the N-glycosylated Fc regions are galactosylated).

In some embodiments of any of the preceding aspects or embodiments, the composition has a relative ADCC potency of about 70% to about 130% in a cell-based ADCC assay compared to a reference standard, e.g., a commercial reference standard (e.g., a relative ADCC potency from 70% to 130% (e.g., from 70% to 100%, from 80% to 120%, from 80% to 100%, from 90% to 110%, from 90% to 100%, or about 100%) in a cell-based ADCC assay compared to a reference standard). In some embodiments, the cell-based ADCC assay is a BAFF-R-expressing NK cell-based ADCC potency assay.

In some embodiments of any of the preceding aspects or embodiments, the composition has a relative ADCC potency of about 80% to about 116% in a cell-based ADCC assay compared to a reference standard (e.g., a commercial reference standard).

In some embodiments of any of the preceding aspects or embodiments, the composition has an ADCC potency within a reference range of ADCC potencies from two or more lots (e.g., two lots) of a reference standard (e.g., a commercial reference standard). In some embodiments, the commercial reference standard is a commercial ianalumab. In some embodiments, the reference standard is an ianalumab reference standard.

In some embodiments of any of the preceding aspects or embodiments, the anti-BAFF-R antibodies have: (a) a binding affinity (K_{D}) to FcγRIIIa^{F158} from 1.3 nM to 3.9 nM; (b) a binding affinity to FcγRIIIa^{V158} from 0.52 nM to 1.59 nM; (c) a relative binding affinity to FcγRIIIa^{F158} and/or FcγRIIIa^{V158} from about 70% to about 130% compared to a commercial reference standard (e.g., a relative binding affinity to FcγRIIIa^{F158} and/or FcγRIIIa^{V158} from 70% to 130% (e.g., from 70% to 100%, from 80% to 120%, from 80% to 100%, from 90% to 110%, from 90% to 100%, or about 100%)).

In some embodiments of any of the preceding aspects or embodiments, the monoclonal anti-BAFF-R antibodies have a serum half-life of at least 5 days.

In some embodiments of any of the preceding aspects or embodiments, the anti-BAFF-R antibodies comprise a variable heavy chain comprising at least 95% (e.g., at least 96%, at least 97%, at least 98%, or at least 99%) sequence identity to the amino acid sequence of SEQ ID NO: 3 and/or a variable light chain comprising at least 95% (e.g., at least 96%, at least 97%, at least 98%, or at least 99%) sequence identity to the amino acid sequence of SEQ ID NO: 4. In some embodiments, the VH comprises the amino acid sequence of SEQ ID NO: 3 and/or the VL comprises the amino acid sequence of SEQ ID NO: 4.

In some embodiments of any of the preceding aspects or embodiments, the anti-BAFF-R antibody comprises a heavy chain having at least 95% (e.g., at least 96%, at least 97%, at least 98%, or at least 99%) identity to the amino acid sequence of SEQ ID NO: 1 and/or a light chain having at least 95% (e.g., at least 96%, at least 97%, at least 98%, or at least 99%) identity to the amino acid sequence of SEQ ID NO: 2. In some embodiments, the heavy chain comprises the amino acid sequence of SEQ ID NO: 1 and/or the light chain comprises the amino acid sequence of SEQ ID NO: 2.

In some embodiments of any of the preceding aspects or embodiments, the anti-BAFF-R antibodies are IgG1 antibodies. In some embodiments, the IgG1 antibodies are IgG1κ antibodies.

In some embodiments of any of the preceding aspects or embodiments, the anti-BAFF-R antibody is ianalumab.

In some embodiments of any of the preceding aspects or embodiments, the composition is a single batch preparation.

In some embodiments of any of the preceding aspects or embodiments, the anti-BAFF-R antibodies are produced in a non-human cell. In some embodiments, the non-human cell is a recombinant Chinese hamster ovary (CHO) cell. In some embodiments, the CHO cell has impaired fucosylation compared to a wild-type CHO cell.

In some embodiments of any of the preceding aspects or embodiments, the composition is formulated into a liquid pharmaceutical formulation suitable for administration to a subject in need thereof.

In another aspect, provided are isolated anti-BAFF-R antibodies produced in a non-human cell. In some embodiments, the antibodies are the anti-BAFF-R antibodies of a composition of any of the preceding aspects or embodiments.

In another aspect, the invention provides a single batch preparation of monoclonal anti-BAFF-R antibodies, wherein the monoclonal anti-BAFF-R antibodies are the isolated anti-BAFF-R antibodies of any of the preceding aspects or embodiments.

In another aspect, provided is a pharmaceutical formulation having: (a) the composition of any one of the preceding aspects or embodiments and a pharmaceutically acceptable carrier, (b) the isolated anti-BAFF-R antibodies of any of the preceding aspects or embodiments and a pharmaceutically acceptable carrier, or (c) the single batch preparation of the preceding aspect and a pharmaceutically acceptable carrier.

In another aspect, provided is a pharmaceutical formulation having a composition of afucosylated anti-BAFF-R antibodies in a pharmaceutically acceptable carrier, wherein the afucosylated anti-BAFF-R antibodies comprise a heavy chain amino acid sequence of SEQ ID NO: 1 and a light chain amino acid sequence of SEQ ID NO: 2, and wherein the anti-BAFF-R antibodies include a high mannose glycan variant and a galactosylated variant.

In another aspect, provided is a lyophilisate obtainable by lyophilisation of the pharmaceutical formulation of any of the preceding aspects.

In another aspect, provided is a pharmaceutical product comprising a container and (e.g., comprising) the pharmaceutical formulation of any of the preceding aspects. In some embodiments, the pharmaceutical formulation is a liquid. In some embodiments, the container is a vial, an injection device, an injection pen, a vial and syringe, a cartridge, a pre-filled syringe, or an autoinjector. In some embodiments, the pharmaceutical product further includes a package insert and/or instructions for use.

In some embodiments, the container is a pre-filled syringe containing the pharmaceutical formulation at 50 mg of any of the anti-BAFF-R antibodies described herein in 1 mL volume or 300 mg of any of the anti-BAFF-R antibodies described herein in 2 mL volume. In some embodiments, the container is a vial containing the pharmaceutical formulation at 150 mg of the anti-BAFF-R antibody in 1 mL volume or 450 mg of the anti-BAFF-R antibody in 3 mL volume.

In another aspect, provided is a composition comprising anti-B cell-activating factor receptor (BAFF-R) antibodies, wherein the anti-BAFF-R antibodies comprise a heavy chain amino acid sequence of SEQ ID NO: 1 and a light chain amino acid sequence of SEQ ID NO: 2, wherein said anti-BAFF-R antibodies comprise N-glycosylated Fc regions, wherein at least 90% of the N-glycosylated Fc regions are afucosylated, and wherein 0% to 10% of the N-glycosylated Fc regions comprise a high mannose glycan. In some embodiments, 0% to 7% of the N-glycosylated Fc regions comprise a high mannose glycan. In some embodiments, 0.5% to 4% of the N-glycosylated Fc regions comprise a high mannose glycan. In some embodiments, 0% to 49% of the N-glycosylated Fc regions are galactosylated. In some embodiments, 15% to 38% of the N-glycosylated Fc regions are galactosylated. In some embodiments, at least 95% of Fc regions of the anti-BAFF-R antibodies are N-glycosylated.

In some embodiments, the anti-BAFF-R antibodies comprise at least one variant selected from a high molecular weight variant, a deamidated variant, a tryptophan-oxidized variant, a methionine-oxidized variant, and a clipped variant. In some embodiments, the anti-BAFF-R antibodies comprise a high molecular weight variant, wherein the high molecular weight variant accounts for up to 6% of the anti-BAFF-R antibodies and fragments thereof in the composition. In some embodiments, the high molecular weight variant accounts for up to 1% of the anti-BAFF-R antibodies in the composition. In some embodiments, the anti-BAFF-R antibodies comprise a deamidated variant comprising deamidation at heavy chain residue 332, wherein the deamidated variant accounts for up to 23% of the anti-BAFF-R antibodies and fragments thereof in the composition. In some embodiments, the deamidated variant accounts for up to 1% of the anti-BAFF-R antibodies in the composition. In some embodiments, the anti-BAFF-R antibodies comprise a tryptophan-oxidized variant comprising oxidation at heavy chain residue 104 and/or heavy chain residue 59, wherein the tryptophan-oxidized variant accounts for up to 16% of the anti-BAFF-R antibodies and fragments thereof in the composition. In some embodiments, the tryptophan-oxidized variant accounts for up to 3% of the anti-BAFF-R antibodies in the composition. In some embodiments, the anti-BAFF-R antibodies comprise a methionine-oxidized variant comprising oxidation at light chain residue 97, wherein the methionine-oxidized variant accounts for up to 62% of the anti-BAFF-R antibodies and fragments thereof in the composition. In some embodiments, the methionine-oxidized variant accounts for up to 4% of the anti-BAFF-R antibodies in the composition. In some embodiments, the anti-BAFF-R antibodies comprise a methionine-oxidized variant comprising oxidation of one or more of heavy chain residues 259, 365, and 435, wherein the methionine-oxidized variant accounts for up to 27% of the anti-BAFF-R antibodies and fragments thereof in the composition. In some embodiments, the methionine-oxidized variant accounts for up to 6% of the anti-BAFF-R antibodies and fragments thereof in the composition. In some embodiments, the anti-BAFF-R antibodies comprise a clipped variant comprising clipping between heavy chain residues 56 and 57. In some embodiments, the clipped variant accounts for up to 10% of the anti-BAFF-R antibodies and fragments thereof in the composition.

In another aspect, provided is a pharmaceutical formulation, wherein the pharmaceutical formulation comprises (a) a composition comprising anti-B cell-activating factor receptor (BAFF-R) antibodies, wherein the anti-BAFF-R antibodies comprise a heavy chain amino acid sequence of SEQ ID NO: 1 and a light chain amino acid sequence of SEQ ID NO: 2, wherein said anti-BAFF-R antibodies comprise N-glycosylated Fc regions, wherein at least 90% of the N-glycosylated Fc regions are afucosylated, and wherein 0% to 10% of the N-glycosylated Fc regions comprise a high mannose glycan.and a (b) pharmaceutically acceptable carrier. In some embodiments, the pharmaceutical formulation comprises a unit dose of the anti-BAFF-R antibody in an amount of 150 mg, 300 mg, or 450 mg. In some embodiments, the pharmaceutical formulation is: (a) a liquid formulation comprising the anti-BAFF-R antibody at a concentration of 150 mg/mL; or (b) a lyophilisate. In some embodiments, the pharmaceutical product comprises a vial, an injection device, an injection pen, a cartridge, a pre-filled syringe, or an autoinjector.

In another aspect, provided is a composition comprising anti-BAFF-R antibodies, wherein the anti-BAFF-R antibodies comprise a heavy chain amino acid sequence of SEQ ID NO: 1 and a light chain amino acid sequence of SEQ ID NO: 2, wherein: (a) at least 95% of the anti-BAFF-R antibodies comprise N-glycosylated Fc regions; (b) at least 95% of the N-glycosylated Fc regions are afucosylated; (c) 0% to 49% of the N-glycosylated Fc regions are galactosylated; and (d) 0% to 7% of the N-glycosylated Fc regions comprise a high mannose glycan. In some embodiments, 15% to 38% of the N-glycosylated Fc regions are galactosylated. In some embodiments, 0.5% to 4% of the N-glycosylated Fc regions comprise a high mannose glycan. In some embodiments, the anti-BAFF-R antibodies comprise one or more of the following: (a) a high molecular weight variant, wherein the high molecular weight variant accounts for up to 6% of the anti-BAFF-R antibodies in the composition; (b) a deamidated variant comprising deamidation at heavy chain residue 332, wherein the deamidated variant accounts for up to 23% of the anti-BAFF-R antibodies in the composition; (c) a tryptophan-oxidized variant comprising oxidation of heavy chain residues 104 and/or 59, wherein the tryptophan-oxidized variant accounts for up to 16% of the anti-BAFF-R antibodies in the composition; (d) a methionine-oxidized variant comprising oxidation at light chain residue 97, wherein the methionine-oxidized variant comprising oxidation at light chain residue 97 accounts for up to 62% of the anti-BAFF-R antibodies in the composition; (e) a methionine-oxidized variant comprising oxidation of one or more of heavy chain residues 259, 365, and 435, wherein the methionine-oxidized variant comprising oxidation of one or more of heavy chain residues 259, 365, and 435 accounts for up to 27% of the anti-BAFF-R antibodies in the composition; and (f) a clipped variant comprising clipping between heavy chain residues 56 and 57, wherein the clipped variant accounts for up to 10% of the anti-BAFF-R antibodies in the composition.

In another aspect, provided is a pharmaceutical product comprising: (i) a composition comprising anti-BAFF-R antibodies, wherein the anti-BAFF-R antibodies comprise a heavy chain amino acid sequence of SEQ ID NO: 1 and a light chain amino acid sequence of SEQ ID NO: 2, wherein: (a) at least 95% of the anti-BAFF-R antibodies comprise N-glycosylated Fc regions; (b) at least 95% of the N-glycosylated Fc regions are afucosylated; (c) 0% to 49% of the N-glycosylated Fc regions are galactosylated; and (d) 0% to 7% of the N-glycosylated Fc regions comprise a high mannose glycan; (ii) a pharmaceutical carrier, and (iii) a container selected from a vial, an injection device, an injection pen, a cartridge, a pre-filled syringe, and an autoinjector, wherein the container contains the composition and the pharmaceutical carrier.

In another aspect, provided is a composition comprising anti-BAFF-R antibodies, wherein the anti-BAFF-R antibodies comprise a heavy chain amino acid sequence of SEQ ID NO: 1 and a light chain amino acid sequence of SEQ ID NO: 2, wherein at least 95% of the anti-BAFF-R antibodies comprise N-glycosylated Fc regions; at least 95% of the N-glycosylated Fc regions are afucosylated; 0% to 49% of the N-glycosylated Fc regions are galactosylated; and (d) 0% to 7% of the N-glycosylated Fc regions comprise a high mannose glycan, and wherein the anti-BAFF-R antibodies comprise one or more of the following: (a) a high molecular weight variant, wherein the high molecular weight variant accounts for up to 1% of the anti-BAFF-R antibodies in the composition; (b) a deamidated variant comprising deamidation at heavy chain residue 332, wherein the deamidated variant accounts for up to 1% of the anti-BAFF-R antibodies in the composition; (c) a tryptophan-oxidized variant comprising oxidation of heavy chain residues 104 and/or 59, wherein the tryptophan-oxidized variant accounts for up to 3% of the anti-BAFF-R antibodies in the composition; (d) a methionine-oxidized variant comprising oxidation at light chain residue 97, and wherein the methionine-oxidized variant comprising oxidation at light chain residue 97 accounts for up to 4% of the anti-BAFF-R antibodies in the composition; (e) a methionine-oxidized variant comprising oxidation of one or more of heavy chain residues 259, 365, and 435, wherein the methionine-oxidized variant comprising oxidation of one or more of heavy chain residues 259, 365, and 435 accounts for up to 6% of the anti-BAFF-R antibodies in the composition; and (f) a clipped variant comprising clipping between heavy chain residues 56 and 57, wherein the clipped variant accounts for up to 4% of the anti-BAFF-R antibodies in the composition.

In another aspect, provided herein is a pharmaceutical product comprising: (i) a composition comprising anti-BAFF-R antibodies, wherein the anti-BAFF-R antibodies comprise a heavy chain amino acid sequence of SEQ ID NO: 1 and a light chain amino acid sequence of SEQ ID NO: 2, wherein at least 95% of the anti-BAFF-R antibodies comprise N-glycosylated Fc regions; at least 95% of the N-glycosylated Fc regions are afucosylated; 0% to 49% of the N-glycosylated Fc regions are galactosylated; and (d) 0% to 7% of the N-glycosylated Fc regions comprise a high mannose glycan, and wherein the anti-BAFF-R antibodies comprise one or more of the following: (a) a high molecular weight variant, wherein the high molecular weight variant accounts for up to 1% of the anti-BAFF-R antibodies in the composition; (b) a deamidated variant comprising deamidation at heavy chain residue 332, wherein the deamidated variant accounts for up to 1% of the anti-BAFF-R antibodies in the composition; (c) a tryptophan-oxidized variant comprising oxidation of heavy chain residues 104 and/or 59, wherein the tryptophan-oxidized variant accounts for up to 3% of the anti-BAFF-R antibodies in the composition; (d) a methionine-oxidized variant comprising oxidation at light chain residue 97, and wherein the methionine-oxidized variant comprising oxidation at light chain residue 97 accounts for up to 4% of the anti-BAFF-R antibodies in the composition; (e) a methionine-oxidized variant comprising oxidation of one or more of heavy chain residues 259, 365, and 435, wherein the methionine-oxidized variant comprising oxidation of one or more of heavy chain residues 259, 365, and 435 accounts for up to 6% of the anti-BAFF-R antibodies in the composition; and (f) a clipped variant comprising clipping between heavy chain residues 56 and 57, wherein the clipped variant accounts for up to 4% of the anti-BAFF-R antibodies in the composition; (ii) a pharmaceutical carrier, and (iii) a container selected from a vial, an injection device, an injection pen, a cartridge, a pre-filled syringe, and an autoinjector, wherein the container contains the composition and the pharmaceutical carrier.

In some embodiments of any of the aforementioned compositions, isolated anti-BAFF-R antibodies, single batch preparations, pharmaceutical formulations, lyophilisate, or pharmaceutical products, the anti-BAFF-R antibody is produced by a method of manufacture comprising a fucosylation-deficient host cell culture (e.g., a fucosylation-deficient CHO cell culture). In some embodiments, the fucosylation-deficient host cell culture produces high mannose N-glycans in less than 10% of the anti-BAFF-R antibodies (e.g., less than 8%, less than 6%, less than 5%, less than 4%, less than 3%, less than 2%, or less than 1%; e.g., from 0.1% to 6%, from 0.5% to 3.5%, or from 1% to 3%; e.g., from 0.1% to 1%, from 1% to 2%, from 2% to 3%, from 3% to 4%, from 4% to 5%, or from 5% to 6% of the anti-BAFF-R antibodies).

In another aspect, the invention provides a method of manufacturing a pharmaceutical formulation comprising anti-BAFF antibodies having an ADCC-dependent mechanism of action, the method including: (a) culturing a recombinant cell line at a pH from about 6.8 to about 7.15 to express a population of anti-BAFF-R antibodies having an ADCC-dependent mechanism of action; and (b) formulating the population of anti-BAFF-R antibodies expressed during step (a) into a pharmaceutical formulation comprising a pharmaceutically acceptable carrier. In some embodiments, step (a) comprises a starting viable cell density from about 1.45x10⁶ cells/mL to about 4x10⁶ cells/mL. In some embodiments, at least 95% of the population of anti-BAFF-R antibodies is afucosylated. In some embodiments, the recombinant cell line is fucosylation deficient. In some embodiments, step (a) comprises a temperature switch from a first temperature to a second temperature, wherein the second temperature is lower than the first temperature. In some embodiments, the second temperature is less than about 36.5° C (e.g., from about 32.5° C to about 34.0° C). In some embodiments, the first temperature is from about 35.8° C to about 37.2° C. In some embodiments, the temperature switch occurs when the recombinant cell line is at a viable cell density from about 10x10⁶ cells/mL to about 16x10⁶ cells/mL. In some embodiments, the duration of step (a) is from about 300 hours to about 350 hours. In some embodiments, the step (a) is in a fed-batch production bioreactor. In some embodiments, the method further includes, prior to step (a), culturing the recombinant cell line in a seed bioreactor. In some embodiments, the method further includes comparing an ADCC potency of the population of anti-BAFF-R antibodies to a target range of ADCC potency, the method including: identifying the target range of ADCC potency, wherein the target range is defined by a range of ADCC potencies of two or more lots of a reference anti-BAFF-R antibody composition; measuring the ADCC potency of the population of anti-BAFF-R antibodies; and determining whether the ADCC potency of the population of anti-BAFF-R antibodies falls within the target range. In some embodiments, the reference anti-BAFF-R antibody composition is ianalumab. In some embodiments, the method further includes recommending the population of anti-BAFF-R antibodies, or a pharmaceutical formulation or drug product thereof, as a treatment of a BAFF-R-related disorder, wherein the determining step results in a determination that the ADCC potency of the population of anti-BAFF-R antibodies falls within the target range.

In another aspect, the invention features a method of manufacturing a pharmaceutical formulation comprising anti-BAFF antibodies having an ADCC-dependent mechanism of action, the method including: (a) culturing a recombinant cell line to express a population of anti-BAFF-R antibodies having an ADCC-dependent mechanism of action, wherein the viable cell density of the recombinant cell line at the start of culturing is from about 1.45x10⁶ cells/mL to about 4x10⁶ cells/mL; and (b) formulating the population of anti-BAFF-R antibodies expressed during step (a) into a pharmaceutical formulation comprising a pharmaceutically acceptable carrier. In some embodiments, step (a) is at a pH from about 6.8 to about 7.15. In some embodiments, at least 95% of the population of anti-BAFF-R antibodies is afucosylated. In some embodiments, the recombinant cell line is fucosylation deficient. In some embodiments, step (a) comprises a temperature switch from a first temperature to a second temperature, wherein the second temperature is lower than the first temperature. In some embodiments, the second temperature is less than about 36.5° C (e.g., from about 32.5° C to about 34.0° C). In some embodiments, the first temperature is from about 35.8° C to about 37.2° C. In some embodiments, the temperature switch occurs when the recombinant cell line is at a viable cell density from about 10x10⁶ cells/mL to about 16x10⁶ cells/mL. In some embodiments, the duration of step (a) is from about 300 hours to about 350 hours. In some embodiments, step (a) is in a fed-batch production bioreactor. In some embodiments, the method further includes, prior to step (a), culturing the recombinant cell line in a seed bioreactor. In some embodiments, the method further includes comparing an ADCC potency of the population of anti-BAFF-R antibodies to a target range of ADCC potency, the method including: identifying the target range of ADCC potency, wherein the target range is defined by a range of ADCC potencies of two or more lots of a reference anti-BAFF-R antibody composition; measuring the ADCC potency of the population of anti-BAFF-R antibodies; and determining whether the ADCC potency of the population of anti-BAFF-R antibodies falls within the target range. In some embodiments, the reference anti-BAFF-R antibody composition is ianalumab. In some embodiments, the method further includes recommending the population of anti-BAFF-R antibodies, or a pharmaceutical formulation or drug product thereof, as a treatment of a BAFF-R-related disorder, wherein the determining step results in a determination that the ADCC potency of the population of anti-BAFF-R antibodies falls within the target range.

In some embodiments of any of the preceding aspects of methods of manufacturing, 95%-100% of the population of antibodies expressed during the culture are afucosylated (e.g., 99% or more of the population of antibodies expressed during culture are afucosylated). In some embodiments, 0-50% of the population of antibodies expressed during the culture comprises galactosylated N-glycans. In some embodiments, the Fc region is a human IgG1 Fc region, e.g., an IgG1κ Fc region. In some embodiments, the anti-BAFF-R antibody comprises a heavy chain having at least 95% identity to the amino acid sequence of SEQ ID NO: 1 and a light chain having at least 95% identity to the amino acid sequence of SEQ ID NO: 2. In some embodiments, the heavy chain comprises the amino acid sequence of SEQ ID NO: 1 and the light chain comprises the amino acid sequence of SEQ ID NO: 2. In some embodiments, the anti-BAFF antibodies having an ADCC-dependent mechanism of action are the isolated anti-BAFF antibodies of any of the preceding aspects or embodiments. In some embodiments, the method further includes lyophilizing the pharmaceutical formulation to produce a lyophilized pharmaceutical formulation. In some embodiments, the method further includes preparing a pharmaceutical product comprising a container by dispensing the pharmaceutical formulation into the container. In some embodiments, the pharmaceutical formulation in the container is a liquid pharmaceutical formulation. In some embodiments, the container is a vial, an injection device, an injection pen, a vial and syringe, a cartridge, a pre-filled syringe, or an autoinjector. In some embodiments, the container comprises a package insert and/or instructions for use (e.g., as a treatment for a BAFF-associated disorder). In some embodiments, the container is a pre-filled syringe containing the pharmaceutical formulation at 50 mg of the anti-BAFF-R antibody in 1 mL volume or 300 mg of the anti-BAFF-R antibody in 2 mL volume. In some embodiments, the container is a vial containing the pharmaceutical formulation at 150 mg of the anti-BAFF-R antibody in 1 mL volume or 450 mg of the anti-BAFF-R antibody in 3 mL volume.

In another aspect, the invention provides a method of matching an ADCC activity of a reference afucosylated anti-BAFF-R antibody composition by: (a) determining an ADCC activity of a reference afucosylated anti-BAFF-R antibody composition; (b) determining an ADCC activity of a second antibody composition comprising an anti-BAFF-R antibody having the same or similar antibody sequence (e.g., at least 95% amino acid sequence identity, at least 96% amino acid sequence identity, at least 97% amino acid sequence identity, at least 98% amino acid sequence identity, at least 99% amino acid sequence identity, or 100% amino acid sequence identity) as the reference anti-BAFF-R antibody; and (c) changing the ADCC activity of the second antibody composition by increasing or decreasing the amount of high-mannose glycans of one or more antibodies within the second antibody composition, wherein the ADCC activity of the second antibody composition after increasing or decreasing the amount of high-mannose is the same as the reference afucosylated anti-BAFF-R antibody composition or within about 10%, about 15%, about 20%, about 25%, about 30%, about 35%, about 40%, about 45% or about 50% of the reference afucosylated anti-BAFF-R antibody composition or within about 1% to about 50% of the reference afucosylated anti-BAFF-R antibody composition.

In another aspect, the invention provides a method for engineering a target ADCC activity of an afucosylated anti-BAFF-R antibody composition by: (a) determining an ADCC activity of a afucosylated anti-BAFF-R antibody composition; (b) determining a target ADCC activity; and (c) increasing or decreasing the ADCC activity of the afucosylated anti-BAFF-R antibody composition by increasing or decreasing the amount high-mannose glycans in the Fc region of the antibody, wherein the ADCC activity of the afucosylated anti-BAFF-R antibody composition after increasing or decreasing the amount of high-mannose glycans is the same as the target ADCC activity or within about 10%, about 15%, about 20%, about 25%, about 30%, about 35%, about 40%, about 45% or about 50% of the target ADCC activity or within about 1% to about 50% of the target ADCC activity. In some embodiments, step (a) occurs before or at the same time as step (b) and/or step (c). In some embodiments, step (a) occurs after step (b) and/or step (c). In some embodiments, an increase of about 1% high-mannose glycans increases ADCC activity by about 20% to about 30%. In some embodiments, the reference anti-BAFF-R antibody is ianalumab.

In some aspects, the invention includes any of the compositions, isolated anti-BAFF-R antibodies, single batch preparations, pharmaceutical formulations, lyophilisates, pharmaceutical products, or pharmaceutical formulations manufactured by any of the methods of any of the preceding aspects or embodiments, for use in a method of treating a BAFF-R-related disorder. In some embodiments, the BAFF-R-related disorder is an autoimmune disease or a B-cell neoplasm. In some embodiments, the subject is a human. In some embodiments, the anti-BAFF-R antibody is administered at a dose of about 3 mg to about 10 mg per kilogram of the subject (mg/kg). In some embodiments, the anti-BAFF-R antibody is administered at a dose of about 3 mg/kg or about 9 mg/kg.

In some aspects, the invention provides methods of treating a BAFF-R-related disorder by administering an effective amount (e.g., a therapeutically effective amount) of any of the compositions, isolated anti-BAFF-R antibodies, single batch preparations, pharmaceutical formulations, lyophilisates, pharmaceutical products, or pharmaceutical formulations manufactured by any of the methods of any of the preceding aspects or embodiments. In some embodiments, the BAFF-R-related disorder is an autoimmune disease or a B-cell neoplasm. In some embodiments, the subject is a human. In some embodiments, the anti-BAFF-R antibody is administered at a dose of about 3 mg to about 10 mg per kilogram of the subject (mg/kg). In some embodiments, the anti-BAFF-R antibody is administered at a dose of about 3 mg/kg or about 9 mg/kg.

In some embodiments, the anti-BAFF-R antibody is administered intravenously. In some embodiments, the anti-BAFF-R antibody is administered at a dose of about 150 mg to about 400 mg, such as about 300 mg. In some embodiments, the dose is 150 mg or 300 mg of the anti-BAFF-R antibody.

In some embodiments, the anti-BAFF-R antibody is administered to a subject in need thereof subcutaneously. In some embodiments, the anti-BAFF-R antibody is administered to a subject in need thereof once every 4 weeks (e.g., monthly, +/- 3 days) or once every 12 weeks (e.g. every 3 months, +/- 3 days).

In some embodiments, the autoimmune disease is autoimmune haematological disorders (including e.g. warm autoimmune hemolytic anaemia, aplastic anaemia, pure red cell anaemia and immune thrombocytopenia), acquired hemophilia A, cold agglutinin disease, cryoglobulinemia, thrombotic thrombocytopenic purpura, Sjögren's Disease, systemic lupus erythematosus, lupus nephritis, inflammatory muscle disorders, polychondritis, sclerodoma, anti-neutrophil cytoplasmic antibody-associated vasculitis, IgM mediated neuropathy, opsoclonus myoclonus syndrome, Wegener granulomatosis, dermatomyositis, chronic active hepatitis, myasthenia gravis, psoriasis, Steven-Johnson syndrome, pemphigus vulgaris, pemphigus foliacius, idiopathic sprue, autoimmune inflammatory bowel disease (including e.g. ulcerative colitis, Crohn's disease and Irritable Bowel Syndrome), endocrine ophthalmopathy, Graves' disease, sarcoidosis, multiple sclerosis, neuromyelitis optica, primary biliary cirrhosis, juvenile diabetes (diabetes mellitus type I), uveitis (anterior, intermediate and posterior as well as panuveitis), keratoconjunctivitis sicca and vernal keratoconjunctivitis, interstitial lung fibrosis, psoriatic arthritis and glomerulonephritis (with and without nephrotic syndrome, e.g. including idiopathic nephrotic syndrome or minimal change nephropathy), tumors, inflammatory disease of skin and cornea, myositis, loosening of bone implants, or metabolic disorders, such as atherosclerosis, diabetes, and dislipidemia.

In some embodiments, the autoimmune disease is warm autoimmune hemolytic anemia, and optionally wherein the anti-BAFF-R antibody is administered to a subject in need thereof at a dose of about 3 mg/kg or about 9 mg/kg, iv., once every 4 weeks (e.g., monthly, +/- 3 days) for a duration of up to 4 months.

In some embodiments, the autoimmune disease is immune thrombocytopenia, and optionally wherein the anti-BAFF-R antibody is administered to a subject in need thereof at a dose of about 3 mg/kg or about 9 mg/kg, iv., once every 4 weeks (e.g., monthly, +/- 3 days) for a duration of up to 4 months.

In some embodiments, the autoimmune disease is Sjögren's Disease, systemic lupus erythematosus, lupus nephritis, hidradenitis suppurativa, systemic sclerosis, or scleroderma, and optionally wherein the anti-BAFF-R antibody is administered to a subject in need thereof at a dose of about 300 mg, subcutaneously, once every 4 weeks (e.g., monthly, +/- 3 days), or once every 12 weeks (e.g. every 3 months, +/- 3 days).

In another aspect, the invention includes methods of controlling ADCC activity of single batch preparation of a population of anti-BAFF-R antibodies comprising: (a) determining an ADCC activity of an afucosylated anti-BAFF-R antibody composition; and (b) increasing or decreasing the ADCC activity of the afucosylated anti-BAFF-R antibody composition by increasing or decreasing the amount of high mannose in the glycan species anti-BAFF-R antibodies within the composition. In some embodiments, the single batch preparation of a population of anti-BAFF-R antibodies is the single batch population of any of the preceding aspects or embodiments.

In another aspect, provided is a composition comprising anti-BAFF-R antibodies, wherein said anti-BAFF-R antibodies comprise N-glycosylated Fc regions, wherein 10% or less of the N-glycosylated Fc regions comprise a high mannose glycan. In some embodiments, at least 90% of the N-glycosylated Fc regions are afucosylated. In some embodiments, 0% to 49% of the N-glycosylated Fc regions are galactosylated. In some embodiments, 0% to 7% of the N-glycosylated Fc regions comprise a high mannose glycan. In some embodiments, 0.5% to 4% of the N-glycosylated Fc regions comprise a high mannose glycan. In some embodiments, the composition has a relative antibody-dependent cell-mediated cytotoxicity (ADCC) potency of 70% to 130% in a cell-based ADCC assay compared to a reference standard. In some embodiments, the composition has a relative ADCC potency of 80% to 116% in a cell-based ADCC assay compared to the reference standard. In some embodiments, the cell-based ADCC assay is a BAFF-R-expressing NK cell-based ADCC potency assay. In some embodiments, the composition has an ADCC potency within a reference range of ADCC potencies from two lots of the reference standard. In some embodiments, the composition has an ADCC potency within a reference range of ADCC potencies from two lots of a reference standard. In some embodiments, the anti-BAFF-R antibodies have a relative binding affinity to FcγRIIIa^{F158} and/or FcγRIIIa^{V158} from about 70% to about 130% compared to the reference standard. In some embodiments, the e anti-BAFF-R antibodies have a relative binding affinity to FcγRIIIa^{F158} and/or FcγRIIIa^{V158} from about 70% to about 130% compared to a reference standard. In some embodiments, the reference standard is ianalumab. In some embodiments, the ianalumab reference standard is manufactured and analyzed in accordance with good manufacturing practices (GMP). In some embodiments, the anti-BAFF-R antibodies have: (a) a binding affinity (K_{D}) to FcγRIIIa^{F158} from 1.3 nM to 3.9 nM; or (b) a binding affinity to FcγRIIIa^{V158} from 0.52 nM to 1.59 nM. In some embodiments, the anti-BAFF-R antibodies have a K_{D} to FcγRIIIa^{F158} from 2.0 nM to 3.0 nM. In some embodiments, the composition depletes human B cells through ADCC with a half-maximal efficacy (EC₅₀): (a) from 10 pM to 1 nM in an *in vitro* culture of human whole blood; and/or (b) from 0.2 pM to 100 pM in an *in vitro* culture of human peripheral blood mononuclear cells. In some embodiments, the composition depletes human B cells through ADCC with a half-maximal efficacy (EC₅₀) from 150 pM to 250 pM in an *in vitro* culture of human whole blood. In some embodiments, the composition depletes human B cells through ADCC with a half-maximal efficacy (EC₅₀) from 2.0 pM to 5 pM in an *in vitro* culture of human peripheral blood mononuclear cells. In some embodiments, the monoclonal anti-BAFF-R antibodies have a serum half-life of at least 5 days. In some embodiments, the anti-BAFF-R antibodies bind to a BAFF-R epitope comprising one or more of residues D26, L27, L28, V29, R30, H31, C32, V33, A34, and C35. In some embodiments, the anti-BAFF-R antibodies comprise one or more anti-BAFF-R paratopic residues comprising one or more of heavy chain residues N32, S33, A34, A35, R52, Y54, R56, S57, Y60, Y102, D103, W104, V110 and/or one or more of light chain residues L92, Y93, and S94. In some embodiments, the anti-BAFF-R antibodies comprise a CDR-H1, a CDR-H2, and a CDR-H3 having the amino acid sequences of SEQ ID NO: 5, SEQ ID NO: 6, and SEQ ID NO: 7, and a CDR-L1, a CDR-L2, and a CDR-L3 having the amino acid sequences of SEQ ID NO: 8, SEQ ID NO: 9, and SEQ ID NO: 10, respectively. In some embodiments, the anti-BAFF-R antibodies comprise at least one variant selected from a high molecular weight variant, a deamidated variant, a tryptophan-oxidized variant, a methionine-oxidized variant, and a clipped variant. In some embodiments, the anti-BAFF-R antibodies comprise a high molecular weight variant. In some embodiments, the high molecular weight variant accounts for up to 6% of the total anti-BAFF-R antibodies and fragments thereof in the composition. In some embodiments, the high molecular weight variant accounts for up to 1% of the total anti-BAFF-R antibodies and fragments thereof in the composition. In some embodiments, the anti-BAFF-R antibodies comprise a deamidated variant comprising deamidation at position N332, and wherein the deamidated variant accounts for up to 23% of the total anti-BAFF-R antibodies and fragments thereof in the composition. In some embodiments, the deamidated variant accounts for up to 1% of the total anti-BAFF-R antibodies and fragments thereof in the composition. In some embodiments, the anti-BAFF-R antibodies comprise a tryptophan-oxidized variant comprising tryptophan oxidation at heavy chain residue 104 and/or heavy chain residue 59. In some embodiments, the tryptophan-oxidized variant accounts for up to 16% of the total anti-BAFF-R antibodies and fragments thereof in the composition. In some embodiments, the tryptophan-oxidized variant accounts for up to 3% of the anti-BAFF-R antibodies, or fragments thereof, in the composition. In some embodiments, the anti-BAFF-R antibodies comprise a methionine-oxidized variant comprising methionine oxidation at one or more of light chain residue 97, heavy chain residue 259, heavy chain residue 365, and heavy chain residue 435. In some embodiments, the methionine-oxidized variant comprises methionine oxidation at light chain residue 97, and wherein the methionine-oxidized variant comprising oxidation at light chain residue 97 accounts for up to 62% of the total anti-BAFF-R antibodies and fragments thereof in the composition. In some embodiments, the methionine-oxidized variant comprising oxidation at light chain residue 97 accounts for up to 4% of the total anti-BAFF-R antibodies and fragments thereof in the composition. In some embodiments, the methionine-oxidized variant comprises methionine oxidation at one or more of heavy chain residues 259, 365, and 435, and wherein the methionine-oxidized variant comprising oxidation at one or more of heavy chain residues 259, 365, and 435 accounts for up to 27% of the total anti-BAFF-R antibodies and fragments thereof in the composition. In some embodiments, the methionine-oxidized variant comprising oxidation at one or more of heavy chain residues 259, 365, and 435 accounts for up to 6% of the total anti-BAFF-R antibodies and fragments thereof in the composition. In some embodiments, the anti-BAFF-R antibodies comprise a clipped variant comprising clipping between heavy chain residues 56 and 57. In some embodiments, the clipped variant accounts for up to 10% of the total anti-BAFF-R antibodies and fragments thereof in the composition.

In another aspect, provided is a composition comprising afucosylated anti-BAFF-R antibodies, wherein the afucosylated anti-BAFF-R antibodies comprise a high molecular weight variant, and wherein the high molecular weight variant accounts for up to 6% of the anti-BAFF-R antibodies in the composition. In some embodiments, the high molecular weight variant accounts for up to 1% of the total anti-BAFF-R antibodies and fragments thereof in the composition. In some embodiments, the afucosylated anti-BAFF-R antibodies comprise a deamidated variant comprising deamidation at heavy chain 332. In some embodiments, the deamidated variant accounts for up to 23% of the total anti-BAFF-R antibodies and fragments thereof in the composition. In some embodiments, the deamidated variant accounts for up to 1% of the total anti-BAFF-R antibodies and fragments thereof in the composition. In some embodiments, the afucosylated anti-BAFF-R antibodies comprise a tryptophan-oxidized variant comprising tryptophan oxidation at heavy chain residue 104 and/or heavy chain residue 59. In some embodiments, the tryptophan-oxidized variant accounts for up to 16% of the total anti-BAFF-R antibodies and fragments thereof in the composition. In some embodiments, the afucosylated anti-BAFF-R antibodies comprise a methionine-oxidized variant comprising methionine oxidation at light chain residue 97, and wherein the methionine-oxidized variant at light chain residue 97 accounts for up to 62% of the total anti-BAFF-R antibodies and fragments thereof in the composition. In some embodiments, the methionine-oxidized variant accounts for up to 4% of the total anti-BAFF-R antibodies and fragments thereof in the composition. In some embodiments, the afucosylated anti-BAFF-R antibodies comprise a methionine-oxidized variant comprising methionine oxidation of one or more of heavy chain residues 259, 365, and 435, and wherein the methionine-oxidized variant comprising oxidation of one or more of heavy chain residues 259, 365, and 435 accounts for up to 27% of the total anti-BAFF-R antibodies and fragments thereof in the composition. In some embodiments, the methionine-oxidized variant comprising methionine oxidation of one or more of heavy chain residues 259, 365, and 435 accounts for up to 6% of the total anti-BAFF-R antibodies and fragments thereof in the composition. In some embodiments, the afucosylated anti-BAFF-R antibodies comprise a clipped variant comprising clipping between heavy chain residues 56 and 57. In some embodiments, the clipped variant accounts for up to 10% of the total anti-BAFF-R antibodies and fragments thereof in the composition.

In another aspect, the invention provides a composition comprising afucosylated anti-BAFF-R antibodies, wherein the afucosylated anti-BAFF-R antibodies comprise a deamidated variant comprising deamidation at position N332, and wherein the deamidated variant accounts for up to 23% of the total anti-BAFF-R antibodies and fragments thereof in the composition. In some embodiments, the deamidated variant accounts for up to 1% of the total anti-BAFF-R antibodies and fragments thereof in the composition. In some embodiments, the afucosylated anti-BAFF-R antibodies comprise a high molecular weight variant, and wherein the high molecular weight variant accounts for up to 6% of the total anti-BAFF-R antibodies and fragments thereof in the composition. In some embodiments, the high molecular weight variant accounts for up to 1% of the total anti-BAFF-R antibodies and fragments thereof in the composition. In some embodiments, the afucosylated anti-BAFF-R antibodies comprise a tryptophan-oxidized variant comprising tryptophan oxidation at heavy chain residue 104 and/or heavy chain residue 59. In some embodiments, the tryptophan-oxidized variant accounts for up to 16% of the anti-BAFF-R antibodies, or fragments thereof, in the composition. In some embodiments, the tryptophan-oxidized variant accounts for up to 3% of the anti-BAFF-R antibodies, or fragments thereof, in the composition. In some embodiments, the afucosylated anti-BAFF-R antibodies comprise a methionine-oxidized variant comprising methionine oxidation at one or more of light chain residue 97, heavy chain residue 259, heavy chain residue 365, and heavy chain residue 435. In some embodiments, the methionine-oxidized variant comprises methionine oxidation at light chain residue 97, and wherein the methionine-oxidized variant comprising oxidation at light chain residue 97 accounts for up to 62% of the total anti-BAFF-R antibodies and fragments thereof in the composition. In some embodiments, the methionine-oxidized variant comprising methionine oxidation at light chain residue 97 accounts for up to 4% of the total anti-BAFF-R antibodies and fragments thereof in the composition. In some embodiments, the methionine-oxidized variant comprises methionine oxidation at one or more of heavy chain residues 259, 365, and 435, and wherein the methionine-oxidized variant comprising oxidation at one or more of heavy chain residues 259, 365, and 435 accounts for up to 27% of the total anti-BAFF-R antibodies or fragments thereof in the composition. In some embodiments, the methionine-oxidized variant comprising methionine oxidation at one or more of heavy chain residues 259, 365, and 435 accounts for up to 6% of the total anti-BAFF-R antibodies and fragments thereof in the composition. In some embodiments, the afucosylated anti-BAFF-R antibodies comprise a clipped variant comprising clipping between heavy chain residues 56 and 57. In some embodiments, the clipped variant accounts for up to 10% of the total anti-BAFF-R antibodies and fragments thereof in the composition.

In another aspect, provided is a composition comprising afucosylated anti-BAFF-R antibodies, wherein the afucosylated anti-BAFF-R antibodies comprise a tryptophan-oxidized variant comprising tryptophan oxidation at heavy chain residue 104 and/or heavy chain residue 59. In some embodiments, the tryptophan-oxidized variant accounts for up to 16% of the total anti-BAFF-R antibodies and fragments thereof in the composition. In some embodiments, the tryptophan-oxidized variant accounts for up to 3% of the total anti-BAFF-R antibodies and fragments thereof in the composition. In some embodiments, the afucosylated anti-BAFF-R antibodies comprise a deamidated variant comprising deamidation at position N332, and wherein the deamidated variant accounts for up to 23% of the anti-BAFF-R antibodies, or fragments thereof, in the composition. In some embodiments, the deamidated variant accounts for up to 1% of the total anti-BAFF-R antibodies and fragments thereof in the composition. In some embodiments, the afucosylated anti-BAFF-R antibodies comprise a high molecular weight variant, and wherein the high molecular weight variant accounts for up to 6% of the total anti-BAFF-R antibodies and fragments thereof in the composition. In some embodiments, the high molecular weight variant accounts for up to 1% of the total anti-BAFF-R antibodies and fragments thereof in the composition. In some embodiments, the afucosylated anti-BAFF-R antibodies comprise a methionine-oxidized variant comprising methionine oxidation at one or more of light chain residue 97, heavy chain residue 259, heavy chain residue 365, and heavy chain residue 435. In some embodiments, the methionine-oxidized variant comprises methionine oxidation at light chain residue 97, and the methionine-oxidized variant comprising oxidation at light chain residue 97 accounts for up to 62% of the total anti-BAFF-R antibodies and fragments thereof in the composition. In some embodiments, the methionine-oxidized variant comprising methionine oxidation at light chain residue 97 accounts for up to 4% of the total anti-BAFF-R antibodies and fragments thereof in the composition. In some embodiments, the methionine-oxidized variant comprises methionine oxidation at one or more of heavy chain residues 259, 365, and 435, and wherein the methionine-oxidized variant comprising oxidation at one or more of heavy chain residues 259, 365, and 435 accounts for up to 27% of the total anti-BAFF-R antibodies and fragments thereof in the composition. In some embodiments, the methionine-oxidized variant comprising methionine oxidation at one or more of heavy chain residues 259, 365, and 435 accounts for up to 6% of the total anti-BAFF-R antibodies and fragments thereof in the composition. In some embodiments, the afucosylated anti-BAFF-R antibodies further comprise a clipped variant comprising clipping between heavy chain residues 56 and 57. In some embodiments, the clipped variant accounts for up to 10% of the total anti-BAFF-R antibodies and fragments thereof in the composition.

In another aspect, provided is a composition comprising afucosylated anti-BAFF-R antibodies, wherein the afucosylated anti-BAFF-R antibodies comprise a methionine-oxidized variant comprising methionine oxidation at one or more of light chain residue 97, heavy chain residue 259, heavy chain residue 365, and heavy chain residue 435. In some embodiments, the methionine-oxidized variant comprises methionine oxidation at light chain residue 97, and wherein the methionine-oxidized variant comprising oxidation at light chain residue 97 accounts for up to 62% of the total anti-BAFF-R antibodies and fragments thereof in the composition. In some embodiments, the methionine-oxidized variant comprising methionine oxidation at light chain residue 97 accounts for up to 4% of the total anti-BAFF-R antibodies and fragments thereof in the composition. In some embodiments, the methionine-oxidized variant comprises methionine oxidation at one or more of heavy chain residues 259, 365, and 435, and wherein the methionine-oxidized variant comprising oxidation at one or more of heavy chain residues 259, 365, and 435 accounts for up to 27% of the total anti-BAFF-R antibodies and fragments thereof in the composition. In some embodiments, the methionine-oxidized variant comprising methionine oxidation at one or more of heavy chain residues 259, 365, and 435 accounts for up to 6% of the total anti-BAFF-R antibodies and fragments thereof in the composition. In some embodiments, the afucosylated anti-BAFF-R antibodies further comprise a deamidated variant comprising deamidation at position N332, and wherein the deamidated variant accounts for up to 23% of the total anti-BAFF-R antibodies and fragments thereof in the composition. In some embodiments, the deamidated variant accounts for up to 1% of the total anti-BAFF-R antibodies and fragments thereof in the composition. In some embodiments, the afucosylated anti-BAFF-R antibodies further comprise a tryptophan-oxidized variant comprising oxidation at heavy chain residue 104 and/or heavy chain residue 59. In some embodiments, the tryptophan-oxidized variant accounts for up to 16% of the total anti-BAFF-R antibodies and fragments thereof in the composition. In some embodiments, the tryptophan-oxidized variant accounts for up to 3% of the total anti-BAFF-R antibodies and fragments thereof in the composition. In some embodiments, the afucosylated anti-BAFF-R antibodies comprise a high molecular weight variant, wherein the high molecular weight variant accounts for up to 6% of the total anti-BAFF-R antibodies and fragments thereof in the composition. In some embodiments, the high molecular weight variant accounts for up to 1% of the total anti-BAFF-R antibodies and fragments thereof in the composition. In some embodiments, the afucosylated anti-BAFF-R antibodies further comprise a clipped variant comprising clipping between heavy chain residues 56 and 57. In some embodiments, the clipped variant accounts for up to 10% of the total anti-BAFF-R antibodies and fragments thereof in the composition. In some embodiments, the afucosylated anti-BAFF-R antibodies comprise a clipped variant comprising clipping between heavy chain residues 56 and 57. In some embodiments, the clipped variant accounts for up to 10% of the total anti-BAFF-R antibodies and fragments thereof in the composition. In some embodiments, the afucosylated anti-BAFF-R antibodies further comprise a deamidated variant comprising deamidation at position N332, and wherein the deamidated variant accounts for up to 23% of the total anti-BAFF-R antibodies and fragments thereof in the composition. In some embodiments, the deamidated variant accounts for up to 1% of the total anti-BAFF-R antibodies and fragments thereof in the composition. In some embodiments, the afucosylated anti-BAFF-R antibodies further comprise a tryptophan-oxidized variant comprising oxidation at heavy chain residue 104 and/or heavy chain residue 59. In some embodiments, the tryptophan-oxidized variant accounts for up to 16% of the total anti-BAFF-R antibodies and fragments thereof in the composition. In some embodiments, the tryptophan-oxidized variant accounts for up to 3% of the total anti-BAFF-R antibodies and fragments thereof in the composition. In some embodiments, the afucosylated anti-BAFF-R antibodies further comprise a methionine-oxidized variant comprising methionine oxidation at one or more of light chain residue 97, heavy chain residue 259, heavy chain residue 365, and heavy chain residue 435. In some embodiments, the methionine-oxidized variant comprises methionine oxidation at light chain residue 97, and wherein the methionine-oxidized variant comprising methionine oxidation at light chain residue 97 accounts for up to 62% of the total anti-BAFF-R antibodies and fragments thereof in the composition. In some embodiments, the methionine-oxidized variant comprising methionine oxidation at light chain residue 97 accounts for up to 4% of the total anti-BAFF-R antibodies and fragments thereof in the composition. In some embodiments, the methionine-oxidized variant comprises methionine oxidation at one or more of heavy chain residues 259, 365, and 435, and the methionine-oxidized variant comprising methionine oxidation at one or more of heavy chain residues 259, 365, and 435 accounts for up to 27% of the total anti-BAFF-R antibodies and fragments thereof in the composition. In some embodiments, the methionine-oxidized variant comprises oxidation at one or more of heavy chain residues 259, 365, and 435 accounts for up to 6% of the total anti-BAFF-R antibodies and fragments thereof in the composition. In some embodiments, the afucosylated anti-BAFF-R antibodies further comprise a high molecular weight variant, and wherein the high molecular weight variant accounts for up to 6% of the total anti-BAFF-R antibodies and fragments thereof in the composition. In some embodiments, the high molecular weight variant accounts for up to 1% of the total anti-BAFF-R antibodies and fragments thereof in the composition.

In some embodiments, the composition comprises N-glycosylated Fc regions (e.g., N-glycosylated Fc regions of the anti-BAFF-R antibodies). In some embodiments, at least 95% of the Fc regions are N-glycosylated. In some embodiments, at least 90% of the N-glycosylated Fc regions are afucosylated. In some embodiments, 0% to 10% of the N-glycosylated Fc regions comprise a high mannose glycan. In some embodiments, 0% to 7% of the N-glycosylated Fc regions comprise a high mannose glycan. In some embodiments, 0.5% to 4% of the N-glycosylated Fc regions comprise a high mannose glycan. In some embodiments, 0% to 49% of the N-glycosylated Fc regions are galactosylated. In some embodiments, 15% to 38% of the N-glycosylated Fc regions are galactosylated. In some embodiments, the composition has a relative ADCC potency of about 70% to about 130% in a cell-based ADCC assay compared to a reference standard. In some embodiments, the composition has a relative ADCC potency of about 80% to about 116% in a cell-based ADCC assay compared to a reference standard. In some embodiments, the composition has an ADCC potency within a reference range of ADCC potencies from two lots of a reference standard.

In some embodiments, the anti-BAFF-R antibodies have a relative ADCC potency from about 70% to about 130% in a cell-based ADCC assay as compared to a reference standard. In some embodiments, the anti-BAFF-R antibodies have: (a) a binding affinity (K_{D}) to FcγRIIIa^{F158} from 1.3 nM to 3.9 nM; (b) a binding affinity to FcγRIIIa^{V158} from 0.52 nM to 1.59 nM; (c) a relative binding affinity to FcγRIIIa^{F158} and/or FcγRIIIa^{V158} from about 70% to about 130% compared to a reference standard. In some embodiments, the reference standard is ianalumab.

In some embodiments, the anti-BAFF-R antibodies have a serum half-life of at least 5 days.

In some embodiments, the anti-BAFF-R antibodies comprise a variable heavy chain (VH) comprising an amino acid sequence of at least 95% sequence identity to SEQ ID NO: 3 and/or a variable light chain (VL) comprising an amino acid sequence of at least 95% sequence identity to SEQ ID NO: 4. In some embodiments, the VH comprises the amino acid sequence of SEQ ID NO: 3 and/or the VL comprises the amino acid sequence of SEQ ID NO: 4. In some embodiments, the anti-BAFF-R antibodies comprise a heavy chain comprising an amino acid sequence of at least 95% sequence identity to SEQ ID NO: 1 and a light chain comprising an amino acid sequence of at least 95% identity to the amino acid sequence of SEQ ID NO: 2. In some embodiments, the heavy chain comprises the amino acid sequence of SEQ ID NO: 1 and the light chain comprises the amino acid sequence of SEQ ID NO: 2.

In some embodiments, the anti-BAFF-R antibodies are IgG1 antibodies. In some embodiments, the IgG1 antibodies are IgG₁κ antibodies. In some embodiments, the anti-BAFF-R antibody is ianalumab.

In some embodiments, the composition is a single batch preparation.

In some embodiments, the anti-BAFF-R antibodies are produced in a non-human cell. In some embodiments, the non-human cell is a recombinant Chinese hamster ovary (CHO) cell. In some embodiments, the CHO cell has impaired fucosylation compared to a wild-type CHO cell.

In some embodiments, the composition is in the form of a liquid pharmaceutical formulation suitable for administration to a subject in need thereof.

In another aspect, provided are isolated anti-BAFF-R antibodies produced in a non-human cell, wherein the antibodies are the anti-BAFF-R antibodies of the composition of any of the preceding embodiments In some embodiments, the anti-BAFF-R antibodies are the isolated anti-BAFF-R antibodies of the previous embodiment.

In another aspect, provided is a pharmaceutical formulation comprising: (a) the composition of any of the preceding embodiments and a pharmaceutically acceptable carrier, the isolated anti-BAFF-R antibodies and a pharmaceutically acceptable carrier, or the single batch preparation and a pharmaceutically acceptable carrier. In some embodiments, the pharmaceutical formulation is in the form of a liquid suitable for administration to a subject in need thereof.

In another aspect, provided is a lyophilisate obtainable by lyophilisation of the pharmaceutical formulation of any preceding embodiment.

In another aspect, provided is a pharmaceutical product comprising a container and the pharmaceutical formulation of any preceding embodiment or the lyophilisate of any preceding embodiment. In some embodiments, the pharmaceutical product comprises a vial, an injection device, an injection pen, a cartridge, a pre-filled syringe, or an autoinjector. In some embodiments, the pharmaceutical produce further comprises a package insert and/or instructions for use. In some embodiments, the pharmaceutical produce comprises a pre-filled syringe containing the pharmaceutical formulation at 50 mg of the anti-BAFF-R antibody in 1 mL volume or 300 mg of the anti-BAFF-R antibody in 2 mL volume. In some embodiments, the pharmaceutical produce further comprises a vial containing the pharmaceutical formulation at 150 mg of the anti-BAFF-R antibody in 1 mL volume or 450 mg of the anti-BAFF-R antibody in 3 mL volume.

In another aspect, provided is the composition of any preceding embodiment, the isolated anti-BAFF-R antibodies of any preceding embodiment, the single batch preparation of any preceding embodiment, the pharmaceutical formulation of any preceding embodiment, the lyophilisate of any preceding embodiment, or the pharmaceutical product of any preceding embodiment, wherein the anti-BAFF-R antibody is produced by a method of manufacture comprising a fucosylation-deficient host cell culture.
In another aspect, provided is the composition, isolated anti-BAFF-R antibodies, single batch preparation, pharmaceutical formulation, lyophilisate, or pharmaceutical product of any previous embodiment, wherein the fucosylation-deficient host cell culture produces high mannose N-glycans in 0% to 10% of the anti-BAFF-R antibodies.

### BRIEF DESCRIPTION OF THE DRAWINGS

FIG. 1A is a histogram showing buried surface area (BSA) by residue of human BAFF-R upon complex formation with ianalumab Fab.
FIG. 1B is a rendering showing steric blocking by ianalumab to BAFF binding to BAFF-R. The composition model was generated with the structure of ianalumab Fab complexed with BAFF-R and that of BAFF-R complexed with BAFF (PDB ID: 1OQE). BAFF-R is shown in ribbon representation with a semi-transparent surface. lanalumab is shown in ribbon, BAFF in a semi-transparent surface. HC refers to the Fab heavy chain, and LC refers to the Fab light chain.
FIGS. 2A and 2B are renderings of the ianalumab Fab : BAFF-R binding interface (heavy chain interactions). FIG. 2A shows the interactions of CDR1, CDR2, and CDR3 of the heavy chain. FIG. 2B shows the central region of the BAFF-R : ianalumab interface, highlighting individual amino acid residues involved in binding.
FIG. 3 is a rendering of the ianalumab Fab : BAFF-R binding interface (light chain interactions).
FIG. 4 is a buried surface area plot of ianalumab showing heavy chain paratopic residues on the top panel and light chain paratopic residues on the bottom panel.
FIG. 5 is a heat map showing the 8HDX-MS differential deuterium uptake for BAFF-R.
FIG. 6 is a heat map showing HDX-MS differential deuterium uptake for ianalumab Fab (heavy chain).
FIG. 7 is a heat map showing HDX-MS differential deuterium uptake for ianalumab Fab (kappa chain).
FIG. 8 is a dose response curve showing potency of ianalumab DS binding to BAFF-R ECD relative to reference standard.
FIGS. 9A and 9B are sensorgram overlays of ianalumaub drug substance (DS) batches showing binding to low affinity Fc gamma receptor FcγRIIIa^{F158} (FIG. 9A) and FcγRIIIa^{V158} (FIG. 9B). X-axis: time [s]; y-axis: relative response [RU]. Association and dissociation phases of duplicate injections are shown. Antibody sequential concentrations on receptor FcyRllla (left to right) are 0.39, 0.78, 1.6, 3.1, 6.2 nM.
FIGS. 10A and 10B are representative overlays of ianalumab drug substance showing binding to low affinity FcγRIIIa^{F158} (FIG. 18A) and FcγRIIIa^{V158} (FIG. 18B). X-axis: time [s]; y-axis: relative response [RU]; One representative fitted sensorgram overlay obtained for sample : BC0001 (process E). Association and dissociation phases of duplicate injections are shown. Antibody duplicate and sequential concentrations (left to right): 0.39, 0.48, 1.6, 3.1, 6.2 nM.
FIG. 11 is a CZE electropherogram overlay of thermal stressed ianalumab (zoomed view). * Annotation according to mass spectrometry identification and relative migration time. L: light chain; HHL: heavy/heavy/light chain; HL: heavy/light chain; HMW: high molecular weight species.
FIG. 12 is a CE-SDS (non-reducing conditions) electropherogram overlay of thermal stressed ianalumab (zoomed view).
FIG. 13 is a CE-SDS (reducing conditions) electropherogram overlay of thermal stressed ianalumab (zoomed view). * Peak assignment based on average mass of corresponding N- or C-terminal fragments. L: light chain; NG: non-glycosylated heavy chain; H: heavy chain; NRV: non reducible variant; HL: heavy/light chain.
FIG. 14 is a SEC chromatogram overlay of thermal stressed ianalumab (zoomed view). AP: aggregation product; DP1: degradation product 1; DP2: degradation product 2.
FIG. 15 shows N-glycan profile by NP-HPLC of ianalumab DS batches. N-glycans were enzymatically cleaved from ianalumab using PNGase F, derivatized with 2-aminobenzamide (2-AB), and analyzed by normal phase liquid chromatography (NP-HPLC) with fluorescence detection.
FIG. 16 is a scatterplot of mannosylation and galactosylation levels of in vitro glycoengineered samples with labels of corresponding ADCC activity. Each dot in the plot represents a sample with varying levels of high-mannose and galactosylated N-glycans, generated by IVGE tools and mixed using a DoE approach. The X-axis shows the measured sum of high-mannose N-glycans, while the Y-axis shows the measured sum of galactosylated N-glycans. Numerical labels denote the measured ADCC activity for each sample.
FIG. 17 is a graph showing predicted vs. measured ADCC activity. ADCC activity (x-axis) for samples with varying levels of high-mannose and galactosylated N-glycans were measured using the validated ADCC bioassay employed for ianalumab release testing, plotted against predicted ADCC activity (y-axis) obtained from the ADCC-N-glycan correlation model. The model considers the sums of high-mannose and galactosylated N-glycans characterized in the samples' glycoprofile.
FIG. 18 is a histogram showing the frequency distribution for N-glycan galactosylation in ianalumab DS batches (n = 34). Acceptable manufacturing ranges (15.0 to 38.0%) are shown by dotted lines.
FIG. 19 is a histogram showing the frequency distribution for N-glycan mannosylation in ianalumab DS batches (n = 34). Acceptable manufacturing ranges (0 to 7.0%) are shown by dotted lines.
FIG. 20 is a schematic depiction of ianalumab. The antibody includes two light chains (SEQ ID NO: 2) and two heavy chains (SEQ ID NO: 1). The two heavy chains are covalently linked by intra- and inter-chain disulfide bridges. Each domain of the heavy and light chains contains one intra-chain disulfide bridge. Two disulfide bridges (C233-C233, C236-C236) in the hinge region connect the two heavy chains and C215 in the light chain and C227 in the heavy chain link the light and heavy chains. The heavy chain is N-glycosylated on the C_{H}2 domain.
FIG. 21 is a diagram showing the amino acid sequences of ianalumab. The complementarity-determining regions (CDRs) of the light and heavy chains are shown in bold. The disulfide bridges are shown as black lines. The cysteine residues that form the inter-chain disulfide bridges between the heavy chains are indicated by asterisks. NSR: the glycosylated motif is underlined and shown in bold and the glycosylation site N304 marked with as asterisk.

### DETAILED DESCRIPTION OF EMBODIMENTS OF THE INVENTION

Provided herein are compositions and methods related to anti-B cell activating factor receptor (BAFF-R) antibodies (e.g., ianalumab) having glycosylation profiles and variant compositions that can confer therapeutic benefit to patients, e.g., patients suffering BAFF-R-related disorders, such as autoimmune diseases or B cell neoplasms. The invention is based, in part, on an observed inverse correlation in afucosylated anti-BAFF-R antibody compositions between high mannose N-glycan levels and antibody dependent cellular cytotoxicity (ADCC) potency. The present invention is further based, in part, on the identification of variants present in anti-BAFF-R antibody compositions that can impact ADCC potency and other functions if present at levels exceeding thresholds identified herein. This observation can be applied to process development criteria to produce ADCC-potent anti-BAFF-R antibodies having low levels of high-mannose glycans and other variants. Accordingly, additionally provided herein are methods of manufacturing and testing such anti-BAFF-R antibodies and compositions thereof, as well as pharmaceutical compositions for use, and methods of using such compositions in treating various suffering BAFF-R-related disorders.

### I. Definitions

In order that the present invention may be more readily understood, certain terms are defined throughout the detailed description. Unless defined otherwise, all technical and scientific terms used herein have the same meaning as commonly understood by those of ordinary skill in the art to which this invention pertains. Unless stated otherwise, the following terms and phrases as used herein are intended to have the following meanings:
As used herein, "ianalumab" refers to an anti-BAFF-R antibody having the heavy chain amino acid sequence of SEQ ID NO: 1 and the light chain amino acid sequence of SEQ ID NO: 2. lanalumab is a human IgG₁κ monoclonal antibody designed to target human BAFF-R and to competitively inhibit binding of BAFF to BAFF-R, thereby blocking BAFF-R-mediated signaling in B cells. lanalumab was engineered to effectively eliminate B cells from circulation *in vivo* by ADCC. ADCC activity of ianalumab is greatly enhanced by elimination of fucose residues from the carbohydrate moiety attached to the Fc part of the antibody. Accordingly, ianalumab has shown potent ADCC activity *in vitro* with an EC₅₀ of 2.0 pM. Thus, ianalumab has been reported to eliminate BAFF-R+ mature and immature B cells via dual mechanisms: (1) antibody-dependent cytotoxicity (ADCC) and (2) induction of B cell apoptosis by blocking BAFF:BAFF-R interaction and downstream survival pathway in B cells. lanalumab may also be referred to as MOR6654B or VAY736.

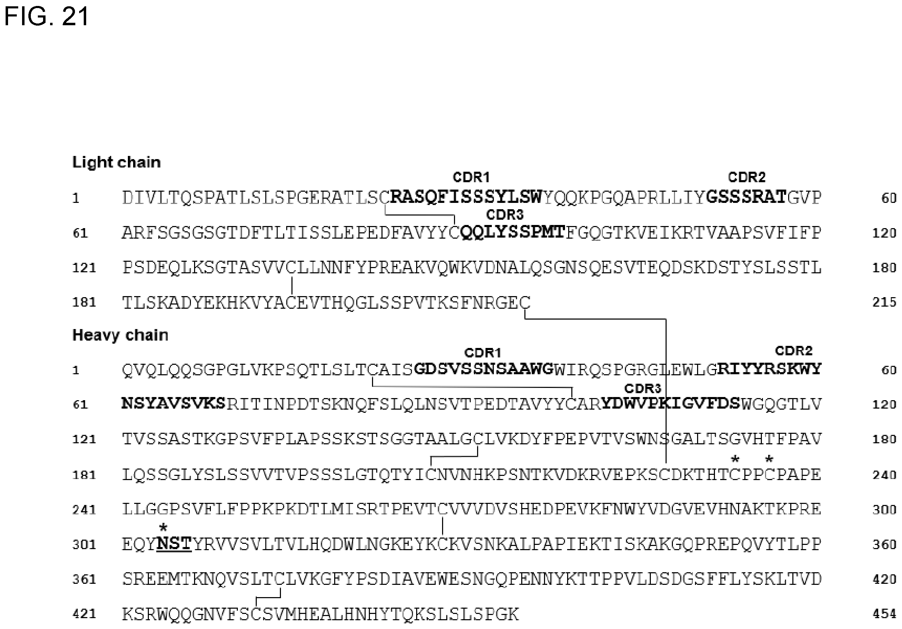

As used herein, reference to amino acid positions (also herein referred to as "residues") of ianalumab corresponds to the numbering of the amino acid positions are as labeled in FIG. 21.

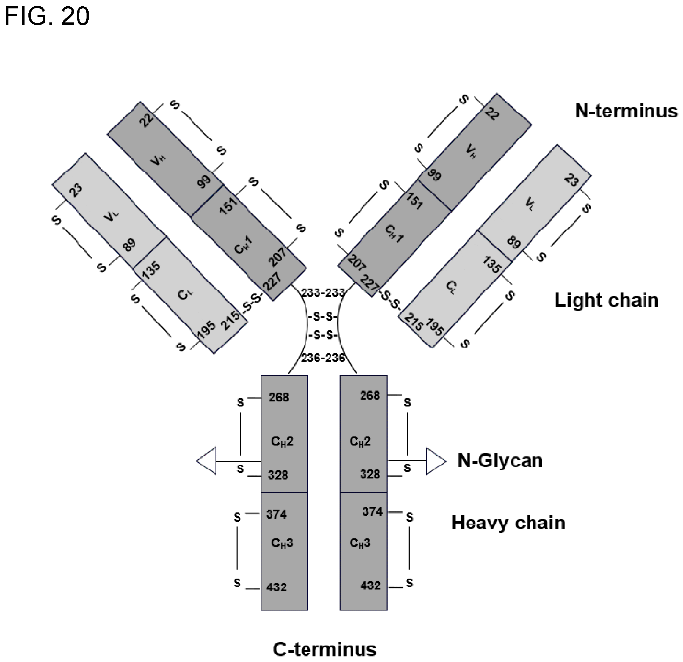

As used herein, the term "N-glycosylated Fc region" means a heavy chain of an antibody's Fc region having an attached N-linked glycan. In instances in which the Fc region is of an IgG antibody (e.g., an IgG₁ antibody, e.g., ianalumab), the N-linked residue can be Asn304 of the CH₂ domain of the Fc region (See FIG. 20).

Reference to a percentage of N-glycosylated Fc regions that have a certain glycoform characteristic (e.g., a percentage of N-glycosylated Fc regions that "comprise a high mannose glycan" or "are afucosylated") corresponds to the relative area of a peak corresponding to that glycoform in a mass spectrometry chromatogram (i.e., relative to the total area under all peaks corresponding to N-glycans in the chromatogram (Empower, Waters)) performed on a sample of antibody that has been enzymatically deglycosylated. As an example, N-glycans (oligosaccharides) present in a test antibody composition can be enzymatically removed from the antibody protein using peptide N-glycosidase F (PNGase F) treatment, followed by separation from the deglycosylated antibody using size exclusion cartridges. Released glycans can be labeled with an excess of 2-aminobenzamide (2-AB). After labeling, the excess of free 2-AB label from the derivatization reaction can be removed by gel filtration. The purified 2-AB labeled glycans can be analyzed on an Acquity UPLC BEH Glycan column (1.7 µm, 2.1 x 100 mm, Waters) by normal phase chromatography with fluorescence detection. Labeled glycans can be separated on a NP-column by applying a gradient from organic to aqueous solvent. A rapid resolution liquid chromatography system coupled to an Orbitrap Fusion Lumos ion trap mass spectrometer (Thermo Fisher) can be used for identification of the separated glycans. See, e.g., Example 8, herein.

As used herein, a "high mannose glycan," or grammatical derivations thereof, refers to any N-glycan containing five or more mannose residues (e.g., M5, M6, M7, or M8 (Oxford Notation)).

As used herein, an "afucosylated" N-glycan refers to an N-glycan that lacks a fucose residue (i.e., has zero fucose residues).

As used herein, a "galactosylated" N-glycan refers to an N-glycan that includes one or more galactose residues. As used herein, the percent "galactosylated" N-glycans is equivalent to the "sum of the percent of galactosylated" N glycans.

As used herein, the term "antibody-dependent cell-mediated cytotoxicity," "ADCC," "ADCC potency," or "ADCC activity," or grammatical derivatives thereof, refers to a form of cytotoxicity in which secreted Ig bound onto Fc receptors (FcRs) present on certain cytotoxic cells (e.g. Natural Killer (NK) cells, neutrophils, and macrophages) enable these cytotoxic effector cells to bind specifically to an antigen-bearing target cell and subsequently kill the target cell with cytotoxins. To assess ADCC activity of a molecule of interest, an ADCC assay, such as that described in herein may be performed. The ADCC activity of anti-BAFF-R antibody, such as ianalumab, is measured based on its ability to trigger ADCC in the presence of BAFF-R-expressing target cells and NK effector cells: Fluorescently labeled HEK-293 cells stably expressing the recombinant human BAFF-R are incubated with different concentrations of the anti-BAFF-R antibody and an excess of natural killer cells (NK3.3 cells). In this ADCC assay the NK3.3 cells serve as effector cells, whereas the labelled HEK-293 cells serve as target cells. Concentration-dependent killing of the HEK-293 target cells is analyzed after incubation (e.g. 1 h), by measuring the release of the fluorochrome from the lysed cells in each well.

Alternatively, ADCC activity of a molecule of interest can be assessed using an *in vitro* assay to monitor B cell depletion as a function of concentration of the molecule of interest. One method is to incubate a cell suspension of human PBMCs with ianalumab or comparator antibodies at various dilutions for 16-24 hours. The cells are then stained with labelled CD19 antibodies (or other antibodies used for detection) and analyzed on a FACS instrument. An alternative method is using purified human B cells (or Ri-1 B cell line) as target cells and purified human NK cells as effector cells. Fluorescently labeled B cells are preincubated with different concentrations of ianalumab or comparator antibodies, then co-cultured with an excess of primary NK cells. Concentration-dependent killing of target cells is analyzed after 1h incubation by measuring the release of the fluorochrome from the lysed cells in the supernatant. In the same system, the ability of ianalumab to activate human NK cells can be evaluated by measuring various inflammatory cytokines (e.g. IFNgamma) from the supernatant.

The relative ADCC activity of the anti-BAFF-R antibody test samples or product control is determined by comparison to a reference standard. The samples and the reference standard are normalized on the basis of protein content. Relative potency is then calculated using a parallel line assay according to the [European Pharmacopeia]. The final result is expressed as relative potency of a sample (in percent) compared to the reference standard.

Also, an ianalumab reference standard inhibited BAFF-induced human B cell proliferation with an IC₅₀ of 17.8 pM in such an *in vitro* assay using whole human blood.

As used herein, a "reference standard" refers to a composition of antibodies having at least 95% sequence identity (e.g., at least 99% sequency identity, or 100% sequence identity) to the antibody in the composition being tested but which was manufactured in a different batch than the composition being tested (e.g., prior to manufacture of the antibody composition being tested). The reference standard may therefore have a different glycosylation profile and/or functional (e.g., ADCC) potency than the antibody composition being tested. In some instances, the reference standard is ianalumab, e.g., ianalumab manufactured according to good manufacturing practices (GMP) standards and/or released with a certificate of analysis (CoA).

As used herein, a "commercial reference standard" refers to a reference standard that is commercially available at the time of assessment of the test antibody composition (e.g., an anti-BAFF-R antibody), e.g., a marketed antibody composition, e.g., for therapeutic use or for research use. For example, the commercial reference standard may be an ianalumab product that is commercially available, e.g. a composition comprising a human IgG₁κ anti-BAFF-R antibody having a heavy chain amino acid sequence of SEQ ID NO: 1 and a light chain amino acid sequence of SEQ ID NO: 2, and the composition has a specific antibody-dependent cellular cytotoxicity (ADCC) potency set as a 100% ADCC potency in a cell-based ADCC assay (e.g., an NK cell-based ADCC potency assay, e.g., as described herein). A reference standard (e.g., a commercial reference standard) may be an ianalumab product batch which has been manufactured and analyzed in accordance with GMP and released according to a Certificate of Analysis (CoA).

As used herein, the term "binding affinity" refers to dissociation equilibrium constant (K_{D}), which can be measured using surface plasmon resonance (SPR, e.g., using a Biacore system) or bio-layer interferometry (BLI, e.g., using Sartorius OCTET^{®} system). For BLI analysis, experimental data can be evaluated with global fitting using Octet Studio 12.0. software to calculate K_{D}.

The terms "disease" and "disorder" are used interchangeably to refer to a condition, in particular, a pathological condition. In certain embodiments, the terms "disease" and "disorder" are used interchangeably to refer to a disease affected by BAFF-R.

As used herein, the terms "treat", "treatment" and "treating" refer to the reduction or amelioration of the progression, severity and/or duration of a disorder, e.g., a proliferative disorder, or the amelioration of one or more symptoms (preferably, one or more discernible symptoms) of the disorder resulting from the administration of one or more therapies. In specific embodiments, the terms "treat," "treatment" and "treating" refer to the amelioration of at least one measurable physical parameter of a proliferative disorder, such as growth of a tumor, not necessarily discernible by the patient. In other embodiments the terms "treat", "treatment" and "treating" -refer to the inhibition of the progression of a proliferative disorder, either physically by, e.g., stabilization of a discernible symptom, physiologically by, e.g., stabilization of a physical parameter, or both. In other embodiments the terms "treat", "treatment" and "treating" refer to the reduction or stabilization of tumor size or cancerous cell count.

As used herein, the terms "therapies" and "therapy" can refer to any protocol(s), method(s), compositions, formulations, and/or agent(s) that can be used in the prevention, treatment, management, or amelioration of a disease, e.g., cancer, infectious disease, lymphopenia, and immunodeficiencies, or a symptom associated therewith. In certain embodiments, the terms "therapies" and "therapy" refer to biological therapy, supportive therapy, and/or other therapies useful in treatment, management, prevention, or amelioration of a disease or a symptom associated therewith known to one of skill in the art.

By "a combination" or "in combination with," it is not intended to imply that the therapy or the therapeutic agents must be administered at the same time and/or formulated for delivery together, although these methods of delivery are within the scope described herein. The therapeutic agents in the combination can be administered concurrently with, prior to, or subsequent to, one or more other additional therapies or therapeutic agents. The therapeutic agents or therapeutic protocol can be administered in any order. In general, each agent will be administered at a dose and/or on a time schedule determined for that agent. It will further be appreciated that the additional therapeutic agent utilized in this combination may be administered together in a single composition or administered separately in different compositions. In general, it is expected that additional therapeutic agents utilized in combination be utilized at levels that do not exceed the levels at which they are utilized individually. In some embodiments, the levels utilized in combination will be lower than those utilized individually. The term "anti-cancer effect" refers to a biological effect which can be manifested by various means, including but not limited to, e.g., a decrease in tumor volume, a decrease in the number of cancer cells, a decrease in the number of metastases, an increase in life expectancy, decrease in cancer cell proliferation, decrease in cancer cell survival, or amelioration of various physiological symptoms associated with the cancerous condition.

The term "cancer" refers to a disease characterized by the rapid and uncontrolled growth of aberrant cells. Cancer cells can spread locally or through the bloodstream and lymphatic system to other parts of the body. Examples of various cancers are described herein and include but are not limited to, breast cancer, prostate cancer, ovarian cancer, cervical cancer, skin cancer, pancreatic cancer, colorectal cancer, renal cancer, liver cancer, brain cancer, lymphoma, leukemia, lung cancer and the like. The terms "tumor" and "cancer" are used interchangeably herein, e.g., both terms encompass solid and liquid, e.g., diffuse or circulating, tumors. As used herein, the term "cancer" or "tumor" includes premalignant, as well as malignant cancers and tumors.

The compositions and methods of the present invention encompass polypeptides and nucleic acids having the sequences specified, or sequences substantially identical or similar thereto, e.g., sequences at least 85%, at least 90%, or at least 95% identical or higher to the sequence specified. In the context of an amino acid sequence, the term "substantially identical" is used herein to refer to a first amino acid that contains a sufficient or minimum number of amino acid residues that are i) identical to, or ii) conservative substitutions of aligned amino acid residues in a second amino acid sequence such that the first and second amino acid sequences can have a common structural domain and/or common functional activity. For example, amino acid sequences that contain a common structural domain having at least about 85%, at least about 90%, at least about 91%, at least about 92%, at least about 93%, at least about 94%, at least about 95%, at least about 96%, at least about 97%, at least about 98%, or at least about 99% identity (e.g., at least 85%, at least 90%, at least 91%, at least 92%, at least 93%, at least 94%, at least 95%, at least 96%, at least 97%, at least 98%, or at least 99% identity) to a reference sequence, e.g., a sequence provided herein.

In the context of nucleotide sequence, the term "substantially identical" is used herein to refer to a first nucleic acid sequence that contains a sufficient or minimum number of nucleotides that are identical to aligned nucleotides in a second nucleic acid sequence such that the first and second nucleotide sequences encode a polypeptide having common functional activity, or encode a common structural polypeptide domain or a common functional polypeptide activity. For example, nucleotide sequences having at least about 85%, at least about 90%, at least about 91%, at least about 92%, at least about 93%, at least about 94%, at least about 95%, at least about 96%, at least about 97%, at least about 98% or at least about 99% identity to a reference sequence, e.g., a sequence provided herein.

The term "functional variant" refers to polypeptides that have a substantially identical amino acid sequence to the naturally-occurring or wild type sequence, or are encoded by a substantially identical nucleotide sequence, and are capable of having one or more activities of the naturally-occurring or wild type sequence. Calculations of homology or sequence identity between sequences (the terms are used interchangeably herein) are performed as follows.

To determine the percent identity of two amino acid sequences, or of two nucleic acid sequences, the sequences are aligned for optimal comparison purposes (e.g., gaps can be introduced in one or both of a first and a second amino acid or nucleic acid sequence for optimal alignment and non-homologous sequences can be disregarded for comparison purposes). In a preferred embodiment, the length of a reference sequence aligned for comparison purposes is at least 70%, preferably at least 80%, more preferably at least 90%, 95%, and even more preferably at least 100% of the length of the reference sequence. The amino acid residues or nucleotides at corresponding amino acid positions or nucleotide positions are then compared. When a position in the first sequence is occupied by the same amino acid residue or nucleotide as the corresponding position in the second sequence, then the molecules are identical at that position (as used herein amino acid or nucleic acid "identity" is equivalent to amino acid or nucleic acid "homology").

The percent identity between the two sequences is a function of the number of identical positions shared by the sequences, taking into account the number of gaps, and the length of each gap, which need to be introduced for optimal alignment of the two sequences. The comparison of sequences and determination of percent identity between two sequences can be accomplished using a mathematical algorithm. In a preferred embodiment, the percent identity between two amino acid sequences is determined using the Needleman and Wunsch ((1970) J. Mol. Biol. 48:444-453 ) algorithm which has been incorporated into the GAP program in the GCG software package (available from the NCBI), using either a Blossum 62 matrix or a PAM250 matrix, and a gap weight of 16, 14, 12, 10, 8, 6, or 4 and a length weight of 1, 2, 3, 4, 5, or 6. In yet another preferred embodiment, the percent identity between two nucleotide sequences is determined using the GAP program in the GCG software package, using a NWSgapdna.CMP matrix and a gap weight of 40, 50, 60, 70, or 80 and a length weight of 1, 2, 3, 4, 5, or 6. A particularly preferred set of parameters (and the one that should be used unless otherwise specified) are a Blossum 62 scoring matrix with a gap penalty of 12, a gap extend penalty of 4, and a frameshift gap penalty of 5.

The percent identity between two amino acid or nucleotide sequences can be determined using the algorithm of E. Meyers and W. Miller ((1989) CABIOS, 4:11-17) which has been incorporated into the ALIGN program (version 2.0), using a PAM120 weight residue table, a gap length penalty of 12 and a gap penalty of 4.

The nucleic acid and protein sequences described herein can be used as a "query sequence" to perform a search against public databases to, for example, identify other family members or related sequences. Such searches can be performed using the NBLAST and XBLAST programs (version 2.0) of Altschul, et al. (1990) J. Mol. Biol. 215:403-10. BLAST nucleotide searches can be performed with the NBLAST program, score = 100, word length = 12 to obtain nucleotide sequences homologous to a nucleic acid molecules of the invention. BLAST protein searches can be performed with the XBLAST program, score = 50, word length = 3 to obtain amino acid sequences homologous to protein molecules of the invention. To obtain gapped alignments for comparison purposes, Gapped BLAST can be utilized as described in Altschul et al., (1997) Nucleic Acids Res. 25:3389-3402. When utilizing BLAST and Gapped BLAST programs, the default parameters of the respective programs (e.g., XBLAST and NBLAST) can be used (available from the NBCI).

It is understood that the molecules of the present invention may have additional conservative or non-essential amino acid substitutions, which do not have a substantial effect on their functions.

The term "amino acid" is intended to embrace all molecules, whether natural or synthetic, which include both an amino functionality and an acid functionality and capable of being included in a polymer of naturally-occurring amino acids. Exemplary amino acids include naturally-occurring amino acids; analogs, derivatives and congeners thereof; amino acid analogs having variant side chains; and all stereoisomers of any of any of the foregoing. As used herein the term "amino acid" includes both the D- or L- optical isomers and peptidomimetics.

A "conservative amino acid substitution" is one in which the amino acid residue is replaced with an amino acid residue having a similar side chain. Families of amino acid residues having similar side chains have been defined in the art. These families include amino acids with basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine), nonpolar side chains (e.g., alanine, valine, leucine, isoleucine, proline, phenylalanine, methionine, tryptophan), beta-branched side chains (e.g., threonine, valine, isoleucine) and aromatic side chains (e.g., tyrosine, phenylalanine, tryptophan, histidine).

The terms "polypeptide", "peptide" and "protein" (if single chain) are used interchangeably herein to refer to polymers of amino acids of any length. The polymer may be linear or branched, it may comprise modified amino acids, and it may be interrupted by non-amino acids. The terms also encompass an amino acid polymer that has been modified; for example, disulfide bond formation, glycosylation, lipidation, acetylation, phosphorylation, or any other manipulation, such as conjugation with a labeling component. The polypeptide can be isolated from natural sources, can be a produced by recombinant techniques from a eukaryotic or prokaryotic host, or can be a product of synthetic procedures.

The terms "nucleic acid," "nucleic acid sequence," "nucleotide sequence," or "polynucleotide sequence," and "polynucleotide" are used interchangeably. They refer to a polymeric form of nucleotides of any length, either deoxyribonucleotides or ribonucleotides, or analogs thereof. The polynucleotide may be either single-stranded or double-stranded, and if single-stranded may be the coding strand or noncoding (antisense) strand. A polynucleotide may comprise modified nucleotides, such as methylated nucleotides and nucleotide analogs. The sequence of nucleotides may be interrupted by non-nucleotide components. A polynucleotide may be further modified after polymerization, such as by conjugation with a labeling component. The nucleic acid may be a recombinant polynucleotide, or a polynucleotide of genomic, cDNA, semisynthetic, or synthetic origin which either does not occur in nature or is linked to another polynucleotide in a nonnatural arrangement.

The term "isolated," as used herein, refers to material that is removed from its original or native environment (e.g., the natural environment if it is naturally occurring). For example, a naturally-occurring polynucleotide or polypeptide present in a living animal is not isolated, but the same polynucleotide or polypeptide, separated by human intervention from some or all of the co-existing materials in the natural system, is isolated. Such polynucleotides could be part of a vector and/or such polynucleotides or polypeptides could be part of a composition and still be isolated in that such vector or composition is not part of the environment in which it is found in nature.

As used herein, the term "glycan" is a sugar, which can be monomers or polymers of sugar residues, such as at least three sugars, and can be linear or branched (e.g., have an α 1,3 arm and an α 1,6 arm). A "glycan" can include natural sugar residues (e.g., glucose, N-acetylglucosamine, N-acetyl neuraminic acid, galactose, mannose, fucose, hexose, arabinose, ribose, xylose, etc.) and/or modified sugars (e.g., 2'-fluororibose, 2'-deoxyribose, phosphomannose, 6'sulfo N-acetylglucosamine, etc.). The term "glycan" includes homo and heteropolymers of sugar residues. The term "glycan" also encompasses a glycan component of a glycoconjugate (e.g., of a glycoprotein, glycolipid, proteoglycan, etc.). The term also encompasses free glycans, including glycans that have been cleaved or otherwise released from a glycoconjugate.

As used herein, the term "glycoprotein" refers to a protein that contains a peptide backbone covalently linked to one or more sugar moieties (i.e., glycans). The sugar moiety(ies) may be in the form of monosaccharides, disaccharides, oligosaccharides, and/or polysaccharides. The sugar moiety(ies) may comprise a single unbranched chain of sugar residues or may comprise one or more branched chains. Glycoproteins can contain O-linked sugar moieties and/or N-linked sugar moieties (N-glycans). The polysaccharide is attached either via the OH group of serine or threonine (O-glycosylated polypeptide) or via the amide group (NH₂) of asparagine (N-glycosylated polypeptide). The glycoprotein may be homologous to the host cell or preferably heterologous to the host cell expressing it, e.g., foreign, e.g., a human protein produced by CHO cells.

The term "glycoconjugate," as used herein, encompasses all molecules in which at least one sugar moiety is covalently linked to at least one other moiety. The term specifically encompasses all biomolecules with covalently attached sugar moieties, including for example N-linked glycoproteins, O-linked glycoproteins, glycolipids, proteoglycans, etc.

The term "glycosylation" refers to the attachment of a polysaccharide to a polypeptide. Preferably, the polysaccharide consists of 2-12 monosaccharides linked together by glycosidic bonds. Glycoproteins can contain O-linked sugar moieties and/or N-linked sugar moieties. The structure and number of sugar moieties attached to a particular glycosylation site can be variable. Such sugar moieties may be, for instance, N-acetyl glucosamine, N-acetyl galactosamine, mannose, galactose, glucose, fucose, xylose, glucuronic acid, iduronic acid and/or sialic acids.

The term "N-linked glycosylation" refers to the attachment of a polysaccharide to an asparagine residue of an amino acid chain.

An "intact antibody" herein is one which comprises two antigen binding regions, and an Fc region. Preferably, the intact antibody has a functional Fc region. In some embodiments, the "intact antibody" is "intact ianalumab". In one embodiment, "intact ianalumab", primary monomeric form of ianalumab, has a molecular weight of about 148,871 Da measuring its peptide chains only. The relative abundance of integral heavy and light chains is reported as a percentage of purity.

The term "main variant antibody" or "wild type antibody" herein refers to the antibody amino acid sequence structure in a composition which is the quantitatively predominant antibody molecule in the composition. Preferably, the main variant antibody is an anti-BAFF-R. In one embodiment, the main variant antibody is one comprising: (a) CDR-H1, CDR-H2, and CDR-H3 having the amino acid sequences of SEQ ID NO: 5, SEQ ID NO: 6, and SEQ ID NO: 7, and CDR-L1, CDR-L2, and CDR-L3 having the amino acid sequences of SEQ ID NO: 8, SEQ ID NO: 9, and SEQ ID NO: 10, respectively; (b) a VH having the amino acid sequence of SEQ ID NO: 3 and a VL having the amino acid sequence of SEQ ID NO: 4; and/or optionally, (c) a heavy chain amino acid sequence of SEQ ID NO: 1 and a light chain amino acid sequence of SEQ ID NO: 2. In one embodiment, the main variant antibody is ianalumab.

Variants described herein can be identified according to any of the methods described herein (e.g., in Examples 5 and 6). The phrase "[X]% of the total anti-BAFF-R antibodies and fragments thereof in the composition" refers to the amount of [X variant] as a percentage of the total amount of sum of anti-BAFF-R antibodies and any fragments of the anti-BAFF-R antibodies that may be in the composition. The analytical methods suitable for the identification and quantification of the anti-BAFF-R antibodies variants described herein are:
- Aggregates/ high molecular weight variants (HMWs): Appearance (Turbidity), SEC, CE-SDS (red.), CE-SDS (nonred.), SEC-MALLS, AUC, DLS;
- Deamidated variants: CZE, Peptide mapping MS;
- Tryptophan-oxidized variant: Peptide mapping MS;
- Methionine-oxidized variant: Peptide mapping MS;
- Clipped variant: CZE, Peptide mapping MS;
- N-glycan site occupancy: CE-SDS (red.),Peptide mapping MS, RP-HPLC-MS; and
- N-glycan galactosylation: N-glycan profiling, Peptide mapping MS.

An "amino acid sequence variant" antibody herein is an antibody with an amino acid sequence which differs from a main variant antibody. Ordinarily, amino acid sequence variants will possess at least about 70% homology with the main variant antibody, and preferably, they will be at least about 80%, and more preferably at least about 90% homologous with the main variant antibody. The amino acid sequence variants possess substitutions, deletions, and/or additions at certain positions within or adjacent to the amino acid sequence of the main variant antibody.

An "acidic variant" is a variant of the main variant antibody which is more acidic than the main variant antibody. An acidic variant has gained negative charge or lost positive charge relative to the main variant antibody. Such acidic variants can be resolved using a separation methodology, such as ion exchange chromatography, that separates proteins according to charge. Acidic variants of a main variant antibody elute earlier than the main peak upon separation by cation exchange chromatography.

A "basic variant" is a variant of the main variant antibody which is more basic than the main variant antibody. A basic variant has gained positive charge or lost negative charge relative to the main variant antibody. Such basic variants can be resolved using a separation methodology, such as ion exchange chromatography, that separates proteins according to charge. Basic variants of a main variant antibody elute later than the main peak upon separation by cation exchange chromatography.

A "clipped variant" is a variant of the main variant antibody that contain one or more cleavages of the heavy or the light chain of the antibody, such as ianalumab. Depending on the position of the clip in the amino acid sequence, these variants have the same or lower molecular weight than the main variant or intact ianalumab (e.g. where the intact ianalumab has a molecular weight of about 148,871 Da measuring its peptide chains only). Clipped variants having the same molecular weight as the main variant are herein considered to have the same amino acid sequences as the main variant, e.g., clipping in the variable heavy chain that does not produce a fragment (i.e., affect the molecular weight of the antibody) does not affect the heavy chain amino acid sequence relative to the main variant without such clipping. For example, a variant of ianalumab that includes clipping between R56 and S57 in the CDR2 loop of the heavy chain herein comprises a heavy chain amino acid sequence of SEQ ID NO: 1 and a light chain amino acid sequence of SEQ ID NO: 2. Such variants can be assessed by treating the composition with a reducing agent and evaluating the resulting composition using a methodology that evaluates protein size, such as Capillary Electrophoresis with Sodium Dodecyl Sulfate (CE-SDS), or CEZ, or peptide mapping MS.

A "high-molecular-weight-variant" or "HMW" comprises a preparation of ianalumab having a molecular weight that is greater than the main variant or intact ianalumab (e.g. where the intact ianalumab has a molecular weight of about 148,871 Da measuring its peptide chains only) or a preparation comprising two or more molecules of ianalumab that are covalently or non-covalently bound to each other. The HMW can be detected by non-reduced Capillary Electrophoresis with Sodium Dodecyl Sulfate (CE-SDS) assay; for example, as in Example 6.

A "low-molecular-weight-variant" or "LMW" comprises a preparation of ianalumab having a molecular weight that is less than the main variant or intact ianalumab (e.g. where the intact ianalumab has a molecular weight of about 148,871 Da measuring its peptide chains only). The LMW can be detected by non-reduced Capillary Electrophoresis with Sodium Dodecyl Sulfate (CE-SDS) assay; for example, as in Example 6.

A "deamidated variant" antibody is one in which one or more asparagine residues thereof has been derivatized, e.g. to an aspartic acid, a succinimide, or an iso-aspartic acid. An example of a deamidated antibody is an ianalumab variant, wherein N332 of ianalumab is deamidated. As used herein, a deamidated variant in which an asparagine residue is deamidated into a different chemical species or residue (e.g., aspartic acid), said deamidated variant is defined herein as having the amino acid sequences of its original form, e.g., the main variant. The deamidated variant can be detected by non-reduced Capillary Zone Electrophoresis (CZE) assay; or by Peptide mapping MS, for example, as in Example 6.

A "tryptophan oxidized variant" antibody is one in which one or more tryptophan residues thereof has been oxidized. An example of a tryptophan oxidized variant is an ianalumab tryptophan oxidized variant, wherein HC-W59 and/or HC-W104 of ianalumab are oxidized. As used herein, a tryptophan oxidized variant in which a tryptophan residue is oxidized to become a different chemical species, said tryptophan oxidized variant is defined herein as having the amino acid sequences of its original form, e.g., the main variant. The tryptophan oxidized variant can be detected by exemplary assays known in the art, e.g. Mass-Spec assay/ peptide mapping MS; for example, as in Example 6.

A "methionine oxidized variant" antibody is one in which one or more methionine residues thereof has been oxidized. An example of a methionine oxidized variant is an ianalumab oxidized variant, wherein M97 of the light chain, M259 of the heavy chain, M365 of the heavy chain and/or M435 of the heavy chain of ianalumab are oxidized. As used herein, a methionine oxidized variant in which a methionine residue is oxidized to become a different chemical species, said methionine oxidized variant is defined herein as having the amino acid sequences of its original form, e.g., the main variant. The methionine oxidized variant can be detected by exemplary assays known in the art, e.g. Mass-Spec assay / peptide mapping MS; for example, as in Example 6.

As used herein, the articles "a" and "an" refer to one or to more than one (e.g., to at least one) of the grammatical object of the article.

The term "or" is used herein to mean, and is used interchangeably with, the term "and/or", unless context clearly indicates otherwise.

The term "about" in relation to a numerical value x means x ± 10%, unless the context dictates otherwise.

Ranges of values stated as "up to [X]," e.g., (up to X%) herein include all values from zero up to and including X. In embodiments "comprising" a variant "accounting for up to [X]%," the variant is present in a detectable amount (e.g., using the methods described herein) and no more than X%.

Ranges of values stated as "[X] to [Y]" or "from [X] to [Y]" herein include X, Y, and all values therebetween.

### II. Anti-BAFF-R Antibodies

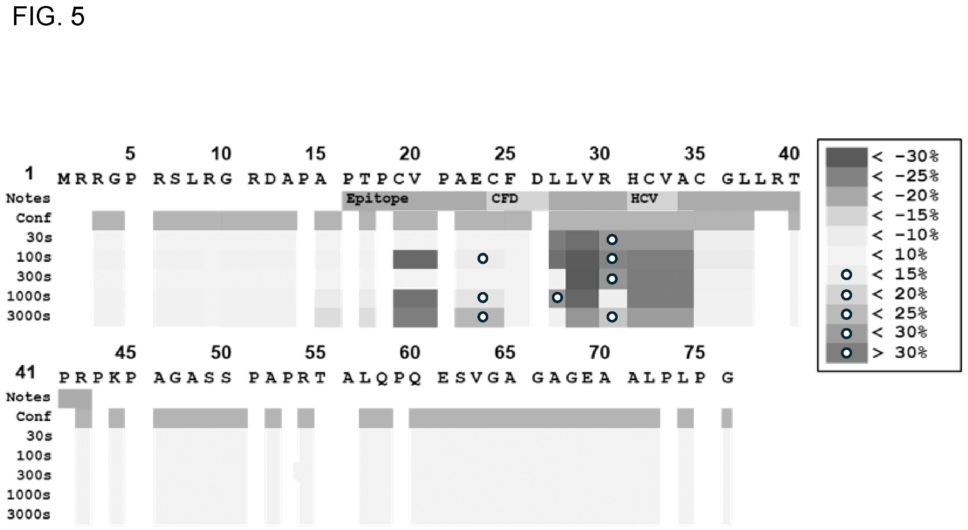

The invention relates to anti-B cell activating factor receptor (BAFF-R) antibodies, and compositions thereof, having structural features that can provide therapeutic benefits. In some instances, the anti-BAFF-R antibodies provided herein are monoclonal anti-BAFF-R antibodies, e.g., monoclonal anti-BAFF-R antibodies that target the ligand-binding domain of BAFF-R. In certain embodiments of the invention, the anti-BAFF-R antibody (e.g., monoclonal anti-BAFF-R antibody) binds to an epitope of BAFF-R having one or more residues of D26, L27, L28, V29, R30, H31, C32, V33, A34, and C35 of SEQ ID NO: 11 (FIG. 5), e.g., one or more contiguous residues of D26, L27, L28, V29, R30, H31, C32, V33, A34, and C35 of SEQ ID NO: 11 (e.g., two or more, three or more, four or more, five or more, six or more, seven or more, eight or more, nine, or all ten contiguous residues of D26, L27, L28, V29, R30, H31, C32, V33, A34, and C35 of SEQ ID NO: 11). Binding such epitopes can lead to inhibition of B cell proliferation through BAFF:BAFF-R signaling.

BAFF-R: SEQ ID NO: 11:
MRRGPRSLRGRDAPAPTPCVPAECFDLLVRHCVACGLLRTPRPKPAGASSPAPRTALQPQESVG
AGAGEAALPLPG

In some embodiments, the paratope of an anti-BAFF-R antibody (e.g., a paratope capable of binding the aforementioned BAFF-R epitopes) includes one or more heavy chain residue numbers 32, 33, 35, 52, 54, 56, 57, 60, 102, 103, 104, and/or 110 (e.g., one or more heavy chain residues N32, S33, A34, A35, R52, Y54, R56, S57, Y60, Y102, D103, W104, and/or V110) and/or one or more light chain residue numbers 92, 93, and/or 94 (e.g., L92, Y93, and/or S94).

An example of an anti-BAFF-R antibody contemplated as part of the present invention is ianalumab, a human IgG₁κ anti-BAFF-R antibody having a heavy chain amino acid sequence of SEQ ID NO: 1 and a light chain amino acid sequence of SEQ ID NO: 2. lanalumab has a heavy chain variable domain amino acid sequence of SEQ ID NO: 3 and a light chain variable domain amino acid sequence of SEQ ID NO: 4.
lanalumab Heavy chain: SEQ ID NO: 1: 
QVQLQQSGPGLVKPSQTLSLTCAISGDSVSSNSAAWGWIRQSPGRGLEWLGRIYYRSKWYNSY
AVSVKSRITINPDTSKNQFSLQLNSVTPEDTAVYYCARYDWVPKIGVFDSWGQGTLVTVSSASTK
GPSVFPLAPSSKSTSGGTAALGCLVKDYFPEPVTVSWNSGALTSGVHTFPAVLQSSGLYSLSSVV
TVPSSSLGTQTYICNVNHKPSNTKVDKRVEPKSCDKTHTCPPCPAPELLGGPSVFLFPPKPKDTL
MISRTPEVTCVVVDVSHEDPEVKFNWYVDGVEVHNAKTKPREEQYNSTYRVVSVLTVLHQDWL
NGKEYKCKVSNKALPAPIEKTISKAKGQPREPQVYTLPPSREEMTKNQVSLTCLVKGFYPSDIAVE
WESNGQPENNYKTTPPVLDSDGSFFLYSKLTVDKSRWQQGNVFSCSVMHEALHNHYTQKSLSL
SPGK
lanalumab Light chain: SEQ ID NO: 2: 
DIVLTQSPATLSLSPGERATLSCRASQFISSSYLSWYQQKPGQAPRLLIYGSSSRATGVPARFSGS
GSGTDFTLTISSLEPEDFAVYYCQQLYSSPMTFGQGTKVEIKRTVAAPSVFIFPPSDEQLKSGTAS
VVCLLNNFYPREAKVQWKVDNALQSGNSQESVTEQDSKDSTYSLSSTLTLSKADYEKHKVYACE
VTHQGLSSPVTKSFNRGEC
lanalumab Variable Domain of Heavy Chain (VH): SEQ ID NO: 3 
QVQLQQSGPGLVKPSQTLSLTCAISGDSVSSNSAAWGWIRQSPGRGLEWLGRIYYRSKWYNSY
AVSVKSRITINPDTSKNQFSLQLNSVTPEDTAVYYCARYDWVPKIGVFDSWGQGTLVTVSS
lanalumab Variable Domain of Light Chain (VL): SEQ ID NO: 4 
DIVLTQSPATLSLSPGERATLSCRASQFISSSYLSWYQQKPGQAPRLLIYGSSSRATGVPARFSGS
GSGTDFTLTISSLEPEDFAVYYCQQLYSSPMTFGQGTKVEIKRT
lanalumab comprises the following Complementarity Determining Regions (CDRs):
**Heavy Chain:**

| | | |
|---|---|---|
| HCDR1: | GDSVSSNSAAWG | SEQ ID NO: 5 |
| HCDR2: | RIYYRSKWYNSYAVSVKS | SEQ ID NO: 6 |
| HCDR3: | YDWVPKIGVFDS | SEQ ID NO: 7 |

**Light Chain:**

| | | |
|---|---|---|
| LCDR1: | RASQFISSSYLS | SEQ ID NO: 8 |
| LCDR2: | GSSSRAT | SEQ ID NO: 9 |
| LCDR3: | QQLYSSPMT | SEQ ID NO: 10 |

The CDRs of ianalumab are also shown within the heavy and light chain amino acid sequences in FIG. 21. Additional structural features contemplated as part of the anti-BAFF-R antibodies of the invention, such as disulfide bond positions, are shown in FIGS. 35 and 36.

Other examples of heavy and light chain amino acid sequences of antibodies are those encoded by corresponding DNA sequences contained in plasmid pBW510 as deposited by Novartis Pharma AG, Forum 1, CH-4002 Basel, Switzerland, at DSMZ on Apr. 29, 2009 with accession number DSM22542.

Other anti-BAFFR antibodies that can be used for preparing the pharmaceutical compositions of the invention include anti-BAFFR antibodies, with amino acids that have been changed (e.g., mutated) by amino acid deletion, insertion or substitution, yet have no more than 1, 2, 3, 4 or 5 amino acid deletion, insertion or substitution in either the heavy or light chain regions described above. In a specific embodiment, such amino acid changes appear only within the framework and/or constant regions and the CDR regions are 100% identical to the heavy chain CDR1, CDR2 and CDR3 regions of SEQ ID NO: 5, 6 and 7 and to the light chain CDR1, CDR2 and CDR3 regions of SEQ ID NO: 8, 9, and 10 respectively. In one more specific embodiment, the changes that have been made are only conservative amino acid substitutions outside of the CDR regions.

In some instances, the anti-BAFF-R antibodies of the invention (e.g., monoclonal anti-BAFF-R antibodies) include a VH having at least 95% (e.g., at least 96%, at least 97%, at least 98%, at least 99%, or 100%) identity to the amino acid sequence of SEQ ID NO: 3 and/or a VL having at least 95% (e.g., at least 96%, at least 97%, at least 98%, at least 99%, or 100%) identity to the amino acid sequence of SEQ ID NO: 4. In some instances, the anti-BAFF-R antibodies of the invention (e.g., monoclonal anti-BAFF-R antibodies) include a VH having the amino acid sequence of SEQ ID NO: 3 and a VL having the amino acid sequence of SEQ ID NO: 4.

In some instances, the anti-BAFF-R antibodies of the invention (e.g., monoclonal anti-BAFF-R antibodies) include a heavy chain having at least 95% (e.g., at least 96%, at least 97%, at least 98%, at least 99%, or 100%) identity to the amino acid sequence of SEQ ID NO: 1 and/or a light chain having at least 95% (e.g., at least 96%, at least 97%, at least 98%, at least 99%, or 100%) identity to the amino acid sequence of SEQ ID NO: 2. In some instances, the anti-BAFF-R antibodies of the invention (e.g., monoclonal anti-BAFF-R antibodies) include a heavy chain having the amino acid sequence of SEQ ID NO: 1 and a light chain having the amino acid sequence of SEQ ID NO: 2.

Conservative amino acid substitutions are ones in which the amino acid residue is replaced with an amino acid residue having a similar side chain. Families of amino acid residues having similar side chains have been defined in the art. These families include amino acids with basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine, tryptophan), nonpolar side chains (e.g., alanine, valine, leucine, isoleucine, proline, phenylalanine, methionine), beta-branched side chains (e.g., threonine, valine, isoleucine) and aromatic side chains (e.g., tyrosine, phenylalanine, tryptophan, histidine). Thus, one or more amino acid residues outside of the CDR regions of an anti-BAFFR antibody, can be replaced with other amino acid residues from the same side chain family, and the altered antibody can be tested for retained function, in particular the same binding properties to BAFFR.

In some embodiments, the monoclonal anti-BAFF-R antibodies of the composition (e.g., monoclonal anti-BAFF-R IgG₁ antibodies, e.g., monoclonal anti-BAFF-R IgG₁κ antibodies) have a CDR-H1, a CDR-H2, and a CDR-H3 having the amino acid sequences of SEQ ID NO: 5, SEQ ID NO: 6, and SEQ ID NO: 7, and/or a CDR-L1, a CDR-L2, and a CDR-L3 having the amino acid sequences of SEQ ID NO: 8, SEQ ID NO: 9, and SEQ ID NO: 10, respectively. In some embodiments, the monoclonal anti-BAFF-R antibodies of the composition (e.g., the monoclonal anti-BAFF-R antibodies of any of the preceding embodiments, e.g., wherein the monoclonal anti-BAFF-R antibody is an afucosylated IgG) have a CDR-H1, a CDR-H2, and a CDR-H3 having the amino acid sequences of SEQ ID NO: 5, SEQ ID NO: 6, and SEQ ID NO: 7, and a CDR-L1, a CDR-L2, and a CDR-L3 having the amino acid sequences of SEQ ID NO: 8, SEQ ID NO: 9, and SEQ ID NO: 10, respectively. In some embodiments, the monoclonal anti-BAFF-R antibodies of the composition (e.g., the monoclonal anti-BAFF-R antibodies of any of the preceding embodiments, e.g., wherein the monoclonal anti-BAFF-R antibody is an afucosylated IgG1) that bind to a BAFF-R epitope comprising one or more of residues D26, L27, L28, V29, R30, H31, C32, V33, A34, and C35; and/or have one or more anti-BAFF-R paratopic residues comprising one or more of heavy chain residues N32, S33, A35, R52, Y54, R56, S57, Y60, Y102, D103, W104, V110 and/or one or more of light chain residues L92, Y93, and S94.

In certain embodiments, any of such anti-BAFF-R antibodies exhibit therapeutic potency, at least in part, by ADCC function. Such antibodies can include an N-glycosylated Fc region suitable to achieve ADCC, e.g., through Fc receptor binding (e.g., FcyRIlla binding). Accordingly, anti-BAFF-R antibodies (e.g., monoclonal anti-BAFF-R antibodies) described herein include IgG antibodies, e.g., IgG₁ antibodies, e.g., IgG₁κ antibodies. In some embodiments, the anti-BAFF-R antibody of the invention (e.g., the monoclonal anti-BAFF-R IgG₁ antibody, e.g., the monoclonal anti-BAFF-R IgG₁κ antibody) is afucosylated (i.e., lacks a fucose residue).

Compositions of anti-BAFF antibodies provided herein include compositions in which at least 95% of the anti-BAFF antibodies (e.g., monoclonal anti-BAFF-R IgG₁ antibodies, e.g., monoclonal anti-BAFF-R IgG₁κ antibodies) are afucosylated. In some embodiments, compositions of anti-BAFF antibodies include compositions in which at least 96%, at least 97%, at least 98%, at least 99%, or about 100% of the anti-BAFF antibodies (e.g., monoclonal anti-BAFF-R IgG₁ antibodies, e.g., monoclonal anti-BAFF-R IgG₁κ antibodies) are afucosylated. In some embodiments, compositions of anti-BAFF antibodies include compositions in which about 99% or more of the anti-BAFF antibodies (e.g., monoclonal anti-BAFF-R IgG₁ antibodies, e.g., monoclonal anti-BAFF-R IgG₁κ antibodies) are afucosylated.

In some embodiments, at least 95% of N-glycosylated Fc regions of the anti-BAFF antibodies (e.g., monoclonal anti-BAFF-R IgG₁ antibodies, e.g., monoclonal anti-BAFF-R IgG₁κ antibodies) are afucosylated. In some embodiments, at least 96%, at least 97%, at least 98%, at least 99%, or about 100% of N-glycosylated Fc regions of the anti-BAFF antibodies (e.g., monoclonal anti-BAFF-R IgG₁ antibodies, e.g., monoclonal anti-BAFF-R IgG₁κ antibodies) are afucosylated. In some embodiments, about 99% or more of N-glycosylated Fc regions of the anti-BAFF antibodies (e.g., monoclonal anti-BAFF-R IgG₁ antibodies, e.g., monoclonal anti-BAFF-R IgG₁κ antibodies) are afucosylated (e.g., at least 99.0%, at least 99.1%, at least 99.2%, at least 99.3%, at least 99.4%, at least 99.5%, at least 99.6%, at least 99.7%, at least 99.8%, or at least 99.9% afucosylated).

The present invention is based, in part, on the discovery that compositions of afucosylated anti-BAFF-R antibodies (e.g., monoclonal anti-BAFF-R IgG₁ antibodies, e.g., monoclonal anti-BAFF-R IgG₁κ antibodies) exhibit suitable ADCC potency when levels of high mannose glycoforms and galactosylated glycoforms satisfy certain conditions that were empirically revealed in a series of *in-vitro* glycoengineering (IVGE) studies described in Example 8. Suitable ADCC potency refers to the ADCC potency of a functionally characterized reference batch of ianalumab drug substance. These conditions can be expressed through Formula I, below: $ADCC = 86.83 - \left(0.819∗sum of high mannose\right) + \left(1.051∗sum of galactosylated\right)$ Using Formula I, an ADCC potency relative to a reference ianalumab (relative ADCC) of a composition of afucosylated anti-BAFF antibodies having a given percentage of high mannose glycoforms can be predicted based on the percentage of galactosylated glycoforms. Table II-a provides the percentage of galactosylated glycoform in a composition of afucosylated anti-BAFF-R IgG₁ antibodies that is predicted to achieve 100% relative ADCC.

**Table II-a. Predicted percent galactosylation (%Gal) for 100% relative ADCC based on percent high mannose (%HM)**

| **% HM** | **% Gal** | **% HM** | **% Gal** |
|---|---|---|---|
| 0 | 12.5 | 11 | 21.1 |
| 1 | 13.3 | 12 | 21.9 |
| 2 | 14.1 | 13 | 22.7 |
| 3 | 14.9 | 14 | 23.4 |
| 4 | 15.6 | 15 | 24.2 |
| 5 | 16.4 | 16 | 25.0 |
| 6 | 17.2 | 17 | 25.8 |
| 7 | 18.0 | 18 | 26.6 |
| 8 | 18.8 | 19 | 27.3 |
| 9 | 19.5 | 20 | 28.1 |
| 10 | 20.3 | 21 | 28.9 |

Based on the data-driven model, Table II-a shows that an afucosylated composition of afucosylated anti-BAFF-R IgG₁ antibodies having, e.g., 7% high mannose is predicted by Formula I to achieve the same ADCC potency as an ianalumab reference standard if 18% of its N-glycosylated Fc regions are galactosylated. An analogous conclusion can be drawn from each pair of values in Table II-a, each of which characterizes an antibody composition part of the present invention.

Additionally, Formula I allows calculation of a target range of galactosylation values around the ianalumab reference standard. Table II-b provides the target range of percentage of galactosylated glycoforms in a composition of afucosylated anti-BAFF-R IgG₁ antibodies that is predicted to achieve 70% to 130% relative ADCC (i.e., +/- 30% variance around the ianalumab reference).

**Table II-b. Predicted %Gal for 70%-130% relative ADCC based on percent %HM**

| **% HM** | **% Gal** | **% HM** | **% Gal** |
|---|---|---|---|
| 0 | 0 - 41.1 | 11 | 0 - 49.6 |
| 1 | 0 - 41.9 | 12 | 0 - 50.4 |
| 2 | 0 - 42.6 | 13 | 0 - 51.2 |
| 3 | 0 - 43.4 | 14 | 0 - 52.0 |
| 4 | 0 - 44.2 | 15 | 0 - 52.8 |
| 5 | 0 - 45.0 | 16 | 0 - 53.5 |
| 6 | 0 - 45.8 | 17 | 0 - 54.3 |
| 7 | 0 - 46.5 | 18 | 0 - 55.1 |
| 8 | 0 - 47.3 | 19 | 0 - 55.9 |
| 9 | 0 - 48.1 | 20 | 0 - 56.7 |
| 10 | 0 - 48.9 | 21 | 0.3 - 57.4 |

Thus, in some instances, about 10% or less (e.g., about 10%) of the N-glycosylated Fc regions in an anti-BAFF-R antibody composition have a high mannose glycan, and about 49% or less of the N-glycosylated Fc regions in the anti-BAFF-R antibody composition are galactosylated (e.g., about 45.0% or less, about 44.2% or less, about 43.4% or less, about 42.6% or less, about 41.9% or less, or about 41.1% or less; e.g., from 10% to 40%, from 15% to 30%, or from 20% to 25%; e.g., about 20%, about 21%, about 22%, about 23%, about 24%, or about 25% of the N-glycosylated Fc regions in the anti-BAFF-R antibody composition are galactosylated). In some instances, according to Formula I, any of the directly aforementioned antibody compositions have a relative ADCC potency from about 70% to about 130% compared to an ianalumab reference standard (e.g., a commercial ianalumab product).

In some instances, about 8% or less (e.g., about 6%) of the N-glycosylated Fc regions in an anti-BAFF-R antibody composition have a high mannose glycan, and about 47% or less of the N-glycosylated Fc regions in the anti-BAFF-R antibody composition are galactosylated (e.g., about 45.0% or less, about 44.2% or less, about 43.4% or less, about 42.6% or less, about 41.9% or less, or about 41.1% or less; e.g., from 10% to 40%, from 15% to 30%, or from 20% to 25%; e.g., about 20%, about 21%, about 22%, about 23%, about 24%, or about 25% of the N-glycosylated Fc regions in the anti-BAFF-R antibody composition are galactosylated). In some instances, according to Formula I, any of the directly aforementioned antibody compositions have a relative ADCC potency from about 70% to about 130% compared to an ianalumab reference standard (e.g., a commercial ianalumab product).

In some instances, about 6% or less (e.g., about 6%) of the N-glycosylated Fc regions in an anti-BAFF-R antibody composition have a high mannose glycan, and about 46% or less of the N-glycosylated Fc regions in the anti-BAFF-R antibody composition are galactosylated (e.g., about 45.0% or less, about 44.2% or less, about 43.4% or less, about 42.6% or less, about 41.9% or less, or about 41.1% or less; e.g., from 10% to 40%, from 15% to 30%, or from 20% to 25%; e.g., about 20%, about 21%, about 22%, about 23%, about 24%, or about 25% of the N-glycosylated Fc regions in the anti-BAFF-R antibody composition are galactosylated). In some instances, according to Formula I, any of the directly aforementioned antibody compositions have a relative ADCC potency from about 70% to about 130% compared to an ianalumab reference standard (e.g., a commercial ianalumab product).

In some instances, about 5% or less (e.g., about 5%) of the N-glycosylated Fc regions in an anti-BAFF-R antibody composition have a high mannose glycan, and about 45% or less of the N-glycosylated Fc regions in the anti-BAFF-R antibody composition are galactosylated (e.g., about 44.2% or less, about 43.4% or less, about 42.6% or less, about 41.9% or less, or about 41.1% or less; e.g., from 10% to 40%, from 15% to 30%, or from 20% to 25%; e.g., about 20%, about 21%, about 22%, about 23%, about 24%, or about 25% of the N-glycosylated Fc regions in the anti-BAFF-R antibody composition are galactosylated). In some instances, according to Formula I, any of the directly aforementioned antibody compositions have a relative ADCC potency from about 70% to about 130% compared to an ianalumab reference standard (e.g., a commercial ianalumab product).

In some instances, about 4% or less (e.g., about 4%) of the N-glycosylated Fc regions in an anti-BAFF-R antibody composition have a high mannose glycan, and about 44% or less of the N-glycosylated Fc regions in the anti-BAFF-R antibody composition are galactosylated (e.g., about 43.4% or less, about 42.6% or less, about 41.9% or less, or about 41.1% or less; e.g., from 10% to 40%, from 15% to 30%, or from 20% to 25%; e.g., about 20%, about 21%, about 22%, about 23%, about 24%, or about 25% of the N-glycosylated Fc regions in the anti-BAFF-R antibody composition are galactosylated). In some instances, according to Formula I, any of the directly aforementioned antibody compositions have a relative ADCC potency from about 70% to about 130% compared to an ianalumab reference standard (e.g., a commercial ianalumab product).

In some instances, about 3% or less (e.g., about 3%) of the N-glycosylated Fc regions in an anti-BAFF-R antibody composition have a high mannose glycan, and about 43% or less of the N-glycosylated Fc regions in the anti-BAFF-R antibody composition are galactosylated (e.g., about 42.6% or less, about 41.9% or less, or about 41.1% or less; e.g., from 10% to 40%, from 15% to 30%, or from 20% to 25%; e.g., about 20%, about 21%, about 22%, about 23%, about 24%, or about 25% of the N-glycosylated Fc regions in the anti-BAFF-R antibody composition are galactosylated). In some instances, according to Formula I, any of the directly aforementioned antibody compositions have a relative ADCC potency from about 70% to about 130% compared to an ianalumab reference standard (e.g., a commercial ianalumab product).

In some instances, about 2% or less (e.g., about 2%) of the N-glycosylated Fc regions in an anti-BAFF-R antibody composition have a high mannose glycan, and about 43% or less of the N-glycosylated Fc regions in the anti-BAFF-R antibody composition are galactosylated (e.g., about 42.6% or less, about 41.9% or less, or about 41.1% or less; e.g., from 10% to 40%, from 15% to 30%, or from 20% to 25%; e.g., about 20%, about 21%, about 22%, about 23%, about 24%, or about 25% of the N-glycosylated Fc regions in the anti-BAFF-R antibody composition are galactosylated). In some instances, according to Formula I, any of the directly aforementioned antibody compositions have a relative ADCC potency from about 70% to about 130% compared to an ianalumab reference standard (e.g., a commercial ianalumab product).

In some instances, about 1% or less (e.g., about 1%) of the N-glycosylated Fc regions in an anti-BAFF-R antibody composition have a high mannose glycan, and about 42% or less of the N-glycosylated Fc regions in the anti-BAFF-R antibody composition are galactosylated (e.g., about 41.9% or less, or about 41.1% or less; e.g., from 10% to 40%, from 15% to 30%, or from 20% to 25%; e.g., about 20%, about 21%, about 22%, about 23%, about 24%, or about 25% of the N-glycosylated Fc regions in the anti-BAFF-R antibody composition are galactosylated). In some instances, according to Formula I, any of the directly aforementioned antibody compositions have a relative ADCC potency from about 70% to about 130% compared to an ianalumab reference standard (e.g., a commercial ianalumab product).

Conversely, a relative ADCC of a composition of afucosylated anti-BAFF antibodies having a given percentage of galactosylated glycoforms can be predicted based on the percentage of high mannose glycoforms. Table II-c provides the percentage of high mannose glycoform in a composition of afucosylated anti-BAFF-R IgG₁ antibodies that is predicted to achieve 100% relative ADCC based on percentage of galactosylation in the composition.

**Table II-c. Predicted %HM for 100% relative ADCC based on %Gal**

| **% Gal** | **% HM** | **% Gal** | **% HM** | **% Gal** | **% HM** |
|---|---|---|---|---|---|
| 13 | 0.6 | 24 | 14.7 | 35 | 28.8 |
| 14 | 1.9 | 25 | 16.0 | 36 | 30.1 |
| 15 | 3.2 | 26 | 17.3 | 37 | 31.4 |
| 16 | 4.4 | 27 | 18.6 | 38 | 32.7 |
| 17 | 5.7 | 28 | 19.8 | 39 | 34.0 |
| 18 | 7.0 | 29 | 21.1 | 40 | 35.2 |
| 19 | 8.3 | 30 | 22.4 | 41 | 36.5 |
| 20 | 9.6 | 31 | 23.7 | 42 | 37.8 |
| 21 | 10.9 | 32 | 25.0 | 43 | 39.1 |
| 22 | 12.1 | 33 | 26.3 | 44 | 40.4 |
| 23 | 13.4 | 34 | 27.5 | 45 | 41.7 |

Table II-c shows that an afucosylated composition of afucosylated anti-BAFF-R IgG₁ antibodies having, e.g., 15% galactose is predicted to achieve the same ADCC potency as an ianalumab reference standard if 3.2% of its N-glycosylated Fc regions contain a high mannose glycan. An analogous conclusion can be drawn from each pair of values in Table II-c, each of which characterizes an antibody composition part of the present invention.

Formula I also provides for a calculation of a target range of high mannose values around the ianalumab reference standard. Table II-d provides the target range of percentage of high mannose glycoforms in a composition of afucosylated anti-BAFF-R IgG₁ antibodies that is predicted to achieve 70% to 130% relative ADCC (i.e., +/- 30% variance around the ianalumab reference), based on a given percentage of galactosylated N-glycans.

**Table II-d. Predicted %HM range for 70%-130% relative ADCC based on percent %Gal**

| **% Gal** | **% HM** | **% Gal** | **% HM** | **% Gal** | **% HM** |
|---|---|---|---|---|---|
| 10 | 0 - 33.4 | 21 | 0 - 47.5 | 32 | 0 - 61.6 |
| 11 | 0 - 34.7 | 22 | 0 - 48.8 | 33 | 0 - 62.9 |
| 12 | 0 - 35.9 | 23 | 0 - 50.1 | 34 | 0 - 64.2 |
| 13 | 0 - 37.2 | 24 | 0 - 51.3 | 35 | 0 - 65.5 |
| 14 | 0 - 38.5 | 25 | 0 - 52.6 | 36 | 0 - 66.7 |
| 15 | 0 - 39.8 | 26 | 0 - 53.9 | 37 | 0 - 68.0 |
| 16 | 0 - 41.1 | 27 | 0 - 55.2 | 38 | 0 - 69.3 |
| 17 | 0 - 42.4 | 28 | 0 - 56.5 | 39 | 0 - 70.6 |
| 18 | 0 - 43.6 | 29 | 0 - 57.8 | 40 | 0 - 71.9 |
| 19 | 0 - 44.9 | 30 | 0 - 59.0 | 41 | 0 - 73.2 |
| 20 | 0 - 46.2 | 31 | 0 - 60.3 | 42 | 1.2 - 74.4 |

Table II-d provides that an anti-BAFF-R antibody composition having 10% to 40% of galactosylated N-glycans can have as little as 0% high mannose and as much as 71.9% high mannose (for a composition having 40% galactosylation, or 33.4% high mannose for a composition having 10% galactosylation) without exceeding a 30% variance in relative ADCC potency compared to a reference ianalumab standard.

While Table II-d suggests a wide range of high mannose tolerance for given galactosylation levels, high levels of high mannose content may be undesirable for reasons not considered in this analysis, such as potential detrimental impact on *in vivo* pharmacokinetics. Therefore, in some instances in which Formula I provides for higher levels of high mannose content in an antibody composition, it will be desirable to keep high mannose levels below 20%, e.g., below 10%, e.g., below 6%, or below 5%. In these instances, the anti-BAFF-R antibody compositions provided herein (e.g., monoclonal, afucosylated anti-BAFF-R IgG₁ antibody compositions, e.g., monoclonal, afucosylated anti-BAFF-R IgG₁κ antibody compositions) typically exhibit an average (e.g., mean or median) serum half-life of at least five days. In some instances, the average (e.g., mean or median) serum half-life of the antibody compositions provided herein (e.g., monoclonal, afucosylated anti-BAFF-R IgG₁ antibody compositions, e.g., monoclonal, afucosylated anti-BAFF-R IgG₁κ antibody compositions) is nine or more days, e.g., 10 or more days.

Thus, in some instances, anti-BAFF-R antibody compositions described herein (e.g., monoclonal, afucosylated anti-BAFF-R IgG₁ antibody compositions, e.g., monoclonal, afucosylated anti-BAFF-R IgG₁κ antibody compositions) include N-glycosylated Fc regions in which about 10-40% of the N-glycosylated Fc regions are galactosylated and less than 20% of the N-glycosylated Fc regions have a high mannose glycan (e.g., less than 15%, less than 10%, less than 8%, less than 6%, less than 5%, less than 4%, less than 3%, less than 2%, or less than 1%; e.g., from 0.1% to 6%, from 0.5% to 3.5%, or from 1% to 3%; e.g., from 0.1% to 1%, from 1% to 2%, from 2% to 3%, from 3% to 4%, from 4% to 5%, or from 5% to 6% of the N-glycosylated Fc regions have a high mannose glycan).

In some instances, the invention includes compositions of anti-BAFF-R as described herein (e.g., monoclonal, afucosylated anti-BAFF-R IgG₁ antibody compositions, e.g., monoclonal, afucosylated anti-BAFF-R IgG₁κ antibody compositions) having N-glycosylated Fc regions in which less than 20% of the N-glycosylated Fc regions have a high mannose glycan (e.g., less than 15%, less than 10%, less than 8%, less than 6%, less than 5%, less than 4%, less than 3%, less than 2%, or less than 1%; e.g., from 0.1% to 6%, from 0.5% to 3.5%, or from 1% to 3%; e.g., from 0.1% to 1%, from 1% to 2%, from 2% to 3%, from 3% to 4%, from 4% to 5%, or from 5% to 6% of the N-glycosylated Fc regions have a high mannose glycan) and about 10-40% of the N-glycosylated Fc regions are galactosylated (e.g., about 15% to about 38% of the N-glycosylated Fc regions are galactosylated, e.g., about 21% to about 25% of the N-glycosylated Fc regions are galactosylated.)

In some instances, the invention includes compositions of anti-BAFF-R as described herein (e.g., monoclonal, afucosylated anti-BAFF-R IgG₁ antibody compositions, e.g., monoclonal, afucosylated anti-BAFF-R IgG₁κ antibody compositions) having N-glycosylated Fc regions in which 0% to about 7% (e.g., about 3% to about 4.8%) of the N-glycosylated Fc regions have a high mannose glycan and about 15% to about 38% (e.g., about 21% to about 25%) of the N-glycosylated Fc regions are galactosylated.

### ADCC

ADCC potency can be quantified by methods known in the art or as described herein. In general, known techniques can measure target cell lysis by detecting intracellular component release or changes in cell viability.

In some instances, flow cytometry-based assays can quantify ADCC with single-cell resolution. Target cells are stained with identifying fluorochromes, and, after co-culture with effector cells and anti-BAFF-R antibodies, viability is measured using propidium iodide (PI) or 7-aminoactinomycin D (7-AAD), which stain dead cells, which can be fluorescently counted using flow cytometry analysis software. Half-maximal effective concentrations (EC₅₀) of anti-BAFF-R antibodies can be derived from a dose titration and quantification of cell killing or lysis by dose of antibody.

In another example, a chromium-51 (⁵¹Cr) release assay can be used to quantify ADCC, in which target cells are pre-labeled with radioactive ⁵¹Cr, which integrates into intracellular proteins. When effector cells lyse the target cells, released ⁵¹Cr is measured using a gamma counter. The detected radioactivity correlates with target cell destruction. Half-maximal effective concentrations (EC₅₀) of anti-BAFF-R antibodies can be derived from a dose titration and quantification of cell killing or lysis by dose of antibody.

Alternatively, fluorometric ADCC assays use enzyme-based detection to measure target cell lysis. Target cells can be labeled with calcein-AM, a membrane-permeable dye that fluoresces upon hydrolysis. When cells lyse, calcein is released, and fluorescence intensity is measured.

In some instances, ADCC potency is measured using a cell-based ADCC assay using NK cells as effector cells and BAFF-R expressing cells (e.g., HEK293 cells).

In some instances, compositions of anti-BAFF-R antibodies herein (e.g., a composition of monoclonal anti-BAFF-R antibodies, e.g., afucosylated IgG₁ antibodies) have a relative antibody-dependent cellular cytotoxicity (ADCC) potency of about 70% to about 130% (e.g., from about 75% to about 125%, from about 80% to about 120%, from about 80% to about 116%, from about 85% to about 115%, from about 90% to about 110%, from about 95% to about 105%, or about 100%) in a cell-based ADCC assay (e.g., an NK cell-based ADCC potency assay, e.g., as described herein) compared to a reference standard (e.g., a commercial reference standard, e.g., ianalumab).

In some instances, the compositions of anti-BAFF-R antibodies herein (e.g., a composition of monoclonal anti-BAFF-R antibodies, e.g., afucosylated IgG₁ antibodies) have an ADCC potency within a reference range of ADCC potencies from two or more lots of a reference standard (e.g., commercial reference standard) (e.g., a reference range of ADCC potencies from two, three, four, five, six, or more lots of a reference standard of ianalumab, e.g., commercially available ianalumab). As used herein, a reference range from more than two lots of a reference standard refers to the broadest range set by the two most disparate ADCC potencies (i.e., the outermost range of the set).

ADCC can be mediated by Fc gamma receptor III (FcγRIII (e.g., FcγRIIIa), CD16) binding by the Fc region of IgG antibodies (e.g., IgG₁ or IgG₃ antibodies), which can trigger intracellular signaling and release of cytotoxic granules containing perforin and granzymes, which can induce apoptosis in the target cell (e.g., a BAFF-R expressing cell). Thus, an anti-BAFF-R antibody's binding affinity (K_{D}) to FcγRIII (e.g., FcγRIIIa) can be used as an indirect readout or surrogate for ADCC activity.

In some instances, the anti-BAFF-R antibodies herein (e.g., a composition of monoclonal anti-BAFF-R antibodies, e.g., afucosylated IgG₁ antibodies) depletes human B cells through ADCC with a half-maximal efficacy (EC₅₀): (a) from 10 pM to 1 nM (e.g., from about 50 pM to about 500 pM, from about 100 pM to about 400 pM, or from about 150 pM to about 250 pM; e.g., about 196 pM) in an *in vitro* culture of human whole blood and/or (b) from 0.2 pM to 100 pM (e.g., from about 1.0 pM to about 50 pM, from about 1.5 pM to about 10 pM, or from 2.0 pM to about 5 pM; e.g., about 2.7 pM) in an *in vitro* culture of human peripheral blood mononuclear cells.

In some instances, the compositions of anti-BAFF-R antibodies herein (e.g., a composition of monoclonal anti-BAFF-R antibodies, e.g., afucosylated IgG₁ antibodies) have: (a) a binding affinity (K_{D}) to FcγRIIIa^{F158} from 1.3 nM to 3.9 nM (e.g., from about 1.5 nM to about 3.5 nM, e.g., from about 2.0 nM to about 3.0 nM, e.g., about 2.6 nM); (b) a binding affinity to FcγRIIIa^{V158} from 0.52 nM to 1.59 nM (e.g., about 1.06 nM); or (c) a relative binding affinity to FcγRIIIa^{F158} and/or FcγRIIIa^{V158} from about 70% to about 130% (e.g., from 80% to 120%, from 90% to 110%, e.g., about 100%) compared to a commercial reference standard, e.g., commercially available ianalumab.

In some instances, the compositions of anti-BAFF-R antibodies herein (e.g., a composition of monoclonal anti-BAFF-R antibodies, e.g., afucosylated IgG₁ antibodies) have a serum half-life (e.g., in humans) of at least 5 days (e.g., at least 6 days, at least 7 days, at least 8 days, or at least 9 days).

### Antibody Variants

Provided herein are compositions of anti-BAFF-R antibodies characterized by the presence of certain variants, which, in some cases, have a therapeutic impact (e.g., a potency impact, e.g., an ADCC impact), as described in Example 5.

In some instances, any of the anti-BAFF-R antibodies of a composition described herein includes at least one variant selected from a high molecular weight variant, a deamidated variant, a tryptophan-oxidized variant, a methionine-oxidized variant, or a clipped variant. In some embodiments, the monoclonal anti-BAFF-R antibodies include a high molecular weight variant, wherein the high molecular weight variant accounts for up to about 11% of the anti-BAFF-R antibodies in the composition, e.g., up to about 6% of the anti-BAFF-R antibodies in the composition, e.g., up to about 1% of the anti-BAFF-R antibodies in the composition. In some embodiments, the monoclonal anti-BAFF-R antibodies include a deamidated variant, wherein the deamidated variant comprises deamidation at position N332 (heavy chain residue 332), and optionally wherein the deamidated variant accounts for up to 23% (e.g., up to 22%) of the anti-BAFF-R antibodies in the composition. In some embodiments, the deamidated variant accounts for up to 1% of the anti-BAFF-R antibodies in the composition. In some embodiments, the monoclonal anti-BAFF-R antibodies include a tryptophan-oxidized variant, e.g., including H-W104 (tryptophan oxidation at heavy chain residue 104) and/or H-W59 (tryptophan oxidation at heavy chain residue 59), e.g., wherein the tryptophan-oxidized variant accounts for up to 23% (e.g., up to 16%, e.g., up to 3%) of the anti-BAFF-R antibodies in the composition. In some embodiments, the anti-BAFF-R antibodies comprise a methionine-oxidized variant, wherein the methionine-oxidized variant comprises methionine oxidation at one or more of light chain residue M97, heavy chain residue M259, heavy chain residue M365, and heavy chain residue M435 (e.g., one, two, three, or all four of light chain residue M97, heavy chain residue M259, heavy chain residue M365, and heavy chain residue M435). In some embodiments, the anti-BAFF-R antibodies comprise a methionine-oxidized variant comprising oxidation at light chain residue 97, wherein the methionine-oxidized variant comprising oxidation at light chain residue 97 accounts for up to 62% of the anti-BAFF-R antibodies and fragments thereof in the composition. In some embodiments, the methionine-oxidized variant accounts for up to 4% of the anti-BAFF-R antibodies in the composition. In some embodiments, the anti-BAFF-R antibodies comprise a methionine-oxidized variant, wherein the methionine-oxidized variant comprises oxidation of M259 on both heavy chains. In some embodiments, the anti-BAFF-R antibodies comprise a methionine-oxidized variant, e.g., a methionine-oxidized variant accounting for about 4% or less of the anti-BAFF-R antibodies in the composition. In some embodiments, the anti-BAFF-R antibodies comprise a clipped variant, e.g., wherein the clipped variant includes clipping between R56 and S57 in the CDR2 loop of the heavy chain. In some embodiments, the clipped variant accounts for up to 10% of the anti-BAFF-R antibodies and fragments thereof in the composition. In some embodiments, the afucosylated anti-BAFF-R antibodies include a high molecular weight variant, e.g., wherein the high molecular weight variant accounts for about 11% or less of the anti-BAFF-R antibodies in the composition, e.g., about 6% or less of the anti-BAFF-R antibodies in the composition, e.g., about 1% or less of the anti-BAFF-R antibodies in the composition. In some embodiments, the afucosylated anti-BAFF-R antibodies comprise a deamidated variant, e.g., wherein the deamidated variant comprises deamidation at position N332 (heavy chain residue 332), e.g., wherein the deamidated variant accounts for up to 23% (e.g., up to 22%) of the anti-BAFF-R antibodies in the composition. In some embodiments, the deamidated variant accounts for up to 1% of the anti-BAFF-R antibodies in the composition. In some embodiments, the afucosylated anti-BAFF-R antibodies include a tryptophan-oxidized variant, e.g., wherein the tryptophan-oxidized variant comprises oxidation at H-W104 and/or H-W59, e.g., wherein the tryptophan-oxidized variant accounts for up to 23% (e.g., up to 16%, e.g., up to 3%) of the anti-BAFF-R antibodies in the composition. In some embodiments, the afucosylated anti-BAFF-R antibodies include a methionine-oxidized variant, e.g., wherein the methionine-oxidized variant comprises one or more of light chain residue M97, heavy chain residue M259, heavy chain residue M365, and heavy chain residue M435, e.g., wherein the methionine-oxidized variant accounts for about 4% or less of the anti-BAFF-R antibodies in the composition. In some embodiments, the anti-BAFF-R antibodies comprise a methionine-oxidized variant comprising oxidation at light chain residue 97, wherein the methionine-oxidized variant comprising oxidation at light chain residue 97 accounts for up to 62% of the anti-BAFF-R antibodies and fragments thereof in the composition. In some embodiments, the methionine-oxidized variant accounts for up to 4% of the anti-BAFF-R antibodies in the composition. In some embodiments, the afucosylated anti-BAFF-R antibodies include a clipped variant, e.g., wherein the clipped variant comprises clipping between R56 and S57 in the CDR2 loop of the heavy chain. In some embodiments, the clipped variant accounts for up to 10% of the anti-BAFF-R antibodies and fragments thereof in the composition.

Additionally provided herein are compositions of any of the afucosylated anti-BAFF-R antibodies described herein (e.g. , wherein the anti-BAFF-R antibodies comprise a heavy chain amino acid sequence of SEQ ID NO: 1 and a light chain amino acid sequence of SEQ ID NO: 2), wherein the afucosylated anti-BAFF-R antibodies include a deamidated variant, e.g., wherein the deamidated variant comprises deamidation at position N332, e.g., wherein the deamidated variant accounts for up to 23% (e.g., up to 22%, e.g., up to 1%) of the anti-BAFF-R antibodies in the composition. In some embodiments, the afucosylated anti-BAFF-R antibodies include a high molecular weight variant, e.g., wherein the high molecular weight variant accounts for up to 11% of the anti-BAFF-R antibodies in the composition, e.g., about 6% or less of the anti-BAFF-R antibodies in the composition, e.g., about 1% or less of the anti-BAFF-R antibodies in the composition. In some embodiments, the afucosylated anti-BAFF-R antibodies comprise a tryptophan-oxidized variant, e.g., wherein the tryptophan-oxidized variant comprises oxidation at H-W104 and/or H-W59, e.g., wherein the tryptophan-oxidized variant accounts for up to 23% (e.g., up to 16%, e.g., up to 3%) of the anti-BAFF-R antibodies in the composition. In some embodiment, the afucosylated anti-BAFF-R antibodies comprise a methionine-oxidized variant, e.g., wherein the methionine-oxidized variant comprises oxidation at one or more of light chain residue M97, heavy chain residue M259, heavy chain residue M365, and heavy chain residue M435, e.g., wherein the methionine-oxidized variant comprises oxidation at heavy chain residue M259 and heavy chain residue M435, e.g., wherein the methionine-oxidized variant accounts for about 4% or less of the anti-BAFF-R antibodies in the composition. In some embodiments, the anti-BAFF-R antibodies comprise a methionine-oxidized variant comprising oxidation at light chain residue 97, wherein the methionine-oxidized variant comprising oxidation at light chain residue 97 accounts for up to 62% of the anti-BAFF-R antibodies and fragments thereof in the composition. In some embodiments, the methionine-oxidized variant accounts for up to 4% of the anti-BAFF-R antibodies in the composition. In some embodiments, the afucosylated anti-BAFF-R antibodies include a clipped variant, e.g., wherein the clipped variant comprises clipping between R56 and S57 in the CDR2 loop of the heavy chain. In some embodiments, the clipped variant accounts for up to 10% of the anti-BAFF-R antibodies and fragments thereof in the composition.

In some instances, provided herein are compositions having afucosylated anti-BAFF-R antibodies (e.g., any of the anti-BAFF-R antibodies described herein, the anti-BAFF-R antibodies comprise a heavy chain amino acid sequence of SEQ ID NO: 1 and a light chain amino acid sequence of SEQ ID NO: 2)), wherein the afucosylated anti-BAFF-R antibodies comprise a tryptophan-oxidized variant, e.g., wherein the tryptophan-oxidized variant comprises oxidation at H-W104 and/or H-W59, and optionally wherein the tryptophan-oxidized variant accounts for up to 23% (e.g., up to 16%, e.g., up to 3%) of the anti-BAFF-R antibodies in the composition. In some embodiments, the afucosylated anti-BAFF-R antibodies further include a deamidated variant, e.g., wherein the deamidated variant comprises deamidation at position N332, and optionally wherein the deamidated variant accounts for up to 23% (e.g., up to 22%, e.g., up to 1%) of the anti-BAFF-R antibodies in the composition. In some embodiments, the afucosylated anti-BAFF-R antibodies further include a high molecular weight variant, e.g., wherein the high molecular weight variant accounts for about 11% or less of the anti-BAFF-R antibodies in the composition, e.g., about 6% or less of the anti-BAFF-R antibodies in the composition, e.g., about 1% or less of the anti-BAFF-R antibodies in the composition. In some embodiments, the afucosylated anti-BAFF-R antibodies further include a methionine-oxidized variant, e.g., wherein the methionine-oxidized variant comprises oxidation at one or more of light chain residue M97, heavy chain residue M259, heavy chain residue M365, and heavy chain residue M435, and optionally wherein the methionine-oxidized variant accounts for about 4% or less of the anti-BAFF-R antibodies in the composition. In some embodiments, the anti-BAFF-R antibodies comprise a methionine-oxidized variant comprising oxidation at light chain residue 97, wherein the methionine-oxidized variant comprising oxidation at light chain residue 97 accounts for up to 62% of the anti-BAFF-R antibodies and fragments thereof in the composition. In some embodiments, the methionine-oxidized variant accounts for up to 4% of the anti-BAFF-R antibodies in the composition. In some embodiments, the afucosylated anti-BAFF-R antibodies further comprise a clipped variant, e.g., wherein the clipped variant comprises clipping between R56 and S57 in the CDR2 loop of the heavy chain. In some embodiments, the clipped variant accounts for up to 10% of the anti-BAFF-R antibodies and fragments thereof in the composition.

In another instance, provided are compositions including afucosylated anti-BAFF-R antibodies (e.g., any of the anti-BAFF-R antibodies described herein, the anti-BAFF-R antibodies comprise a heavy chain amino acid sequence of SEQ ID NO: 1 and a light chain amino acid sequence of SEQ ID NO: 2)) having a methionine-oxidized variant comprising one or more of M97, M259, M365, and M435. In some embodiments, the methionine-oxidized variant accounts for about 4% or less of the anti-BAFF-R antibodies in the composition. In some embodiments, the anti-BAFF-R antibodies comprise a methionine-oxidized variant comprising oxidation at light chain residue 97, wherein the methionine-oxidized variant comprising oxidation at light chain residue 97 accounts for up to 62% of the anti-BAFF-R antibodies and fragments thereof in the composition. In some embodiments, the methionine-oxidized variant accounts for up to 4% of the anti-BAFF-R antibodies in the composition. In some embodiments, the afucosylated anti-BAFF-R antibodies further include a deamidated variant, e.g., wherein the deamidated variant comprises deamidation at position N332, and optionally wherein the deamidated variant accounts for up to 23% (e.g., up to 22%, e.g., up to 1%) of the anti-BAFF-R antibodies in the composition. In some embodiments, the afucosylated anti-BAFF-R antibodies further include a tryptophan-oxidized variant, e.g., wherein the tryptophan-oxidized variant comprises oxidation at H-W104 and/or H-W59, and optionally wherein the tryptophan-oxidized variant accounts for up to 23% (e.g., up to 16%, e.g., up to 3%) of the anti-BAFF-R antibodies in the composition. In some embodiments, the afucosylated anti-BAFF-R antibodies include a high molecular weight variant, e.g., wherein the high molecular weight variant accounts for about 11% or less of the anti-BAFF-R antibodies in the composition, e.g., about 6% or less of the anti-BAFF-R antibodies in the composition, e.g., about 1% or less of the anti-BAFF-R antibodies in the composition. In some embodiments, the afucosylated anti-BAFF-R antibodies further include a clipped variant, wherein the clipped variant comprises clipping between R56 and S57 in the CDR2 loop of the heavy chain. In some embodiments, the clipped variant accounts for up to 10% of the anti-BAFF-R antibodies and fragments thereof in the composition.

In another instance, provided are compositions including the afucosylated anti-BAFF-R antibodies (e.g. , wherein the anti-BAFF-R antibodies comprise a heavy chain amino acid sequence of SEQ ID NO: 1 and a light chain amino acid sequence of SEQ ID NO: 2) which afucosylated anti-BAFF-R antibodies comprise a clipped variant comprising clipping between R56 and S57 in the CDR2 loop of the heavy chain. In some embodiments, the clipped variant accounts for up to 10% of the anti-BAFF-R antibodies and fragments thereof in the composition. In some embodiments, the afucosylated anti-BAFF-R antibodies further include a deamidated variant, e.g., wherein the deamidated variant comprises deamidation at position N332, and optionally wherein the deamidated variant accounts for up to 23% (e.g., up to 22%, e.g., up to 3%) of the anti-BAFF-R antibodies in the composition. In some embodiments, the afucosylated anti-BAFF-R antibodies further comprise a tryptophan-oxidized variant comprising oxidation at H-W104 and/or H-W59, and optionally wherein the tryptophan-oxidized variant accounts for up to 23% (e.g., up to 16%, e.g., up to 3%) of the anti-BAFF-R antibodies in the composition. In some embodiments, the afucosylated anti-BAFF-R antibodies further comprise a methionine-oxidized variant, e.g., wherein the methionine-oxidized variant comprises oxidation at one or more of light chain residue M97, heavy chain residue M259, heavy chain residue M365, and heavy chain residue M435, and optionally wherein the methionine-oxidized variant accounts for about 4% or less of the anti-BAFF-R antibodies in the composition. In some embodiments, the anti-BAFF-R antibodies comprise a methionine-oxidized variant comprising oxidation at light chain residue 97, wherein the methionine-oxidized variant comprising oxidation at light chain residue 97 accounts for up to 62% of the anti-BAFF-R antibodies and fragments thereof in the composition. In some embodiments, the methionine-oxidized variant accounts for up to 4% of the anti-BAFF-R antibodies in the composition. The afucosylated anti-BAFF-R antibodies further comprise a high molecular weight variant, wherein the high molecular weight variant accounts for about 11% or less of the anti-BAFF-R antibodies in the composition, e.g., about 6% or less of the anti-BAFF-R antibodies in the composition, e.g., about 1% or less of the anti-BAFF-R antibodies in the composition.

### III. Production

Chinese hamster ovary (CHO) cells are most commonly used for the production of glycosylated polypeptides for therapeutic use. These cells produce a defined glycosylation profile and allow the creation of genetically stable, high-productivity cell lines. Furthermore, CHO cells can be cultured at high cell density in serum-free media to develop safe and reproducible biological processes.

A CHO cell line comprising a deletion in the telomeric region of the q arm of chromosome 8 is provided. The deletion was induced by chromosome breakage. The deleted portion comprised gene FAM60A as well as among others, gene C12orf35 which is located telomeric from gene FAM60A. Said cell line was obtained from a parental cell line derived from CHO-K1. Said cell line with a chromosome break in chromosome 8 can be prepared as follows. Parental cells from a single vial are treated in several rounds with 0.5 µM, 1 µM or 2 µM methotrexate (known to induce chromosome aberrations). After six days the cell viabilities are around 30-40%. Cells are centrifuged at 180xg for 5 min and cultivated in culture medium without methotrexate to allow the cells to recover until viabilities were above 95% (after ca. 21 days). This procedure is repeated two more times. Single cell clones are obtained from cell pools. Single cells are sorted by fluorescence activated cell sorting (FACS) and clones are screened for lack of the telomeric region of chromosome 8 by PCR. For the final selected clone, loss of the chromosome 8 telomeric region to be confirmed by fluorescence in situ hybridization (FISH) and a master cell bank can be prepared.

In some aspects, the anti-BAFF-R antibody is produced by a CHO cell line, for example as described above, that has been further altered to impair the function of fucose, e.g. by reducing or eliminating the functional expression of the fucose gene. Cells with an altered glycosylation machinery have been described in the art and can be used as host cells in which to express recombinant antibodies of the invention to thereby produce an antibody with impaired fucosylation. For example, EP 1,176,195 by Hang et al. describes a cell line with a functionally disrupted FUT8 gene, which encodes a fucosyl transferase, such that antibodies expressed in such a cell line exhibit hypofucosylation or are devoid of fucose residues. Therefore, in one embodiment, the anti-BAFFR antibodies that are included in the pharmaceutical compositions of the invention are produced by recombinant expression in a cell line which exhibit hypofucosylation or non-fucosylation pattern, for example, a mammalian cell line (e.g., CHO cell line) with deficient expression of the FUT8 gene encoding fucosyltransferase, or a mammalian cell line (e.g., CHO cell line) expressing GDP-6-deoxy-D-lyxo-4-hexulose reductase (RMD).

In certain aspects, provided herein is a CHO cell line generated according to the methods disclosed in WO/2015/092735 or in WO/2015/092737, which are incorporated herein by reference. The CHO cell is comprising a deletion in the telomeric region of the q arm of chromosome 8 and is fucosyltransferase-deficient (e.g., a CHO-C8TD ΔFUT-8 cell line) or the CHO cell is comprising a deletion in the telomeric region of the q arm of chromosome 8 and expressing GDP-6-deoxy-D-lyxo-4-hexulose reductase (RMD) (e.g., a CHO-C8TD RMD cell line).

PCT Publication WO 03/035835 by Presta describes a variant CHO cell line, Lecl3 cells, with reduced ability to attach fucose to N-linked carbohydrates, also resulting in hypofucosylation of antibodies expressed in that host cell (see also Shields, RL et al., 2002J. Biol. Chem. 277:26733-26740). PCT Publication WO 99/54342 by Umana et al. describes cell lines engineered to express glycoprotein-modifying glycosyl transferases (e.g., beta(1,4)-N acetylglucosaminyltransferase III (GnTIII)) such that antibodies expressed in the engineered cell lines exhibit increased bisecting GIcNac structures which results in increased ADCC activity of the antibodies (see also Umana et al., 1999 Nat. Biotech. 17:176-180). Eureka Therapeutics further describes genetically engineered CHO mammalian cells capable of producing antibodies with altered mammalian glycosylation pattern devoid of fucose residues (www.eurekainc.com/about_us/companyoverview.html).

Alternatively, the anti-BAFFR antibodies can be produced in yeasts or filamentous fungi engineered for mammalian-like glycosylation pattern and capable of producing antibodies lacking fucose as glycosylation pattern (see for example EP1297172B1).

In accordance with the methods of the present invention, host-cells are cultured in a medium that allows for the expression of recombinant glycoproteins. Suitable cell culture procedures and conditions are well known in the art. Host-cells (e.g., CHO cells) may be cultured in a wide variety of formats and culture vessels. For example, host-cells may be cultured in formats designed for large scale or small scale production of glycoproteins. Additionally, host-cells may be cultured adherent to the bottom of culture flasks or dishes, or they may be in suspension in stirred flasks, bioreactors or in roller bottle cultures. In certain embodiments, for production of recombinant glycoproteins in commercially relevant quantities, host-cells may be grown in bioreactors, and preferably bioreactors having a capacity of about 2 liters or more, or about 5 liters or more, or about 10 liters or more, or about 50 liters or more, or about 100 liters or more, or about 500 liters or more, or about 1000 liters or more, or about 1500 liters or more, or about 2000 liters or more.

In certain embodiments, host-cells can be cultured (e.g., maintained and/or grown) in liquid media and preferably are cultured, either continuously or intermittently, by conventional culturing methods such as standing culture, test tube culture, shaking culture (e.g., rotary shaking culture, shake flask culture, etc.), aeration spinner culture, or fermentation. In certain embodiments, host-cells are cultured in shake flasks. In yet other embodiments, host-cells are cultured in a fermentor (e.g., in a fermentation process). Fermentation processes include, but are not limited to, batch, fed-batch and continuous methods of fermentation. The terms "batch process" and "batch fermentation" refer to a closed system in which the composition of media, nutrients, supplemental additives and the like is set at the beginning of the fermentation and not subject to alteration during the fermentation; however, attempts may be made to control such factors as pH and oxygen concentration to prevent excess media acidification and/or microorganism death. The terms "fed-batch process" and "fed-batch" fermentation refer to a batch fermentation with the exception that one or more substrates or supplements are added (e.g., added in increments or continuously) or the cell culture conditions are changed as the fermentation progresses. The terms "continuous process" and "continuous fermentation" refer to a system in which a defined fermentation media is added continuously to a fermentor and an equal amount of used or "conditioned" media is simultaneously removed, for example, for recovery of the desired product (e.g. antibody). A variety of such processes have been developed and are well-known in the art.

Glucose and other nutrients will typically be present in or added to the culture medium, i.e. the base medium into which the monoclonal antibody producing cells are transferred for the production phase. Transfer may be, for example, from a medium tailored for growth of the cells. The precise nature of the base medium is not essential to the present invention. Chemically defined media have been extensively developed and published in recent history, including such media for culture of mammalian cells. All components of defined media are well characterized and such media do not contain complex additives such as serum and hydrolysates. Typically these media include defined quantities of purified growth factors, proteins, lipoproteins and other substances which may otherwise be provided by serum or extract supplement. Such media have been produced with the sole purpose of supporting highly productive cell cultures. Certain defined media may be termed low protein media or may be protein free if the typical components of low protein media, insulin and transferrin, are not included. Serum free media may otherwise be used in the methods of the present invention. Such media normally do not contain serum or protein fractions, but may contain undefined components.

Examples of commercially available culture media include Ham's F10 (Sigma), Minimal Essential Medium (MEM, Sigma), RPMI-1640 (Sigma) and Dulbecco's Modified Eagle's Medium (DMEM, Sigma) and chemically defined media and feed supplements sold by Life Technologies. Any such media may be supplemented as necessary with hormones and/or other growth factors (such as insulin, transferrin or epidermal growth factor); salts (such as sodium chloride, calcium, magnesium and phosphate), amino acids, buffers (such as HEPES); nucleosides (such as adenosine and thymidine), antibiotics (such as GENTAMYCIN^{™}), and glucose or an equivalent energy source. Any of these media may be used as the base medium, with addition of glucose and other nutrients as necessary to follow the methods described herein. In a preferred embodiment, the cells are cultured in a chemically defined medium comprising glucose and other nutrients typically required for cell culture and growth and expression of antibody.

The duration of the production phase may depend on the culture method used and/or may depend on the cell density used for inoculation of the medium, for example an inoculation cell density of about 1 x 10⁵ cells/mL to about 20 x 10⁵ cells/mL will typically require a production phase of up to 10-18 days. However, when a higher inoculation cell density is used, e.g. from about 21 x 10⁵ cells/mL to about 200 x 10⁵ cells/mL, the duration of the production phase may decrease to, for example, 6 to 10 days. A high inoculation cell density can be reached with, for example, an intensified process, e.g. a perfusion process at the n-1 step of cultivation. In one embodiment of the present invention the production phase is up to 7-18 days from inoculation, preferably 7-10 days, 10-18 days, or 14-17 days.

Following the antibody production phase, the antibody of interest can be recovered from the culture medium using techniques which are well established in the art. The antibody of interest preferably is recovered from the culture medium as a secreted antibody, although it also may be recovered from host cell lysates.

In certain aspects, the culture medium or lysate is centrifuged to remove particulate cell debris. The antibody thereafter is purified from contaminant soluble proteins and polypeptides using a suitable purification procedures. Exemplary purification procedures include, but are not limited to, fractionation on immunoaffinity or ion-exchange columns; ethanol precipitation; reverse phase HPLC; chromatography on silica or on a cation-exchange resin such as DEAE; chromatofocusing; SDS-PAGE; ammonium sulfate precipitation; gel filtration using, for example, Sephadex G-75; and protein A Sepharose columns to remove contaminants such as IgG. A protease inhibitor such as phenyl methyl sulfonyl fluoride (PMSF) also may be useful to inhibit proteolytic degradation during purification. One skilled in the art will appreciate that purification methods suitable for the antibody of interest may require modification to account for changes in the character of the antibody upon expression in recombinant cell culture.

### IV. Pharmaceutical Formulations and Pharmaceutical Products

Provided herein are compositions comprising an anti-BAFF-R antibody. The compositions include bulk drug compositions useful in the manufacture of pharmaceutical compositions (e.g., impure or non-sterile compositions) and pharmaceutical compositions (i.e., compositions that are suitable for administration to a subject or patient) which can be used in the preparation of unit dosage forms. The compositions (e.g., pharmaceutical compositions) comprise an effective amount of an anti-BAFF-R antibody, or a combination of an anti-BAFF-R antibody and a pharmaceutically acceptable carrier. Such compositions are suitably free of visible particulate matter. The formulation may be in liquid form or lyophilized form. A composition in a liquid formulation may be filled into containers and frozen. In certain embodiments, aliquots of the frozen formulation comprising the composition may be lyophilized. Lyophilisate may be reconstituted by the addition of water or other aqueous solution to produce a reconstituted formulation comprising the composition.

In a specific embodiment, the term "pharmaceutically acceptable" means approved by a regulatory agency of the Federal or a state government or listed in the U.S. Pharmacopeia or other generally recognized pharmacopeia for use in animals, and more particularly in humans. The term "carrier" refers to a diluent, adjuvant (e.g., Freund's adjuvant (complete and incomplete) or, more preferably, MF59C.1 adjuvant), excipient, or vehicle with which the therapeutic is administered. Such pharmaceutical carriers can be sterile liquids, such as water and oils, including those of petroleum, animal, vegetable or synthetic origin, such as peanut oil, soybean oil, mineral oil, sesame oil and the like. In one embodiment, water is a carrier when the pharmaceutical composition is administered intravenously. Saline solutions and aqueous dextrose and glycerol solutions can also be employed as liquid carriers, particularly for injectable solutions. Suitable pharmaceutical excipients include starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol and the like. The composition, if desired, can also contain wetting or emulsifying agents, or pH buffering agents. These compositions can take the form of solutions, suspensions, emulsion, tablets, pills, capsules, powders, sustained-release formulations and the like.

Pharmaceutical compositions may be formulated in any conventional manner using one or more pharmaceutically acceptable carriers or excipients. In a specific embodiment, an anti-BAFF-R antibody as defined herein, administered to a subject in accordance with the methods described herein is administered as a pharmaceutical composition.

Generally, the components of the pharmaceutical compositions comprising an anti-BAFF-R antibody is supplied in unit dosage form, for example, as a dry lyophilized powder or water free concentrate in a hermetically sealed container such as an ampoule or sachette indicating the quantity of the anti-BAFF-R antibody. Where the anti-BAFF-R antibody is to be administered by infusion, it can be dispensed with an infusion bottle containing sterile pharmaceutical grade water or saline (e.g., PBS). Where the anti-BAFF-R antibody is administered by injection, an ampoule of sterile water for injection or saline can be provided so that the ingredients may be mixed prior to administration.

In some embodiments, the anti-BAFF-R antibody may be formulated for administration by any method known to one of skill in the art, including but not limited to, parenteral (e.g., subcutaneous, intravenous, intratumoral or intramuscular) administration. In one embodiment, the anti-BAFF-R antibody is formulated for local or systemic parenteral administration. In a specific embodiment, the anti-BAFF-R antibody is formulated for subcutaneous or intravenous administration, respectively. In one embodiment, the anti-BAFF-R antibody is formulated in a pharmaceutically compatible solution.

The anti-BAFF-R antibody can be formulated for parenteral administration by injection, e.g., by bolus injection or continuous infusion. Formulations for injection may be presented in unit dosage form, e.g., in ampoules or in multi-dose containers, with an added preservative. The compositions may take such forms as suspensions, solutions or emulsions in oily or aqueous vehicles, and may contain formulatory agents such as suspending, stabilizing and/or dispersing agents. Alternatively, the anti-BAFF-R antibody may be in powder form for constitution with a suitable vehicle, e.g., sterile pyrogen-free water, before use.

WO2012076670, WO2013186700 describe formulations of anti-BAFF-R antibodies, which are incorporated herein by reference.

In one embodiment the pharmaceutical composition, also called drug product (DP), is a lyophilized formulation prepared from an aqueous formulation, or an aqueous pharmaceutical composition comprising:
(i) the anti-BAFF-R antibody, e.g., ianalumab
(ii) sucrose, trehalose or mannitol,
(iii) histidine, citrate or succinate,
(iv) polysorbate 20, poloxamer 188 or hydroxyproyl-b-cyclodextrin, and, optionally,
(v) arginine.

In one embodiment, the aqueous composition of the invention as described herein in the various embodiments comprises the anti-BAFF-R antibody in a concentration from about 20 mg/mL up to about 150 mg/mL, particularly of from about 80 mg/mL up to about 150 mg/mL, particularly of from about 100 mg/mL up to about150 mg/mL.

In one embodiment, the aqueous composition of the invention as described herein in the various embodiments comprises a sugar, particularly sucrose, mannitol or trehalose, in a concentration of from about 80 mM up to about 300 mM, particularly of from about120 mM up to about 270 mM, particularly of from about 120 mM up to about 220 mM.

In one embodiment, the aqueous composition of the invention as described herein in the various embodiments comprises a surfactant, particularly polysorbate 20 or poloxamer 188, in a concentration of from about 0.01% up to about 0.1 %, particularly of from about 0.02% up to about 0.06%.

In one embodiment, the aqueous composition of the invention as described herein in the various embodiments comprises a buffering agent, particularly histidine, citrate or succinate, in a concentration of from about 5 mM up to about 50 mM, particularly in a concentration of from about 15 mM up to about 25 mM, particularly of from about 18 mM up to about 22 mM, particularly about 20 mM.

In one embodiment, the aqueous composition of the invention as described herein in the various embodiments further comprises an amino acid, particularly arginine or arginine -HCI, in a concentration of from about 2 mM up to about 80 mM.

Techniques for lyophilisation of antibodies are well known in the art e.g. see John F. Carpenter and Michael J. Pikal, 1997 (Pharm. Res. 14, 969-975); Xialin (Charlie) Tang and Michael J. Pikal, 2004 (Pharm. Res. 21, 191-200). For example, the monoclonal antibody products SYNAGIS^{™}, REMICADE^{™}, RAPTIVA^{™}, SIMULECT^{™}, XOLAIR^{™} and HERCEPTIN^{™} are supplied as lyophilisates. These antibodies are reconstituted to various final concentrations e.g. SIMULECT^{™} is reconstituted to a concentration of 4 mg/ml antibody, REMICADE^{™} is reconstituted to a concentration of 10 mg/ml, HERCEPTIN^{™} to 21 mg/ml, SYNAGIS^{™} and RAPTIVAT^{™} to 100 mg/ml, and XOLAIR^{™} to 125 mg/ml.

Before a lyophilisate can be administered to a patient it should be reconstituted with an aqueous reconstituent. This step permits antibody and other components in the lyophilisate to re-dissolve to give a solution which is suitable for injection to a patient. Alternatively, a suspension could be formed.

The volume of aqueous material used for reconstitution dictates the concentration of the antibody in a resulting pharmaceutical composition. Reconstitution with a smaller volume of reconstituent than the pre-lyophilisation volume provides a composition which is more concentrated than before lyophilisation. The reconstitution factor (volume of formulation after lyophilization:volume of formulation before lyophilization) may be from 1:0.5 to 1:6. A reconstitution factor of 1:3 is useful. As mentioned above, lyophilisates of the invention can be reconstituted to give aqueous compositions with an anti-BAFFR antibody concentration of at least 50 mg/ml, 100 mg/ml, 150 mg/ml. 200 mg/ml, 250 mg/ml or 300 mg/ml, and the volume of reconstituent will be selected accordingly. If required, the reconstituted formulation can be diluted prior to administration to a patient as appropriate to deliver the intended dose.

Typical reconstituents for lyophilized antibodies include sterile water or buffer, optionally containing a preservative. If the lyophilisate includes a buffering agent then the reconstituent may include further buffering agent (which may be the same as or different from the lyophilisate's buffering agent) or it may instead include no buffering agent (e.g. WFI (water for injection), or physiological saline).

When present, lyophilisate components ill be at a pre-lyophilisation concentration sufficient to maintain the anti-BAFFR antibody in a form which is active and soluble after storage (under normal conditions) and reconstitution. The components will also be present after reconstitution.

Thus a sugar, such as sucrose or trehalose, may be present before lyophilisation at a concentration of from about 3 up to about 300 mM e.g. 15-200 mM, 30-150 mM, 80-100 mM. A concentration of 90 mM sucrose is useful. A buffering agent, such as histidine, may be present before lyophilisation at a concentration of from about 1 up to about 60 mM e.g. 3-30 mM, 5-20 mM, 5-15 mM. A concentration of 7 mM histidine buffer is useful. A surfactant, such as polysorbate 80 or polysorbate 20 may be present before lyophilisation at a concentration of up to 0.2% (by volume) e.g., 0.01-0.1%, 0.01-0.08%, 0.01-0.04%. A concentration of 0.02% polysorbate 80 or polysorbate 20 is useful. A free amino acid, such as arginine or glycine, may be present before lyophilisation at a concentration of from about 2 up to about 80 mM e.g. 3-50 mM, 6-30 mM, 10-25 mM, 15-20 mM. A concentration of 17 mM arginine-HCI or 20 mM glycine-HCl is useful. The anti-BAFFR antibody is present before lyophilization at a concentration of from about 20 mg/ml up to about 120 mg/ml, e.g. 20 mg/ml, 30 mg/ml, 40 mg/ml, 50 mg/ml, 60 mg/ml, 66.6 mg/ml, 70 mg/ml, 80 mg/ml, 90 mg/ml, 100 mg/ml, 110 mg/ml, or 120 mg/ml. A concentration of 50 mg/ml or of 150 mg/ml is useful.

The pre-lyophilisate of the invention has a pH from about 5.0 up to about 8.0, from about5.0 up to about7.0, from about 6.0 up to about 8.0, or from about 6.0 up to about 7.0. In a specific embodiment, the pre-lyophilisate of the invention has a pH of about 6.5.

In one embodiment the pre-lyophilisate of the invention has a molar ratio of sucrose:antibody of 270:1 and a molar ratio of histidine:antibody of 21:1.

In one embodiment the pre-lyophilisate of the invention has a molar ratio of sucrose:antibody of 270:1, a molar ratio of histidine:antibody of 21:1, and a molar ratio of arginine-HCl:antibody of 51:1.

In one embodiment the pre-lyophilisate of the invention has a molar ratio of sucrose:antibody of 270:1, a molar ratio of histidine:antibody of 21:1, and a molar ratio of glycine-HCl:antibody of 60:1.

In one embodiment the pre-lyophilisate of the invention has a molar ratio of sucrose:antibody of 203:1, a molar ratio of histidine:antibody of 16:1, and a molar ratio of arginine-HCl:antibody of 38:1.

A formulation containing histidine buffer, sucrose, polysorbate 80 or polysorbate 20 or poloxamer 188 and, optionally arginine or glycine has been shown to be suitable for lyophilisation of anti-BAFF-R antibodies. After reconstitution, the components of the lyophilisate may be present at a concentration of the aqueous pharmaceutical compositions as described herein.

In a specific aspect, provided is an aqueous composition having a pH of 5.5-6.5 and comprising:
(i) an anti-BAFFR antibody wherein the antibody has a concentration of 18 mg/mL -165 mg/mL, and wherein said anti-BAFFR antibody includes heavy chain CDR1, CDR2 and CDR3 of SEQ ID NOs 5, 6 and 7 respectively, and light chain CDR1, CDR2 and CDR3 of SEQ ID NOs: 8, 9 and 10,
(ii) 80 mM - 300 mM sucrose, trehalose or mannitol,
(iii) 5 mM -50 mM histidine, citrate or succinate as a buffering agent,
(iv) 0.01% - 0.1% polysorbate 20 or poloxamer 188, or 1 mM - 3 mM hydroxyproyl-b- cyclodextrin, and, optionally,
(v) 2 mM - 80 mM arginine, particularly arginine-HCl.

In another specific aspect, provided is an aqueous composition having a pH of 5.5-6.5 comprising:
(i) an anti-BAFFR antibody wherein the antibody has a concentration of 20 mg/mL - 150 mg/mL and wherein said anti-BAFFR antibody includes heavy chain CDR1, CDR2 and CDR3 of SEQ ID NOs 5, 6 and 7 respectively, and light chain CDR1, CDR2 and CDR3 of SEQ ID NOs: 8, 9 and 10,
(ii) 110 mM - 250 mM sucrose or trehalose,
(iii) 15 mM - 25 mM histidine, citrate or succinate as a buffering agent,
(iv) up to 0.02% - 0.06% polysorbate 20 or poloxamer 188 or 2 mM - 3 mM hydro xyproyl-b-cyclodextrin, and, optionally,
(v) 2 mM - 80 mM arginine, particularly arginine-HCI.

In one specific aspect the composition, also called drug product (DP) is a lyophilized formulation prepared from an aqueous formulation having a pH of 5.7-6.3, e.g. about 6.0, and comprising:
(i) about 150 mg/mL ianalumab,
(ii) 200-270 mM sucrose,
(iii) 15-30 mM L-histidine, and
(iv) 0.03%- 0.06% Polysorbate 20.

In another specific aspect the pharmaceutical composition, also called drug product (DP), is an aqueous pharmaceutical composition has a pH of 5.7-6.3, e.g. about 6.0, and comprising:
(i) about 150 mg/mL ianalumab,
(ii) about 220 mM sucrose,
(iii) about 20 mM L-histidine, and
(iv) about 0.04% Polysorbate 20.

Other contemplated excipients, which may be utilized in the aqueous pharmaceutical compositions of the invention include, for example, flavoring agents, antimicrobial agents, sweeteners, antioxidants, antistatic agents, lipids such as phospholipids or fatty acids, steroids such as cholesterol, protein excipients such as serum albumin (human serum albumin), recombinant human albumin, gelatin, casein, salt-forming counterions such sodium and the like. These and additional known pharmaceutical excipients and/or additives suitable for use in the formulations of the invention are known in the art, e.g., as listed in "The Handbook of Pharmaceutical Excipients, 4th edition, Rowe et al., Eds., American Pharmaceuticals Association (2003); and Remington: the Science and Practice of Pharmacy, 21th edition, Gennaro, Ed., Lippincott Williams & Wilkins (2005).

The aqueous pharmaceutical compositions of the invention may include further active ingredients in addition to the anti-BAFF-R antibody. Further pharmacological agents may include, for instance, chemotherapeutic compounds.

A "stable" formulation is one in which the protein/antibody therein essentially retains its physical and/or chemical stability during manufacturing, transport, storage, and administration. Stability can be measured at a selected temperature for a selected time period. For example, for a product stored at a recommended temperature of 2° C to 8° C., the formulation is stable at room temperature, about 30° C, or at 40° C, for at least 1 month and/or stable at about 2 to 8° C for at least 1 year and preferably for at least 2 years. For example, the extent of aggregation during storage can be used as an indicator of protein stability. Thus, a "stable" formulation may be one wherein, about 10% or less, about 5% or less, for example about 4% or less aggregation is present in the formulation.

In certain aspects provided herein is a formulation which allows the composition to remain stable to storage at about 2 to 8° C. for at least 18 months, freezing, thawing, and/or mixing. In one embodiment, a "stable" formulation may be one wherein, about 4% or less aggregation is present in the formulation.

In another specific aspect the pharmaceutical composition, also called drug product (DP), is aqueous pharmaceutical composition has a pH of about 5.7- 6.3, has a turbidity of ≤ 18 NTU (Nephelometric Turbidity Unit) and comprising:
(i) about 150 mg/mL ianalumab,
(ii) about 0.04% Polysorbate 20.

In yet another specific aspect the pharmaceutical composition, also called drug product (DP), is aqueous pharmaceutical composition has a pH of about 5.7- 6.3, comprising:
(i) about 150 mg/mL ianalumab,
(ii) about 0.04% Polysorbate 20,
and wherein the pharmaceutical composition is colorless to slightly brownish-yellow, not more intensely colored than reference solution BY4 (Color reference solution BY4 according to the European Pharmacopeia).

In certain aspects provided herein is a formulation comprising the anti-BAFF-R antibody, e.g. ianalumab, as herein disclosed, and wherein the pharmaceutical formulation comprises a polysorbate with a reduced hydrolysis activity rate, wherein the shelf-life of the pharmaceutical formulation is more than 24 months.

In yet another aspect, provided herein is an article of manufacture, e.g. a kit, comprising a container holding a composition in a formulation described herein. In one aspect there is provided an injection device comprising the formulation. The injection device may comprise a pen injector device or an autoinjector device. In one embodiment, the formulation is contained in a prefilled syringe.

In certain aspects provided herein is injection device comprising the formulation comprising the anti-BAFF-R antibody, e.g. ianalumab, as herein disclosed, and wherein the injection time of the injection device is of about 17 seconds or less.

In yet another aspect, provided is a pharmaceutical formulation in a container, wherein the pharmaceutical formulation is a pharmaceutical formulation as described herein. The container may be a vial, an injection device, an injection pen, a vial and syringe, a cartridge, a pre-filled syringe, or an autoinjector. In certain aspects, the container further comprises a package insert and/or instructions for use.

In some aspects, provided are pharmaceutical products that include any of the antibody compositions or pharmaceutical formulations described herein, optionally packaged in a container (e.g., a container described herein, such as a vial, an injection device, an injection pen, a cartridge, a pre-filled syringe, or an autoinjector) or together with any of the containers described herein (e.g., as a lyophilisate in a vial, wherein the pharmaceutical product further includes a syringe for injecting the reconstituted lyophilisate.)

### V. Therapeutic Methods and Uses

In one aspect, provided herein are methods for treating or preventing a BAFF-R-related disorder, e.g. an autoimmune disease, or B-cells neoplasm, comprising administering to subjects in need thereof the anti-BAFF-R antibody in a specific dose regimen. Non-limiting examples of a BAFFR-related disorder include autoimmune disease, wherein the autoimmune disease is autoimmune haematological disorders (including e.g. warm autoimmune hemolytic anaemia, aplastic anaemia, pure red cell anaemia and immune thrombocytopenia), acquired hemophilia A, cold agglutinin disease, cryoglobulinemia, thrombotic thrombocytopenic purpura, Sjögren's Disease, systemic lupus erythematosus, lupus nephritis, inflammatory muscle disorders, polychondritis, sclerodoma, anti-neutrophil cytoplasmic antibody-associated vasculitis, IgM mediated neuropathy, opsoclonus myoclonus syndrome, Wegener granulomatosis, dermatomyositis, chronic active hepatitis, myasthenia gravis, psoriasis, Steven-Johnson syndrome, pemphigus vulgaris, pemphigus foliacius, idiopathic sprue, autoimmune inflammatory bowel disease (including e.g. ulcerative colitis, Crohn's disease and Irritable Bowel Syndrome), endocrine ophthalmopathy, Graves' disease, sarcoidosis, multiple sclerosis, neuromyelitis optica, primary biliary cirrhosis, juvenile diabetes (diabetes mellitus type I), uveitis (anterior, intermediate and posterior as well as panuveitis), keratoconjunctivitis sicca and vernal keratoconjunctivitis, interstitial lung fibrosis, psoriatic arthritis and glomerulonephritis (with and without nephrotic syndrome, e.g. including idiopathic nephrotic syndrome or minimal change nephropathy), tumors, inflammatory disease of skin and cornea, myositis, loosening of bone implants, or metabolic disorders, such as atherosclerosis, diabetes, and dislipidemia.

In one embodiment, the anti-BAFF-R antibody, especially ianalumab, is administered to a subject, especially in need thereof, at a dose from about 150 to about 450 mg, from about 200 to about 350 mg. Preferably the dose is from about 150 mg to about 300 mg. More preferably the dose is about 300 mg.

In one embodiment, the antibody, especially ianalumab, is administered to a subject, especially in need thereof, once every 4 weeks (q4w, monthly, +/- 3 days).

In one embodiment, the antibody is ianalumab, wherein ianalumab is administered to a subject, especially in need thereof, at a dose of about 150 mg, subcutaneously, once every four weeks (q4w, monthly, +/- 3 days).

In one embodiment, the antibody is ianalumab, wherein ianalumab is administered to a subject, especially in need thereof, at a dose of about 300 mg, subcutaneously, once every four weeks (q4w, monthly, +/- 3 days).

In one embodiment, the anti-BAFF-R antibody, especially ianalumab, is administered to a subject, especially in need thereof, as a monotherapy. The subject does not receive any other treatment of the disease during the time period of being treated with the Drug of the Invention.

In one embodiment, are provided methods for treating or preventing an autoimmune disease, wherein the autoimmune disease is warm autoimmune hemolytic anemia, and optionally wherein the anti-BAFF-R antibody is administered to a subject in need thereof at a dose of about 3 mg/kg or about 9 mg/kg, iv., once every 4 weeks (e.g., monthly, +/- 3 days) for a duration of up to 4 months.

In one embodiment, are provided methods for treating or preventing an autoimmune disease, wherein the autoimmune disease is immune thrombocytopenia, and optionally wherein the anti-BAFF-R antibody is administered to a subject in need thereof at a dose of about 3 mg/kg or about 9 mg/kg, iv., once every 4 weeks (e.g., monthly, +/- 3 days) for a duration of up to 4 months.

In one embodiment, are provided methods for treating or preventing an autoimmune disease, wherein the autoimmune disease is Sjögren's Disease, systemic lupus erythematosus, lupus nephritis, hidradenitis suppurativa, or scleroderma, and optionally wherein the anti-BAFF-R antibody is administered to a subject in need thereof at a dose of about 300 mg, subcutaneously, once every 4 weeks (e.g., monthly, +/- 3 days), or once every 12 weeks (e.g. every 3 months, +/- 3 days).

In one embodiment, the route of administration is subcutaneous or intravenous of the antibody according to the first aspect, or a combination of subcutaneous or intravenous.

The dose may be about 3 mg to about 10 mg anti-BAFF-R antibody per kilogram of a human subject. The dose may be given weekly, every two weeks or every four weeks.

In one embodiment, the dose is about 150 mg to about 600 mg anti-BAFF-R antibody. The dose may be given weekly, every two weeks or every four weeks.

In one embodiment, the dose is about 300 mg anti-BAFF-R antibody. The dose may be given weekly, every two weeks or every four weeks.

In one preferred embodiment, the dose is 150 mg anti-BAFF-R antibody. In another preferred embodiment, the dose is 300 mg anti-BAFF-R antibody. In yet another preferred embodiment, the dose is 300 mg anti-BAFF-R antibody and may be given every four weeks.

In one embodiment, the antibody is administered through a loading dosing and a maintenance dosing. In one embodiment, the loading dosing is administered via subcutaneous injections of a first dose and the maintenance dosing is administered via subcutaneous injections of a second dose. The first dose may be the same as the second dose or higher than the second dose.

Typically, the antibodies or proteins are administered by injection, for example, either intravenously, intraperitoneally, or subcutaneously. Methods to accomplish this administration are known to those of ordinary skill in the art. It may also be possible to obtain compositions that may be topically or orally administered, or which may be capable of transmission across mucous membranes. As will be appreciated by a person skilled in the art, any suitable means for administering can be used, as appropriate for a particular selected route of administration.

Examples of possible routes of administration include parenteral, (e.g., intravenous (I.V. or IV), intramuscular (IM), intradermal, subcutaneous (S.C. or SC), or infusion), oral and pulmonary (e.g., inhalation), nasal, transdermal (topical), transmucosal, and rectal administration. Solutions or suspensions used for parenteral, intradermal, or subcutaneous application can include the following components: a sterile diluent such as water for injection, saline solution, fixed oils, polyethylene glycols, glycerin, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. pH can be adjusted with acids or bases, such as hydrochloric acid or sodium hydroxide. The parenteral preparation can be enclosed in ampoules, disposable syringes, or multiple dose vials made of glass or plastic.

Therapeutically effective doses can be administered, for example, according to a weekly dosing schedule, or once every two weeks (bi-weekly), once every three weeks, or once every four weeks. In such embodiments, the therapeutically effective doses generally fall within the dosing range (i.e. about 3 mg/kg to about 10 mg/kg, such as about 3 mg/kg, e.g. 9 mg/kg IV or about 150 mg or about 300 mg administered weekly, bi-weekly or every 4 weeks subcutaneously).

The timing of dosing is generally measured from the day of the first dose of the active compound (e.g., the anti-BAFFR antibody or ianalumab), which is also known as "baseline." However, different health care providers use different naming conventions.

Notably, week zero may be referred to as week 1 by some health care providers, while day zero may be referred to as day one by some health care providers. Thus, it is possible that different physicians will designate, e.g., a dose as being given during week 3 / on day 21, during week 3 / on day 22, during week 4 / on day 21, during week 4 / on day 22, while referring to the same dosing schedule. For consistency, the first week of dosing will be referred to herein as week 0, while the first day of dosing will be referred to as day 1. However, it will be understood by a skilled artisan that this naming convention is simply used for consistency and should not be construed as limiting, i.e., weekly dosing is the provision of a weekly dose of the anti-BAFF-R antibody, e.g., ianalumab, regardless of whether the physician refers to a particular week as "week 1" or "week 2". It will be understood that a dose need not be provided at an exact time point, e.g., a dose due approximately on day 29 could be provided, e.g., on day 24 to day 34, e.g., day 30, as long as it is provided in the appropriate week.

As used herein, the phrase "container having a sufficient amount of the anti-BAFF-R antibody to allow delivery of [a designated dose]" is used to mean that a given container (e.g., vial, pen, syringe) has disposed therein a volume of an anti-BAFF-R antibody (e.g., as part of a pharmaceutical composition) that can be used to provide a desired dose. As an example, if a desired dose is 300 mg, then a clinician may use 2 ml from a container that contains an anti-BAFF-R antibody formulation with a concentration of 150 mg/ml, 1 ml from a container that contains an anti-BAFF-R antibody formulation with a concentration of 300 mg/ml, 0.5 ml from a container contains an anti-BAFF-R antibody formulation with a concentration of 600 mg/ml, etc. In each such case, these containers have a sufficient amount of the anti-BAFF-R antibody to allow delivery of the desired 300 mg dose.

As used herein, the phrase "formulated at a dosage to allow [route of administration] delivery of [a designated dose]" is used to mean that a given pharmaceutical composition can be used to provide a desired dose of an anti-BAFF-R antibody, e.g., ianalumab, via a designated route of administration (e.g., s.c. or i.v.). As an example, if a desired subcutaneous dose is 300 mg, then a clinician may use 2 ml of an anti-BAFF-R antibody formulation having a concentration of 150 mg/ml, 1 ml of an anti-BAFF-R antibody formulation having a concentration of 300 mg/ml, 0.5 ml of an anti-BAFF-R antibody formulation having a concentration of 600 mg/ml, etc. In each such case, these anti-BAFF-R antibody formulations are at a concentration high enough to allow subcutaneous delivery of the anti-BAFF-R antibody. Subcutaneous delivery typically requires delivery of volumes of less than about 2 ml, preferably a volume of about 1ml or less. However, higher volumes may be delivered over time using, e.g., a patch/pump mechanism.

Disclosed herein is the use of an anti-BAFF-R antibody (e.g., ianalumab) for the manufacture of a medicament for the treatment of an autoimmune disease, or B-cells neoplasm as herein defined, in a patient, wherein the medicament is formulated to comprise containers, each container having a sufficient amount of the anti-BAFF-R antibody to allow delivery of at least about 75 mg, 150 mg, or 300 mg anti-BAFF-R antibody or antigen binding fragment thereof (e.g., ianalumab) per unit dose.

Disclosed herein is the use of an anti-BAFF-R antibody (e.g., ianalumab) for the manufacture of a medicament for the treatment of an autoimmune disease, or B-cells neoplasm as herein defined, in a patient, wherein the medicament is formulated at a dosage to allow systemic delivery (e.g., i.v. or s.c. delivery) about 75 mg, about 150 mg, or about 300 mg anti-BAFF-R antibody or antigen binding fragment thereof (e.g., ianalumab) per unit dose.

### VI. ADCC activity level control

The present disclosure also provides methods of monitoring product quality of an antibody composition, wherein the ADCC activity level of the antibody composition is a criterion upon which product quality of the antibody composition is based. In exemplary embodiments, the method comprises determining product quality of an antibody composition in accordance with a method of the present disclosures, with a first sample obtained at a first timepoint and with a second sample taken at a second timepoint which is different from the first timepoint. In various instances, each of the first sample and second sample is a sample of in-process material. In various aspects, the first sample is a sample of in-process material and the second sample is a sample of a manufacturing lot. Optionally, the first sample is a sample obtained before one or more conditions of the cell culture are modified and the second sample is a sample obtained after the one or more conditions of the cell culture are modified. In exemplary instances, the high mannose glycan content is determined for each of the first sample and second sample. Product quality of the antibody composition depends on whether the high mannose glycan content is within a target range and the ADCC activity level is within a target range. In various instances, the method comprises determining the product quality of the antibody composition as acceptable and/or achieving the ADCC activity level criterion when the high mannose glycan content determined is within a target range, as defined herein. In various aspects, the target range of ADCC activity level is known for the antibody of the antibody composition. The antibody of the antibody composition, in various aspects, is a biosimilar of a reference antibody. In exemplary instances, the reference antibody is ianalumab.

In exemplary aspects of the presently disclosed methods, the target % ADCC is within a target % ADCC range. Optionally, the target % ADCC range is greater than or about 40 and less than or about 170. In various aspects, the target % ADCC range is greater than or about 44 and less than or about 165. In various instances, the target % ADCC range is greater than or about 60 and less than or about 130.

In various aspects, the % ADCC is determined by a quantitative cell-based assay which measures the ability of the antibodies of the antibody composition to mediate cell cytotoxicity in a dose-dependent manner in cells expressing the antigen of the antibodies and engaging FcγRIIIa receptors on effector cells through the Fc domain of the antibodies. In various instances, the % ADCC is determined by the assay described in Example 5. In exemplary aspects, the determining step is carried out after a harvest step. Optionally, the determining step is carried out after a chromatography step. In various aspects, the chromatography step is a Protein A chromatography step. In various instances of the presently disclosed methods, the one or more downstream processing steps comprise(s): a dilution step, a filling step, a filtration step, a formulation step, a chromatography step, a viral filtration step, a viral inactivation step, or a combination thereof. Optionally, the chromatography step is an ion exchange chromatography step, optionally, a cation exchange chromatography step or an anion exchange chromatography step.

The details of one or more embodiments of the disclosure are set forth in the accompanying description above. Although any methods and materials similar or equivalent to those described herein can be used in the practice or testing of the present disclosure, the preferred methods and materials are now described. Other features, objects, and advantages of the disclosure will be apparent from the description and from the claims. In the specification and the appended claims, the singular forms include plural referents unless the context clearly dictates otherwise. Unless defined otherwise, all technical and scientific terms used herein have the same meaning as commonly understood by one of ordinary skill in the art to which this disclosure belongs. All patents and publications cited in this specification are incorporated by reference as applicable, unless otherwise indicated. The following Examples are presented in order to more fully illustrate the preferred embodiments of the disclosure. These examples should in no way be construed as limiting the scope of the disclosed subject matter, which is defined by the appended claims.

### VII. Examples

The following examples are included for illustrative purposes only and are not intended to limit the scope of the disclosure.

### Example 1. Structure of ianalumab Fab in complex with BAFF-R

The three-dimensional structures of ianalumab Fab in its free form and bound to human BAFF-R were determined by X-ray crystallography to resolutions of 2.48 and 2.18 Å, respectively.

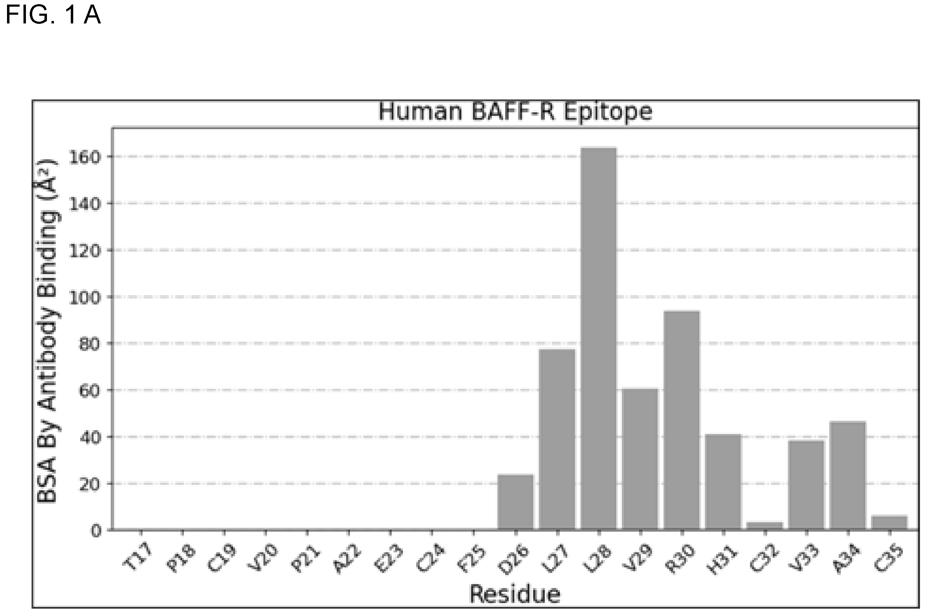

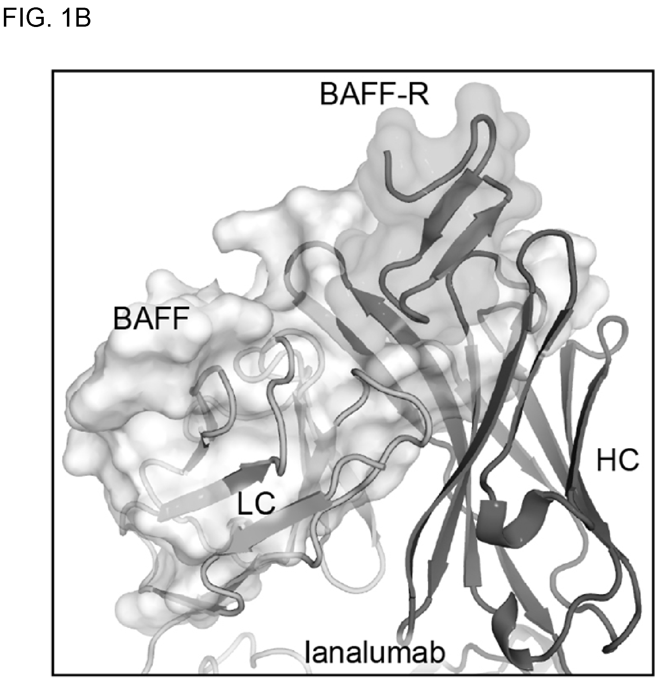

In the crystal structure of ianalumab Fab complexed with BAFF-R, four cysteine residues within the BAFF-R ligand binding domain form two disulfide bridges (C19-C32 and C24-C35), which are clearly visible in an electron density map and help stabilize the β-hairpin structure (Cys19 to Cys35) at the core of the BAFF-R ligand binding domain. The core of the structure consists of two β-strands linked by a non-canonical β-turn. This loop, consisting of six residues (²⁶Asp-Leu-Leu-Val-Arg-His³¹), is centered around a conserved Asp-Xxx-Leu motif critical for ianalumab binding, which is buried by ianalumab binding shown in FIG. 1A. Overall, this loop contributes to 83% of the antibody binding surface. Structural alignment with BAFF-R in complex with its nature ligand, BAFF revealed that both heavy and light chains of ianalumab sterically blocks BAFF binding (FIG. 1B), leading to effective inhibition of BAFF binding to BAFF-R.

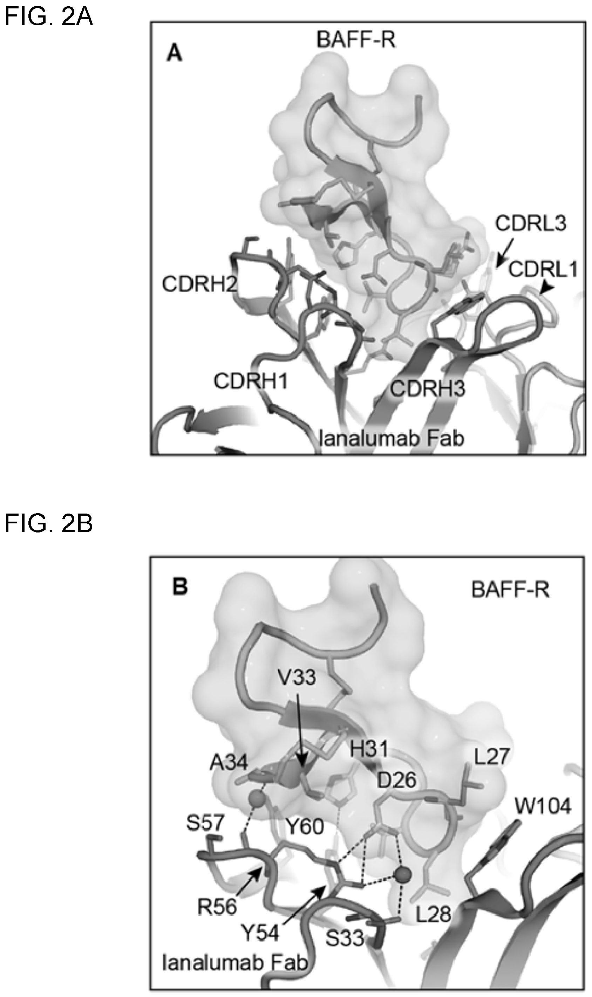

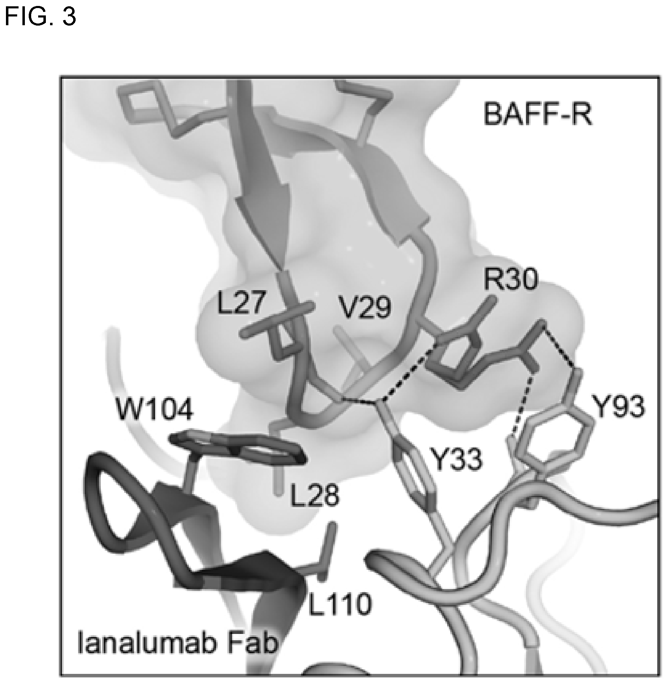

Individual residues located at the ianalumab : BAFF-R binding interface were identified by the decrease of solvent-accessible surface area upon formation of the complex. All complementarity determining regions (CDRs) except CDR2 of ianalumab light chain contribute to the binding interface. This is evidenced by the reduction in their solvent-accessible surface area upon antigen binding, with a notable concentration over the three heavy-chain CDRs (FIGS. 2A and 2B), along with light-chain CDR1 and CDR3 loops (FIG. 3). The ianalumab heavy chain contributes to 74% of the buried surface area by BAFF-R while the light-chain contributes to 26%.

FIGS. 2A, 2B, and 3 illustrate the ianalumab Fab - BAFF-R interaction, specifically highlighting the binding interface of the heavy chain in FIGS. 2A and 2B and of the light chain in FIG. 3. It is clearly shown that the heavy chain of ianalumab dominates the interaction with BAFF-R and it is also evident from the higher buried surface area (BSA) of the heavy chain compared to the light chain.

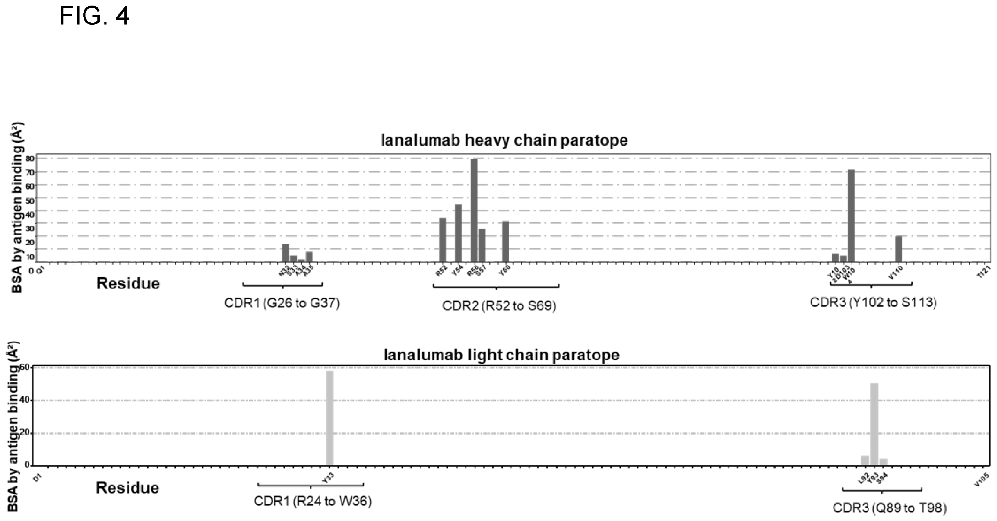

According to buried surface area plot shown in FIG. 4, mainly the amino acid residues N32, S33, A34, A35 in CDR1, R52, Y54, R56, S57, Y60 in CDR2 and Y102, D103, W104, V110 in CDR3 of the heavy chain as well as residues Y33 in CDR1 and L92, Y93, S94 in the CDR3 of the light chain were shown to be involved in the binding interaction with BAFF-R. The epitope of BAFF-R comprises residues D26, L27, L28, V29, R30, H31, C32, V33, A34, and C35, which are crucial for the antibody-antigen interaction (FIG. 1A).

### Example 2. Epitope mapping of ianalumab on BAFF-R

Hydrogen deuterium exchange mass spectrometry (HDX-MS) was employed as orthogonal methodology to identify key residues involved in the ianalumab : BAFF-R interaction. In the HDX-MS experiment, the ianalumab : BAFF-R complex and apo proteins are first incubated in a deuterium-containing buffer, allowing hydrogen atoms in the protein backbone to be exchanged with deuterium. Subsequently the reaction is quenched, and the protein is digested into peptides, and the resulting peptides are analyzed via mass spectrometry to determine the extent of deuterium incorporation. Residues involved in binding are less exposed to the solvent and show a reduced hydrogen deuterium exchange rate. HDX-MS was performed to map the putative epitope of ianalumab Fab on human BAFF-R extracellular domain (amino acid 1 to 76) and the corresponding paratope of BAFF-R on ianalumab Fab.

A total of 13 peptides of the BAFF-R were monitored by HDX-MS experiments resulting in 100% sequence coverage. For differential experiments, the levels of deuterium exchange between apo BAFF-R and ianalumab Fab : BAFF-R complex were compared. FIG. 5 shows the difference in deuterium uptakes between the ianalumab Fab : BAFF-R complex and apo BAFF-R.. The data indicates that ianalumab induces protection from deuterium exchange close to the N-terminal side of BAFF-R including amino acid residues 19-20, 27-29 and 31-34. Therefore, these residues are expected to contribute to the ianalumab-BAFF-R interaction, which is in good agreement with the results from X-ray crystallography, where D26 to C35 of BAFF-R showed higher BSA values (see also Example 1, FIG. 1A).

### Example 3. Paratope mapping of BAFF-R on ianalumab Fab

A total of 106 ianalumab Fab peptides were monitored by HDX-MS experiments resulting in 84% sequence coverage.

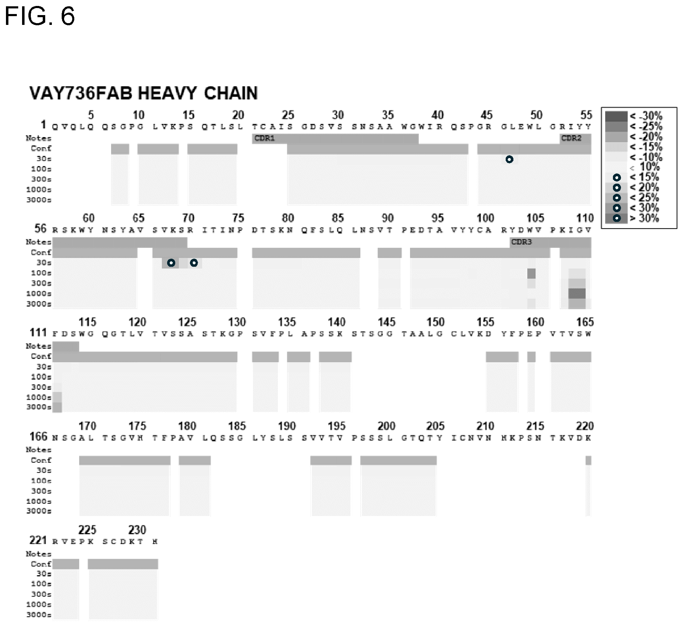

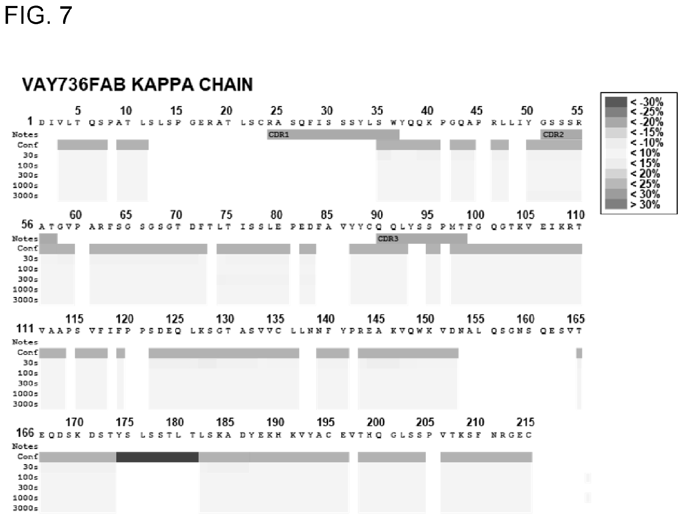

For differential experiments, the levels of deuterium exchange between apo ianalumab Fab and ianalumab Fab : BAFF-R complex were compared. FIGS. 6 and 7 showing the difference in deuterium uptake between the ianalumab Fab : BAFF-R complex and apo ianalumab Fab.. The data shows that BAFF-R induces a protection from deuterium exchange in the heavy chain CDR3 of ianalumab Fab containing amino acid residues 104, 108-109. Therefore, these residues are expected to contribute to the ianalumab Fab-BAFF-R interaction, which is in good agreement with the results from X-ray crystallography, where W104 in the CDR3 of the heavy chain showed the highest BSA value (see also Example 1, FIG. 4).

In hydrogen deuterium exchange differential experiments, some of the observed protection and deprotection can be the result of allosteric changes or other changes in higher order structure not directly related to the antibody-antigen binding. Nevertheless, the observed protection regions are nearly contiguous in sequence and so have a high likelihood of being directly related to the binding of ianalumab to BAFF-R. In summary, the HDX experiment define a paratope and epitope in the ianalumab Fab : BAFF-R interaction with 1:1 stoichiometry.

### Example 4. ADCC potency of ianalumab drug substance

### Relative ADCC potency of ianalumab drug substance by cell-based assay

In this example, relative ADCC potency of ianalumab was characterized using a cell-based assay. ADCC activity of ianalumab was measured based on its ability to mediate ADCC in the presence of BAFF-R-expressing target cells and NK effector cells. Fluorescently labeled HEK-293 cells stably expressing the recombinant human BAFF-R were incubated with varying concentrations of ianalumab and an excess of natural killer cells (NK3.3 cells). In this ADCC assay, the NK3.3 cells served as effector cells, whereas the labeled HEK-293 cells served as target cells. The cytotoxic response, indicated by the concentration-dependent killing of the HEK-293 target cells, was analyzed after a one-hour incubation by measuring the release of the fluorochrome from the lysed cells in each well.

The ADCC activity of ianalumab test samples or product control was determined by comparison to a reference standard. The samples and the standard were normalized on the basis of protein content. Relative potency was then calculated using a parallel line assay according to the European Pharmacopeia. The final result was expressed as relative potency of a sample (in percent) compared to the reference standard. Table 4-1 shows the potency results for ianalumab DS batches. All values were within 105-108% relative potency.

**Table 4-1. Potency of ianalumab drug substance by ADCC assay**

| **Sample** | **Relative biological potency [%]** |
|---|---|
| DS-1 | 105 |
| DS-2 | 108 |
| DS-3 | 108 |

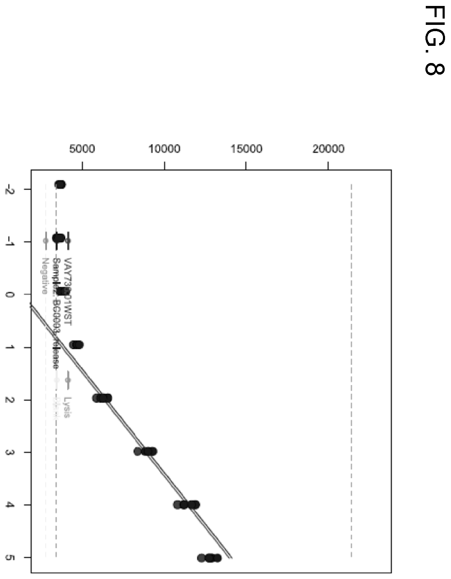

FIG. 8 shows a representative example of a dose-response curve from single determination performed for an ianalumab drug substance sample. X-axis is the logarithm of dose in ng/mL, and Y-axis is the response measured as fluorescence emission at 535 nm (excitation at 485 nm). The potency of ianalumab was measured in comparison to the reference standard VAY736.01WST (light blue). Relative potency was 107.4% for the determination shown.

### Fc receptor binding of ianalumab drug substance

The binding of IgG to FcγR is an important step for the initiation and control of cell-mediated effector functions, and each subclass of IgG (IgG1, IgG2, IgG3, and IgG4) can differ in profile of effector functions, dictated by differential binding to each of the FcγRs. Antibodies of the IgG₁ class have the potential to exert Fc-mediated effector functions, such as ADCC, by binding to the extracellular domain of FcγRs on effector cells and simultaneously to a membrane bound target.

To assess the binding of ianalumab drug substance to FcγRIIIa receptors, the soluble extracellular domain of FcγRIIIa^{F158/V158} were captured on SPR sensor chips via an anti-his antibody platform.

The affinity of ianalumab to FcγRIII was determined by real-time analysis of the interaction kinetics using a Biacore instrument. It was determined four times in two independent experiments. A carboxymethyl dextran-coated CM5 sensor ship (Cytiva) was covalently coupled with an anti-His antibody (Cytiva), which served as a capture platform to immobilize recombinant human FcγR. lanalumab was injected over the sensorchip surface at different concentrations (Table 4-2). The sensorchip was regenerated using a solution of 10 mM Glycine pH 1.5. The running buffer used during the experiment was HBS-P+ (Cytiva). From the obtained SPR sensorgrams, the dissociation equilibrium constant K_{D} was determined.

**Table 4-2. Recombinant human Fc receptors used in this study**

| **Receptor** | **Source** | **Concentrations [µM]** |
|---|---|---|
| FcγRIIIa^{F158} | R&D Systems | 0.39, 0.78, 1.6, 3.1, 6.2 |
| FcγRIIIa^{V158} | R&D Systems | 0.39, 0.78, 1.6, 3.1, 6.2 |

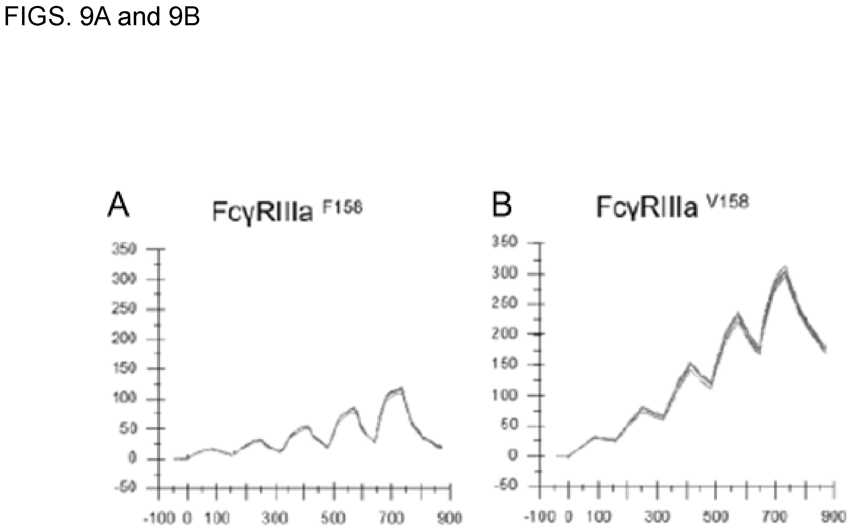

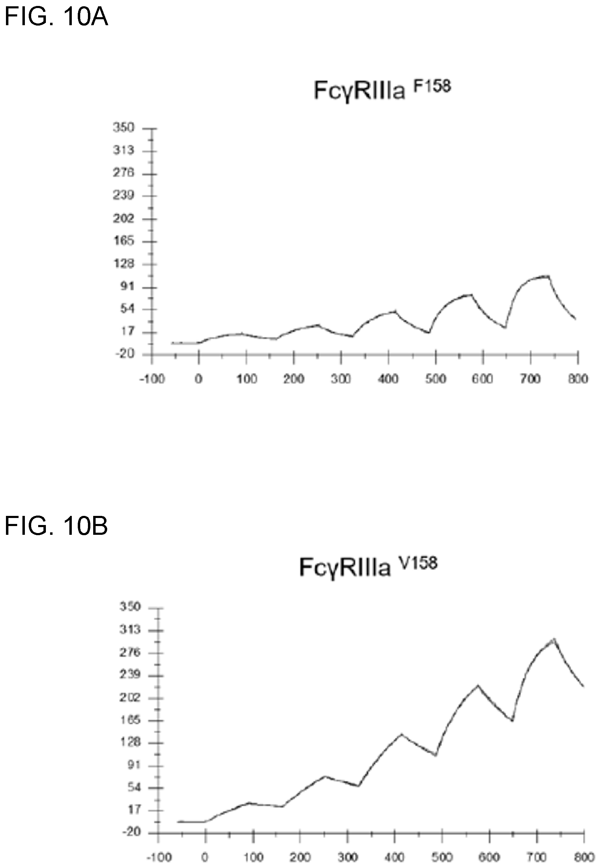

Table 4-3 summarizes the relative average (geometric mean) +/- global relative standard deviation of kₒₙ, k_{off}, and/or K_{D} for each batch. A sensorgram overlay, from one representative determination, of all batches is shown in FIGS. 9A and 9B. To further compare all batches, a fitted sensorgram for FcγRIIIa^{F158} and FcγRIIIa^{V158} is provided in FIGS. 10A and 10B, respectively. The affinity of ianalumab to FcγRIIIa was elevated compared to other IgG₁ with fucosylated glycan structures in the Fc (data not shown).

**Table 4-3. Relative affinity of ianalumab drug substance to FcγRIIIa (percentage of reference¹)**

| **Batch** | FcγRIIIa^{F158} | | | FcγRIIIa^{V158} | | |
|---|---|---|---|---|---|---|
| | Relative Average | Relative Average | Relative Average | Relative Average | Relative Average | Relative Average |
| | kₒₙ | k_{off} | K_{D} | kₒₙ | k_{off} | K_{D} |
| REF (BC0001) | 100 | 100 | 100 | 100 | 100 | 100 |
| DS-1 (BC0001) | 112 ± 5 | 101 ± 2 | 90 ± 2 | 114 ± 6 | 101 ± 1 | 89 ± 4 |
| DS-2 (BC0002) | 105 ± 6 | 97 ± 3 | 93 ± 3 | 103 ± 7 | 98 ± 3 | 95 ± 4 |
| DS-3 (BC0003) | 104 ± 5 | 98 ± 2 | 94 ± 3 | 103 ± 5 | 98 ± 2 | 96 ± 3 |

| | | | | | | |
|---|---|---|---|---|---|---|
| ¹ REF = reference ianalumab DS; measured constants for FcγRIIIa^{F158}: kₒₙ [10⁶ M⁻¹ s⁻¹]: 8.31 ± 0.42, k_{off} [10⁻³ s⁻¹]: 21.2 ± 2.7, K_{D} [10⁻⁹ M]: 2.57 ± 0.45, FcγRIIIa^{V158}; kₒₙ [10⁶ M⁻¹ s⁻¹]: 5.20 ± 0.11, k_{off} [10⁻³ s⁻¹]: 5.51 ± 0.37, K_{D} [10⁻⁹ M]: 1.06 ± 0.09. | | | | | | |

Comparable kinetic rate constants were obtained for all batches analyzed.

### Example 5. Quality attributes of ianalumab drug substance and drug product

The control strategy for manufacturing ianalumab drug substance (DS) and drug product (DP) forms is based on a comprehensive understanding of the product, its manufacturing processes, and its critical quality attributes. Integrated release and stability testing are important components, designed to detect any potential adverse impacts on patient safety and efficacy, ensuring process consistency.

The criticality of each quality attribute in ianalumab was evaluated using information such as stability data, non-clinical and clinical data, and detailed product characterization of variants underlying the degradation pathways. Each quality attribute was assigned a criticality score based on its known or potential impact on immunogenicity, safety, pharmacokinetics/pharmacodynamics (PK/PD), and biological activity/potency.

Criticality was determined in accordance with ICH Q9, by assessing each quality attribute towards potential impact and uncertainty (or certainty) of that impact. This assessment considers the known or potential impact or consequence on (1) biological activity, either through (pre-) clinical experience or results from potency assays(s) representing the mode of action, (2) PK/PD, either through (pre-) clinical experience or results from representative in vitro assays, (3) immunogenicity, i.e., the risk of anti-drug-antibody formation, either through clinical experience or results from representative *in vitro* assays, and (4) safety, i.e., the risk for adverse effects, though clinical experience. The impact for biological activity and PK/PD is assessed without considering the quantity of the quality attribute (for API-related quality attributes); whereas criticality assessment with respect to safety and immunogenicity takes into account actual levels present and clinical experience.

The identity and structure of ianalumab are intrinsically linked to its biological activity, PK/PD, immunogenicity, and safety profile. Therefore, both primary and higher-order structures are considered critical quality attributes. Similarly, potency is directly related to efficacy and considered a critical quality attribute. In establishing the appropriate potency assay for routine release and stability testing, several factors were thoroughly assessed, including method sensitivity and the relationship between ianalumab structure and function as linked to critical quality attributes related to biological activity.

An overview of the critical quality attributes having a moderate to high impact on potency, detailing their impact on potency linked to ianalumab's dual mode of action, is shown in Tables 5-1A to 5-1E. (+) represents a moderate to high impact of the quality attribute; (-) represents no or low impact, EoS refers to end of shelf life.

**Table 5-1A. Summary of potency-related critical quality attributes: Product related variants**

| **Critical Quality Attribute** | **Impact on BAFF-R blockade** | **Impact on ADCC potency** |
|---|---|---|
| **Clipping/fragmentation** | **+** | **+** |
| *Fragments (native):* Fragmentation in the hinge region and at a characterized HC clipping site can reduce BAFF-R blockade and ADCC activity. Primarily induced under severe and prolonged thermal stress, followed by light, oxidative, and basic pH stress. Under normal manufacturing and storage conditions, fragments and HC clipping are consistently low (up to approx. 2.5% and 3.7% respectively at DP EoS) and are not linked to significant impacts on potency. | | |
| Thermally-stressed material with increased fragments and HC clipping (above approx. 9.5% and 8.8% respectively) accompanied by other concomitant degradations moderately impacts potency. Fab-Fc fragments reduce blockade of BAFF-R and potency due to lower avidity. Fab or F(ab')2 fragments | | |
| without Fc domain abrogate ADCC. Isolated fragments from stressed samples or enzymatically generated demonstrate decreased potency compared to monomeric species. | | |
| *Fragments (non-native):* Fragmentation between arginine at position 56(R56) and serine at position 57 (S58) in the CDR2 loop of the heavy chain (HC) of ianalumab can have a moderate to minimal impact on blockade of BAFF-R. Under normal manufacturing and storage conditions, observed cleavage levels between R56/S57 are not linked to significant potency impacts. | | |
| Harsh thermal-stressed material with increased cleavage levels (<10%), accompanied by other concomitant degradations, moderately to minimally effect blockade of BAFF-R. Further reductions in potency are observed in ADCC assays due to modifications in the Fc domain. | | |
| **Aggregation (HMWs)** | **+** | **+** |
| Aggregation may reduce BAFF-R blockade and ADCC activity by obstructing binding domains. lanalumab aggregation is found as dimers with no larger species (e.g., trimers, tetramers or more complex oligomeric structures) observed. Primarily induced under harsh and prolonged light stress and, to a lesser extent, under thermal and basic pH stress. Under normal manufacturing and storage conditions, dimer levels are consistently low (up to approx. 0.7% at DP EoS) and are not linked to significant impacts on potency. | | |
| Photo-stressed material with increased dimers (above approx. 5.9%) and other concomitant degradations shows a moderate to high impact on potency. Forced degradation studies also show a moderate to high impact on blockade of BAFF-R at increased dimer levels (<15%) upon harsh thermal and light stress. | | |
| **Deamidation / isomerization in the constant region** | **-** | **+** |
| Deamidation of asparagine (N) at position 332 to aspartate (D) in the Fc region reduces ADCC activity by altering FcγRIIIa binding. Primarily induced under severe and prolonged thermal stress. Under normal manufacturing and storage conditions, N332D levels consistently low (up to approx. 0.8% at DP EoS) and are not linked to significant impacts on potency. Worst-case stability modeling, based on advanced Arrhenius-derived kinetics, confirmed robust predictability, with an upper tolerance limit of 3.24% at 36 months. Degradation studies performed under severe and prolonged thermal stress conditions (40°C) demonstrated a consistent reduction in ADCC potency to below 70 % relative activity when N332D levels exceeded approximately 22.9%. Since BAFF-R blockade is independent of Fc functionality, only ADCC is responsive to this modification. | | |
| **Methionine oxidation in the CDR** | **+** | **+** |
| Methionine oxidation, particularly at residue 97 in the LC near the binding interface to BAFF-R, may reduce BAFF-R blockade and ADCC activity. Under normal manufacturing and storage conditions, M97 oxidation levels are consistently low (up to approx. 3.8% at DP EoS) and are not linked to significant impacts on potency. Worst-case stability modeling, based on advanced Arrhenius-derived kinetics, confirmed robust predictability, with an upper tolerance limit of 4.89 % at 36 months. | | |
| Oxidized material upon severe peroxide stress with increased M97 oxidation (above approx. 61.9%) and other concomitant degradations shows a moderate to high impact on potency. | | |
| **Tryptophan oxidation in the CDR** | **+** | **+** |
| Tryptophan oxidation, particularly at HC residues: W104, within the binding interface to BAFF-R, and W59, in its vicinity; may reduce BAFF-R blockade and ADCC activity. Primarily induced under severe and prolonged light stress. Under normal manufacturing and storage conditions, W104 and W59 oxidation levels consistently low (up to approx. 2.9% at DP EoS; modeled worst-case upper tolerance limit of 3.07% at 36 months) and are not linked to significant impacts on potency. | | |
| Photostressed material with increased W104 and W59 oxidation (above approx. 15.8%) and other concomitant degradations showed a moderate to high impact on potency in competitive ELISA for BAFF-R binding, as well as in ADCC and antiproliferation assays. | | |
| **Mannosylation (High mannose N-glycans; Fc)** | **-** | **+** |
| High mannose N-glycans may reduce ADCC activity. If these species were more abundant in ianalumab, potency could decrease to less than 50%, as thoroughly investigated by *in vitro* glycoengineering tools. Its abundance is defined by the manufacturing cell line and culture conditions. | | |
| In ianalumab, the consistent low levels of high mannose N-glycans (up to approximately 4%) are not linked to significant impacts on potency. Since BAFF-R blockade is independent of Fc functionality, only ADCC is responsive to this modification. | | |
| **N-glycan site occupancy (Fc)** | **-** | **+** |
| N-glycan site occupancy is crucial for ADCC activity, as the absence of N-glycans (e.g., at N304) abrogates ADCC. It is defined by the manufacturing cell line and culture conditions. In ianalumab, consistently marginal levels of antibodies lacking N-glycans (up to approx. 0.7%) do not impact potency. Since BAFF-R blockade is independent of Fc functionality, only ADCC is responsive to this modification. | | |
| **N-glycan galactosylation (Fc)** | - | + |
| N-glycan galactosylation may enhance ADCC activity. If these species were more abundant in ianalumab, potency could increase beyond 100%, as thoroughly investigated by *in vitro* glycoengineering tools. Its abundance is defined by the manufacturing cell line and culture conditions. | | |
| In ianalumab, consistent levels of N-glycan galactosylation (up to approximately 40%) are not linked to | | |
| significant impacts on potency. Since BAFF-R blockade is independent of Fc functionality, only ADCC is responsive to this modification. | | |

**Table 5-1B. Summary of potency-related critical quality attributes: Process related impurities and contaminants**

| **Critical Quality Attribute** | **Impact on BAFF-R blockade** | **Impact on ADCC potency** |
|---|---|---|
| **Bioburden** | **+** | **+** |
| Bioburden represents the total number of viable micro-organisms prior to sterilization. Ianalumab could potentially be modified by microbial enzymes resulting in decreased or increased biological activity. | | |
| **Protein A** | **+** | **+** |
| Protein A may have a high impact biological activity through interference with Fc receptor binding of ianalumab or by inducing oligomerization of ianalumab. | | |
| **Sterility** | **+** | **+** |
| Non-sterile primary packaging could potentially result in decreased or increased biological activity of the drug product. | | |

**Table 5-1C. Summary of potency-related critical quality attributes: Particles**

| **Critical Quality Attribute** | **Impact on BAFF-R blockade** | **Impact on ADCC potency** |
|---|---|---|
| **Visible particles** | **+** | **+** |
| Visible particles may have a very high influence on biological activity as species with increased or decreased potency are possible. | | |
| **Subvisible particles** | **+** | **+** |
| Subvisible particles may have a very high influence on biological activity as species with increased or decreased potency are possible. | | |

**Table 5-1D. Summary of potency-related critical quality attributes: Strength and composition**

| **Critical Quality Attribute** | **Impact on BAFF-R blockade** | **Impact on ADCC potency** |
|---|---|---|
| **pH - i.v. and s.c. administration** | **+** | **+** |
| pH can influence the conformational structure of a protein, its physical and chemical degradation, and thereby may moderately impact its biological activity. | | |

**Table 5-1E. Summary of potency-related critical quality attributes: Appearance and description**

| **Critical Quality Attribute** | **Impact on BAFF-R blockade** | **Impact on ADCC potency** |
|---|---|---|
| **Container closure integrity** | **+** | **+** |
| Within a non-sterile primary packaging, the drug product could potentially be modified resulting in decreased or increased biological activity. | | |
| **Color** | **+** | **+** |
| Color is a non-specific and insensitive indicator for product changes and has a moderate impact on biological activity. | | |
| **Turbidity/clarity** | **+** | **+** |
| Turbidity may indicate the presence of sub-visible particles and/or protein aggregation, or changes in composition which may impact biological activity. No significant change of turbidity has been observed for ianalumab drug substance and drug product on stability or during formulation development studies. | | |

As detailed in Tables 5-1A to 5-1E, the ADCC activity of ianalumab is influenced or potentially influenced by all potency-relevant critical quality attributes, reflecting its superiority in terms of sensitivity to potential changes in potency-related quality attributes. In this context, ADCC assays offers robust potency control to maintain the safety and efficacy profile of ianalumab, as it can effectively monitor changes in relevant quality attributes impacting potency.

An overview of the critical quality attributes having or potentially having a moderate to high impact on PK/PD of ianalumab is shown in Tables 5-2A to 5-2E.

**Table 5-2A. Summary of PK/PD-related critical quality attributes: Product related variants**

| **Quality Attribute** |
|---|
| **Clipping/fragmentation** |
| *Fragments (native):* detectable using analytical techniques such as Size Exclusion Chromatography (SEC), non-reducing Capillary Electrophoresis-Sodium Dodecyl Sulfate (CE-SDS), intact Mass Spectrometry (LC-MS), and peptide mapping with MS detection. |
| Fragmentation is primarily induced under severe and prolonged thermal stress, followed by light, oxidative, and basic pH stress. The primary fragmentation site is in the hinge region, leading to the formation of ianalumab species missing one Fab arm and the corresponding Fab fragment. Further fragmentation can disrupt peptide bonds or disulfide bridges resulting in light or heavy chain fragments. Due to their significantly altered structure, these fragments can exhibit different binding properties or clearance rates, impacting PK/PD. Due to the lack of the Fc part, the Fab fragment cannot bind to FcRn. Consequently, the impact on PK/PD is very high. |
| Under normal manufacturing and storage conditions, fragments and HC clipping are consistently low (up to approx. 2.5% and 3.7% respectively at DP EoS). |

| **Aggregation (HMWs)** |
|---|
| Aggregation: characterized by non-reducing CE-SDS and by the formation of non-reducible thioether-bridged heavy-heavy or heavy-light chains, as characterized by reducing CE-SDS and mass spectrometry. |
| Aggregation can affect biding to the neonatal Fc receptor (FcRn) compared to monomers, depending on the nature of the aggregation. Dimeric species can exhibit different PK/PD profiles due to altered biding behavior to their epitopes and subsequent receptor-mediated endocytosis. Additionally, dimeric forms of IgGs have shown increased retention time on FcRn affinity columns compared to monomers, indicating potential changes in PK. The potential influence of aggregation on PK/PD is considered very high as it could lead to altered clearance. |
| Under normal manufacturing and storage conditions, dimer levels are consistently low (up to approx. 0.7% at DP EoS) and are not linked to significant impacts on potency. |

| **Methionine oxidation in the constant region** |
|---|
| Methionine oxidation: characterized using peptide mapping with MS detection Methionine oxidation, particularly at M259 in the constant region of both heavy chains could potentially cause faster clearance and therefore its influence on PK/PD may be high. Under normal manufacturing and storage conditions, M259 oxidation levels are consistently low (up to approx. 5%) and are not linked to significant impacts on PK/PD. |

| **Mannosylation (High mannose N-glycans; Fc)** |
|---|
| Mannosylation. detectable after cleavage and derivatization, using normal phase liquid chromatography with fluorescence detection |
| High mannose N-glycans have been demonstrated to have faster clearance rates compared to IgGs bearing native complex-type glycans. Therefore, substantial changes in high-mannose glycan content are expected to impact PK, although variations of up to 10% in mannosylation levels have shown no measurable effect on PK. In ianalumab, the consistent low levels of high mannose N-glycans (up to approximately 4%) are not linked to significant impacts on PK/PD. |

**Table 5-2B. Summary of PK/PD-related critical quality attributes: Process related impurities and contaminants**

| **Critical Quality Attribute** |
|---|
| **Bioburden** |
| lanalumab could potentially be modified by microbial enzymes resulting in decreased or increased PK/PD. The influence on PK/PD may be very high. |

| **Host cell proteins (HCPs)** |
|---|
| A host cell protein may bind to ianalumab and affect its PK. The influence on PK/PD may be moderate. |

| **Protein A** |
|---|
| Protein A may influence the pharmacokinetics of ianalumab and its impact may be high. |

| **Sterility** |
|---|
| Non-sterile primary packaging could potentially lead to the drug product being modified resulting in decreased or increased PK/PD. Its impact could be very high. |

**Table 5-2C. Summary of PK/PD-related critical quality attributes: Particles**

| **Critical Quality Attribute** |
|---|
| **Visible particles** |
| Visible particles may have a very high influence on the PK/PD as species with increased or decreased potency are possible. |

| **Subvisible particles** |
|---|
| Subvisible particles may have a very high influence on PK/PD as species with increased or decreased potency are possible. |

**Table 5-2D. Summary of PK/PD-related critical quality attributes: Strength and composition**

| **Critical Quality Attribute** |
|---|
| **API concentration** |
| API concentration determines the correct dosage and thus can have a high influence on PK/PD. |

| **pH - s.c. administration** |
|---|
| pH can result in differently charged protein molecules which might moderately impact the PK/PD profile after administration. |

**Table 5-2E. Summary of PK/PD-related critical quality attributes: Appearance and description**

| **Critical Quality Attribute** |
|---|
| **Container closure integrity** |
| PK/PD may be compromised through product leakage or modification of the drug product through non-sterile primary packaging. The impact could be very high. |

| **Extractable volume** |
|---|
| Extractable volume is connected with correct dosage and thus its influence on PK/PD may be high. |

| **Color** |
|---|
| Color is a non-specific and insensitive indicator for product changes and has a moderate impact on PK/PD. |

| **Turbidity/clarity** |
|---|
| Turbidity can principally be an unspecific indicator for the presence of sub-visible particles and/or protein aggregates, or changes in composition, which itself might have an impact on PK/PD of the therapeutic protein. No significant change of turbidity has been observed for ianalumab drug substance and drug product on stability or during formulation development studies. |

| **Osmolarity - s.c. administration** |
|---|
| Osmolarity may impact PK/PD of a s.c. administered biologic. |

An overview of the critical quality attributes having or potentially having a moderate to high impact on immunogenicity of ianalumab is shown in Tables 5-3A to 5-3E.

**Table 5-3A. Summary of immunogenicity-related critical quality attributes: Product related variants**

| **Quality Attribute** |
|---|
| **Aggregation (HMWs)** |
| Anti-drug antibody-mediated immunogenic responses may occur due to aggregation with significant chemical modifications or altered antigen presentation. However, dimers are generally considered of low risk, as immunogenicity is primarily linked to higher-order aggregates. |
| Under normal manufacturing and storage conditions, dimer levels are consistently low (up to approx. 0.7% at DP EoS). |

**Table 5-3B. Summary of immunogenicity-related critical quality attributes: Process related impurities and contaminants**

| **Critical Quality Attribute** |
|---|
| **Bioburden** |
| Bioburden can be assessed using the microbial enumeration test (MET) consisting of a Total Aerobic Microbial Count (TAMC) and a Total combined Yeasts and Molds Count (TYMC) procedure. It represents the total number of viable micro-organisms prior to sterilization. The residual microbial contaminants could act as adjuvants increasing drug immunogenicity and may therefore have a high influence on immunogenicity of drug product. |

| **Endotoxin** |
|---|
| Endotoxin, assessed for example using the bacterial endotoxin test (BET), may induce systemic inflammation and sepsis. The presence of endotoxin may augment the immunogenic risk of biotherapeutics. The influence of endotoxin on immunogenicity is very high. |

| **Mycoplasma** |
|---|
| Mycoplasma's influence on immunogenicity is very high as mycoplasma can act as adjuvant and induce unwanted immune response. Several species are known to be pathogenic in humans. |

| **Viral impurities** |
|---|
| The influence of viral impurities on immunogenicity is very high. |

| **Residual host cell DNA** |
|---|
| DNA can act as adjuvant and modify immunogenicity of ianalumab. The influence on immunogenicity may be very high. |

| **Host cell proteins (HCPs)** |
|---|
| Host cell proteins might be immunogenic by themselves and/or might act as adjuvants, thereby increasing the risk of immunogenicity directed against ianalumab. |

| **Sterility** |
|---|
| Non-sterile primary packaging could potentially lead to the drug product being modified resulting in decreased or increased immunogenicity. Its impact may be very high. |

| **Leachables and extractables** |
|---|
| Leachables may increase the immunogenicity of the drug product, having a moderate impact, by interaction with excipients or the active pharmaceutical ingredient. |

**Table 5-3C. Summary of immunogenicity-related critical quality attributes: Particles**

| **Critical Quality Attribute** |
|---|
| **Visible particles** |
| Visible particles may have a very high influence on immunogenicity. Immunogenic responses to protein particles with significant chemical modifications or foreign visible particles can be life-threatening. |

| **Subvisible particles** |
|---|
| Sub-v, or SECparticles may have a very high influence on immunogenicity. Immunogenic responses to protein particles with significant chemical modifications or foreign sub-visible particles can be life-threatening. Presence of visible particles by visual inspection is a general compendial requirement for parenteral dosage forms. Particles larger than approximately 100 um are generally classified as "visible particles" |

**Table 5-3D. Summary of immunogenicity-related critical quality attributes: Strength and composition**

| **Critical Quality Attribute** |
|---|
| **pH - i.v. and s.c. administration** |
| pH can indirectly influence the conformational structure of a protein and thereby have influence its immunogenicity. |

**Table 5-3E. Summary of immunogenicity-related critical quality attributes: Appearance and description**

| **Critical Quality Attribute** |
|---|
| **Container closure integrity** |
| Within a non-sterile primary packaging, the drug product may be modified resulting in increased immunogenicity. |

| **Color** |
|---|
| The impact of color on immunogenicity is moderate as it is a non-specific and insensitive indicator for product changes. |

| **Turbidity/Clarity** |
|---|
| Turbidity (measured by ratio turbidimetry according to compendial methods) can principally be an unspecific indicator for the presence of sub-visible particles and/or protein aggregates, or changes in composition, which itself might have an impact immunogenicity of the therapeutic protein. No significant change of turbidity has been observed for ianalumab drug substance and drug product on stability or during formulation development studies. |

An overview of the critical quality attributes having or potentially having a moderate to high impact on safety of ianalumab is shown in Tables 5-4A to 5-CD.

**Table 5-4A. Summary of safety-related critical quality attributes: Process related impurities and contaminants**

| **Critical Quality Attribute** |
|---|
| **Bioburden** |
| The impact of bioburden on safety is very high as it is directly linked to possible life-threatening adverse events. |

| **Endotoxin** |
|---|
| The presence of endotoxin may lead to life threatening adverse events when administered to humans. |

| **Mycoplasma** |
|---|
| Mycoplasma may cause human disease and therefore its influence on safety is very high. |

| **Viral impurities** |
|---|
| Viruses may cause serious human disease and therefore its influence on safety is very high. |

| **Residual host cell DNA** |
|---|
| Residual host cel DNA my highly influence safety as there is a theoretical risk of oncogenicity or infectivity with eukaryotic DNA. |

| **Host cell proteins (HCPs)** |
|---|
| The general impact on safety is considered high as biologically active HCPs might lead to advese events. |

| **Sterility** |
|---|
| The impact of sterility on safety is very high as directly linked to possible life-threatening adverse events. |

| **Leachables and extractables** |
|---|
| If the presence of toxic, including mutagenic or sensitizing/irritating, substances cannot be excluded the potential impact on safety is considered as high. |

**Table 5-4B. Summary of safety-related critical quality attributes: Particles**

| **Critical Quality Attribute** |
|---|
| **Visible particles** |
| Life-threatening adverse events by immunogenic response to protein particles with significant chemical modifications or visible foreign particles are possible. |

| **Subvisible particles** |
|---|
| Life-threatening adverse events by immunogenic response to protein particles with significant chemical modifications or sub-visible foreign particles are possible. |

**Table 5-4C. Summary of safety-related critical quality attributes: Appearance and description**

| **Critical Quality Attribute** |
|---|
| **Container closure integrity** |
| The impact of container closure integrity on safety is very high as directly linked to sterility. |

| **Color** |
|---|
| The impact of color on safety is moderate as it is a non-specific and insensitive indicator for product changes. |

| **Turbidity/Clarity** |
|---|
| Turbidity can principally be an unspecific indicator for the presence of sub-visible particles and/or protein aggregates, or changes in composition, which itself might have an impact safety of the therapeutic protein. No significant change of turbidity has been observed for ianalumab drug substance and drug product on stability or during formulation development studies. |

| **Osmolarity - s.c. administration** |
|---|
| The impact of osmolarity is considered moderate as the possible adverse events are reversible and manageable by clinical treatment. |

### Example 6. Detection of ianalumab variants in drug substance batches

Assessment of ianalumab's purity employs three validated analytical technologies that reliably separate the main product from variants and impurities, and possess proven stability-indicating capabilities:
1. *Charge heterogeneity by Capillary Zone Electrophoresis (CZE):* This technique separates proteins based on their charge-to-size ratio in an electric field, effectively assessing charge variant heterogeneity by distinguishing between acidic and basic variants relative to the main ianalumab variant. The method reports the percentage of the main variant and monitors acidic variants for potential alterations in charge heterogeneity, ensuring process consistency. Changes in acidic variants can monitor increases in major ianalumab-related variants, including fragmentation and deamidation/isomerization in the constant region, both critical quality attributes with potential impact on biological activity.
*2. Purity by Sodium Dodecyl Sulfate Capillary Gel Electrophoresis under non-reducing conditions (CE-SDS nr):* This technique uses mild denaturing conditions to separate size variants relative to the primary monomeric form of ianalumab (main peak). It separates size-related variants such as incomplete antibodies, fragments, and covalently linked aggregates. The method reports the percentage of the main peak as a measure of purity relative to the overall size variants observed. It also directly reports the abundance of fragments considered a critical quality attribute.
*3. Purity by Sodium Dodecyl Sulfate Capillary Gel Electrophoresis under reducing conditions (CE-SDS r):* This technique chemically reduces disulfide bonds to separately analyze ianalumab's heavy and light chains. It confirms the structural integrity of these subunits and measures degradation products and by-products based on molecular size. The method reports the relative abundance of integral heavy and light chains as a percentage of purity. Size-related variants, such as clipping/fragmentation in light or heavy chains, covalently linked aggregates, and by-products like non-glycosylated heavy chains, are detected. Variations in purity percentage serve as an indicator and control measure for increases in these size-related variants, which are considered critical quality attributes due to their potential impact on biological activity.

These analytical techniques are useful for ensuring product consistency and providing a comprehensive quality assessment. Utilized in routine batch release and stability testing, they serve as process Performance Indicators (Pls) within ianalumab's control strategy. Furthermore, the reported 'purity' by these methodologies effectively and indirectly monitors product-related variants, including critical quality attributes, thereby minimizing risks to patient safety and ensuring product efficacy.

The degradation pathways of ianalumab have been thoroughly elucidated using CZE, CE-SDS nr, and CE-SDS r analytical methods, supported by several orthogonal characterization techniques. Comprehensive characterization was performed on product-related variants resolved by these methods, particularly using stressed ianalumab material, to conclusively establish their identification and impact.

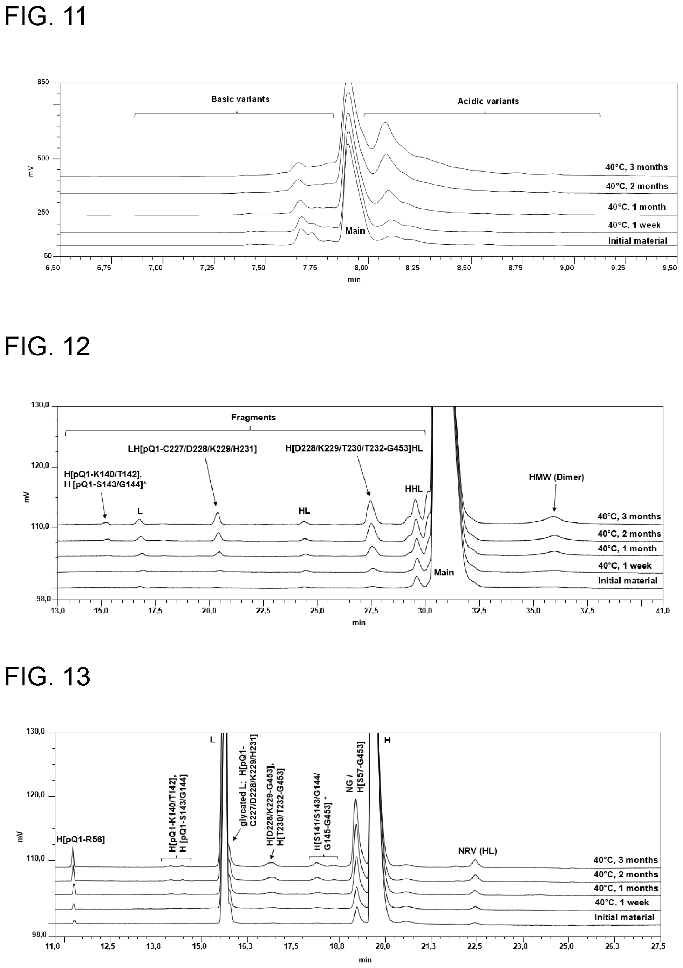

FIGS. 11-13 show representative purity profiles of ianalumab material obtained using CZE, CE-SDS nr, and CE-SDS r on drug substance material subjected to thermal stress (40°C for up to three months). These profiles, along with chromatograms and graphical peak assignments, illustrate degradation pathways observed in ianalumab. The overlay chromatograms highlight product stability and potential changes, reinforcing the robustness of these methods in ensuring consistent product quality.

In this context, the purity tests using CZE, CE-SDSnr, and CE-SDSr were further complemented by the assessment of high molecular weight (HMW) species using Size Exclusion Chromatography (SEC) in routine release and stability testing. Notably, the primary pathway leading to HMW species in ianalumab is dimerization.

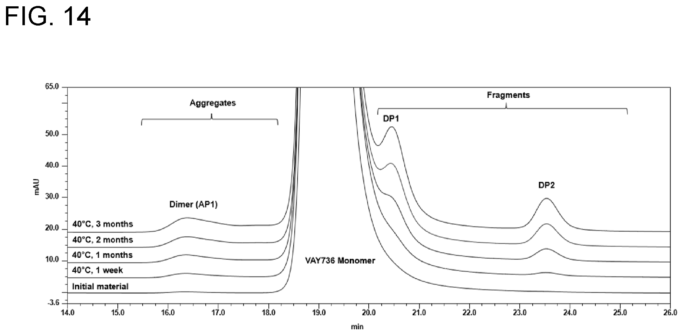

FIG. 14 shows representative profiles of ianalumab obtained using SEC on drug substance material subjected to thermal stress (40°C for up to three months). These profiles, along with graphical peak assignments, illustrate SEC's resolution capability for HMWs and its limited resolution for fragments, impacting its overall monomer purity assessment capabilities. In contrast, CE-SDS nr and CE-SDS r were superior in assessing size variants, including fragments and monomeric species, surpassing the performance of SEC in these aspects.

As a result of the observed limitations of SEC method itself in the quantitative analysis of monomer purity and fragments, the control strategy for ianalumab employed CE-SDS nr and CE-SDS r to robustly control monomer purity and fragments under both native and denaturing conditions. SEC was used for monitoring HMWs in ianalumab.

By integrating validated methodologies such as CZE, CE-SDS nr, and CE-SDS r, along with SEC specifically for HMWs, a robust and comprehensive testing strategy for ianalumab was established.

### Example 7. Characterization of the N-glycan profile of ianalumab

lanalumab was produced in engineered CHO-C8TD cells expressing GDP-6-deoxy-D-lyxo-4-hexulose reductase (RMD).

As an afucosylated IgG1 antibody, ianalumab features well-characterized biantennary complex N-glycan structures with terminal galactose heterogeneity and low levels of high-mannose structures, typical of monoclonal antibody therapeutics. Comprehensive characterization involved cleaving N-glycans from ianalumab and analysis by normal phase liquid chromatography (NP-HPLC), followed by identification via mass spectrometry (MS).

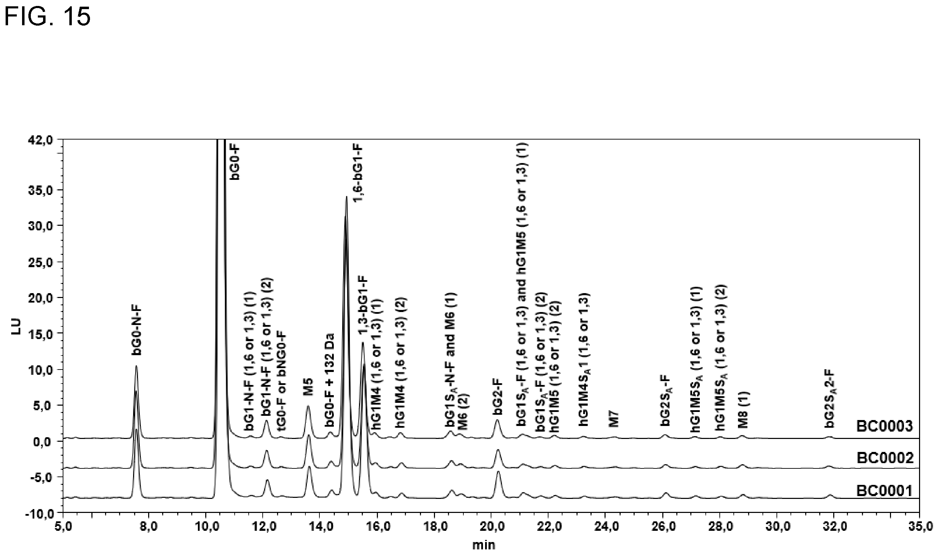

FIG. 15 shows the N-glycan distribution of three representative ianalumab drug substance (DS) batches, reflecting product quality. For more details, Table 7 provides a summary of the relative amounts of all identified N-glycan species and their identities confirmed by MS.

lanalumab's N-glycans were confirmed to be non-fucosylated, primarily bi-antennary (i.e., A2, A2G1, and A2G2) and mono-antennary glycans (i.e., A1 and A1G1). Low levels of high-mannose structures (M5, M6, M7, and M8) were observed (well below 7%), while sialylated glycans were present at very low levels, near or below the limit of quantitation (0.10%).

**Table 7. N-glycans identified in ianalumab DS batches with relative abundance and identity evaluation by MS**

| **Peak** | **Glycan species** | | **Peak area [%]** | | | **Expected mass [Da]** | **Observed mass [Da]** |
|---|---|---|---|---|---|---|---|
| | **Oxford nomenclature** | **Alternate nomenclature** | **DS-1** | **DS-2** | **DS-3** | | |
| 1 | A1 | bG0-N-F | 2.4 | 3.1 | 3.2 | 1233.5 | 1233.5 |
| 2 | A2 | bG0-F | 70.6 | 72.0 | 71.3 | 1436.6 | 1436.6 |
| 3 | A1G1 | bG1-N-F (1,6 or 1,3) (1) | 0.11 | 0.15 | 0.17 | 1395.5 | 1395.5 |
| 4 | A1G1 | bG1-N-F (1,6 or 1,3) (2) | 0.83 | 0.95 | 1.0 | 1395.5 | 1395.5 |
| 5 | A3 or A2B | tG0-F or bNG0-F (1) | 0.12 | 0.13 | 0.13 | 1639.6 | 1639.6 |
| 6 | M5 | M5 | 1.4 | 1.7 | 1.8 | 1354.5 | 1354.5 |
| 7 | A2 + 132Da | bG0-F + 132Da | 0.36 | 0.40 | 0.39 | 1568.6 | 1568.6 |
| 8 | A2[6]G1 | 1,6 bG1-F | 13.1 | 11.3 | 11.7 | 1598.6 | 1598.6 |
| 9 | A2[3]G1 | 1,3 bG1-F | 5.9 | 5.1 | 5.2 | 1598.6 | 1598.6 |
| 10 | M4A1G1 | hG1M4 (1,6 or 1,3) (1) | 0.27 | 0.30 | 0.32 | 1557.6 | 1557.6 |
| 11 | A2G1 or A1BG1 | bG1-F or bNG1 | 0.09 | 0.09 | 0.09 | 1598.6 | 1598.6 |
| 12 | M4A1G1 | hG1M4 (1,6 or 1,3) (2) | 0.24 | 0.30 | 0.33 | 1557.6 | 1557.6 |
| 13 | M6 (1) // A1G1S1 | M6 (1) // bG1S_{A}-N-F (1,6 or 1,3) | 0.42 | 0.45 | 0.47 | 1516.6 // 1686.6 | 1516.6 // 1686.6 |
| 14 | M6 (2) | M6 (2) | 0.25 | 0.28 | 0.30 | 1516.6 | 1516.6 |
| 15 | A2G1 +132Da | bG1-F +132Da (1,6 or 1,3) (1) // bG1-F +132Da (1,6 or 1,3) (2) | 0.07 // 0.05 | 0.06 // 0.04 | 0.06 // 0.04 | 1730.7 | 1730.6 |
| 16 | A2G2 | bG2-F | 1.3 | 1.0 | 1.1 | 1760.7 | 1760.7 |
| 17 | A2G1S1 // M5A1G1 | bG1S_{A}-F (1,6 or 1,3) // hG1M5 (1,6 or 1,3) (1) | 0.37 | 0.33 | 0.34 | 1889.7 // 1719.6 | 1889.7 // 1719.6 |
| 18 | A2G1S1 | bG1S_{A}-F (1,6 or 1,3) | 0.13 | 0.11 | 0.10 | 1889.7 | 1889.7 |
| 19 | M5A1G1 | hG1M5 (1,6 or 1,3) (2) | 0.14 | 0.17 | 0.18 | 1719.6 | 1719.6 |
| 20 | M4A1G1S1 | hG1M4S_{A}1 (1,6 or 1,3) | 0.13 | 0.14 | 0.15 | 1848.7 | 1848.7 |
| 21 | M7 | M7 | 0.11 | 0.12 | 0.13 | 1678.6 | 1678.6 |
| 22 | A2G2S1 | bG2S_{A}-F (1,6 or 1,3) | 0.26 | 0.21 | 0.21 | 2051.8 | 2051.8 |
| 23 | M5A1G1S1 | hG1M5S_{A} (1,6 or 1,3) (1) | 0.11 | 0.09 | 0.09 | 2010.7 | 2010.7 |
| 24 | M5A1G1S1 | hG1M5S_{A} (1,6 or 1,3) (2) | 0.09 | 0.10 | 0.11 | 2010.7 | 2010.7 |
| 25 | M8 | M8 (1) | 0.16 | 0.18 | 0.18 | 1840.7 | 1840.7 |
| 26 | M8 | M8 (2) | 0.04 | 0.05 | 0.05 | 1840.7 | 1840.7 |
| 27 | A2G2S2 | bG2S_{A}2-F | 0.14 | 0.11 | 0.10 | 2342.9 | 2342.9 |
| n.a. | n.a. | Sum of Unknown ¹ | 0.86 | 0.86 | 0.89 | n.a. | n.a. |

| | | | | | | | |
|---|---|---|---|---|---|---|---|
| ¹Sum is calculated as: 100% - all identified species (including peaks <LOQ); LOQ: 0.10% | | | | | | | |

### Example 8. The impact of ianalumab's N-glycans on ADCC activity

The essential role of N-glycans in the Fc region of IgG1 monoclonal antibodies, particularly regarding their effector functions, is well established. Removing the proximal fucose from N-glycans significantly enhances binding affinity to FcγRIIIa and increases ADCC activity both *in vitro* and *in vivo,* without affecting binding to other Fcγ receptors (FcγRs) or other effector functions. This modification can lead to a 50- to 100-fold increase in binding affinity for FcyRllla, thereby substantially enhancing ADCC activity.

During the development of ianalumab, the influence of N-glycans on its mode of action, particularly its ADCC mechanism, which is uniquely responsive to this attribute, was thoroughly investigated. Ianalumab's ability to mediate rapid and profound B cell depletion through increased ADCC activity has been confirmed *in vitro* using human B cells from peripheral blood mononuclear cells and in similar assays with human and cynomolgus monkey B cells. Further studies on human B cells in whole blood confirmed this depletion activity.

Compared to other B-cell-targeted therapeutic antibodies, afucosylation in ianalumab substantially enhances binding affinity (K_{D}) to FcγRIIIa, observed in the low nanomolar range (10⁻⁹ M), whereas IgG₁ antibodies typically exhibit binding affinities in the mid-nanomolar range (10⁻⁷ M). This enhancement results in increased and consistent NK cell activation, cytokine production, and B-cell depletion.

To see the impact of ianalumab N-glycosylation patterns on ADCC activity, a regression model was developed using *in vitro* glycoengineering (IVGE) tools. Major glycan species of ianalumab, including mannosylated (high-mannose N-glycans), non-galactosylated and galactosylated species, were selectively generated and enriched. Their impact on ADCC activity was assessed using a design of experiments approach, enabling detailed evaluation of the relationship between N-glycosylation patterns and ADCC activity as measured by the validated ADCC cell-based bioassay used for release and stability testing of ianalumab.

The three main glycoforms present in the drug substance (DS) were mixed according to design of experiment (DoE) principles to sample the experimental space s. Seven samples were generated to cover the entire design space. All samples, including the initial DS, the primary samples (Max_HM, Max_bG0-F, Max_bG-F) and the seven mixing samples (sample 1 - 7) were analyzed by N-glycan mapping, target binding ELISA, ADCC and anti-proliferation assays. The bG2-F was measured in duplicate to reduce assay variability for highly potent samples, the final number of samples was 12.

The findings revealed that mannosylation and galactosylation have inverse and direct impacts, respectively, on ADCC activity compared to the afucosylated baseline species (non-galactosylated, non-sialylated glycan structures) predominant in ianalumab. Specifically, increased galactosylation levels were associated with enhanced ADCC activity, while higher levels of mannosylation were associated with reduced ADCC activity.

The negative relationship observed between mannosylation and ADCC was unexplored in prior studies showing that increasing percentage of high mannose structures in fucosylated IgG1 monoclonal antibodies corresponded with *increased* ADCC activity. (See Pace et al., Biotechnol. Prog., 2016, Vol.32, No.5. at page 1185 and Figure 2B).

The findings revealed that mannosylation (high-mannose N-glycans M5-M8) and galactosylation have inverse and direct impacts, respectively, on ADCC activity, compared to the afucosylated baseline species (non-galactosylated, non-sialylated glycan structures) of ianalumab. Specifically, increased galactosylation levels were associated with enhanced ADCC activity, while higher levels of mannosylation were associated with reduced ADCC activity.

This result reveals an important set of criteria to achieve ianalumab's proven ADCC potency range in a new anti-BAFF antibody. For example, a new anti-BAFF-R antibody having 10% high mannose content could exhibit similar ADCC potency as a reference ianalumab antibody having 0% high mannose content, provided that the new anti-BAFF-R antibody had a suitably elevated galactosylation level relative to the reference antibody (in this case, a ~8% higher galactosylation level in the new antibody could compensate for the negative impact on ADCC resulting from its high mannose content).

This impact of high mannose levels on biological activity is both unexpected and relevant to ADCC potency, one of the primary mechanisms of action of ianalumab.

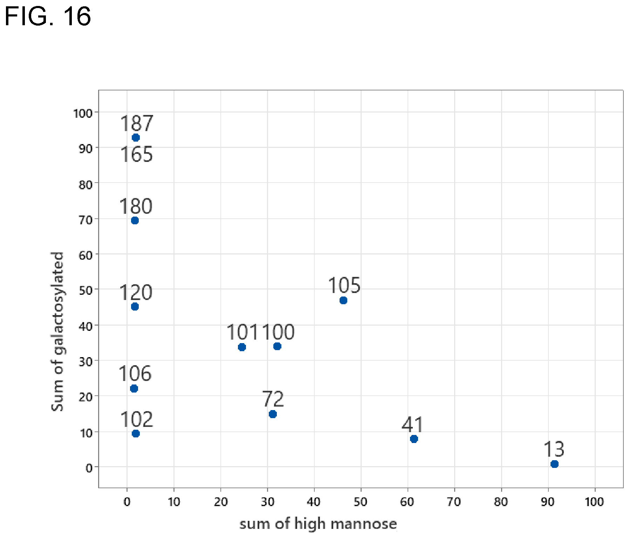

The N-glycan distribution of all 12 IVGE samples was determined by N-glycan mapping. Table 8-1 presents the results observed for *in vitro* glycoengineered samples of ianalumab, detailing the content of mannosylation and galactosylation, along with the corresponding ADCC activity. ADCC activity results are also depicted graphically in FIG. 16.

**Table 8-1 Mannosylation and galactosylation levels of ianalumab in vitro glycoengineered samples and corresponding ADCC activity**

| **Sample** | **Mannosylation (Sum of high-mannose N-glycans) [%]** | **Galactosylation (Sum of galactosylated N-glycans) [%]** | **Relative ADCC biological activity compared to the reference substance [%]** |
|---|---|---|---|
| Control (Initial DS) | 1.7 | 22.1 | 106 |
| Max. high-mannose species ¹ | 91.3 | 0.8 | <50* |
| Min. galactosylation and high-mannose species ¹ | 1.9 | 9.4 | 102 |
| Max. galactosylation_1 ¹ | 2.0 | 92.7 | 187 |
| Mix1 ² | 61.3 | 7.9 | <50* |
| Mix2² | 31.1 | 14.9 | 72 |
| Mix3² | 1.8 | 69.4 | 180 |
| Mix4 ² | 1.8 | 45.2 | 120 |
| Mix5² | 46.3 | 47.0 | 105 |
| Mix6² | 32.1 | 33.9 | 100 |
| Mix7² | 24.6 | 33.8 | 101 |

| | | | |
|---|---|---|---|
| * Non reportable result, below the validated range of the method. ¹ lanalumab samples with varying high-mannose and galactosylated N-glycans were generated using IVGE tools. Cells producing ianalumab were cultured with a glycosylation inhibitor to produce immature N-glycans (M9). Post-treatment with mannosidase, galactosyltransferase, or galactosidase, including standard ianalumab material, resulted in mature N-glycans. This process increased the abundance of glycoforms with high-mannose, non-galactosylated non-fucosylated, and galactosylated non-fucosylated species. ² The main enriched glycoform species were mixed according to design of experiment (DoE) principles to evenly sample the experimental space and generate seven additional samples or mixes. | | | |

For the calculation of the respective sum following N-glycan species were considered, as shown in Table 8-2:

**Table 8-2: Glycoforms counted in each category for IVGE study**

| **Oxford nomenclature** | **Alternate nomenclature** | **Included in calculation of sums used for IVGE study** |
|---|---|---|
| A1G1 | bG1-N-F (1,6 or 1,3) (2) | Sum of galactosylated |
| M5 | M5 | Sum of High Mannose |
| A2[6]G1 | bG1-F (1,6) | Sum of galactosylated |
| A2[3]G1 | bG1-F (1,3) | Sum of galactosylated |
| A1G1S1 / M6 (1) | bG1S_{A}-N-F (1,6 or 1,3) / M6(1) | Sum of galactosylated |
| M6 (2) | M6 (2) | Sum of High Mannose |
| A2G2 | bG2-F | Sum of galactosylated |
| A2G2S1 | bG2S_{A}-F (1,6 or 1,3) | Sum of galactosylated |
| M8 | M8 (1) | Sum of High Mannose |

Of note, samples with varied N-glycan species abundance showed no impact on the BAFF-R blockade and inhibition of the BAFF-mediated signaling mechanism of ianalumab, which occur independently of effector cells or Fc domain functionality.

These results were used to develop a regression model for ADCC activity based on the N-glycan profile. The model was validated by comparing predicted values against actual values, showing no systematic deviations. High determination coefficients (R-sq: 96.24%; predicted R-sq: 93.32%; 10-fold R-sq: 93.25%) indicate excellent model fit and reliability for predicting ADCC activity. In particular, the sum of high-mannose N-glycans and the sum of galactosylated N-glycans are significant predictors of changes in ADCC activity. As such, the model explains 96% of the observed variability in ADCC response and predicts 93% of the variability (10-fold R-sq).

The formula for the prediction of ADCC activity with N-glycan species is given as Formula I: $ADCC activity = 86.83 - \left(0.819\times Sum of high-mannose\right) + \left(1.051\times Sum of galactosylation\right) .$

Tables 8-2 and 8-3 detail the model performance summary for ADCC activity prediction and the statistical significance of its coefficients.

**Table 8-2 Model Performance Summary for ADCC Response Prediction**

| **S** | **R-sq** | **R-sq(adj)** | **PRESS** | **R-sq(pred)** | **AICc** | **BIC** | **10-fold S** | **10-fold R-sq** |
|---|---|---|---|---|---|---|---|---|
| 11.1590 | 96.24% | 95.40% | 1988.45 | 93.32% | 102.21 | 98.44 | 12.9439 | 93.25% |

| | | | | | | | | |
|---|---|---|---|---|---|---|---|---|
| S: Standard deviation of residuals (Standard Error of the Estimate); R-squared (R-sq): Coefficient of Determination for the fit; R-squared Adjusted (R-sq(adj)): Adjusted Coefficient of Determination; PRESS: Predicted Residual Error Sum of Squares; R-squared Predicted (R-sq(pred)): Predicted Coefficient of Determination; AICc: Corrected Akaike Information Criterion; BIC: Bayesian Information Criterion; 10-fold S: Standard deviation of residuals in 10-fold Cross-Validation; 10-fold R-squared (10-fold R-sq): Coefficient of Determination in 10-fold Cross-Validation. | | | | | | | | |

**Table 8-3 Statistical Coefficients and Significance for ADCC Activity Prediction Model**

| **Term** | **Coef** | **SE Coef** | **95% CI** | **T-Value** | **P-Value** | **VIF** |
|---|---|---|---|---|---|---|
| Constant | 86.83 | 8.33 | (67.98; 105.68) | 10.42 | 0.000 | |
| sum of high mannose | -0.819 | 0.141 | (-1.137; -0.500) | -5.81 | 0.000 | 1.51 |
| sum of galactosylated | 1.051 | 0.131 | (0.756; 1.347) | 8.05 | 0.000 | 1.51 |

| | | | | | | |
|---|---|---|---|---|---|---|
| Coef: Coefficient estimate for the term; SE Coef: Standard Error of the Coefficient; 95% CI: 95% Confidence Interval for the Coefficient; T-Value: T-statistic for testing the null hypothesis that the coefficient is zero; P-Value: Probability value for the statistical significance of the coefficient; VIF: Variance Inflation Factor, to assess multicollinearity. | | | | | | |

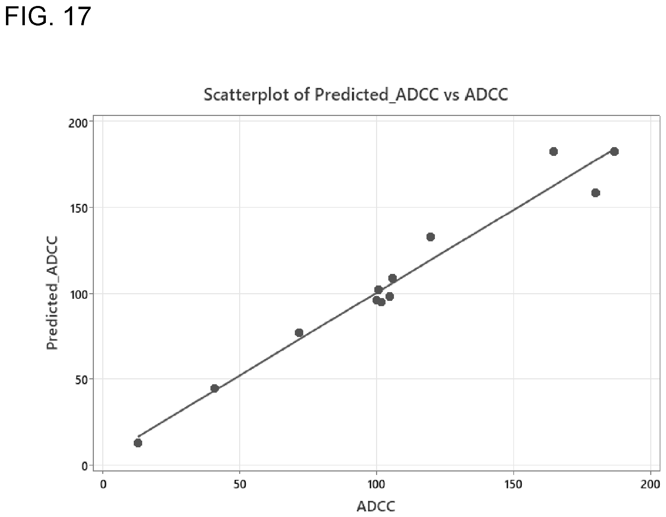

The comparison of predicted ADCC versus measured ADCC activity, as shown in FIG. 17 compares predicted versus measured ADCC activity, illustrating the model's accuracy and reliability. ADCC activity for samples with varying levels of high-mannose and galactosylated N-glycans was measured by the ADCC cell-based bioassay and compared to predicted values using this correlation model. These predicted values considered the sums of high-mannose and galactosylated N-glycans observed in the characterized glycoprofiles for each sample.

### Example 9. Manufacturing process characterization on the N-glycan profile and other variants of ianalumab

Process characterization for ianlaumab manufacturing included data evaluation and a series of studies to categorize the criticality of process parameters and to establish acceptable ranges, to ensure robustness of the manufacturing process, capable of constantly delivering the required product quality. Those studies were carried out utilizing multivariate experiments defined by design of experiment principles (DoE), as well as univariate or worst-case experiments.

The thorough understanding of the N-glycan profile of ianalumab, including critical potency-relevant species such as high-mannose and galactosylated variants, formed the basis for developing of a control strategy that ensures consistent manufacturing quality.

The main-stage bioreactor cultivation primarily defines the N-glycan composition of ianalumab. During upstream manufacturing process (USP) characterization (PC) the impact of various process parameters and stages (e.g. main-stage fed-batch bioprocess, and bulk harvest hold time) on N-glycan species was thoroughly evaluated.

Input parameters were classified based on their impact by comparing observed effects at different settings to predicted values from reference runs, using impact ratios (IR) to reflect observed change rates. These were categorized as follows:
- No relevant impact: no impact (IR up to and including5%), non-relevant (IR above 5% up to and including20%);
- Relevant impact: moderate (IR above 20% up to and including50%), strong (IR above 50% up to and including100%), or unacceptable (IR over 100%).

Parameters with relevant impact over critical N-glycan species or other critical quality attributes were identified as critical process parameters (CPPs, IR > 20%). Their acceptable ranges (AR) were adjusted following characterization.

Within the studied acceptable ranges, all process parameters impacting critical N-glycan species in ianalumab resulted in no, non-relevant, or at most, moderate impacts. No parameter has strong or unacceptable changes in the N-glycan profile when operated within the acceptable ranges.

### Main-stage bioreactor:

A comprehensive Design of Experiments (DoE) characterized the main-stage bioreactor, including seven continuous factors and one categorical factor (scale): seeding viable cell density, pH, temperature at start, feed accuracy relative to initial reactor volume, continuous feeds start criterion, temperature shift criterion, temperature after shift, and scale.

Based on the DoE results, regression models were developed, with regression coefficients indicating a good fit. Specifically, the main galactosylated N-glycan species, bG1-F (1,6), and the sum of high-mannose N-glycan species showed correlation coefficients (R²) of 0.81 and 0.83, respectively. Most process parameters had no or non-relevant impact on the critical N-glycan species in ianalumab. However, in the evaluated characterization range pH had a strong impact on high-mannose N-glycan species, while the 'continuous feeds start criterion' had a moderate impact on both galactosylated and high-mannose N-glycan species, along with other quality attributes like content, charge heterogeneity, aggregation, or fragmentation. These were classified as critical process parameters (CPPs), and their ranges were tightened to ensure acceptable performance across the affected attributes.

Table 9-1 summarizes selected process characterization (PC) results for the main-stage bioreactor, showing impacts on N-glycan species for characterized parameters and the adjusted ARs.

**Table 9-1 Summary of PC results for the main-stage bioreactor parameters**

| **Process Parameter** | **Characterizatio n Range** | **Acceptable Range (AR)** | **Impact on critical N-glycan species** |
|---|---|---|---|
| pH [-] | 6.75 - 7.15 | 6.80 - 7.15 | Relevant impact for high-mannose species; not relevant for galactosylation |
| Continuous feed 0016 Start criterion (capacitance)¹ [pF/ cm] | 1.4 - 7.0 | 2.5 - 7.0 | Relevant impact for high-mannose species; and galactosylation |
| Continuous feed 0016 Start criterion (VCD)¹ [×10⁶ cells/mL] | 1.0 - 4.0 | 1.45 - 4.00² | |

| | | | |
|---|---|---|---|
| ¹ Process can be steered by either VCD or capacitance (which is correlated to VCD). ² CR for VCD adapted and additional digit added (equivalent to AR) based on larger data set for regression model VCD vs. capacitance. | | | |

The DoE main-stage bioreactor study revealed that process parameters pH, VCD at feed start, T after T-shift, feed accuracy and T at start have relevant impact of fragments purity, change variants, glycan structure and content.

### Harvest criteria and bulk harvest hold time:

Further investigations were conducted before primary separation after the main stage. In all cases no, or non-relevant impact was observed for all critical N-glycan species.

It should be additionally noted that, as expected, following the downstream processing (DSP) of ianalumab, no impact was observed on the determination and quantification of N-glycans at any stage. The comprehensive characterization and control of process parameters impacting the N-glycan profile of ianalumab demonstrate the robustness of the manufacturing process design. Maintaining critical parameters within acceptable ranges ensures consistent product quality. This is further confirmed by extensive GMP batch manufacturing experience, including process validation and cell age studies at LIVCA, highlighting the process's control capabilities and the consistency of N-glycan profiles under controlled conditions.

### Storage conditions

Impact of storage conditions on physico-chemical properties/attributes such as conductivity, pH, osmolality, and amino-acid content of all media were determined.

*Thermal stress*: incubation of drug substance at 40C/ 75% RH for 1 week, 1 month, 2 months and 3 months. CE-SDS performed under non-reducing and reducing conditions was used for determination of purity and the amounts of size variants. Thermal stress resulted in increase of the acidic variants, decrease of fragments due to the clips in the hinge region, with a concomitant decrease of the main variant and a minor decrease of the basic variants. Oxidation of methionine was also observed.

*Oxidative stress*: drug substance was incubated with 0.25% hydrogen peroxide at room temperature for 1, 3, 6, and 24h, and then analyzed by SEC, CE-SDS, CZE, RP-HLPC etc. Analysis of oxidative stressed samples showed increase in fragments, oxidation of all eight methionine residues, as well as formation of HHL and L species.

*pH stress*: drug substance was incubated at pH 4.0 and pH 9.0 at room temperature for 1, 3, 7 and 14 days. Analysis of pH stressed samples showed: (a) basic stress resulted in an increase of acidic variants (deamidated variants), precipitation and particle formation; (b) acidic stress: fragmentation of heavy chain, oxidation, and decrease of the main variant.

lanalumab is not prone to aggregation and fragmentation after applied freeze/thaw stress, nor after the applied shaking stress.

*Light stress*: drug substance samples were prepared by exposure to light stress under the 0.1 ICH, 0.5 ICH and 1 ICH conditions. Licht stress resulted in an increase in acidic variants, with concomitant decrease in main and basic variants; oxidation of methionine and tryptophan and histidine residues; clipping of the heavy chain hinge region

Conclusion Storage conditions: elevated temperature and prolonged high pH exposure, and light stress should be avoided.

HCP: Residual CHO host cell protein levels in the ianalumab composition are measured using an enzyme-linked immunosorbent assay (ELISA). This method uses antibodies produced against native antigens of the CHO cell line grown under conditions that mimic the production process conditions of ianalumab. It was determined that for the ianalumab composition, an acceptable range for HCP content is ≤20 ppm, e.g., ≤10 ppm.

### Example 10. Manufacturing process capabilities related to the N-glycan profile of ianalumab

The GMP production of over 30 ianalumab DS batches, representative of commercial quality, demonstrates high N-glycan consistency within established acceptable manufacturing ranges. To evaluate process capability for critical N-glycan species, manufacturing batch data is assessed against acceptable ranges that exclude impacts on safety and efficacy. The ranges are 15.0% to 38.0% for galactosylated N-glycan species and 0% to 7.0% for high-mannose N-glycan species.

For galactosylated species, the lower threshold of ≥15.0% permits a reduction in abundance of no more than 8.1% from the mean baseline level of approximately 23.1%, while the upper threshold of ≤38.0% allows for an increase of up to 14.9%. Within this range, and supported by the robust ADCC potency correlation model, ADCC activity variations between -8.5% and +15.7% remain controlled. These potential ADCC activity variations are deemed acceptable and without clinically meaningful impact.

For high-mannose species, the maximum potential ADCC variability within the evaluation range is below 3.0%, based on a mean baseline level of approximately 3.7% and supported by the robust ADCC potency correlation model. These changes are minimal and not clinically meaningful.

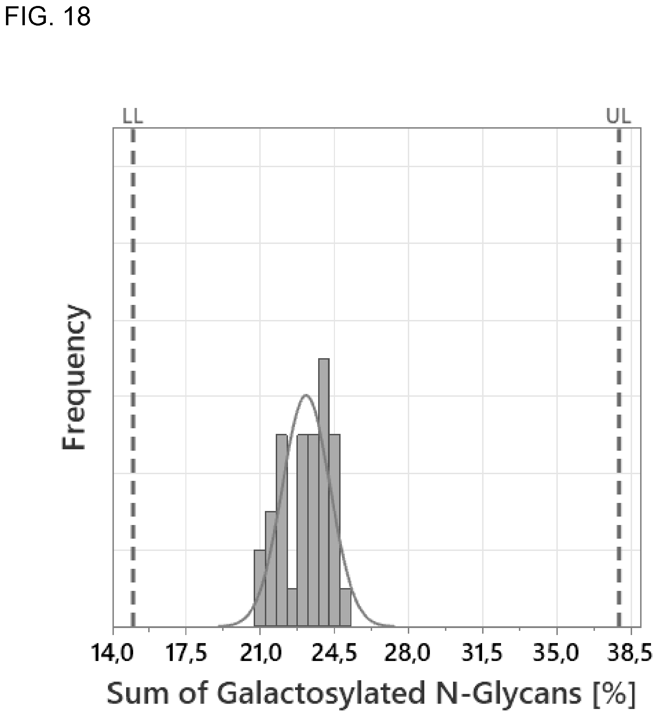

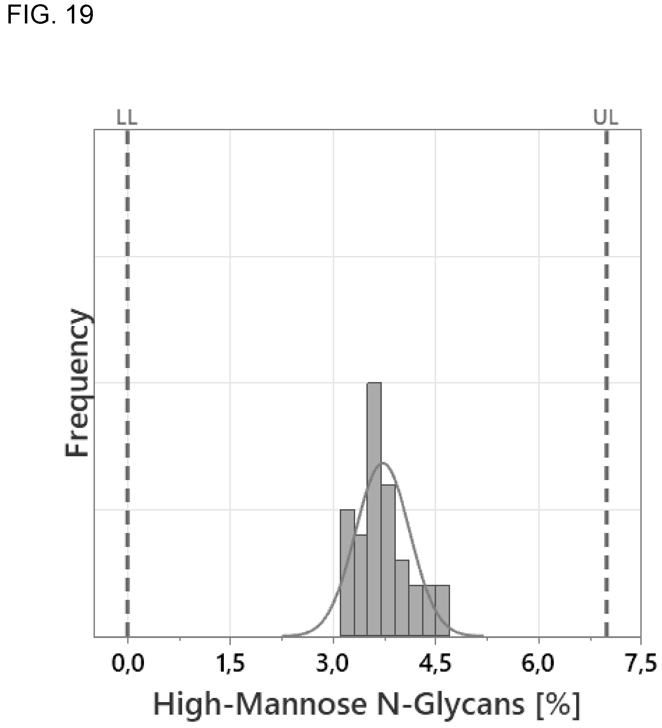

FIG. 18 and FIG. 19 illustrate the distribution results for high-mannose and galactosylated N-glycans, respectively, across manufactured ianalumab DS batches.

The acceptable manufacturing ranges are depicted with dotted red lines for each species. For further details, please refer to Table 10-1, which provides the detailed characterization results for these N-glycan species for each batch.

**Table 10-1 Mannosylation and galactosylation levels in ianalumab DS batches**

| **DS Batch** | **Sum of high-mannose N-glycans [%]** | **Sum of galactosylated N-glycans [%]** |
|---|---|---|
| 1 | 4.7 | 21.5 |
| 2 | 4.6 | 22.0 |
| 3 | 3.7 | 21.9 |
| 4 | 3.9 | 22.2 |
| 5 | 3.6 | 23.3 |
| 6 | 4.2 | 20.8 |
| 7 | 4.2 | 21.3 |
| 8 | 3.5 | 22.6 |
| 9 | 3.5 | 22.1 |
| 10 | 4.0 | 23.1 |
| 11 | 3.9 | 21.1 |
| 12 | 4.3 | 24.0 |
| 13 | 3.9 | 23.5 |
| 14 | 3.7 | 23.8 |
| 15 | 3.3 | 24.1 |
| 16 | 3.3 | 24.1 |
| 17 | 3.6 | 21.7 |
| 18 | 3.2 | 24.4 |
| 19 | 3.2 | 25.0 |
| 20 | 3.3 | 24.5 |
| 21 | 3.3 | 22.2 |
| 22 | 3.6 | 24.1 |
| 23 | 3.6 | 23.0 |
| 24 | 3.5 | 24.1 |
| 25 | 3.4 | 23.1 |
| 26 | 3.9 | 23.1 |
| 27 | 3.8 | 23.4 |
| 28 | 3.3 | 23.9 |
| 29 | 3.6 | 23.2 |
| 30 | 4.0 | 24.4 |
| 31 | 3.7 | 24.6 |
| 32 | 4.3 | 23.4 |
| 33 | 3.5 | 23.4 |
| 34 | 3.5 | 24.5 |

N-glycan data was evaluated either at the DS level or in a process solution prior to final DS filling. The results shown above consider latest integration principles, including consideration of M6 in the calculation of the sum of galactosylated species due to limited resolution at M6 peak.

The evaluation of ianalumab DS manufacturing batch data demonstrates a robust and consistent process, particularly concerning critical N-glycan mannosylation and galactosylation levels. Low process standard deviations (0.4 for mannosylation and 1.1 for galactosylation) confirm this reliability. Consequently, the existing control strategy for critical N-glycan species, supported by process capability data, indicates that additional routine release testing for the N-glycan profile is unnecessary. The comprehensive manufacturing design ensures the safety and efficacy profile.

This conclusion is reinforced by the strong correlation between the abundance of critical N-glycan species in ianalumab and ADCC bioassay potency results. Incorporating this glycan-correlated ADCC bioassay in routine release and stability testing for ianalumab DS and drug product (DP) further minimizes residual risks.

The producing cell line and commercial cell banks are fully characterized under cGMP, including extensive analysis of the N-glycan profile. Routine analytical controls, particularly the validated ADCC potency bioassay, utilize appropriate suitability measures, including a product control evaluated with each assay and a qualified reference standard. This ensures consistent method performance and product quality, preventing potential drifts in potency. Potency assignment for the reference standard follows a rigorous two-tiered qualification and retest process with harmonized test procedures.

### Example 11. Product Summary

The manufacturing process for ianalumab drug substance (DS) used a recombinant Chinese hamster ovary (CHO) cell line. lanalumab DS was generated using a process typical for monoclonal antibodies consisting of initial cell expansion and fed-batch cultivation, followed by primary separation and a series of purification steps, including chromatography steps as well as virus removal and inactivation steps.

Multiple DS production batches were generated from a single working cell bank (WCB) vial (rolling inoculum) via cell expansion, protein production during the upstream process (cultivation) and purification during the subsequent downstream process.

lanalumab DS was manufactured in a 3000 L bioreactor with an approximate working volume of 2650 L, yielding approximately 70 L purified DS. The cell culture was harvested as a single bulk harvest batch which was subsequently purified as a single DS batch.

The composition of the two presentations liquid in syringe, liquid in vial, was (i) 150 mg/mL ianalumab, (ii) 220 mM sucrose, (iii) about 20 mM L-histidine, and (iv) 0.04% Polysorbate 20.

For DP manufacturing, the DS was provided frozen in 5L PET bottles stored at ≤ -60 degrees C and protected from light. The frozen DS solution was thawed and homogenized. The DS solution was then pooled into the compounding vessel, homogenized by stirring and filtered (i.e., microbial reduction filtration) into the storage vessel.

The liquid in vial formulations were packaged in glass vials with coated rubber stoppers sealed with aluminum caps with plastic flip-off disks. The liquid in syringe formulations were filled in a glass syringe with a staked stainless-steel needle, a coated rubber plunger stopper and a rigid needle shield. The syringes were assembled with needle safety devices or autoinjectors as single dose products. The container closure systems are suitable packaging materials for pharmaceutical parenteral products.

The finished products were stored at 2-8°C.

### Example 12. A randomized, open label, multiple dose, parallel group study to assess the safety and PK comparability of two ianalumab drug products

A randomized, open label, multiple dose, parallel group study to assess the safety and pharmacokinetic comparability of two ianalumab drug products (DP), at the subcutaneous dose of 300 mg q4w (three doses), in patients with rheumatoid arthritis : ianalumab Reference DP (Ref DP; ianalumab as described in WO2010/007082) and ianalumab Test DP (as disclosed herein).

Patients were eligible for the trial if they met the following inclusion criteria:
- Fulfill 2010 ACR/EULAR criteria for RA Aletaha et al 2010 at screening
- Active disease defined as ≥ 2 swollen joints (of 58 evaluable joints) and two or more tender joints (of 60 evaluable joints) despite stable MTX ≤ 25 mg/week and/or hydroxychloroquine ≤ 400 mg/day treatment for at least two months prior to randomization

Patients were ineligible for the trial if they met any one or more of the following exclusion criteria:
- Prior or previous use of (specific dosages and intervals prior to study start may apply): other investigational drugs, B-cell depleting therapy (e.g. rituximab), monoclonal antibodies (mAb), i.v. / s.c. Ig, thymoglobulin, intravenous or oral cyclophosphamide, oral cyclosporine, soluble cytokine receptors, azathioprine.
- Currently receiving prednisone >10 mg/day (or equivalent oral glucocorticoid) or dose adjustment within two weeks prior to randomization
- Active viral, bacterial or other infections requiring systemic treatment at the time of screening or enrollment, or history of recurrent clinically significant infection or of bacterial infections with encapsulated organisms
- Receipt of live/attenuated vaccine within a two-month period before randomization
- Pregnant or nursing (lactating) women
- Women of child-bearing potential, defined as all women physiologically capable of becoming pregnant, unless they are using highly effective methods of contraception from screening and for four months after stopping of investigational drug

### Results

A total of 48 patients were exposed to ianalumab, 23 on Ref DP and 25 on Test DP, from which 22 on Ref DP and 23 on Test DP completed EOT visit by the data cut-off. At final analysis, the primary objective, which was to assess the steady state pharmacokinetic comparability of two ianalumab DP in rheumatoid arthritis subjects, was met. The geometric mean ratios of the primary PK parameters AUCtau and Cₘₐₓ after the third dose were very close to 1 and the two-sided 90% confidence intervals were all contained in the pre-specified interval boundaries (0.70, 1.43):
- AUCₜₐᵤ 1.01 (0.82, 1.24)
- Cₘₐₓ 0.92 (0.76, 1.12)

B cell kinetics was comparable between the Ref DP and Test DP. The existing safety profile of ianalumab remains unchanged with similar safety profile between the two drug DPs. Overall, the results from this study show that the pharmacokinetic profiles are comparable between the Reference and Test DP.

### Numerated Embodiments - Set A:

1. A composition comprising monoclonal anti-BAFF-R antibodies, wherein said monoclonal anti-BAFF-R antibodies comprise N-glycosylated Fc regions, wherein about 10% or less of the N-glycosylated Fc regions comprise a high mannose glycan.
2. The composition of embodiment 1, wherein at least 95% of the N-glycosylated Fc regions are afucosylated.
3. The composition of embodiment 1 or 2, wherein about 49% or less of the N-glycosylated Fc regions are galactosylated.
4. The composition of any one of embodiments 1-3, wherein about 0.1% to about 7% of the N-glycosylated Fc regions comprise a high mannose glycan.
5. The composition of embodiment 4, wherein about 0.5% to about 3.5% of the N-glycosylated Fc regions comprise a high mannose glycan.
6. The composition of any one of embodiments 1-5, wherein the composition has a relative ADCC potency of about 70% to about 130% in a cell-based ADCC assay compared to a commercial reference standard.
7. The composition of embodiment 6, wherein the composition has a relative ADCC potency of about 80% to about 116% in a cell-based ADCC assay compared to a commercial reference standard.
8. The composition of embodiment 6 or 7, wherein the cell-based ADCC assay is a BAFF-R-expressing NK cell-based ADCC potency assay.
9. The composition of any one of embodiments 1-8, wherein the composition has an ADCC potency within a reference range of ADCC potencies from two or more lots of a commercial reference standard.
10. The composition of any one of embodiments 6-9, wherein the commercial reference standard is a commercial ianalumab.
11. The composition of any one of embodiments 1-10, wherein the composition depletes human B cells through ADCC with a half-maximal efficacy (EC₅₀): (a) from 10 pM to 1 nM in an *in vitro* culture of human whole blood and/or (b) from 0.2 pM to 100 pM in an *in vitro* culture of human peripheral blood mononuclear cells.
12. The composition of any one of embodiments 1-11, wherein the anti-BAFF-R antibodies have: (a) a binding affinity (K_{D}) to FcγRIIIa^{F158} from 1.3 nM to 3.9 nM; (b) a binding affinity to FcγRIIIa^{V158} from 0.52 nM to 1.59 nM; or (c) a relative binding affinity to FcγRIIIa^{F158} and/or FcγRIIIa^{V158} from about 70% to about 130% compared to a commercial reference standard.
13. The composition of any one of embodiments 1-12, wherein the monoclonal anti-BAFF-R antibodies have a serum half-life of at least 5 days.
14. The composition of any one of embodiments 1-13, wherein the anti-BAFF-R antibodies:
   (a) comprise a CDR-H1, a CDR-H2, and a CDR-H3 having the amino acid sequences of SEQ ID NO: 5, SEQ ID NO: 6, and SEQ ID NO: 7, and a CDR-L1, a CDR-L2, and a CDR-L3 having the amino acid sequences of SEQ ID NO: 8, SEQ ID NO: 9, and SEQ ID NO: 10, respectively;
   (b) bind to a BAFF-R epitope comprising one or more of residues D26, L27, L28, V29, R30, H31, C32, V33, A34, and C35; and/or
   (c) comprise one or more anti-BAFF-R paratopic residues comprising one or more of heavy chain residues N32, S33, A34, A35, R52, Y54, R56, S57, Y60, Y102, D103, W104, V110 and/or one or more of light chain residues L92, Y93, and S94.
15. The composition of any one of embodiments 1-14, wherein the anti-BAFF-R antibodies comprise at least one variant selected from a high molecular weight variant, a deamidated variant, a tryptophan-oxidized variant, a methionine-oxidized variant, or a clipped variant.
16. The composition of embodiment 15, wherein the anti-BAFF-R antibodies comprise a high molecular weight variant, wherein the high molecular weight variant accounts for up to about 11% of the anti-BAFF-R antibodies in the composition.
17. The composition of embodiment 15 or 16, wherein the anti-BAFF-R antibodies comprise a deamidated variant, wherein the deamidated variant comprises deamidation at position N332, and optionally wherein the deamidated variant accounts for up to 42.6% of the anti-BAFF-R antibodies in the composition.
18. The composition of any one of embodiments 15-17, wherein the anti-BAFF-R antibodies comprise a tryptophan-oxidized variant, wherein the tryptophan-oxidized variant comprises H-W104 and/or H-W59, and optionally wherein the tryptophan-oxidized variant accounts for up to 25% of the anti-BAFF-R antibodies in the composition.
19. The composition of any one of embodiments 15-18, wherein the anti-BAFF-R antibodies comprise a methionine-oxidized variant, wherein the methionine-oxidized variant comprises one or more of M97, M259, M365, and M435.
20. The composition of any one of embodiments 15-19, wherein the anti-BAFF-R antibodies comprise a methionine-oxidized variant, wherein the methionine-oxidized variant comprises oxidation of M259 on both heavy chains.
21. The composition of any one of embodiments 15-20, wherein the anti-BAFF-R antibodies comprise a methionine-oxidized variant, wherein the methionine-oxidized variant accounts for about 4% or less of the anti-BAFF-R antibodies in the composition.
22. The composition of any one of embodiments 15-21, wherein the anti-BAFF-R antibodies comprise a clipped variant, wherein the clipped variant comprises clipping between R56 and S57 in the CDR2 loop of the heavy chain.
23. A composition comprising afucosylated anti-BAFF-R antibodies, wherein the afucosylated anti-BAFF-R antibodies comprise a high molecular weight variant, wherein the high molecular weight variant accounts for about 11% or less of the anti-BAFF-R antibodies in the composition.
24. The composition of embodiment 23, wherein the afucosylated anti-BAFF-R antibodies comprise a deamidated variant, wherein the deamidated variant comprises deamidation at position N332, and optionally wherein the deamidated variant accounts for up to 42.6% of the anti-BAFF-R antibodies in the composition.
25. The composition of embodiment 23 or 24, wherein the afucosylated anti-BAFF-R antibodies comprise a tryptophan-oxidized variant, wherein the tryptophan-oxidized variant comprises H-W104 and/or H-W59, and optionally wherein the tryptophan-oxidized variant accounts for up to 23.8% of the anti-BAFF-R antibodies in the composition.
26. The composition of any one of embodiments 23-25, wherein the afucosylated anti-BAFF-R antibodies comprise a methionine-oxidized variant, wherein the methionine-oxidized variant comprises one or more of M97, M259, M365, and M435 wherein the methionine-oxidized variant accounts for about 12.4% or less of the anti-BAFF-R antibodies in the composition.
27. The composition of any one of embodiments 23-26, wherein the afucosylated anti-BAFF-R antibodies comprise a clipped variant, wherein the clipped variant comprises clipping between R56 and S57 in the CDR2 loop of the heavy chain.
28. A composition comprising afucosylated anti-BAFF-R antibodies, wherein the afucosylated anti-BAFF-R antibodies comprise a deamidated variant, wherein the deamidated variant comprises deamidation at position N332, and optionally wherein the deamidated variant accounts for up to 42.6% of the anti-BAFF-R antibodies in the composition.
29. The composition of embodiment 28, the afucosylated anti-BAFF-R antibodies comprise a high molecular weight variant, wherein the high molecular weight variant accounts for about 11% or less of the anti-BAFF-R antibodies in the composition.
30. The composition of embodiment 28 or 29, wherein the afucosylated anti-BAFF-R antibodies comprise a tryptophan-oxidized variant, wherein the tryptophan-oxidized variant comprises H-W104 and/or H-W59, and optionally wherein the tryptophan-oxidized variant accounts for up to 23.8% of the anti-BAFF-R antibodies in the composition.
31. The composition of any one of embodiments 28-30, wherein the afucosylated anti-BAFF-R antibodies comprise a methionine-oxidized variant, wherein the methionine-oxidized variant comprises one or more of M97, M259, M365, and M435, optionally wherein the methionine-oxidized variant comprises M259 and M435, and wherein the methionine-oxidized variant accounts for about 4% or less of the anti-BAFF-R antibodies in the composition.
32. The composition of any one of embodiments 28-31, wherein the afucosylated anti-BAFF-R antibodies comprise a clipped variant, wherein the clipped variant comprises clipping between R56 and S57 in the CDR2 loop of the heavy chain.
33. A composition comprising afucosylated anti-BAFF-R antibodies, wherein the afucosylated anti-BAFF-R antibodies comprise a tryptophan-oxidized variant, wherein the tryptophan-oxidized variant comprises H-W104 and/or H-W59, and optionally wherein the tryptophan-oxidized variant accounts for up to 23.8% of the anti-BAFF-R antibodies in the composition.
34. The composition of embodiment 33, wherein the afucosylated anti-BAFF-R antibodies further comprise a deamidated variant, wherein the deamidated variant comprises deamidation at position N332, and optionally wherein the deamidated variant accounts for up to 42.6% of the anti-BAFF-R antibodies in the composition.
35. The composition of embodiment 33 or 34, wherein the afucosylated anti-BAFF-R antibodies further comprise a high molecular weight variant, wherein the high molecular weight variant accounts for about 11% or less of the anti-BAFF-R antibodies in the composition.
36. The composition of any one of embodiments 33-35, wherein the afucosylated anti-BAFF-R antibodies further comprise a methionine-oxidized variant, wherein the methionine-oxidized variant comprises one or more of M97, M259, M365, and M435, and wherein the methionine-oxidized variant accounts for about 12.4% or less of the anti-BAFF-R antibodies in the composition.
37. The composition of any one of embodiments 33-36, wherein the afucosylated anti-BAFF-R antibodies further comprise a clipped variant, wherein the clipped variant comprises clipping between R56 and S57 in the CDR2 loop of the heavy chain.
38. A composition comprising afucosylated anti-BAFF-R antibodies, wherein the afucosylated anti-BAFF-R antibodies comprise a methionine-oxidized variant comprising one or more of M97, M259, M365, and M435.
39. The composition of embodiment 38, wherein the methionine-oxidized variant accounts for about 12.4% or less of the anti-BAFF-R antibodies in the composition.
40. The composition of embodiment 38 or 39, wherein the afucosylated anti-BAFF-R antibodies further comprise a deamidated variant, wherein the deamidated variant comprises deamidation at position N332, and optionally wherein the deamidated variant accounts for up to 42.6% of the anti-BAFF-R antibodies in the composition.
41. The composition of any one of embodiments 38-40, wherein the afucosylated anti-BAFF-R antibodies further comprise a tryptophan-oxidized variant, wherein the tryptophan-oxidized variant comprises H-W104 and/or H-W59, and optionally wherein the tryptophan-oxidized variant accounts for up to 23.8% of the anti-BAFF-R antibodies in the composition.
42. The composition of any one of embodiments 38-41, wherein the afucosylated anti-BAFF-R antibodies comprise a high molecular weight variant, wherein the high molecular weight variant accounts for about 11% or less of the anti-BAFF-R antibodies in the composition.
43. The composition of any one of embodiments 38-42, wherein the afucosylated anti-BAFF-R antibodies further comprise a clipped variant, wherein the clipped variant comprises clipping between R56 and S57 in the CDR2 loop of the heavy chain.
44. A composition comprising afucosylated anti-BAFF-R antibodies, wherein the afucosylated anti-BAFF-R antibodies comprise a clipped variant comprising clipping between R56 and S57 in the CDR2 loop of the heavy chain.
45. The composition of embodiment 44, wherein the afucosylated anti-BAFF-R antibodies further comprise a deamidated variant, wherein the deamidated variant comprises deamidation at position N332, and optionally wherein the deamidated variant accounts for up to 42.6% of the anti-BAFF-R antibodies in the composition.
46. The composition of embodiment 44 or 45, wherein the afucosylated anti-BAFF-R antibodies further comprise a tryptophan-oxidized variant comprising H-W104 and/or H-W59, and optionally wherein the tryptophan-oxidized variant accounts for up to 23.8% of the anti-BAFF-R antibodies in the composition.
47. The composition of any one of embodiments 44-46, wherein the afucosylated anti-BAFF-R antibodies further comprise a methionine-oxidized variant, wherein the methionine-oxidized variant comprises one or more of M97, M259, M365, and M435, and wherein the methionine-oxidized variant accounts for about 4% or less of the anti-BAFF-R antibodies in the composition.
48. The composition of any one of embodiments 44-47, wherein the afucosylated anti-BAFF-R antibodies further comprise a high molecular weight variant, wherein the high molecular weight variant accounts for about 11% or less of the anti-BAFF-R antibodies in the composition.
49. The composition of any one of embodiments 23-48, wherein at least 95% of N-glycosylated Fc regions in the anti-BAFF-R antibodies are afucosylated.
50. The composition of any one of embodiments 23-49, wherein about 10% or less of N-glycosylated Fc regions in the afucosylated anti-BAFF-R antibodies comprise a high mannose glycan.
51. The composition of any one of embodiments 23-50, wherein 50% or less of the N-glycosylated Fc regions are galactosylated.
52. The composition of any one of embodiments 23-51, wherein the composition has a relative ADCC potency of about 70% to about 130% in a cell-based ADCC assay compared to a commercial reference standard.
53. The composition of embodiment 52, wherein the composition has a relative ADCC potency of about 80% to about 116% in a cell-based ADCC assay compared to a commercial reference standard.
54. The composition of any one of embodiments 23-53, wherein the composition has an ADCC potency within a reference range of ADCC potencies from two or more lots of a commercial reference standard.
55. The composition of any one of embodiments 23-54, wherein the commercial reference standard is a commercial ianalumab.
56. The composition of any one of embodiments 23-55, wherein the anti-BAFF-R antibodies have a mean relative ADCC cytotoxicity from about 70% to about 130% in a cell-based ADCC assay.
57. The composition of any one of embodiments 23-56, wherein the cell-based ADCC assay is a BAFF-R-expressing NK cell-based ADCC potency assay.
58. The composition of any one of embodiments 23-57, wherein the anti-BAFF-R antibodies have: (a) a binding affinity (K_{D}) to FcγRIIIa^{F158} from 1.3 nM to 3.9 nM; (b) a binding affinity to FcγRIIIa^{V158} from 0.52 nM to 1.59 nM; (c) a relative binding affinity to FcγRIIIa^{F158} and/or FcγRIIIa^{V158} from about 70% to about 130% compared to a commercial reference standard.
59. The composition of any one of embodiments 23-58, wherein the monoclonal anti-BAFF-R antibodies have a serum half-life of at least 5 days.
60. The composition of any one of embodiments 1-59, wherein the anti-BAFF-R antibodies comprise a variable heavy chain comprising at least 95% sequence identity to SEQ ID NO: 3 and/or a variable light chain comprising at least 95% sequence identity to SEQ ID NO: 4.
61. The composition of embodiment 60, wherein the VH comprises the amino acid sequence of SEQ ID NO: 3 and/or the VL comprises the amino acid sequence of SEQ ID NO: 4.
62. The composition of any one of embodiments 1-61, wherein the anti-BAFF-R antibody comprises a heavy chain having at least 95% identity to the amino acid sequence of SEQ ID NO: 1 and a light chain having at least 95% identity to the amino acid sequence of SEQ ID NO: 2.
63. The composition of embodiment 62, wherein the heavy chain comprises the amino acid sequence of SEQ ID NO: 1 and the light chain comprises the amino acid sequence of SEQ ID NO: 2.
64. The composition of any one of embodiments 1-63, wherein the anti-BAFF-R antibodies are IgG1 antibodies.
65. The composition of embodiment 64, wherein the IgG1 antibodies are IgG1κ antibodies.
66. The composition of embodiment 65, wherein the anti-BAFF-R antibody is ianalumab.
67. The composition of any one of embodiments 1-66, wherein the composition is a single batch preparation.
68. The composition of any one of embodiments 1-67, wherein the anti-BAFF-R antibodies are produced in a non-human cell.
69. The composition of embodiment 68, wherein the non-human cell is a recombinant Chinese hamster ovary (CHO) cell.
70. The composition of embodiment 69, wherein the CHO cell has impaired fucosylation compared to a wild-type CHO cell.
71. The composition of any one of embodiments 1-70, wherein the composition is formulated into a liquid pharmaceutical formulation suitable for administration to a subject in need thereof.
72. Isolated anti-BAFF-R antibodies produced in a non-human cell, wherein the antibodies are the anti-BAFF-R antibodies of the composition of any one of embodiments 1-71.
73. A single batch preparation of monoclonal anti-BAFF-R antibodies, wherein the monoclonal anti-BAFF-R antibodies are the isolated anti-BAFF-R antibodies of embodiment 72.
74. A pharmaceutical formulation comprising: (a) the composition of any one of embodiments 1-71 and a pharmaceutically acceptable carrier, (b) the isolated anti-BAFF-R antibodies of embodiment 72 and a pharmaceutically acceptable carrier, or (c) the single batch preparation of embodiment 73 and a pharmaceutically acceptable carrier.
75. A pharmaceutical formulation comprising a composition of afucosylated anti-BAFF-R antibodies in a pharmaceutically acceptable carrier, wherein the afucosylated anti-BAFF-R antibodies comprise a heavy chain amino acid sequence of SEQ ID NO: 1 and a light chain amino acid sequence of SEQ ID NO: 2, and wherein the anti-BAFF-R antibodies include a high mannose glycan variant and a galactosylated variant.
76. A lyophilisate obtainable by lyophilisation of the pharmaceutical formulation of embodiment 74 or 75.
77. A pharmaceutical product comprising a container comprising the pharmaceutical formulation of embodiment 74 or 75.
78. The pharmaceutical product of embodiment 77, wherein the pharmaceutical formulation is a liquid.
79. The pharmaceutical product of embodiment 78, wherein the container is a vial, an injection device, an injection pen, a vial and syringe, a cartridge, a pre-filled syringe, or an autoinjector.
80. The pharmaceutical product of any one of embodiments 77-79, further comprising a package insert and/or instructions for use.
81. The pharmaceutical product of any one of embodiments 78-80, wherein the container is a pre-filled syringe containing the pharmaceutical formulation at 50 mg of the anti-BAFF-R antibody in 1 mL volume or 300 mg of the anti-BAFF-R antibody in 2 mL volume.
82. The pharmaceutical product of any one of embodiments 78-80, wherein the container is a vial containing the pharmaceutical formulation at 150 mg of the anti-BAFF-R antibody in 1 mL volume or 450 mg of the anti-BAFF-R antibody in 3 mL volume.
83. The composition of any one of embodiments 1-71, the isolated anti-BAFF-R antibodies of embodiment 72, the single batch preparation of embodiment 73, the pharmaceutical formulation of embodiment 74 or 75, the lyophilisate of embodiment 76, or the pharmaceutical product of any one of embodiments 77-82, wherein the anti-BAFF-R antibody is produced by a method of manufacture comprising a fucosylation-deficient host cell culture.
84. The composition, isolated anti-BAFF-R antibodies, single batch preparation, pharmaceutical formulation, lyophilisate, or pharmaceutical product of embodiment 83, wherein the fucosylation-deficient host cell culture produces high mannose N-glycans in less than 10% of the anti-BAFF-R antibodies.
85. A method of manufacturing a pharmaceutical formulation comprising anti-BAFF antibodies having an ADCC-dependent mechanism of action, the method comprising:
   (a) culturing a recombinant cell line at a pH from about 6.8 to about 7.15 to express a population of anti-BAFF-R antibodies having an ADCC-dependent mechanism of action; and
   (b) formulating the population of anti-BAFF-R antibodies expressed during step (a) into a pharmaceutical formulation comprising a pharmaceutically acceptable carrier.
86. The method of embodiment 85, wherein step (a) comprises a starting viable cell density from about 1.45x10⁶ cells/mL to about 4x10⁶ cells/mL.
87. A method of manufacturing a pharmaceutical formulation comprising anti-BAFF antibodies having an ADCC-dependent mechanism of action, the method comprising:
   (a) culturing a recombinant cell line to express a population of anti-BAFF-R antibodies having an ADCC-dependent mechanism of action, wherein the viable cell density of the recombinant cell line at the start of culturing is from about 1.45x10⁶ cells/mL to about 4x10⁶ cells/mL; and
   (b) formulating the population of anti-BAFF-R antibodies expressed during step (a) into a pharmaceutical formulation comprising a pharmaceutically acceptable carrier.
88. The method of embodiment 87, wherein step (a) is at a pH from about 6.8 to about 7.15.
89. The method of any one of embodiments 85-88, wherein at least 95% of the population of anti-BAFF-R antibodies is afucosylated.
90. The method of any one of embodiments 85-89, wherein the recombinant cell line is fucosylation deficient.
91. The method of any one of embodiments 85-90, wherein step (a) comprises a temperature switch from a first temperature to a second temperature, wherein the second temperature is lower than the first temperature.
92. The method of embodiment 91, wherein the second temperature is less than about 36.5 C.
93. The method of embodiment 92, wherein the second temperature is from about 32.5 C to about 34.0 C.
94. The method of any one of embodiments 91-93, wherein the first temperature is from about 35.8 C to about 37.2 C.
95. The method of any one of embodiments 91-94, wherein the temperature switch occurs when the recombinant cell line is at a viable cell density from about 10x10⁶ to about 16x10⁶ cells/mL.
96. The method of any one of embodiments 85-95, wherein the duration of step (a) is from about 300 hours to about 350 hours.
97. The method of any one of embodiments 85-96, wherein the step (a) is in a fed-batch production bioreactor.
98. The method of any one of embodiments 85-97, further comprising, prior to step (a), culturing the recombinant cell line in a seed bioreactor.
99. The method of any one of embodiments 85-98, further comprising comparing an ADCC potency of the population of anti-BAFF-R antibodies to a target range of ADCC potency, the method comprising:
   (c) identifying the target range of ADCC potency, wherein the target range is defined by a range of ADCC potencies of two or more lots of a reference anti-BAFF-R antibody composition;
   (d) measuring the ADCC potency of the population of anti-BAFF-R antibodies; and
   (e) determining whether the ADCC potency of the population of anti-BAFF-R antibodies falls within the target range.
100. The method of embodiment 99, wherein the reference anti-BAFF-R antibody composition is ianalumab.
101. The method of embodiment 99 or 100, further comprising recommending the population of anti-BAFF-R antibodies, or a pharmaceutical formulation or drug product thereof, as a treatment of a BAFF-R-related disorder, wherein step (e) results in a determination that the ADCC potency of the population of anti-BAFF-R antibodies falls within the target range.
102. The method of any one of embodiments 85-101, wherein 95%-100% of the population of antibodies expressed during the culture are afucosylated.
103. The method of embodiment 102, wherein 99% or more of the population of antibodies expressed during culture are afucosylated.
104. The method of any one of embodiments 85-103, wherein 0-50% of the population of antibodies expressed during the culture comprises galactosylated N-glycans.
105. The method of embodiment any one of embodiments 85-104, wherein the Fc region is a human IgG1 Fc region.
106. The method of embodiment 105, wherein the IgG1 Fc region is an IgG1κ Fc region.
107.The method of any one of embodiments 85-106, wherein the anti-BAFF-R antibody comprises a heavy chain having at least 95% identity to the amino acid sequence of SEQ ID NO: 1 and a light chain having at least 95% identity to the amino acid sequence of SEQ ID NO: 2.
108. The method of embodiment 107, wherein the heavy chain comprises the amino acid sequence of SEQ ID NO: 1 and the light chain comprises the amino acid sequence of SEQ ID NO: 2.
109. The method of any one of embodiments 85-108, wherein the anti-BAFF antibodies having an ADCC-dependent mechanism of action are the isolated anti-BAFF antibodies of embodiment 24.
110. The method of any one of embodiments 85-109, further comprising lyophilizing the pharmaceutical formulation to produce a lyophilized pharmaceutical formulation.
111. The method of any one of embodiments 85-109, further comprising preparing a pharmaceutical product comprising a container by dispensing the pharmaceutical formulation into the container.
112. The method of embodiment 111, wherein the pharmaceutical formulation in the container is a liquid pharmaceutical formulation.
113. The method of embodiment 112, wherein the container is a vial, an injection device, an injection pen, a vial and syringe, a cartridge, a pre-filled syringe, or an autoinjector.
114. The method of any one of embodiments 111-113, wherein the container comprises a package insert and/or instructions for use.
115. The method of embodiment 113 or 114, wherein the container is a pre-filled syringe containing the pharmaceutical formulation at 150 mg of the anti-BAFF-R antibody in 1 mL volume or 300 mg of the anti-BAFF-R antibody in 2 mL volume.
116. The method of embodiment 113 or 114, wherein the container is a vial containing the pharmaceutical formulation at 150 mg of the anti-BAFF-R antibody in 1 mL volume or 450 mg of the anti-BAFF-R antibody in 3 mL volume.
117.A method of matching an ADCC activity of a reference afucosylated anti-BAFF-R antibody composition comprising:
   (a) determining an ADCC activity of a reference afucosylated anti-BAFF-R antibody composition;
   (b) determining an ADCC activity of a second antibody composition comprising an anti-BAFF-R antibody having the same antibody sequence as the reference anti-BAFF-R antibody; and
   (c) changing the ADCC activity of the second antibody composition by increasing or decreasing the amount of high-mannose glycans of one or more antibodies within the second antibody composition, wherein the ADCC activity of the second antibody composition after increasing or decreasing the amount of high-mannose is the same as the reference afucosylated anti-BAFF-R antibody composition or within about 10%, about 15%, about 20%, about 25%, about 30%, about 35%, about 40%, about 45% or about 50% of the reference and afucosylated anti-BAFF-R antibody composition or within about 1% to about 50% of the reference and afucosylated anti-BAFF-R antibody composition.
118. A method for engineering a target ADCC activity of an afucosylated anti-BAFF-R antibody composition comprising:
   (a) determining an ADCC activity of a afucosylated anti-BAFF-R antibody composition;
   (b) determining a target ADCC activity; and
   (c) increasing or decreasing the ADCC activity of the glycosylated and afucosylated anti-BAFF-R antibody composition by increasing or decreasing the amount high-mannose glycans in the Fc region of the antibody, wherein the ADCC activity of the glycosylated and afucosylated anti-BAFF-R antibody composition after increasing or decreasing the amount of high-mannose glycans is the same as the target ADCC activity or within about 10%, about 15%, about 20%, about 25%, about 30%, about 35%, about 40%, about 45% or about 50% of the target ADCC activity or within about 1% to about 50% of the target ADCC activity.
119. The method of embodiment 117 or 118, wherein step (a) occurs before or at the same time as step (b) and/or step (c).
120. The method of embodiment 117 or 118, wherein step (a) occurs after step (b) and/or step (c).
121.The method of any one of embodiments 117-120, wherein an increase of about 1% high-mannose glycans increases ADCC activity by about 20% to about 30%.
122. The method any one of embodiments 117-121, wherein the reference anti-BAFF-R antibody is ianalumab.
123. The composition of any one of embodiments 1-71, the isolated anti-BAFF-R antibodies of embodiment 72, the single batch preparation of embodiment 73, the pharmaceutical formulation of embodiment 74 or 75, the lyophilisate of embodiment 76, the pharmaceutical product of any one of embodiments 77-82, or the pharmaceutical formulation manufactured by the method of any one of embodiments 85-116, for use in a method of treating a BAFF-R-related disorder.
124.A method of treating a BAFF-R-related disorder, the method comprising administering an effective amount of the composition of any one of embodiments 1-71, the isolated anti-BAFF-R antibodies of embodiment 72, the single batch preparation of embodiment 73, the pharmaceutical formulation of embodiment 74 or 75, the lyophilisate of embodiment 76, the pharmaceutical product of any one of embodiments 77-82, or the pharmaceutical formulation manufactured by the method of any one of embodiments 85-116.
125. The composition, isolated anti-BAFF-R antibodies, single batch preparation, pharmaceutical formulation, or pharmaceutical product for use of embodiment 123, or the method of embodiment 124, wherein the BAFF-R-related disorder is an autoimmune disease or a B-cell neoplasm.
126. The composition, isolated anti-BAFF-R antibodies, single batch preparation, pharmaceutical formulation, or pharmaceutical product for use, or method, of embodiment 125, wherein the subject is a human.
127. The composition, isolated anti-BAFF-R antibodies, single batch preparation, pharmaceutical formulation, or pharmaceutical product for use, or method, of any one of embodiments 123-126, wherein the anti-BAFF-R antibody is administered at a dose of about 3 mg to about 10 mg active ingredient per kilogram of the subject (mg/kg).
128. The composition, isolated anti-BAFF-R antibodies, single batch preparation, pharmaceutical formulation, or pharmaceutical product for use, or method, of embodiment 127, wherein the anti-BAFF-R antibody is administered at a dose of about 3 mg/kg or about 9 mg/kg.
129. The composition, isolated anti-BAFF-R antibodies, single batch preparation, pharmaceutical formulation, or pharmaceutical product for use, or method, of any one of embodiments 123-128, wherein the anti-BAFF-R antibody is administered intravenously.
130. The composition, isolated anti-BAFF-R antibodies, single batch preparation, pharmaceutical formulation, or pharmaceutical product for use, or method, of embodiment 129, wherein the anti-BAFF-R antibody is administered at a dose of about 150 mg to about 400 mg active ingredient, such as about 300 mg active ingredient.
131. The composition, isolated anti-BAFF-R antibodies, single batch preparation, pharmaceutical formulation, or pharmaceutical product for use, or method, of embodiment 130, wherein the dose is 150 mg active ingredient or 300 mg active ingredient.
132. The composition, isolated anti-BAFF-R antibodies, single batch preparation, pharmaceutical formulation, or pharmaceutical product for use, or method, of embodiment 130 or 131, wherein the anti-BAFF-R antibody is administered to a subject in need thereof subcutaneously.
133. The composition, isolated anti-BAFF-R antibodies, single batch preparation, pharmaceutical formulation, or pharmaceutical product for use, or method, of any one of embodiments 130-132, wherein the anti-BAFF-R antibody is administered to a subject in need thereof once every 4 weeks (e.g., monthly, +/- 3 days) or once every 12 weeks (e.g. every 3 months, +/- 3 days).
134. The composition, isolated anti-BAFF-R antibodies, single batch preparation, pharmaceutical formulation, or pharmaceutical product for use, or method, of any one of embodiments 123-133, wherein the autoimmune disease is autoimmune haematological disorders (including e.g. warm autoimmune hemolytic anaemia, aplastic anaemia, pure red cell anaemia and immune thrombocytopenia), acquired hemophilia A, cold agglutinin disease, cryoglobulinemia, thrombotic thrombocytopenic purpura, Sjögren's Disease, systemic lupus erythematosus, lupus nephritis, inflammatory muscle disorders, polychondritis, sclerodoma, anti-neutrophil cytoplasmic antibody-associated vasculitis, IgM mediated neuropathy, opsoclonus myoclonus syndrome, Wegener granulomatosis, dermatomyositis, chronic active hepatitis, myasthenia gravis, psoriasis, Steven-Johnson syndrome, pemphigus vulgaris, pemphigus foliacius, idiopathic sprue, autoimmune inflammatory bowel disease (including e.g. ulcerative colitis, Crohn's disease and Irritable Bowel Syndrome), endocrine ophthalmopathy, Graves' disease, sarcoidosis, multiple sclerosis, neuromyelitis optica, primary biliary cirrhosis, juvenile diabetes (diabetes mellitus type I), uveitis (anterior, intermediate and posterior as well as panuveitis), keratoconjunctivitis sicca and vernal keratoconjunctivitis, interstitial lung fibrosis, psoriatic arthritis and glomerulonephritis (with and without nephrotic syndrome, e.g. including idiopathic nephrotic syndrome or minimal change nephropathy), tumors, inflammatory disease of skin and cornea, myositis, loosening of bone implants, metabolic disorders, such as atherosclerosis, diabetes, and dislipidemia.
135. The composition, isolated anti-BAFF-R antibodies, single batch preparation, pharmaceutical formulation, or pharmaceutical product for use, or method, of embodiment 134, wherein the autoimmune disease is warm autoimmune hemolytic anemia, and optionally wherein the anti-BAFF-R antibody is administered to a subject in need thereof at a dose of about 3 mg/kg or about 9 mg/kg, iv., once every 4 weeks (e.g., monthly, +/- 3 days) for a duration of up to 4 months.
136. The composition, isolated anti-BAFF-R antibodies, single batch preparation, pharmaceutical formulation, or pharmaceutical product for use, or method, of embodiment 134, wherein the autoimmune disease is immune thrombocytopenia, and optionally wherein the anti-BAFF-R antibody is administered to a subject in need thereof at a dose of about 3 mg/kg or about 9 mg/kg, iv., once every 4 weeks (e.g., monthly, +/- 3 days) for a duration of up to 4 months.
137. The composition, isolated anti-BAFF-R antibodies, single batch preparation, pharmaceutical formulation, or pharmaceutical product for use, or method, of embodiment 103, wherein the autoimmune disease is Sjögren's Disease, systemic lupus erythematosus, lupus nephritis, hidradenitis suppurativa, or scleroderma, and optionally wherein the anti-BAFF-R antibody is administered to a subject in need thereof at a dose of about 300 mg, subcutaneously, once every 4 weeks (e.g., monthly, +/- 3 days), or once every 12 weeks (e.g. every 3 months, +/- 3 days).
138. A method of controlling ADCC activity of single batch preparation of a population of anti-BAFF-R antibodies comprising:
   (a) determining an ADCC activity of an afucosylated anti-BAFF-R antibody composition; and
   (b) increasing or decreasing the ADCC activity of the afucosylated anti-BAFF-R antibody composition by increasing or decreasing the amount of high mannose in the glycan species anti-BAFF-R antibodies within the composition.
139. The method of embodiment 138, wherein the single batch preparation of a population of anti-BAFF-R antibodies is the single batch population of embodiment 73.

### Numerated Embodiments - Set B:

1. A composition comprising anti-BAFF-R antibodies (e.g., monoclonal anti-BAFF-R antibodies), wherein said anti-BAFF-R antibodies comprise N-glycosylated Fc regions, wherein 10% or less of the N-glycosylated Fc regions comprise a high mannose glycan.
2. The composition of embodiment 1, wherein at least 90% of the N-glycosylated Fc regions are afucosylated.
3. The composition of embodiment 1 or 2, wherein 0% to 49% of the N-glycosylated Fc regions are galactosylated.
4. The composition of any one of embodiments 1-3, wherein 0% to 7% of the N-glycosylated Fc regions comprise a high mannose glycan.
5. The composition of embodiment 4, wherein 0.5% to 4% of the N-glycosylated Fc regions comprise a high mannose glycan.
6. The composition of any one of embodiments 1-5, wherein the composition has a relative antibody-dependent cell-mediated cytotoxicity (ADCC) potency of 70% to 130% in a cell-based ADCC assay compared to a reference standard.
7. The composition of embodiment 6, wherein the composition has a relative ADCC potency of 80% to 116% in a cell-based ADCC assay compared to the reference standard.
8. The composition of any one of embodiment 6 or 7, wherein the composition has an ADCC potency within a reference range of ADCC potencies from two lots of the reference standard
9. The composition of any one of embodiments 1-5, wherein the composition has an ADCC potency within a reference range of ADCC potencies from two lots of a reference standard.
10. The composition of any one of embodiments 6-8, wherein the cell-based ADCC assay is a BAFF-R-expressing NK cell-based ADCC potency assay.
11. The composition of any one of embodiments 6-10, wherein the anti-BAFF-R antibodies have a relative binding affinity to FcγRIIIa^{F158} and/or FcγRIIIa^{V158} from about 70% to about 130% compared to the reference standard.
12. The composition of any one of embodiments 1-5, wherein the e anti-BAFF-R antibodies have a relative binding affinity to FcγRIIIa^{F158} and/or FcγRIIIa^{V158} from about 70% to about 130% compared to a reference standard.
13. The composition of any one of embodiments 6-12, wherein the reference standard is ianalumab.
14. The composition of embodiment 13, wherein the ianalumab reference standard is manufactured and analyzed in accordance with good manufacturing practices (GMP).
15. The composition of any one of embodiments 1-14, wherein the anti-BAFF-R antibodies have: (a) a binding affinity (K_{D}) to FcγRIIIa^{F158} from 1.3 nM to 3.9 nM; or (b) a binding affinity to FcγRIIIa^{V158} from 0.52 nM to 1.59 nM.
16. The composition of embodiment 15, wherein the anti-BAFF-R antibodies have a K_{D} to FcγRIIIa^{F158} from 2.0 nM to 3.0 nM.
17. The composition of any one of embodiments 1-16, wherein the composition depletes human B cells through ADCC with a half-maximal efficacy (EC₅₀): (a) from 10 pM to 1 nM in an *in vitro* culture of human whole blood; and/or (b) from 0.2 pM to 100 pM in an *in vitro* culture of human peripheral blood mononuclear cells.
18. The composition of embodiment 17, wherein the composition depletes human B cells through ADCC with a half-maximal efficacy (EC₅₀) from 150 pM to 250 pM in an *in vitro* culture of human whole blood.
19. The composition of embodiment 17 or 18, wherein the composition depletes human B cells through ADCC with a half-maximal efficacy (EC₅₀) from 2.0 pM to 5 pM in an *in vitro* culture of human peripheral blood mononuclear cells.
20. The composition of any one of embodiments 1-19, wherein the anti-BAFF-R antibodies have a serum half-life of at least 5 days.
21. The composition of any one of embodiments 1-20, wherein the anti-BAFF-R antibodies bind to a BAFF-R epitope comprising one or more of residues D26, L27, L28, V29, R30, H31, C32, V33, A34, and C35.
22. The composition of any one of embodiments 1-21, wherein the anti-BAFF-R antibodies comprise one or more anti-BAFF-R paratopic residues comprising one or more of heavy chain residues N32, S33, A34, A35, R52, Y54, R56, S57, Y60, Y102, D103, W104, V110 and/or one or more of light chain residues L92, Y93, and S94.
23. The composition of any one of embodiments 1-22, wherein the anti-BAFF-R antibodies comprise a CDR-H1, a CDR-H2, and a CDR-H3 having the amino acid sequences of SEQ ID NO: 5, SEQ ID NO: 6, and SEQ ID NO: 7, and a CDR-L1, a CDR-L2, and a CDR-L3 having the amino acid sequences of SEQ ID NO: 8, SEQ ID NO: 9, and SEQ ID NO: 10, respectively.
24. The composition of any one of embodiments 1-23, wherein the anti-BAFF-R antibodies comprise at least one variant selected from a high molecular weight variant, a deamidated variant, a tryptophan-oxidized variant, a methionine-oxidized variant, and a clipped variant.
25. The composition of embodiment 24, wherein the anti-BAFF-R antibodies comprise a high molecular weight variant.
26. The composition of embodiment 25, wherein the high molecular weight variant accounts for up to 6% of the total anti-BAFF-R antibodies and fragments thereof in the composition.
27. The composition of embodiment 26, wherein the high molecular weight variant accounts for up to 1% of the total anti-BAFF-R antibodies and fragments thereof in the composition.
28. The composition of any one of embodiments 24-27, wherein the anti-BAFF-R antibodies comprise a deamidated variant comprising deamidation at position N332, and wherein the deamidated variant accounts for up to 23% of the total anti-BAFF-R antibodies and fragments thereof in the composition.
29. The composition of embodiment 28, wherein the deamidated variant accounts for up to 1% of the total anti-BAFF-R antibodies and fragments thereof in the composition.
30. The composition of any one of embodiments 24-29, wherein the anti-BAFF-R antibodies comprise a tryptophan-oxidized variant comprising tryptophan oxidation at heavy chain residue 104 and/or heavy chain residue 59.
31. The composition of embodiment 30, wherein the tryptophan-oxidized variant accounts for up to 16% of the total anti-BAFF-R antibodies and fragments thereof in the composition.
32. The composition of embodiment 31, wherein the tryptophan-oxidized variant accounts for up to 3% of the total anti-BAFF-R antibodies and fragments thereof in the composition.
33. The composition of any one of embodiments 24-32, wherein the anti-BAFF-R antibodies comprise a methionine-oxidized variant comprising methionine oxidation at one or more of light chain residue 97, heavy chain residue 259, heavy chain residue 365, and heavy chain residue 435.
34. The composition of embodiment 33, wherein the methionine-oxidized variant comprises methionine oxidation at light chain residue 97, and wherein the methionine-oxidized variant comprising oxidation at light chain residue 97 accounts for up to 62% of the total anti-BAFF-R antibodies and fragments thereof in the composition.
35. The composition of embodiment 34, wherein the methionine-oxidized variant comprising oxidation at light chain residue 97 accounts for up to 4% of the total anti-BAFF-R antibodies and fragments thereof in the composition.
36. The composition of embodiment 33, wherein the methionine-oxidized variant comprises methionine oxidation at one or more of heavy chain residues 259, 365, and 435, and wherein the methionine-oxidized variant comprising oxidation at one or more of heavy chain residues 259, 365, and 435 accounts for up to 27% of the total anti-BAFF-R antibodies and fragments thereof in the composition.
37. The composition of embodiment 36, wherein the methionine-oxidized variant comprising oxidation at one or more of heavy chain residues 259, 365, and 435 accounts for up to 6% of the total anti-BAFF-R antibodies and fragments thereof in the composition.
38. The composition of any one of embodiments 24-37, wherein the anti-BAFF-R antibodies comprise a clipped variant comprising clipping between heavy chain residues 56 and 57.
39. The composition of embodiment 38, wherein the clipped variant accounts for up to 10% of the total anti-BAFF-R antibodies and fragments thereof in the composition.
40. A composition comprising afucosylated anti-BAFF-R antibodies, wherein the afucosylated anti-BAFF-R antibodies comprise a high molecular weight variant, and wherein the high molecular weight variant accounts for up to 6% of the total anti-BAFF-R antibodies and fragments thereof in the composition.
41. The composition of embodiment 40, wherein the high molecular weight variant accounts for up to 1% of the total anti-BAFF-R antibodies and fragments thereof in the composition.
42. The composition of embodiment 40 or 41, wherein the afucosylated anti-BAFF-R antibodies comprise a deamidated variant comprising deamidation at heavy chain 332.
43. The composition of embodiment 42, wherein the deamidated variant accounts for up to 23% of the total anti-BAFF-R antibodies and fragments thereof in the composition.
44. The composition of alim 43, wherein the deamidated variant accounts for up to 1% of the total anti-BAFF-R antibodies and fragments thereof in the composition.
45. The composition of any one of embodiments 40-44, wherein the afucosylated anti-BAFF-R antibodies comprise a tryptophan-oxidized variant comprising tryptophan oxidation at heavy chain residue 104 and/or heavy chain residue 59.
46. The composition of embodiment 45, wherein the tryptophan-oxidized variant accounts for up to 16% of the total anti-BAFF-R antibodies and fragments thereof in the composition.
47. The composition of any one of embodiments 40-46, wherein the afucosylated anti-BAFF-R antibodies comprise a methionine-oxidized variant comprising methionine oxidation at light chain residue 97, and wherein the methionine-oxidized variant at light chain residue 97 accounts for up to 62% of the total anti-BAFF-R antibodies and fragments thereof in the composition.
48. The composition of embodiment 47, wherein the methionine-oxidized variant at light chain residue 97 accounts for up to 4% of the total anti-BAFF-R antibodies and fragments thereof in the composition.
49. The composition of any one of embodiments 40-48, wherein the afucosylated anti-BAFF-R antibodies comprise a methionine-oxidized variant comprising methionine oxidation of one or more of heavy chain residues 259, 365, and 435, and wherein the methionine-oxidized variant comprising oxidation of one or more of heavy chain residues 259, 365, and 435 accounts for up to 27% of the total anti-BAFF-R antibodies and fragments thereof in the composition.
50. The composition of embodiment 49, wherein the methionine-oxidized variant comprising methionine oxidation of one or more of heavy chain residues 259, 365, and 435 accounts for up to 6% of the total anti-BAFF-R antibodies and fragments thereof in the composition.
51. The composition of any one of embodiments 40-50, wherein the afucosylated anti-BAFF-R antibodies comprise a clipped variant comprising clipping between heavy chain residues 56 and 57.
52. The composition of embodiment 51, wherein the clipped variant accounts for up to 10% of the total anti-BAFF-R antibodies and fragments thereof in the composition.
53. A composition comprising afucosylated anti-BAFF-R antibodies, wherein the afucosylated anti-BAFF-R antibodies comprise a deamidated variant comprising deamidation at position N332, and wherein the deamidated variant accounts for up to 23% of the total anti-BAFF-R antibodies and fragments thereof in the composition.
54. The composition of embodiment 53, wherein the deamidated variant accounts for up to 1% of the total anti-BAFF-R antibodies and fragments thereof in the composition.
55. The composition of embodiment 53 or 54, wherein the afucosylated anti-BAFF-R antibodies comprise a high molecular weight variant, and wherein the high molecular weight variant accounts for up to 6% of the total anti-BAFF-R antibodies and fragments thereof in the composition.
56. The composition of embodiment 55, wherein the high molecular weight variant accounts for up to 1% of the total anti-BAFF-R antibodies and fragments thereof in the composition.
57. The composition of any one of embodiments 53-56, wherein the afucosylated anti-BAFF-R antibodies comprise a tryptophan-oxidized variant comprising tryptophan oxidation at heavy chain residue 104 and/or heavy chain residue 59.
58. The composition of embodiment 57, wherein the tryptophan-oxidized variant accounts for up to 16% of the total anti-BAFF-R antibodies and fragments thereof in the composition.
59. The composition of embodiment 58, wherein the tryptophan-oxidized variant accounts for up to 3% of the total anti-BAFF-R antibodies and fragments thereof in the composition.
60. The composition of any one of embodiments 53-59, wherein the afucosylated anti-BAFF-R antibodies comprise a methionine-oxidized variant comprising methionine oxidation at one or more of light chain residue 97, heavy chain residue 259, heavy chain residue 365, and heavy chain residue 435.
61. The composition of embodiment 60, wherein the methionine-oxidized variant comprises methionine oxidation at light chain residue 97, and wherein the methionine-oxidized variant comprising oxidation at light chain residue 97 accounts for up to 62% of the total anti-BAFF-R antibodies and fragments thereof in the composition.
62. The composition of embodiment 61, wherein the methionine-oxidized variant comprising methionine oxidation at light chain residue 97 accounts for up to 4% of the total anti-BAFF-R antibodies and fragments thereof in the composition.
63. The composition of any one of embodiments 60-62, wherein the methionine-oxidized variant comprises methionine oxidation at one or more of heavy chain residues 259, 365, and 435, and wherein the methionine-oxidized variant comprising oxidation at one or more of heavy chain residues 259, 365, and 435 accounts for up to 27% of the total anti-BAFF-R antibodies and fragments thereof in the composition.
64. The composition of embodiment 63, wherein the methionine-oxidized variant comprising methionine oxidation at one or more of heavy chain residues 259, 365, and 435 accounts for up to 6% of the total anti-BAFF-R antibodies and fragments thereof in the composition.
65. The composition of any one of embodiments 53-64, wherein the afucosylated anti-BAFF-R antibodies comprise a clipped variant comprising clipping between heavy chain residues 56 and 57.
66. The composition of embodiment 65, wherein the clipped variant accounts for up to 10% of the total anti-BAFF-R antibodies and fragments thereof in the composition.
67. A composition comprising afucosylated anti-BAFF-R antibodies, wherein the afucosylated anti-BAFF-R antibodies comprise a tryptophan-oxidized variant comprising tryptophan oxidation at heavy chain residue 104 and/or heavy chain residue 59.
68. The composition of embodiment 67, wherein the tryptophan-oxidized variant accounts for up to 16% of the total anti-BAFF-R antibodies and fragments thereof in the composition.
69. The composition of embodiment 68, wherein the tryptophan-oxidized variant accounts for up to 3% of the total anti-BAFF-R antibodies and fragments thereof in the composition.
70. The composition of any one of embodiments 67-69, wherein the afucosylated anti-BAFF-R antibodies comprise a deamidated variant comprising deamidation at position N332, and wherein the deamidated variant accounts for up to 23% of the total anti-BAFF-R antibodies and fragments thereof in the composition.
71. The composition of embodiment 70, wherein the deamidated variant accounts for up to 1% of the total anti-BAFF-R antibodies and fragments thereof in the composition.
72. The composition of any one of embodiments 67-71, wherein the afucosylated anti-BAFF-R antibodies comprise a high molecular weight variant, and wherein the high molecular weight variant accounts for up to 6% of the total anti-BAFF-R antibodies and fragments thereof in the composition.
73. The composition of embodiment 72, wherein the high molecular weight variant accounts for up to 1% of the total anti-BAFF-R antibodies and fragments thereof in the composition.
74. The composition of any one of embodiments 67-73, wherein the afucosylated anti-BAFF-R antibodies comprise a methionine-oxidized variant comprising methionine oxidation at one or more of light chain residue 97, heavy chain residue 259, heavy chain residue 365, and heavy chain residue 435.
75. The composition of embodiment 74, wherein the methionine-oxidized variant comprises methionine oxidation at light chain residue 97, and the methionine-oxidized variant comprising oxidation at light chain residue 97 accounts for up to 62% of the total anti-BAFF-R antibodies and fragments thereof in the composition.
76. The composition of embodiment 75, wherein the methionine-oxidized variant comprising methionine oxidation at light chain residue 97 accounts for up to 4% of the total anti-BAFF-R antibodies and fragments thereof in the composition.
77. The composition of any one of embodiments 74-76, wherein the methionine-oxidized variant comprises methionine oxidation at one or more of heavy chain residues 259, 365, and 435, and wherein the methionine-oxidized variant comprising oxidation at one or more of heavy chain residues 259, 365, and 435 accounts for up to 27% of the total anti-BAFF-R antibodies and fragments thereof in the composition.
78. The composition of embodiment 77, wherein the methionine-oxidized variant comprising methionine oxidation at one or more of heavy chain residues 259, 365, and 435 accounts for up to 6% of the total anti-BAFF-R antibodies and fragments thereof in the composition.
79. The composition of any one of embodiments 67-78, wherein the afucosylated anti-BAFF-R antibodies further comprise a clipped variant comprising clipping between heavy chain residues 56 and 57.
80. The composition of embodiment 79, wherein the clipped variant accounts for up to 10% of the total anti-BAFF-R antibodies and fragments thereof in the composition.
81. A composition comprising afucosylated anti-BAFF-R antibodies, wherein the afucosylated anti-BAFF-R antibodies comprise a methionine-oxidized variant comprising methionine oxidation at one or more of light chain residue 97, heavy chain residue 259, heavy chain residue 365, and heavy chain residue 435.
82. The composition of embodiment 81, wherein the methionine-oxidized variant comprises methionine oxidation at light chain residue 97, and wherein the methionine-oxidized variant comprising oxidation at light chain residue 97 accounts for up to 62% of the total anti-BAFF-R antibodies and fragments thereof in the composition.
83. The composition of embodiment 82, wherein the methionine-oxidized variant comprising methionine oxidation at light chain residue 97 accounts for up to 4% of the total anti-BAFF-R antibodies and fragments thereof in the composition.
84. The composition of any one of embodiments 81-83, wherein the methionine-oxidized variant comprises methionine oxidation at one or more of heavy chain residues 259, 365, and 435, and wherein the methionine-oxidized variant comprising oxidation at one or more of heavy chain residues 259, 365, and 435 accounts for up to 27% of the total anti-BAFF-R antibodies and fragments thereof in the composition.
85. The composition of embodiment 84, wherein the methionine-oxidized variant comprising methionine oxidation at one or more of heavy chain residues 259, 365, and 435 accounts for up to 6% of the total anti-BAFF-R antibodies and fragments thereof in the composition.
86. The composition of any one of embodiments 81-85, wherein the afucosylated anti-BAFF-R antibodies further comprise a deamidated variant comprising deamidation at position N332, and wherein the deamidated variant accounts for up to 23% of the total anti-BAFF-R antibodies and fragments thereof in the composition.
87. The composition of embodiment 86, wherein the deamidated variant accounts for up to 1% of the total anti-BAFF-R antibodies and fragments thereof in the composition.
88. The composition of any one of embodiments 81-87, wherein the afucosylated anti-BAFF-R antibodies further comprise a tryptophan-oxidized variant comprising oxidation at heavy chain residue 104 and/or heavy chain residue 59.
89. The composition of embodiment 88, wherein the tryptophan-oxidized variant accounts for up to 16% of the total anti-BAFF-R antibodies and fragments thereof in the composition.
90. The composition of embodiment 89, wherein the tryptophan-oxidized variant accounts for up to 3% of the total anti-BAFF-R antibodies and fragments thereof in the composition.
91. The composition of any one of embodiments 81-90, wherein the afucosylated anti-BAFF-R antibodies comprise a high molecular weight variant, wherein the high molecular weight variant accounts for up to 6% of the total anti-BAFF-R antibodies and fragments thereof in the composition.
92. The composition of embodiment 91, wherein the high molecular weight variant accounts for up to 1% of the total anti-BAFF-R antibodies and fragments thereof in the composition.
93. The composition of any one of embodiments 81-92, wherein the afucosylated anti-BAFF-R antibodies further comprise a clipped variant comprising clipping between heavy chain residues 56 and 57.
94. The composition of embodiment 93, wherein the clipped variant accounts for up to 10% of the total anti-BAFF-R antibodies and fragments thereof in the composition.
95. A composition comprising afucosylated anti-BAFF-R antibodies, wherein the afucosylated anti-BAFF-R antibodies comprise a clipped variant comprising clipping between heavy chain residues 56 and 57.
96. The composition of embodiment 95, wherein the clipped variant accounts for up to 10% of the total anti-BAFF-R antibodies and fragments thereof in the composition.
97. The composition of embodiment 95 or 96, wherein the afucosylated anti-BAFF-R antibodies further comprise a deamidated variant comprising deamidation at position N332, and wherein the deamidated variant accounts for up to 23% of the total anti-BAFF-R antibodies and fragments thereof in the composition.
98. The composition of embodiment 97, wherein the deamidated variant accounts for up to 1% of the total anti-BAFF-R antibodies and fragments thereof in the composition.
99. The composition of any one of embodiments 95-98, wherein the afucosylated anti-BAFF-R antibodies further comprise a tryptophan-oxidized variant comprising oxidation at heavy chain residue 104 and/or heavy chain residue 59.
100. The composition of embodiment 99, wherein the tryptophan-oxidized variant accounts for up to 16% of the total anti-BAFF-R antibodies and fragments thereof in the composition.
101. The composition of embodiment 100, wherein the tryptophan-oxidized variant accounts for up to 3% of the total anti-BAFF-R antibodies and fragments thereof in the composition.
102. The composition of any one of embodiments 95-101, wherein the afucosylated anti-BAFF-R antibodies further comprise a methionine-oxidized variant comprising methionine oxidation at one or more of light chain residue 97, heavy chain residue 259, heavy chain residue 365, and heavy chain residue 435.
103. The composition of embodiment 102, wherein the methionine-oxidized variant comprises methionine oxidation at light chain residue 97, and wherein the methionine-oxidized variant comprising methionine oxidation at light chain residue 97 accounts for up to 62% of the total anti-BAFF-R antibodies and fragments thereof in the composition.
104. The composition of embodiment 103, wherein the methionine-oxidized variant comprising methionine oxidation at light chain residue 97 accounts for up to 4% of the total anti-BAFF-R antibodies and fragments thereof in the composition.
105. The composition of any one of embodiments 102-104, wherein the methionine-oxidized variant comprises methionine oxidation at one or more of heavy chain residues 259, 365, and 435, and the methionine-oxidized variant comprising methionine oxidation at one or more of heavy chain residues 259, 365, and 435 accounts for up to 27% of the total anti-BAFF-R antibodies and fragments thereof in the composition.
106. The composition of embodiment 105, wherein the methionine-oxidized variant comprising oxidation at one or more of heavy chain residues 259, 365, and 435 accounts for up to 6% of the total anti-BAFF-R antibodies and fragments thereof in the composition.
107. The composition of any one of embodiments 95-106, wherein the afucosylated anti-BAFF-R antibodies further comprise a high molecular weight variant, and wherein the high molecular weight variant accounts for up to 6% of the total anti-BAFF-R antibodies and fragments thereof in the composition.
108. The composition of embodiment 107, wherein the high molecular weight variant accounts for up to 1% of the total anti-BAFF-R antibodies and fragments thereof in the composition.
109. The composition of any one of embodiments 40-108, wherein the composition comprises N-glycosylated Fc regions (e.g., N-glycosylated Fc regions of the anti-BAFF-R antibodies).
110. The composition of embodiment 109, wherein at least 95% of the Fc regions are N-glycosylated.
111. The composition of embodiment 109 or 110, wherein at least 90% of the N-glycosylated Fc regions are afucosylated.
112. The composition of any one of embodiments 109-111, wherein 0% to 10% of the N-glycosylated Fc regions comprise a high mannose glycan.
113. The composition of embodiment 112, wherein 0% to 7% of the N-glycosylated Fc regions comprise a high mannose glycan.
114. The composition of embodiment 113, wherein 0.5% to 4% of the N-glycosylated Fc regions comprise a high mannose glycan.
115. The composition of any one of embodiments 109-114, wherein 0% to 49% of the N-glycosylated Fc regions are galactosylated.
116. The composition of embodiment 115, wherein 15% to 38% of the N-glycosylated Fc regions are galactosylated.
117. The composition of any one of embodiments 40-116, wherein the composition has a relative ADCC potency of about 70% to about 130% in a cell-based ADCC assay compared to a reference standard (e.g., wherein the reference standard is ianalumab, e.g., ianalumab made according to GMP).
118. The composition of embodiment 117, wherein the composition has a relative ADCC potency of about 80% to about 116% in a cell-based ADCC assay compared to a reference standard (e.g., wherein the reference standard is ianalumab, e.g., ianalumab made according to GMP).
119. The composition of any one of embodiments 40-118, wherein the composition has an ADCC potency within a reference range of ADCC potencies from two lots of a reference standard (e.g., wherein the reference standard is ianalumab, e.g., ianalumab made according to GMP).
120. The composition of any one of embodiments 40-119, wherein the anti-BAFF-R antibodies have a relative ADCC potency from about 70% to about 130% in a cell-based ADCC assay as compared to a reference standard (e.g., wherein the reference standard is ianalumab, e.g., ianalumab made according to GMP).
121. The composition of any one of embodiments 40-120, wherein the anti-BAFF-R antibodies have: (a) a binding affinity (K_{D}) to FcγRIIIa^{F158} from 1.3 nM to 3.9 nM; (b) a binding affinity to FcγRIIIa^{V158} from 0.52 nM to 1.59 nM; (c) a relative binding affinity to FcγRIIIa^{F158} and/or FcγRIIIa^{V158} from about 70% to about 130% compared to a reference standard (e.g., wherein the reference standard is ianalumab, e.g., ianalumab made according to GMP).
122. The composition of any one of embodiments 117-121, wherein the reference standard is ianalumab (e.g., ianalumab made according to GMP).
123. The composition of any one of embodiments 40-122, wherein the anti-BAFF-R antibodies have a serum half-life of at least 5 days.
124. The composition of any one of embodiments 1-123, wherein the anti-BAFF-R antibodies comprise a variable heavy chain (VH) comprising an amino acid sequence of at least 95% sequence identity to SEQ ID NO: 3 and/or a variable light chain (VL) comprising an amino acid sequence of at least 95% sequence identity to SEQ ID NO: 4.
125. The composition of embodiment 124, wherein the VH comprises the amino acid sequence of SEQ ID NO: 3 and/or the VL comprises the amino acid sequence of SEQ ID NO: 4.
126. The composition of any one of embodiments 1-125, wherein the anti-BAFF-R antibodies comprise a heavy chain comprising an amino acid sequence of at least 95% sequence identity to SEQ ID NO: 1 and a light chain comprising an amino acid sequence of at least 95% identity to the amino acid sequence of SEQ ID NO: 2.
127. The composition of embodiment 126, wherein the heavy chain comprises the amino acid sequence of SEQ ID NO: 1 and the light chain comprises the amino acid sequence of SEQ ID NO: 2.
128. The composition of any one of embodiments 1-127, wherein the anti-BAFF-R antibodies are IgG1 antibodies.
129. The composition of embodiment 128, wherein the IgG1 antibodies are IgG1κ antibodies.
130. The composition of embodiment 129, wherein the anti-BAFF-R antibody is ianalumab.
131. The composition of any one of embodiments 1-130, wherein the composition is a single batch preparation.
132. The composition of any one of embodiments 1-131, wherein the anti-BAFF-R antibodies are produced in a non-human cell.
133. The composition of embodiment 132, wherein the non-human cell is a recombinant Chinese hamster ovary (CHO) cell.
134. The composition of embodiment 133, wherein the CHO cell has impaired fucosylation compared to a wild-type CHO cell.
135. The composition of any one of embodiments 1-134, wherein the composition is in the form of a liquid pharmaceutical formulation suitable for administration to a subject in need thereof.
136. Isolated anti-BAFF-R antibodies produced in a non-human cell, wherein the antibodies are the anti-BAFF-R antibodies of the composition of any one of embodiments 1-135.
137. A single batch preparation of anti-BAFF-R antibodies, wherein the anti-BAFF-R antibodies are the isolated anti-BAFF-R antibodies of embodiment 136.
138. A pharmaceutical formulation comprising: (a) the composition of any one of embodiments 1-135 and a pharmaceutically acceptable carrier, (b) the isolated anti-BAFF-R antibodies of embodiment 136 and a pharmaceutically acceptable carrier, or (c) the single batch preparation of embodiment 137 and a pharmaceutically acceptable carrier.
139. The pharmaceutical formulation of embodiment 138, wherein the pharmaceutical formulation is in the form of a liquid suitable for administration to a subject in need thereof.
140. A lyophilisate obtainable by lyophilisation of the pharmaceutical formulation of embodiment 138 or 139.
141. A pharmaceutical product comprising a container and the pharmaceutical formulation of embodiment 138 or 139 or the lyophilisate of embodiment 140.
142. The pharmaceutical product of embodiment 141, comprising a vial, an injection device, an injection pen, a cartridge, a pre-filled syringe, or an autoinjector.
143. The pharmaceutical product of embodiment 141 or 142, further comprising a package insert and/or instructions for use.
144. The pharmaceutical product of embodiments 142 or 143, comprising a pre-filled syringe containing the pharmaceutical formulation at 150 mg of the anti-BAFF-R antibody in 1 mL volume or 300 mg of the anti-BAFF-R antibody in 2 mL volume.
145. The pharmaceutical product of embodiments 142 or 143, comprising a vial containing the pharmaceutical formulation at 150 mg of the anti-BAFF-R antibody in 1 mL volume or 450 mg of the anti-BAFF-R antibody in 3 mL volume.
146. The composition of any one of embodiments 1-135, the isolated anti-BAFF-R antibodies of embodiment 136, the single batch preparation of embodiment 137, the pharmaceutical formulation of embodiment 138 or 139, the lyophilisate of embodiment 140, or the pharmaceutical product of any one of embodiments 141-145, wherein the anti-BAFF-R antibody is produced by a method of manufacture comprising a fucosylation-deficient host cell culture.
147. The composition, isolated anti-BAFF-R antibodies, single batch preparation, pharmaceutical formulation, lyophilisate, or pharmaceutical product of embodiment 146, wherein the fucosylation-deficient host cell culture produces high mannose N-glycans in 0% to 10% of the anti-BAFF-R antibodies.
148. A method of manufacturing a pharmaceutical formulation comprising anti-BAFF antibodies having an ADCC-dependent mechanism of action, the method comprising:
   (a) culturing a recombinant cell line at a pH from about 6.8 to about 7.15 to express a population of anti-BAFF-R antibodies having an antibody-dependent cellular cytotoxicity (ADCC) dependent mechanism of action; and
   (b) formulating the population of anti-BAFF-R antibodies expressed during step (a) into a pharmaceutical formulation comprising a pharmaceutically acceptable carrier.
149. The method of embodiment 148, wherein step (a) comprises a starting viable cell density from 1.45x10⁶ cells/mL to 4x10⁶ cells/mL.
150. A method of manufacturing a pharmaceutical formulation comprising anti-BAFF antibodies having an antibody-dependent cellular cytotoxicity (ADCC) dependent mechanism of action, the method comprising:
   (a) culturing a recombinant cell line to express a population of anti-BAFF-R antibodies having an ADCC-dependent mechanism of action, wherein the viable cell density of the recombinant cell line at the start of culturing is from 1.45x10⁶ cells/mL to 4x10⁶ cells/mL; and
   (b) formulating the population of anti-BAFF-R antibodies expressed during step (a) into a pharmaceutical formulation comprising a pharmaceutically acceptable carrier.
151. The method of embodiment 150, wherein step (a) is at a pH from 6.8 to 7.15.
152. The method of any one of embodiments 148-151, wherein at least 95% of the population of anti-BAFF-R antibodies comprises afucosylated N-glycosylated Fc regions.
153. The method of any one of embodiments 148-152, wherein the recombinant cell line is fucosylation deficient.
154. The method of any one of embodiments 148-153, wherein step (a) comprises a temperature switch from a first temperature to a second temperature, wherein the second temperature is lower than the first temperature.
155. The method of embodiment 154, wherein the second temperature is less than about 36.5 °C.
156. The method of embodiment 155, wherein the second temperature is from about 32.5 ° C to about 34.0 ° C.
157. The method of any one of embodiments 154-156, wherein the first temperature is from about 35.8 °C to about 37.2 °C.
158. The method of any one of embodiments 154-157, wherein the temperature switch occurs when the recombinant cell line is at a viable cell density from 10x10⁶ cells/mL to 16x10⁶ cells/mL.
159. The method of any one of embodiments 148-158, wherein the duration of step (a) is from about 300 hours to about 350 hours.
160. The method of any one of embodiments 148-159, wherein step (a) is in a fed-batch production bioreactor.
161. The method of any one of embodiments 148-160, further comprising, prior to step (a), culturing the recombinant cell line in a seed bioreactor.
162. The method of any one of embodiments 148-161, further comprising comparing an ADCC potency of the population of anti-BAFF-R antibodies to a target range of ADCC potency, the method comprising:
   (c) identifying the target range of ADCC potency, wherein the target range is defined by a range of ADCC potencies of two lots of a reference standard;
   (d) measuring the ADCC potency of the population of anti-BAFF-R antibodies; and
   (e) determining whether the ADCC potency of the population of anti-BAFF-R antibodies falls within the target range.
163. The method of embodiment 162, wherein the reference standard is ianalumab (e.g., ianalumab made according to good manufacturing practices).
164. The method of embodiment 162 or 163, further comprising recommending the population of anti-BAFF-R antibodies, or a pharmaceutical formulation or pharmaceutical product thereof, as a treatment of a BAFF-R-related disorder, wherein step (e) results in a determination that the ADCC potency of the population of anti-BAFF-R antibodies falls within the target range.
165. The method of any one of embodiments 148-164, wherein 95% to 100% of the population of antibodies expressed during the culturing step (a) are afucosylated.
166. The method of embodiment 165, wherein 99% or more of the population of antibodies expressed during the culturing step (a) are afucosylated.
167. The method of any one of embodiments 148-166, wherein 0% to 50% of the population of antibodies expressed during the culturing step (a) comprises galactosylated N-glycans.
168. The method of any one of embodiments 148-167, wherein the Fc region is a human IgG1 Fc region.
169. The method of embodiment 168, wherein the IgG1 Fc region is an IgG1κ Fc region.
170. The method of any one of embodiments 148-169, wherein the anti-BAFF-R antibody comprises a heavy chain having at least 95% identity to the amino acid sequence of SEQ ID NO: 1 and a light chain having at least 95% identity to the amino acid sequence of SEQ ID NO: 2.
171. The method of embodiment 170, wherein the heavy chain comprises the amino acid sequence of SEQ ID NO: 1 and the light chain comprises the amino acid sequence of SEQ ID NO: 2.
172. The method of any one of embodiments 148-171, wherein the anti-BAFF antibodies having an ADCC-dependent mechanism of action are the isolated anti-BAFF antibodies of embodiment 136.
173. The method of any one of embodiments 148-172, further comprising lyophilizing the pharmaceutical formulation to produce a lyophilized pharmaceutical formulation.
174. The method of any one of embodiments 148-172, further comprising preparing a pharmaceutical product comprising a container by dispensing the pharmaceutical formulation into the container.
175. The method of embodiment 174, wherein the pharmaceutical formulation in the container is a liquid pharmaceutical formulation.
176. The method of embodiment 175, wherein the container is a vial, an injection device, an injection pen, a vial and syringe, a cartridge, a pre-filled syringe, or an autoinjector.
177. The method of embodiment 176, wherein the container is a pre-filled syringe containing the pharmaceutical formulation at 150 mg of the anti-BAFF-R antibody in 1 mL volume or 300 mg of the anti-BAFF-R antibody in 2 mL volume.
178. The method of embodiment 176, wherein the container is a vial containing the pharmaceutical formulation at 150 mg of the anti-BAFF-R antibody in 1 mL volume or 450 mg of the anti-BAFF-R antibody in 3 mL volume.
179. A method of matching an antibody-dependent cellular cytotoxicity (ADCC) activity of a reference standard comprising:
   (a) determining an ADCC activity of the reference standard;
   (b) determining an ADCC activity of a second antibody composition comprising an anti-BAFF-R antibody having the same antibody sequence as the reference standard; and
   (c) changing the ADCC activity of the second antibody composition by increasing or decreasing the amount of high-mannose glycans of one or more antibodies within the second antibody composition, and wherein the ADCC activity of the second antibody composition after increasing or decreasing the amount of high-mannose is the same as the reference standard or within about 10%, about 15%, about 20%, about 25%, about 30%, about 35%, about 40%, about 45% or about 50% of the reference standard or within about 1% to about 50% of the reference standard.
180. A method for engineering a target antibody-dependent cellular cytotoxicity (ADCC) activity of an afucosylated anti-BAFF-R antibody composition comprising:
   (a) determining an ADCC activity of the afucosylated anti-BAFF-R antibody composition;
   (b) determining a target ADCC activity; and
   (c) increasing or decreasing the ADCC activity of the afucosylated anti-BAFF-R antibody composition by increasing or decreasing the amount high-mannose glycans in the Fc region of the antibody, and wherein the ADCC activity of the afucosylated anti-BAFF-R antibody composition after increasing or decreasing the amount of high-mannose glycans is the same as the target ADCC activity or within about 10%, about 15%, about 20%, about 25%, about 30%, about 35%, about 40%, about 45% or about 50% of the target ADCC activity or within about 1% to about 50% of the target ADCC activity.
181. The method of embodiment 179 or 180, wherein step (a) occurs before or at the same time as step (b) and/or at the same time as step (c).
182. The method of embodiment 179 or 180, wherein step (a) occurs after step (b) and before step (c).
183. The method of any one of embodiments 179-182, wherein an increase of about 1% high-mannose glycans increases ADCC activity by about 20% to about 30%.
184. The method of embodiment 179, wherein the reference standard is ianalumab.
185. The composition of any one of embodiments 1-135, the isolated anti-BAFF-R antibodies of embodiment 136, the single batch preparation of embodiment 137, the pharmaceutical formulation of embodiment 138 or 139, the lyophilisate of embodiment 140, the pharmaceutical product of any one of embodiments 141-145, or the pharmaceutical formulation manufactured by the method of any one of embodiments 148-178, for use in a method of treating a BAFF-R-related disorder in a subject.
186. A method of treating a BAFF-R-related disorder in a subject, the method comprising administering an effective amount of the composition of any one of embodiments 1-135, the isolated anti-BAFF-R antibodies of embodiment 136, the single batch preparation of embodiment 137, the pharmaceutical formulation of embodiment 138 or 139, the lyophilisate of embodiment 140, the pharmaceutical product of any one of embodiments 141-145, or the pharmaceutical formulation manufactured by the method of any one of embodiments 148-178.
187. The composition, isolated anti-BAFF-R antibodies, single batch preparation, pharmaceutical formulation, lyophilisate, or pharmaceutical product for use of embodiment 185, or the method of embodiment 186, wherein the BAFF-R-related disorder is an autoimmune disease or a B-cell neoplasm.
188. The composition, isolated anti-BAFF-R antibodies, single batch preparation, pharmaceutical formulation, lyophilisate, or pharmaceutical product for use, or method, of embodiment 187, wherein the subject is a human.
189. The composition, isolated anti-BAFF-R antibodies, single batch preparation, pharmaceutical formulation, lyophilisate, or pharmaceutical product for use, or method, of any one of embodiments 185-188, wherein the anti-BAFF-R antibody is administered at a dose of about 3 mg to about 10 mg anti-BAFF-R antibody per kilogram of the subject (mg/kg).
190. The composition, isolated anti-BAFF-R antibodies, single batch preparation, pharmaceutical formulation, lyophilisate, or pharmaceutical product for use, or method, of embodiment 189, wherein the anti-BAFF-R antibody is administered at a dose of about 3 mg/kg or about 9 mg/kg.
191. The composition, isolated anti-BAFF-R antibodies, single batch preparation, pharmaceutical formulation, lyophilisate, or pharmaceutical product for use, or method, of any one of embodiments 185-190, wherein the anti-BAFF-R antibody is administered intravenously.
192. The composition, isolated anti-BAFF-R antibodies, single batch preparation, pharmaceutical formulation, lyophilisate, or pharmaceutical product for use, or method, of embodiment 191, wherein the anti-BAFF-R antibody is administered at a dose of about 150 mg to about 400 mg anti-BAFF-R antibody, such as about 300 mg active ingredient.
193. The composition, isolated anti-BAFF-R antibodies, single batch preparation, pharmaceutical formulation, or pharmaceutical product for use, or method, of embodiment 192, wherein the dose is 150 mg anti-BAFF-R antibody or 300 mg anti-BAFF-R antibody.
194. The composition, isolated anti-BAFF-R antibodies, single batch preparation, pharmaceutical formulation, or pharmaceutical product for use, or method, of embodiment 192 or 193, wherein the anti-BAFF-R antibody is administered to a subject in need thereof subcutaneously.
195. The composition, isolated anti-BAFF-R antibodies, single batch preparation, pharmaceutical formulation, or pharmaceutical product for use, or method, of any one of embodiments 192-194, wherein the anti-BAFF-R antibody is administered to a subject in need thereof once every 4 weeks.
196. The composition, isolated anti-BAFF-R antibodies, single batch preparation, pharmaceutical formulation, or pharmaceutical product for use, or method, of any one of embodiments 192-194, wherein the anti-BAFF-R antibody is administered to a subject monthly.
197. The composition, isolated anti-BAFF-R antibodies, single batch preparation, pharmaceutical formulation, or pharmaceutical product for use, or method, of any one of embodiments 192-194, wherein the anti-BAFF-R antibody is administered to a subject once every 12 weeks.
198. The composition, isolated anti-BAFF-R antibodies, single batch preparation, pharmaceutical formulation, or pharmaceutical product for use, or method, of any one of embodiments 192-194, wherein the anti-BAFF-R antibody is administered to a subject once every three months.
199. The composition, isolated anti-BAFF-R antibodies, single batch preparation, pharmaceutical formulation, or pharmaceutical product for use, or method, of any one of embodiments 185-198, wherein the BAFF-R-related disorder is an autoimmune disease selected from an autoimmune haematological disorder, acquired hemophilia A, cold agglutinin disease, cryoglobulinemia, thrombotic thrombocytopenic purpura, Sjögren's Disease, systemic lupus erythematosus, lupus nephritis, inflammatory muscle disorders, polychondritis, sclerodoma, anti-neutrophil cytoplasmic antibody-associated vasculitis, IgM mediated neuropathy, opsoclonus myoclonus syndrome, Wegener granulomatosis, dermatomyositis, chronic active hepatitis, myasthenia gravis, psoriasis, Steven-Johnson syndrome, pemphigus vulgaris, pemphigus foliacius, idiopathic sprue, autoimmune inflammatory bowel disease, endocrine ophthalmopathy, Graves' disease, sarcoidosis, multiple sclerosis, neuromyelitis optica, primary biliary cirrhosis, juvenile diabetes, uveitis, keratoconjunctivitis sicca and vernal keratoconjunctivitis, interstitial lung fibrosis, psoriatic arthritis, glomerulonephritis, an inflammatory disease of skin, an inflammatory disease of the cornea, myositis, loosening of bone implants, and a metabolic disorder.
200. The composition, isolated anti-BAFF-R antibodies, single batch preparation, pharmaceutical formulation, or pharmaceutical product for use, or method, of embodiment 199, wherein the BAFF-R-related disorder is an autoimmune haematological disorder selected from warm autoimmune hemolytic anaemia, aplastic anaemia, pure red cell anaemia, and immune thrombocytopenia.
201. The composition, isolated anti-BAFF-R antibodies, single batch preparation, pharmaceutical formulation, or pharmaceutical product for use, or method, of embodiment 199, wherein the BAFF-R-related disorder is an autoimmune inflammatory bowel disease selected from ulcerative colitis, Crohn's disease, and irritable bowel syndrome.
202. The composition, isolated anti-BAFF-R antibodies, single batch preparation, pharmaceutical formulation, or pharmaceutical product for use, or method, of embodiment 199, wherein the BAFF-R-related disorder is a glomerulonephritis including idiopathic nephrotic syndrome or minimal change nephropathy.
203. The composition, isolated anti-BAFF-R antibodies, single batch preparation, pharmaceutical formulation, or pharmaceutical product for use, or method, of embodiment 199, wherein the BAFF-R-related disorder is a metabolic disorder selected from atherosclerosis, diabetes, and dislipidemia.
204. The composition, isolated anti-BAFF-R antibodies, single batch preparation, pharmaceutical formulation, or pharmaceutical product for use, or method, of embodiment 200, wherein the autoimmune haematological disorder is warm autoimmune hemolytic anemia.
205. The composition, isolated anti-BAFF-R antibodies, single batch preparation, pharmaceutical formulation, or pharmaceutical product for use, or method, of embodiment 204, wherein the anti-BAFF-R antibody is intravenously administered to a subject in need thereof at a dose of about 3 mg/kg or about 9 mg/kg once every 4 weeks for a duration of up to 4 months.
206. The composition, isolated anti-BAFF-R antibodies, single batch preparation, pharmaceutical formulation, or pharmaceutical product for use, or method, of embodiment 200, wherein the autoimmune haematological disorder is immune thrombocytopenia.
207. The composition, isolated anti-BAFF-R antibodies, single batch preparation, pharmaceutical formulation, or pharmaceutical product for use, or method, of embodiment 206, wherein the anti-BAFF-R antibody is intravenously administered to a subject in need thereof at a dose of about 3 mg/kg or about 9 mg/kg once every 4 weeks for a duration of up to 4 months.
208. The composition, isolated anti-BAFF-R antibodies, single batch preparation, pharmaceutical formulation, or pharmaceutical product for use, or method, of any one of embodiments 185-198, wherein the BAFF-R-related disorder is an autoimmune disease selected from Sjögren's Disease, systemic lupus erythematosus, lupus nephritis, hidradenitis suppurativa, and scleroderma.
209. The composition of embodiment 208, wherein the anti-BAFF-R antibody is subcutaneously administered to a subject in need thereof at a dose of about 300 mg once every 4 weeks or once every 12 weeks.

### Numerated Embodiments - Set C

1. A composition comprising anti-BAFF-R antibodies, wherein the anti-BAFF-R antibodies comprise at least one variant selected from:
   (a) a high mannose N-glycan variant,
   (b) a high molecular weight variant,
   (c) a deamidated variant,
   (d) a tryptophan-oxidized variant,
   (e) a methionine-oxidized variant, and
   (f) a clipped variant.
2. The composition of embodiment 1, wherein the anti-BAFF-R antibodies comprise at least two variants selected from:
   (a) a high mannose N-glycan variant,
   (b) a high molecular weight variant,
   (c) a deamidated variant,
   (d) a tryptophan-oxidized variant,
   (e) a methionine-oxidized variant, and
   (f) a clipped variant.
3. The composition of embodiment 2, wherein the anti-BAFF-R antibodies comprise at least three variants selected from:
   (a) a high mannose N-glycan variant,
   (b) a high molecular weight variant,
   (c) a deamidated variant,
   (d) a tryptophan-oxidized variant,
   (e) a methionine-oxidized variant, and
   (f) a clipped variant.
4. The composition of embodiment 3, wherein the anti-BAFF-R antibodies comprise at least four variants selected from:
   (a) a high mannose N-glycan variant,
   (b) a high molecular weight variant,
   (c) a deamidated variant,
   (d) a tryptophan-oxidized variant,
   (e) a methionine-oxidized variant, and
   (f) a clipped variant.
5. The composition of embodiment 4, wherein the anti-BAFF-R antibodies comprise at least five variants selected from:
   (a) a high mannose N-glycan variant,
   (b) a high molecular weight variant,
   (c) a deamidated variant,
   (d) a tryptophan-oxidized variant,
   (e) a methionine-oxidized variant, and
   (f) a clipped variant.
6. The composition of embodiment 5, wherein the anti-BAFF-R antibodies comprise each of the following variants:
   (a) a high mannose N-glycan variant,
   (b) a high molecular weight variant,
   (c) a deamidated variant,
   (d) a tryptophan-oxidized variant,
   (e) a methionine-oxidized variant, and
   (f) a clipped variant.
7. The composition of any one of embodiments 1-6, wherein the high mannose N-glycan variant, if present, accounts for up to 10% of the total anti-BAFF-R antibodies and fragments thereof in the composition.
8. The composition of embodiment 7, wherein the high mannose N-glycan variant, if present, accounts for up to 7% of the total anti-BAFF-R antibodies and fragments thereof in the composition.
9. The composition of embodiment 8, wherein the high mannose N-glycan variant, if present, accounts for 0.5% to 4% of the total anti-BAFF-R antibodies and fragments thereof in the composition.
10. The composition of any one of embodiments 1-9, wherein the anti-BAFF-R antibodies comprise N-glycosylated Fc regions.
11. The composition of embodiment 10, wherein at least 95% of the Fc regions of the anti-BAFF-R antibodies are N-glycosylated.
12. The composition of embodiment 10 or 11, wherein at least 90% of the N-glycosylated Fc regions are afucosylated.
13. The composition of any one of embodiments 10-12, wherein 0% to 49% of the N-glycosylated Fc regions are galactosylated.
14. The composition of embodiment 13, wherein 15% to 38% of the N-glycosylated Fc regions are galactosylated.
15. The composition of any one of embodiments 1-14, wherein the high mannose glycan N-glycan variant is present in the composition.
16. The composition of any one of embodiments 1-15, wherein the high molecular weight variant, if present, accounts for up to 6% of the total anti-BAFF-R antibodies and fragments thereof in the composition.
17. The composition of embodiment 16, wherein the high molecular weight variant, if present, accounts for up to 1% of the total anti-BAFF-R antibodies and fragments thereof in the composition.
18. The composition of embodiment 16 or 17, wherein the percentage of the high molecular weight variant in the composition is determined by capillary electrophoresis with sodium dodecyl sulfate (CE-SDS) under non-reducing conditions.
19. The composition of embodiment 16 or 17, wherein the percentage of the high molecular weight variant in the composition is determined by CE-SDS under reducing conditions, or size exclusion chromatography (SEC).
20. The composition of any one of embodiments 1-19, wherein the deamidated variant, if present, comprises deamidation at position N332.
21. The composition of embodiment 20, wherein deamidation at position N332, if present, accounts for up to 23% of the total anti-BAFF-R antibodies and fragments thereof in the composition.
22. The composition of embodiment 21, wherein deamidation at position N332, if present, accounts for up to 1% of the total anti-BAFF-R antibodies and fragments thereof in the composition.
23. The composition of embodiment 21 or 22, wherein the percentage of the deamidated variant comprising deamidation at position N332 in the composition is determined by charge-based capillary zone electrophoresis (CZE).
24. The composition of any one of embodiments 1-23, wherein the tryptophan-oxidized variant, if present, comprises oxidation at heavy chain residue 104 and/or heavy chain residue 59.
25. The composition of embodiment 24, wherein oxidation at heavy chain residue 104 and/or heavy chain residue 59, if present, accounts for up to 16% of the total anti-BAFF-R antibodies and fragments thereof in the composition.
26. The composition of embodiment 25, wherein oxidation at heavy chain residue 104 and/or heavy chain residue 59, if present, accounts for up to 3% of the total anti-BAFF-R antibodies and fragments thereof in the composition.
27. The composition of embodiment 25 or 26, wherein the percentage of the tryptophan-oxidized variant comprising oxidation at heavy chain residue 104 and/or heavy chain residue 59 in the composition is determined by peptide mapping with mass spectrometry (MS) detection.
28. The composition of any one of embodiments 1-27, wherein the methionine-oxidized variant, if present, comprises oxidation at one or more of light chain residue 97, heavy chain residue 259, heavy chain residue 365, and heavy chain residue 435.
29. The composition of embodiment 28, wherein the methionine-oxidized variant, if present, comprises oxidation at light chain residue 97.
30. The composition of embodiment 29, wherein oxidation at light chain residue 97, if present, accounts for up to 62% of the total anti-BAFF-R antibodies and fragments thereof in the composition.
31. The composition of embodiment 30, wherein oxidation at light chain residue 97, if present, accounts for up to 4% of the total anti-BAFF-R antibodies and fragments thereof in the composition.
32. The composition of embodiment 30 or 31, wherein the percentage of the methionine-oxidized variant comprising oxidation at light chain residue 97 in the composition is determined by peptide mapping with mass spectrometry (MS) detection.
33. The composition of embodiment 28, wherein the methionine-oxidized variant, if present, comprises oxidation at one or more of heavy chain residues 259, 365, and 435.
34. The composition of embodiment 33, wherein oxidation at one or more of heavy chain residues 259, 365, and 435, if present, accounts for up to 27% of the total anti-BAFF-R antibodies and fragments thereof in the composition.
35. The composition of embodiment 34, wherein oxidation at one or more of heavy chain residues 259, 365, and 435, if present, accounts for up to 6% of the total anti-BAFF-R antibodies and fragments thereof in the composition.
36. The composition of embodiment 34 or 35, wherein the percentage of the methionine-oxidized variant comprising oxidation at one or more of heavy chain residues 259, 365, and 435 in the composition is determined by peptide mapping with mass spectrometry (MS) detection.
37. The composition of any one of embodiments 1-36, wherein the clipped variant, if present, comprises clipping between heavy chain residues 56 and 57.
38. The composition of embodiment 36, wherein the clipped variant, if present, accounts for up to 10% of the total anti-BAFF-R antibodies and fragments thereof in the composition.
39. The composition of embodiment 38, wherein the percentage of the clipped variant comprising clipping between heavy chain residues 56 and 57 in the composition is determined by charge-based capillary zone electrophoresis (CZE).
40. The composition of any one of embodiments 1-39, wherein the composition has a relative antibody-dependent cell-mediated cytotoxicity (ADCC) potency of 70% to 130% in a cell-based ADCC assay compared to a reference standard.
41. The composition of embodiment 40, wherein the composition has a relative ADCC potency of 80% to 116% in a cell-based ADCC assay compared to the reference standard.
42. The composition of any one of embodiments 1-39, wherein the composition has an antibody-dependent cell-mediated cytotoxicity (ADCC) potency in a cell-based ADCC assay within a reference range of ADCC potencies from two lots of a reference standard.
43. The composition of embodiment 40 or 41, wherein the composition has an antibody-dependent cell-mediated cytotoxicity (ADCC) potency in a cell-based ADCC assay within a reference range of ADCC potencies from two lots of the reference standard.
44. The composition of any one of embodiments 40-43, wherein the cell-based ADCC assay is a BAFF-R-expressing NK cell-based ADCC potency assay.
45. The composition of any one of embodiments 1-44, wherein the composition depletes human B cells through antibody-dependent cell-mediated cytotoxicity (ADCC) with a half-maximal efficacy (EC₅₀): (a) from 10 pM to 1 nM in an *in vitro* culture of human whole blood; and/or (b) from 0.2 pM to 100 pM in an *in vitro* culture of human peripheral blood mononuclear cells.
46. The composition of any one of embodiments 1-39, wherein the anti-BAFF-R antibodies have: (a) a binding affinity (K_{D}) to FcγRIIIa^{F158} from 1.3 nM to 3.9 nM; (b) a binding affinity to FcγRIIIa^{V158} from 0.52 nM to 1.59 nM; or (c) a relative binding affinity to FcγRIIIa^{F158} and/or FcγRIIIa^{V158} from about 70% to about 130% compared to a reference standard.
47. The composition of any one of embodiments 40-45, wherein the anti-BAFF-R antibodies have: (a) a binding affinity (K_{D}) to FcγRIIIa^{F158} from 1.3 nM to 3.9 nM; (b) a binding affinity to FcγRIIIa^{V158} from 0.52 nM to 1.59 nM; or (c) a relative binding affinity to FcγRIIIa^{F158} and/or FcγRIIIa^{V158} from about 70% to about 130% compared to the reference standard.
48. The composition of any one of embodiments 40-47, wherein the reference standard is ianalumab.
49. The composition of embodiment 48, wherein the reference standard is ianalumab made according to good manufacturing practices.
50. The composition of any one of embodiments 1-49, wherein the anti-BAFF-R antibodies have a serum half-life of at least 5 days.
51. The composition of any one of embodiments 1-50, wherein the anti-BAFF-R antibodies bind to a BAFF-R epitope comprising one or more of residues D26, L27, L28, V29, R30, H31, C32, V33, A34, and C35..
52. The composition of any one of embodiments 1-51, wherein the anti-BAFF-R antibodies comprise one or more anti-BAFF-R paratopic residues comprising one or more of heavy chain residues N32, S33, A34, A35, R52, Y54, R56, S57, Y60, Y102, D103, W104, V110 and/or one or more of light chain residues L92, Y93, and S94.
53. The composition of any one of embodiments 1-52, wherein the anti-BAFF-R antibodies comprise a CDR-H1, a CDR-H2, and a CDR-H3 having the amino acid sequences of SEQ ID NO: 5, SEQ ID NO: 6, and SEQ ID NO: 7, and a CDR-L1, a CDR-L2, and a CDR-L3 having the amino acid sequences of SEQ ID NO: 8, SEQ ID NO: 9, and SEQ ID NO: 10, respectively.
54. The composition of any one of embodiments 1-53, wherein the anti-BAFF-R antibodies comprise a variable heavy chain (VH) comprising an amino acid sequence of at least 95% sequence identity to SEQ ID NO: 3 and/or a variable light chain (VL) comprising an amino acid sequence of at least 95% sequence identity to SEQ ID NO: 4.
55. The composition of embodiment 54, wherein the VH comprises the amino acid sequence of SEQ ID NO: 3 and/or the VL comprises the amino acid sequence of SEQ ID NO: 4.
56. The composition of any one of embodiments 1-55, wherein the anti-BAFF-R antibodies comprise a heavy chain comprising an amino acid sequence of at least 95% sequence identity to SEQ ID NO: 1 and a light chain comprising an amino acid sequence of at least 95% identity to the amino acid sequence of SEQ ID NO: 2.
57. The composition of embodiment 56, wherein the heavy chain comprises the amino acid sequence of SEQ ID NO: 1 and the light chain comprises the amino acid sequence of SEQ ID NO: 2.
58. The composition of any one of embodiments 1-57, wherein the anti-BAFF-R antibodies are IgG1 antibodies.
59. The composition of embodiment 58, wherein the IgG1 antibodies are IgG1κ antibodies.
60. The composition of embodiment 59, wherein the anti-BAFF-R antibody is ianalumab.
61. The composition of any one of embodiments 1-60, wherein the composition is a single batch preparation.
62. The composition of any one of embodiments 1-61, wherein the anti-BAFF-R antibodies are produced in a non-human cell.
63. The composition of embodiment 62, wherein the non-human cell is a recombinant Chinese hamster ovary (CHO) cell.
64. The composition of embodiment 63, wherein the CHO cell has impaired fucosylation compared to a wild-type CHO cell.
65. Isolated anti-BAFF-R antibodies produced in a non-human cell, wherein the antibodies are the anti-BAFF-R antibodies of the composition of any one of embodiments 1-64.
66. A single batch preparation of anti-BAFF-R antibodies, wherein the anti-BAFF-R antibodies are the isolated anti-BAFF-R antibodies of embodiment 65.
67. A pharmaceutical formulation comprising: (a) the composition of any one of embodiments 1-64 and a pharmaceutically acceptable carrier, (b) the isolated anti-BAFF-R antibodies of embodiment 65 and a pharmaceutically acceptable carrier, or (c) the single batch preparation of embodiment 66 and a pharmaceutically acceptable carrier.
68. The pharmaceutical formulation of embodiment 67, wherein the pharmaceutical formulation is in the form of a liquid suitable for administration to a subject in need thereof.
69. A lyophilisate obtainable by lyophilisation of the pharmaceutical formulation of embodiment 67 or 68.
70. A pharmaceutical product comprising a container comprising the pharmaceutical formulation of embodiment 67 or 68, or the lypophilisate of embodiment 69.
71. A method of manufacturing a pharmaceutical formulation comprising anti-BAFF antibodies having an ADCC-dependent mechanism of action, the method comprising:
   (a) culturing a recombinant cell line at a pH from about 6.8 to about 7.15 to express a population of anti-BAFF-R antibodies having an ADCC-dependent mechanism of action; and
   (b) formulating the population of anti-BAFF-R antibodies expressed during step (a) into a pharmaceutical formulation comprising a pharmaceutically acceptable carrier.
72.A method of matching an ADCC activity of a reference afucosylated anti-BAFF-R antibody composition comprising:
   (a) determining an ADCC activity of a reference afucosylated anti-BAFF-R antibody composition;
   (b) determining an ADCC activity of a second antibody composition comprising an anti-BAFF-R antibody having the same antibody sequence as the reference anti-BAFF-R antibody; and
   (c) changing the ADCC activity of the second antibody composition by increasing or decreasing the amount of high-mannose glycans of one or more antibodies within the second antibody composition, wherein the ADCC activity of the second antibody composition after increasing or decreasing the amount of high-mannose is the same as the reference afucosylated anti-BAFF-R antibody composition or within about 10%, about 15%, about 20%, about 25%, about 30%, about 35%, about 40%, about 45% or about 50% of the reference and afucosylated anti-BAFF-R antibody composition or within about 1% to about 50% of the reference and afucosylated anti-BAFF-R antibody composition.
73. A method for engineering a target ADCC activity of an afucosylated anti-BAFF-R antibody composition comprising:
   (a) determining an ADCC activity of a afucosylated anti-BAFF-R antibody composition;
   (b) determining a target ADCC activity; and
   (c) increasing or decreasing the ADCC activity of the glycosylated and afucosylated anti-BAFF-R antibody composition by increasing or decreasing the amount high-mannose glycans in the Fc region of the antibody, wherein the ADCC activity of the glycosylated and afucosylated anti-BAFF-R antibody composition after increasing or decreasing the amount of high-mannose glycans is the same as the target ADCC activity or within about 10%, about 15%, about 20%, about 25%, about 30%, about 35%, about 40%, about 45% or about 50% of the target ADCC activity or within about 1% to about 50% of the target ADCC activity.
74. A method of treating a BAFF-R-related disorder, the method comprising administering an effective amount of the composition of any one of embodiments 1-64, the isolated anti-BAFF-R antibodies of embodiment 65, the single batch preparation of embodiment 66, the pharmaceutical formulation of embodiment 67 or 68, the lyophilisate of embodiment 69, the pharmaceutical product of embodiment 70, or the pharmaceutical formulation manufactured by the method of embodiment 71.

## Claims

1. A composition comprising anti-B cell-activating factor receptor (BAFF-R) antibodies, wherein the anti-BAFF-R antibodies comprise a heavy chain amino acid sequence of SEQ ID NO: 1 and a light chain amino acid sequence of SEQ ID NO: 2, wherein said anti-BAFF-R antibodies comprise N-glycosylated Fc regions, wherein at least 90% of the N-glycosylated Fc regions are afucosylated, and wherein 0% to 10% of the N-glycosylated Fc regions comprise a high mannose glycan.

2. The composition of claim 1, wherein 0% to 7% of the N-glycosylated Fc regions comprise a high mannose glycan.

3. The composition of claim 2, wherein 0.5% to 4% of the N-glycosylated Fc regions comprise a high mannose glycan.

4. The composition of claim 1, wherein 0% to 49% of the N-glycosylated Fc regions are galactosylated.

5. The composition of claim 4, wherein 15% to 38% of the N-glycosylated Fc regions are galactosylated.

6. The composition of claim 1, wherein at least 95% of Fc regions of the anti-BAFF-R antibodies are N-glycosylated.

7. The composition of claim 1, wherein the anti-BAFF-R antibodies comprise at least one variant selected from a high molecular weight variant, a deamidated variant, a tryptophan-oxidized variant, a methionine-oxidized variant, and a clipped variant.

8. The composition of claim 1, wherein the anti-BAFF-R antibodies comprise a high molecular weight variant, wherein the high molecular weight variant accounts for up to 6% of the total anti-BAFF-R antibodies and fragments thereof in the composition.

9. The composition of claim 8, wherein the high molecular weight variant accounts for up to 1% of the total anti-BAFF-R antibodies and fragments thereof in the composition.

10. The composition of claim 1, wherein the anti-BAFF-R antibodies comprise a deamidated variant comprising deamidation at heavy chain residue 332, wherein the deamidated variant accounts for up to 23% of the total anti-BAFF-R antibodies and fragments thereof in the composition.

11. The composition of claim 10, wherein the deamidated variant accounts for up to 1% of the total anti-BAFF-R antibodies and fragments thereof in the composition.

12. The composition of claim 1, wherein the anti-BAFF-R antibodies comprise a tryptophan-oxidized variant comprising oxidation at heavy chain residue 104 and/or heavy chain residue 59, wherein the tryptophan-oxidized variant accounts for up to 16% of the total anti-BAFF-R antibodies and fragments thereof in the composition.

13. The composition of claim 12, wherein the tryptophan-oxidized variant accounts for up to 3% of the total anti-BAFF-R antibodies and fragments thereof in the composition.

14. The composition of claim 1, wherein the anti-BAFF-R antibodies comprise a methionine-oxidized variant comprising oxidation at light chain residue 97, wherein the methionine-oxidized variant accounts for up to 62% of the total anti-BAFF-R antibodies and fragments thereof in the composition.

15. The composition of claim 14, wherein the methionine-oxidized variant accounts for up to 4% of the total anti-BAFF-R antibodies and fragments thereof in the composition.

16. The composition of claim 1, wherein the anti-BAFF-R antibodies comprise a methionine-oxidized variant comprising oxidation of one or more of heavy chain residues 259, 365, and 435, wherein the methionine-oxidized variant accounts for up to 27% of the total anti-BAFF-R antibodies and fragments thereof in the composition.

17. The composition of claim 16, wherein the methionine-oxidized variant accounts for up to 6% of the total anti-BAFF-R antibodies and fragments thereof in the composition.

18. The composition of claim 1, wherein the anti-BAFF-R antibodies comprise a clipped variant comprising clipping between heavy chain residues 56 and 57.

19. The composition of claim 18, wherein the clipped variant accounts for up to 10% of the total anti-BAFF-R antibodies and fragments thereof in the composition.

20. A pharmaceutical formulation, wherein the pharmaceutical formulation comprises the composition of claim 1 and a pharmaceutically acceptable carrier.

21. The pharmaceutical formulation of claim 20, wherein the pharmaceutical formulation comprises a unit dose of the anti-BAFF-R antibody in an amount of 150 mg, 300 mg, or 450 mg.

22. The pharmaceutical formulation of claim 20, wherein the pharmaceutical formulation is: (a) a liquid formulation comprising the anti-BAFF-R antibody at a concentration of 150 mg/mL; or (b) a lyophilisate.

23. A pharmaceutical product comprising the pharmaceutical formulation of any one of claims 20-22, wherein the pharmaceutical product comprises a vial, an injection device, an injection pen, a cartridge, a pre-filled syringe, or an autoinjector.

24. A composition comprising anti-BAFF-R antibodies, wherein the anti-BAFF-R antibodies comprise a heavy chain amino acid sequence of SEQ ID NO: 1 and a light chain amino acid sequence of SEQ ID NO: 2, wherein:
(a) at least 95% of the anti-BAFF-R antibodies comprise N-glycosylated Fc regions;
(b) at least 95% of the N-glycosylated Fc regions are afucosylated;
(c) 0% to 49% of the N-glycosylated Fc regions are galactosylated; and
(d) 0% to 7% of the N-glycosylated Fc regions comprise a high mannose glycan.

25. The composition of claim 24, wherein 15% to 38% of the N-glycosylated Fc regions are galactosylated.

26. The composition of claim 24, wherein 0.5% to 4% of the N-glycosylated Fc regions comprise a high mannose glycan.

27. The composition of claim 24, wherein the anti-BAFF-R antibodies comprise one or more of the following:
(a) a high molecular weight variant, wherein the high molecular weight variant accounts for up to 6% of the anti-BAFF-R antibodies in the composition;
(b) a deamidated variant comprising deamidation at heavy chain residue 332, wherein the deamidated variant accounts for up to 23% of the anti-BAFF-R antibodies in the composition;
(c) a tryptophan-oxidized variant comprising oxidation of heavy chain residues 104 and/or 59, wherein the tryptophan-oxidized variant accounts for up to 16% of the anti-BAFF-R antibodies in the composition;
(d) a methionine-oxidized variant comprising oxidation at light chain residue 97, wherein the methionine-oxidized variant comprising oxidation at light chain residue 97 accounts for up to 62% of the anti-BAFF-R antibodies in the composition;
(e) a methionine-oxidized variant comprising oxidation of one or more of heavy chain residues 259, 365, and 435, wherein the methionine-oxidized variant comprising oxidation of one or more of heavy chain residues 259, 365, and 435 accounts for up to 27% of the anti-BAFF-R antibodies in the composition; and
(f) a clipped variant comprising clipping between heavy chain residues 56 and 57, wherein the clipped variant accounts for up to 10% of the anti-BAFF-R antibodies in the composition.

28. A pharmaceutical product comprising: (a) the composition of claim 27, (b) a pharmaceutical carrier, and (c) a container selected from a vial, an injection device, an injection pen, a cartridge, a pre-filled syringe, and an autoinjector, wherein the container contains the composition and the pharmaceutical carrier.

29. A composition comprising anti-BAFF-R antibodies, wherein the anti-BAFF-R antibodies comprise a heavy chain amino acid sequence of SEQ ID NO: 1 and a light chain amino acid sequence of SEQ ID NO: 2, wherein at least 95% of the anti-BAFF-R antibodies comprise N-glycosylated Fc regions; at least 95% of the N-glycosylated Fc regions are afucosylated; 0% to 49% of the N-glycosylated Fc regions are galactosylated; and (d) 0% to 7% of the N-glycosylated Fc regions comprise a high mannose glycan, and wherein the anti-BAFF-R antibodies comprise one or more of the following:
(a) a high molecular weight variant, wherein the high molecular weight variant accounts for up to 1% of the anti-BAFF-R antibodies in the composition;
(b) a deamidated variant comprising deamidation at heavy chain residue 332, wherein the deamidated variant accounts for up to 1% of the anti-BAFF-R antibodies in the composition;
(c) a tryptophan-oxidized variant comprising oxidation of heavy chain residues 104 and/or 59, wherein the tryptophan-oxidized variant accounts for up to 3% of the anti-BAFF-R antibodies in the composition;
(d) a methionine-oxidized variant comprising oxidation at light chain residue 97, and wherein the methionine-oxidized variant comprising oxidation at light chain residue 97 accounts for up to 4% of the anti-BAFF-R antibodies in the composition;
(e) a methionine-oxidized variant comprising oxidation of one or more of heavy chain residues 259, 365, and 435, wherein the methionine-oxidized variant comprising oxidation of one or more of heavy chain residues 259, 365, and 435 accounts for up to 6% of the anti-BAFF-R antibodies in the composition; and
(f) a clipped variant comprising clipping between heavy chain residues 56 and 57, wherein the clipped variant accounts for up to 4% of the anti-BAFF-R antibodies in the composition.

30. A pharmaceutical product comprising: (a) the composition of claim 29, (b) a pharmaceutical carrier, and (c) a container selected from a vial, an injection device, an injection pen, a cartridge, a pre-filled syringe, and an autoinjector, wherein the container contains the composition and the pharmaceutical carrier.
